# EUROPEAN PATENT SPECIFICATION

(11) **EP 2 585 596 B1**
(45) Date of publication and mention of the grant of the patent: **30.12.2020**
(21) Application number: 11798937.6
(22) Date of filing: 23.06.2011
(51) Int. Cl.: C12N 15/113, A61K 48/00, A61K 31/7088, C12N 15/63

(54) **TREATMENT OF SODIUM CHANNEL, VOLTAGE-GATED, ALPHA SUBUNIT (SCNA) RELATED DISEASES BY INHIBITION OF NATURAL ANTISENSE TRANSCRIPT TO SCNA**
BEHANDLUNG VON SCNA-VERMITTELTEN ERKRANKUNGEN MITTELS HEMMUNG DES NATÜRLICHEN ANTISENSE-TRANSKRIPTS GEGEN SCNA
TRAITEMENT DE MALADIES LIÉES À LA SOUS-UNITÉ ALPHA DU CANAL SODIQUE VOLTAGE-DÉPENDANT (SCNA) PAR INHIBITION DU PRODUIT DE TRANSCRIPTION NATUREL ANTISENS À LA SCNA

(30) Priority: 23.06.2010 US 357774 P
(43) Date of publication of application: 01.05.2013
(73) Proprietor: CuRNA, Inc., Miami, FL 33137 (US)
(72) Inventor: COLLARD, Joseph, Delray Beach Florida 33483 (US); KHORKOVA SHERMAN, Olga, Tequesta Florida 33469 (US); DE LEON, Belinda, San Francisco California 94123 (US); COITO, Carlos, West Palm Beach Florida 33417 (US); HSIAO, Jane H., Dr., Miami Florida 33137 (US)
(74) Representative: Mathys & Squire
(86) International application number: PCT/US2011/041664
(87) International publication number: WO 2011/163499

(56) References cited:
- EP-A1- 1 642 966
- WO-A1-03/066893
- WO-A1-2006/067056
- WO-A1-2006/133508
- JP-A- 2004 275 115
- US-A1- 2004 203 024
- US-A1- 2008 027 216
- US-A1- 2009 118 333
- NAKAJIMA T. ET AL.: 'Eicosapentaenoic acid inhibits voltage-gated sodium channels and invasiveness in prostate cancer cells' BRITISH JOURNAL OF PHARMACOLOGY vol. 156, 19 January 2009, pages 420 - 431, XP002540501
- MEGURO K. ET AL.: 'Function and role of voltage-gated sodium channel Nav1.7 expressed in aortic smooth muscle cells' AM. J. PHYSIOL. HEART CIRC. PHYSIOL. vol. 296, 31 October 2008, pages H211 - H219, XP009121180
- DATABASE Geneseq [Online] 27 January 2005 (2005-01-27), "Connectron nucleic acid #234.", XP55578760, retrieved from EBI accession no. GSN:ADU59612 Database accession no. ADU59612 & WO 2004/095216 A2 (FELDMAN RICHARD J [US]) 4 November 2004 (2004-11-04)
- DATABASE Geneseq [Online] 30 October 2008 (2008-10-30), "Influenza A virus viral replication siRNA target SEQ ID NO 16385.", XP55578763, retrieved from EBI accession no. GSN:ASQ74198 Database accession no. ASQ74198 & WO 2008/043561 A2 (MAX PLANCK GESELLSCHAFT [DE]; MEYER THOMAS F [DE] ET AL.) 17 April 2008 (2008-04-17)
- DATABASE Geneseq [Online] 4 September 2001 (2001-09-04), "Human SCN2A PCR-SSCP PCR primer SEQ ID NO:305.", XP55578776, retrieved from EBI accession no. GSN:AAH56061 Database accession no. AAH56061 & WO 01/38564 A2 (UNIV MCGILL [CA]; ROULEAU GUY A [CA] ET AL.) 31 May 2001 (2001-05-31)
- DATABASE EMBL [Online] 31 October 2009 (2009-10-31), "Sequence 145 from Patent EP2112234.", XP055578785, retrieved from EBI accession no. EM_PAT:HC048936 Database accession no. HC048936 & EP 2 112 234 A2 (UNIV MCGILL [CA]) 28 October 2009 (2009-10-28)

## Description

### FIELD OF THE DISCLOSURE

The present application claims the priority of U.S. provisional patent application No. 61/357,774 filed June 23, 2010.

Instances of the disclosure comprise oligonucleotides modulating expression and/or function of SCNA and associated molecules.

### BACKGROUND

DNA-RNA and RNA-RNA hybridization are important to many aspects of nucleic acid function including DNA replication, transcription, and translation. Hybridization is also central tο a variety of technologies that either detect a particular nucleic acid or alter its expression. Antisense nucleotides, for example, disrupt gene expression by hybridizing to target RNA, thereby interfering with RNA splicing, transcription, translation, and replication. Antisense DNA has the added feature that DNA-RNA hybrids serve as a substrate for digestion by ribonuclease H, an activity that is present in most cell types. Antisense molecules can be delivered into cells, as is the case for oligodeoxynucleotides (ODNs), or they can be expressed from endogenous genes as RNA molecules. The FDA recently approved an antisense drug, VITRAVENE™ (for treatment of cytomegalovirus retinitis), reflecting that antisense has therapeutic utility.

### SUMMARY

The invention is defined by the claims. Those aspects/instances of the present disclosure which constitute the invention. are defined by the claims.

This Summary is provided to present a summary of the disclosure to briefly indicate the nature and substance of the disclosure. It is submitted with the understanding that it will not be used to interpret or limit the scope or meaning of the claims.

In one instance, the disclosure provides methods for inhibiting the action of a natural antisense transcript by using antisense oligonucleotide(s) targeted to any region of the natural antisense transcript resulting in upregalation of the corresponding sense gene wherein said sense gene is selected from at least one member of the SCNA gene family and variants thereof. It is also contemplated herein that inhibition of the natural antisense transcript can be achieved by siRNA, ribozymes and small molecules, which are considered to be within the scope of the present disclosure.

One instance provides a method of modulating function and/or expression of an SCNA polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of a polynucleotide comprising 5 to 30 consecutive nucleotides within nucleotides 1 to 1123 of SEQ ID NO: 12 and 1 to 2352 of SEQ ID NO: 13, 1 to 267 of SEQ ID NO: 14, 1 to 1080 of SEQ ID NO:15, 1 to 173 of SEQ ID NO: 16, 1 to 618 of SEQ ID NO: 17, 1 to 871 of SEQ ID NO: 18,1 to 304 of SEQ ID NO: 19, 1 to 293 of SEQ ID NO: 20, 1 to 892 of SEQ ID NO: 21,1 to 260 of SEQ ID NO: 22, 1 to 982 of SEQ ID NO: 23, 1 to 906 of SEQ ID NO: 24, 1 to 476 of SEQ ID NO: 25, 1 to 285 of SEQ ID NO: 26, 1 to 162 of SEQ ID NO: 27 and 1 to 94 of SEQ ID NO: 28 thereby modulating function and/or expression of the SCNA polynucleotide in patient cells or tissues *in vivo* or *in* vitro.

In an instance, an oligonucleotide targets a natural antisense sequence of SCNA polynucleotides, for example, nucleotides set forth in SEQ ID NOS: 12 to 28, and any variants, alleles, homologs, mutants, derivatives, fragments and complementary sequences thereto. Examples of antisense oligonucleotides are set forth as SEQ ID NOS: 29 to 94.

Another instance provides a method of modulating function and/or expression of an SCNA polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of an antisense of the SCNA polynucleotide; thereby modulating function and/or expression of the SCNA polynucleotide in patient cells or tissues in vivo or in vitro.

Another instance provides a method of modulating function and/or expression of an SCNA polynucleotide in patient cells or tissues in vivo or in vitro comprising contacting said cells or tissues with an antisense oligonucleotide 5 to 30 nucleotides in length wherein said oligonucleotide has at least 50% sequence complementary to an SCNA natural antisense transcript; thereby modulating function and/or expression of the SCNA polynucleotide in patient cells or tissues in vivo or in vitro.

In an instance, a composition comprises one or more antisense oligonucleotides which bind to sense and/or antisense SCNA polynucleotides wherein said polynucleotides are selected from the group consisting of SCNA to SCN12A and variants thereof. In a preferred instance, the target polynucleotide is selected from SCNA.

In an instance, the oligonucleotides comprise one or more modified or substituted nucleotides.

In an instance, the oligonucleotides comprise one or more modified bonds.

In yet another instance, the modified nucleotides comprise modified bases comprising phosphorothioate, methylphosphonate, peptide nucleic acids, 2'-O-methy, fluoro- or carbon, methylene or other locked nucleic acid (LNA) molecules. Preferably, the modified nucleotides are locked nucleic acid molecules, including α-L-LNA.

In an instance, the oligonucleotides are administered to a patient subcutaneously, intramuscularly, intravenously or intraperitoneally.

In an instance, the oligonucleotides are administered in a pharmaceutical composition. A treatment regimen comprises administering the antisense compounds at least once to patient; however, this treatment can be modified to include multiple doses over a period of time. The treatment can be combined with one or more other types of therapies.

In an instance, the oligonucleotides are encapsulated in a liposome or attached to a carrier molecule (e.g. cholesterol, TAT peptide).

Other aspects are described *infra.*

### BRIEF DESCRIPTION OF THE DRAWINGS

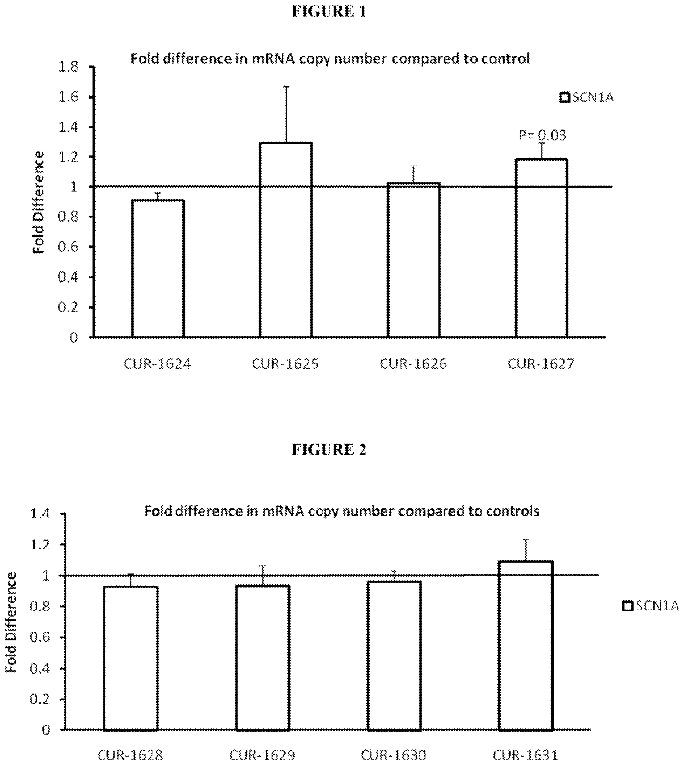

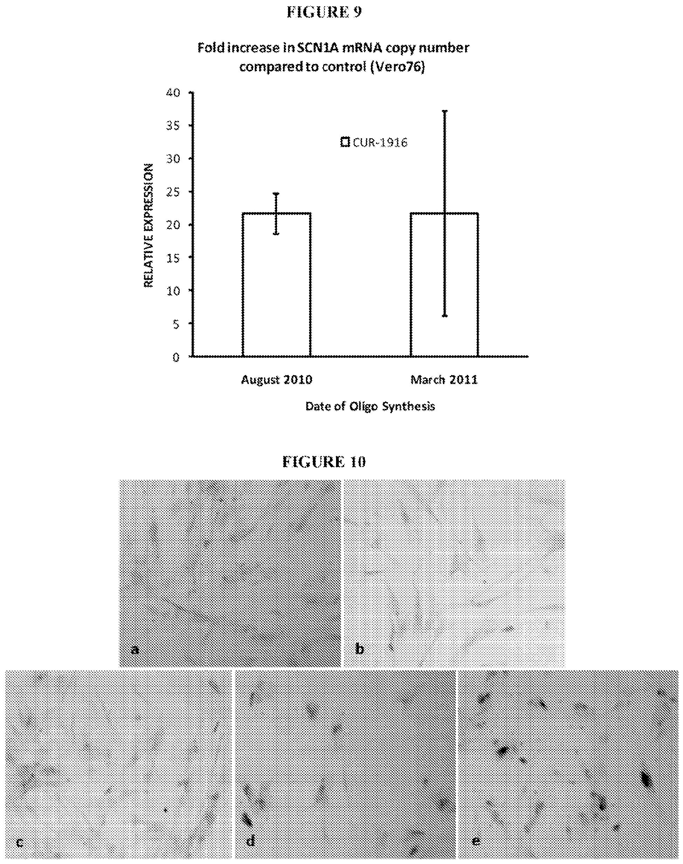

Figure 1 is a graph of real time PCR results showing the fold change + standard deviation in SCN1A mRNA after treatment of HepG2 cells with phosphorothioate oligonucleotides introduced using Lipofectamine 2000, as compared to control. Real Time PCR results show that levels of SCNIA mRNA in HepG2 cells are significantly increased 48h after treatment with one of the antisense oligonucleotides to SCN1A antisense BG724147. Bars denoted as CUR-1624 to CUR-1627 correspond to samples treated with SEQ ID NOS: 30 to 33 respectively.
Figure 2 is a graph of real time PCR results showing the fold change + standard deviation in SCN1A mRNA after treatment of HepG2 cells with phosphorothioate oligonucleotides introduced using Lipofectamine 2000, as compared to control. Bars denoted as CUR-1628 to CUR-1631 correspond to samples treated with SEQ ID NOS: 34 to 37 respectively.

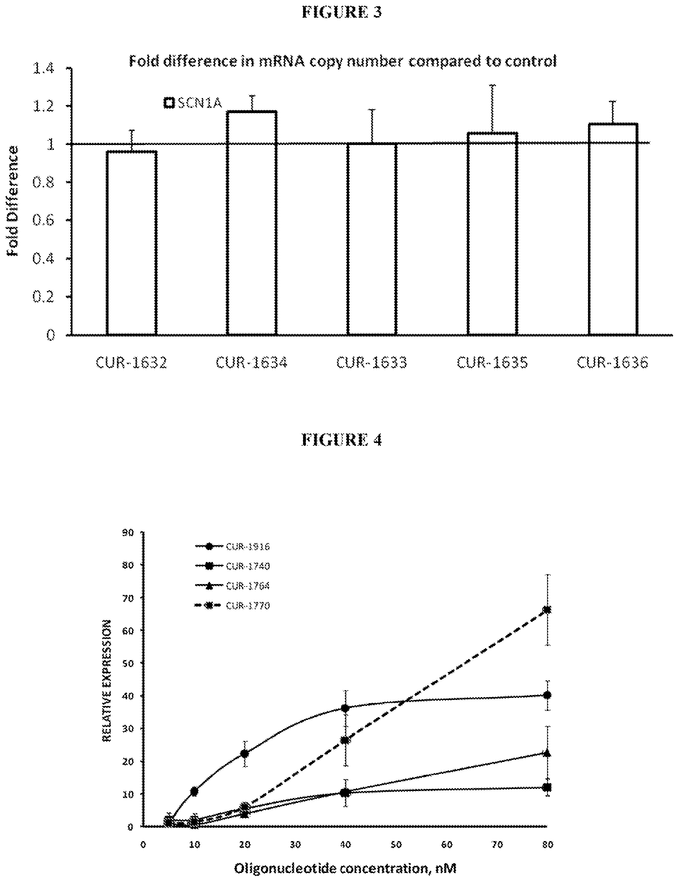

Figure 3 is a graph of real time PCR results showing the fold change + standard deviation in SCN1A mRNA after treatment of HepG2 cells with phosphorothioate oligonucleotides introduced using Lipofectamine 2000, as compared to control. Bars denoted as CUR-1632 to CUR-1636 correspond to samples treated with SEQ ID NOS: 38 to 42 respectively.
Figure 4 shows dose-dependent upregulation of SCNIA mRNA in primary human skin fibroblasts carrying a Dravet syndrome-associated mutation. CUR-1916, CUR-1740, CUR-1764 and CUR-1770 correspond to samples treated with SEQ ID NOS: 70, 45, 52 and 57 respectively.

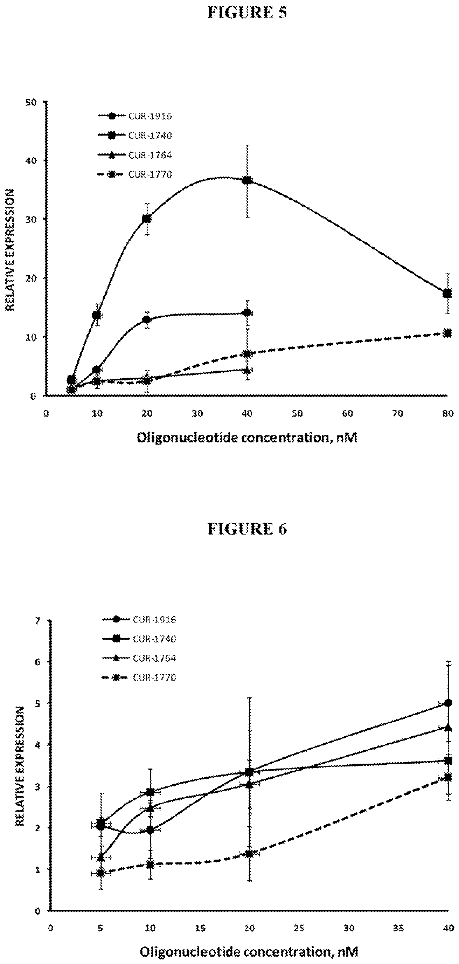

Figure 5 shows dose-dependent upregulation of SCN1A mRNA in SK-N-AS cells- CUR-1916, CUR-1740, CUR-1764 and CUR-1770 correspond to samples treated with SEQ ID NOS: 70,45,52 and 57 respectively.
Figure 6 shows dose-dependent upregulation of SCN1A mRNA in Vero76 cells. CUR-1916, CUR-1740, CUR-1764 and CUR-1770 correspond to samples treated with SEQ ID NOS: 70,45,52 and 57 respectively.

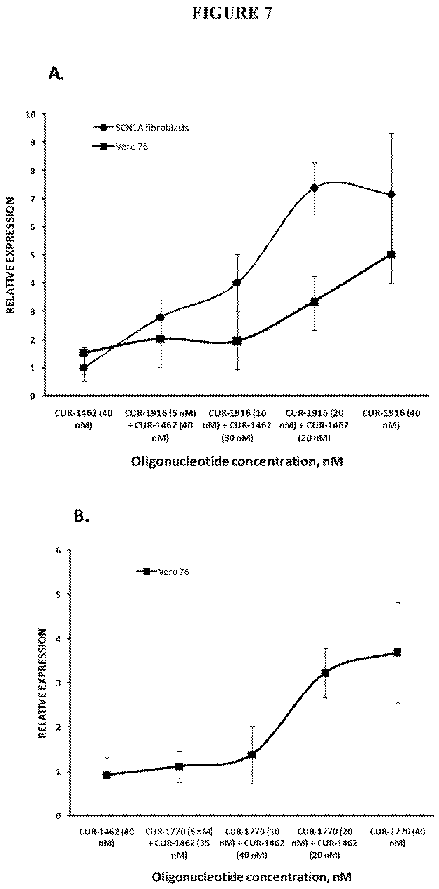

Figure 7 shows that upregulation of SCN1A mRNA is not caused by non-specific toxicity of antisense oligonucleotides. A - upregulation by CUR-1916; B - upregulation by CUR-1770. CUR-1462 is an inactive control oligonucleotide of similar chemistry.

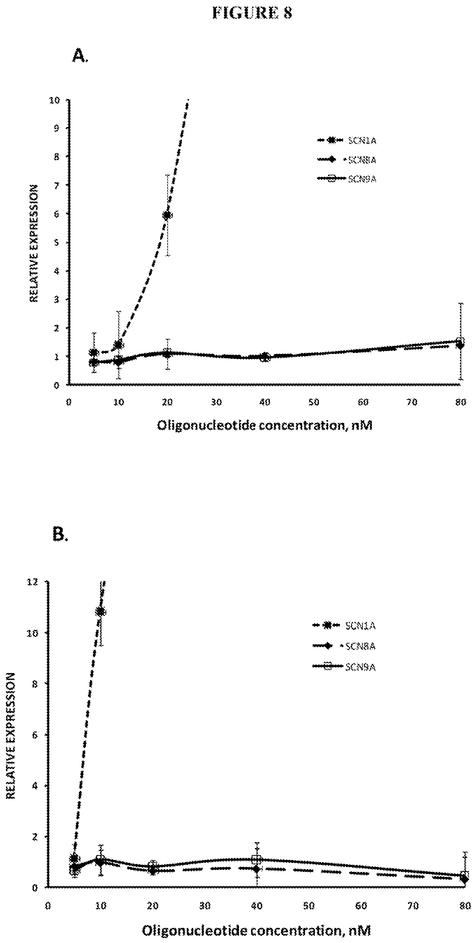

Figure 8 shows that expression of the SCNSA and SCN9A channels in human fibroblasts carrying a Dravet-associated mutation is not significantly affected by the treatment with antisense oligonucleotides targeted against SCN1A natural antisense transcript. A - treatment with CUR-1770; B - treatment with CUR-1916.
Figure 9 shows stability of antisense oligonucleotides targeting SCN1A-specific natural antisense transcript. Vero 76 cells were treated as described in Example 2 with two different batches of CUR-1916 synthesized in August 2010 and March 2011. The oligonucleotide synthesized in August 2010 was stored as a 1 mM aqueous solution at 4°C. The oligonucleotide synthesized in March 2011 was shipped in lyophilized form and tested immediately upon arrival.
Figure 10 shows upregulation of SCN1A protein in fibroblasts carrying a Dravet syndrome mutation treated with antisense oligonucleotides complementary to the SCN1A natural antisense. Fibroblasts were grown in 24 well plates and treated with antisense oligonucleotides complementary to the SCN1A natural antisense at 20nM. (panel c: CUR-1740; d: CUR-1770; c: CUR-1916) and at 0 nM (b). The cells were stained for SCN1A (b-e) by indirect immunohistochemistry using an anti-SCN1A antibody (Abeam cat# ab24820) and a secondary antibody staining/amplification with the avidin/biotin method (Vector Laboratories cat# SP-2001; Vector Laboratories cat# PK-6101; Vector Laboratories cat# SK-4105); panel a - negative control, a rabbit anti-mouse antibody was used as a primary antibody followed by the same staining procedure as in panels b-e.

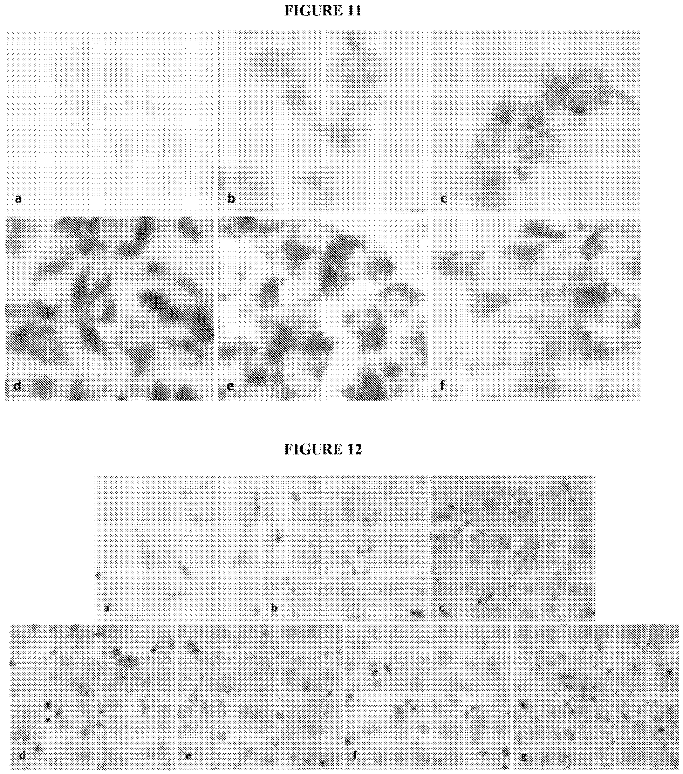

Figure 11 shows upregulation of SCN1A protein in SK-N-AS cells treated with antisense oligonucleotides complementary to the SCN1A natural antisense. SK-N-AS cells were grown in 24 well plates and treated with oligonucleotides at 20nM (c: CUR-1740; d: CUR-1764; e: CUR-1770; f: CUR-1916) and at (b: 0 nM). The SK-N-AS cells were stained for SCN1A (b-f) by indirect immunobistochemistry using an anti- SCN1A antibody (Abcam cat# ab24820) and secondary antibody staining/amplification using the avidin/biotin method (Vector Laboratories cat# SP-2001; Vector Laboratories cat# PK-6101; Vector Laboratories cat# SK-4105); as a negative control, a rabbit anti-mouse antibody was used as a primary antibody followed by the same staining procedure as in panels b-f (panel a).
Figure 12 shows upregulation of SCN1A protein in Vero 76 cells treated with antisense oligonucleotides complementary to the SCN1A natural antisense. Vero 76 cells were grown in 24 well plates and treated with antisense oligonucleotides complementary to the SCN1A natural antisense at 2.0nM (c: CUR-1740; d: CUR-1945; e: CUR-1770; f: CUR-1916; g: CUR-1924) and at 0 nM. (b). The Vero 76 cells were stained for SCN1A (b-f) by indirect immunohistochemistry using an anti- SCN1A antibody (Abcam cat# ab24820) and secondary antibody staining/amplification with the avidin/biotin method (Vector Laboratories cat# SP-2001; Vector Laboratories cat# PK-6101; Vector Laboratories cat# SK-4105); panel a - as a negative control, a rabbit anti-mouse antibody was used as primary antibody followed by the same staining procedure as in panels b-g.

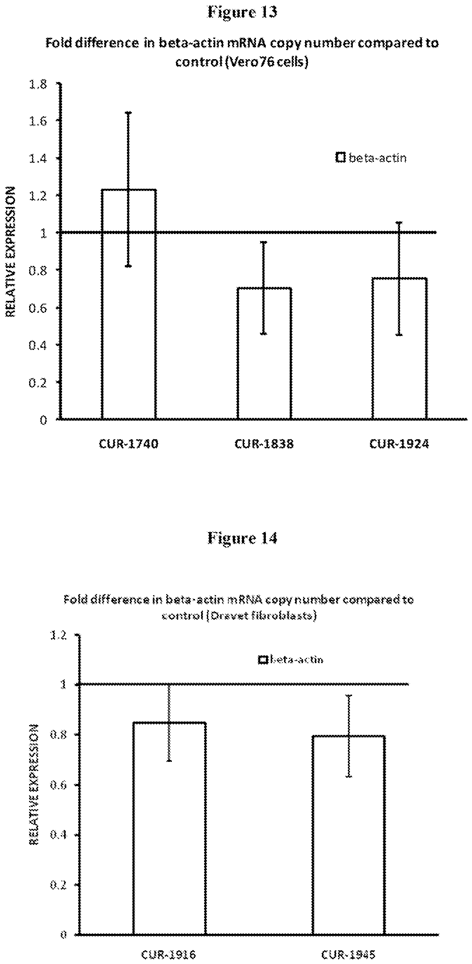

Figure 13 show that oligonucleotides targeting SCN1A-specific natural antisense transcript that upregulate SCN1AmRNA do not upregulate actin in Vero76 cells. The same antisense oligonucleotides (CUR-1740, CUR-1838, CUR-1924) targeting SCN1A-specific natural antisense transcript that were shown in Examples 5 and 12 to upregulate SCN1A mRNA and protein were tested for their effect on beta-actin mRNA expression in Vero 76 cells. The data confirms that oligonucleotide targeting SCN1A-specific natural antisense transcript do not upregulate a non-related gene such as actin. Bars denoted as CUR-1740, CUR-1838 and CUR-1924 correspond to samples treated with SEQ ID NOS: 45, 62 and 78 respectively.
Figure 14 shows that oligonucleotide targeting SCN1A-specific natural antisense transcript shown to upregulate SCN1A mRNA and protein do not upregulate actin in fibroblasts carrying a Dravet-associated mutation. The oligonucleotides (CUR-1916, CUR-1945) targeting SCN1A-specific natural antisense transcript that were shown in Examples 2 and 7 to upregulate SCN1A mRNA and protein were tested for their effect on actin mRNA expression in fibroblasts carrying a Dravet-associated mutation. The data below confirms that oligonucleotides targeting SCN1A-specific natural antisense transcript do not upregulate a non-related gene such as actin. Bars denoted as CUR-1916, and CUR-1945 correspond to samples treated with SEQ ID NOS: 70 and 93 respectively.

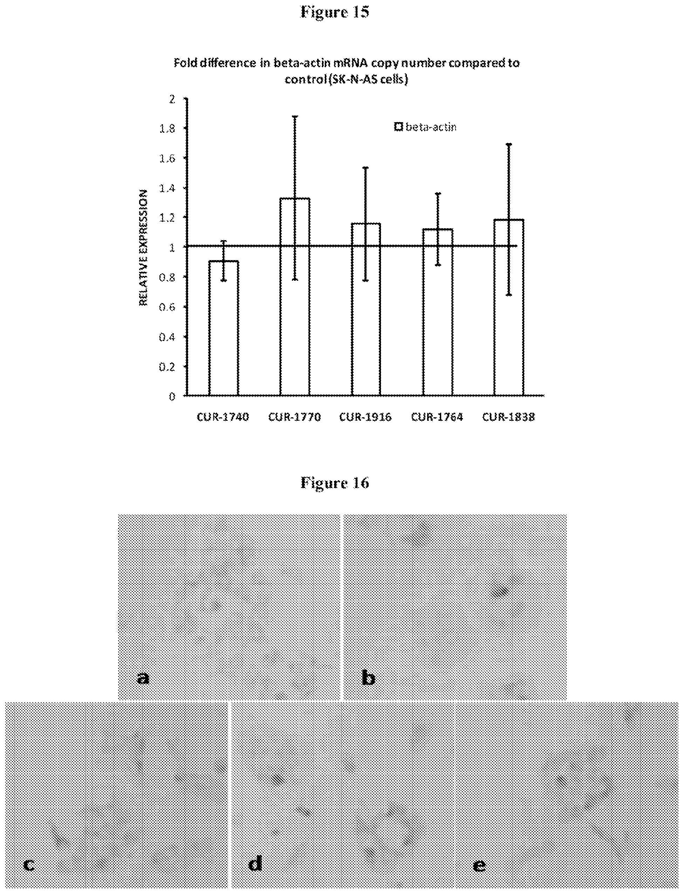

Figure 15 show that oligonucleotides targeting SCN1A-specific natural antisense transcript shown to upregulate SCN1A mRNA and protein do not upregulate actin in SK-N-AS cells. The same antisense oligonucleotides (CUR-1740, CUR-1770, CUR-1916, CUR-1764, CUR-1838) that were shown in Examples to upregulate SCN1A mRNA and protein were tested for their effect on actin mRNA expression in SK-N-AS cells. The data confirms that oligonucleotides targeting SCN1A-specific natural antisense transcript do not upregulate a non-related gene such as actin. Bars denoted as CUR-1740, CUR-1770, CUR-1916, CUR-1764, CUR-1838 correspond to samples treated with SEQ ID NOS: 45, 57, 70, 52 and 62 respectively.
Figure 16 shows staining of actin protein in SK-N-AS cells treated with antisense oligonucleotides complementary to the SCN1A natural antisense. SK-N-AS cells were grown in 24 well plates and treated with oligonucleotides at 20 nM (b: CUR-1740; c: CUR-1764; d: CUR-1770; e: CUR-1916) and at 0 nM (a). The SK-N-AS cells were stained for actin (a-e) by indirect immunohistochemisiry using an anti-actin antibody (Abcam cat#ab1801) and secondary antibody staining/amplification using the avidin/biotin method (Vector Laboratories cat# SP-2001; Vector Laboratories cat# PK-6101; Vector Laboratories cat# SK-4105).

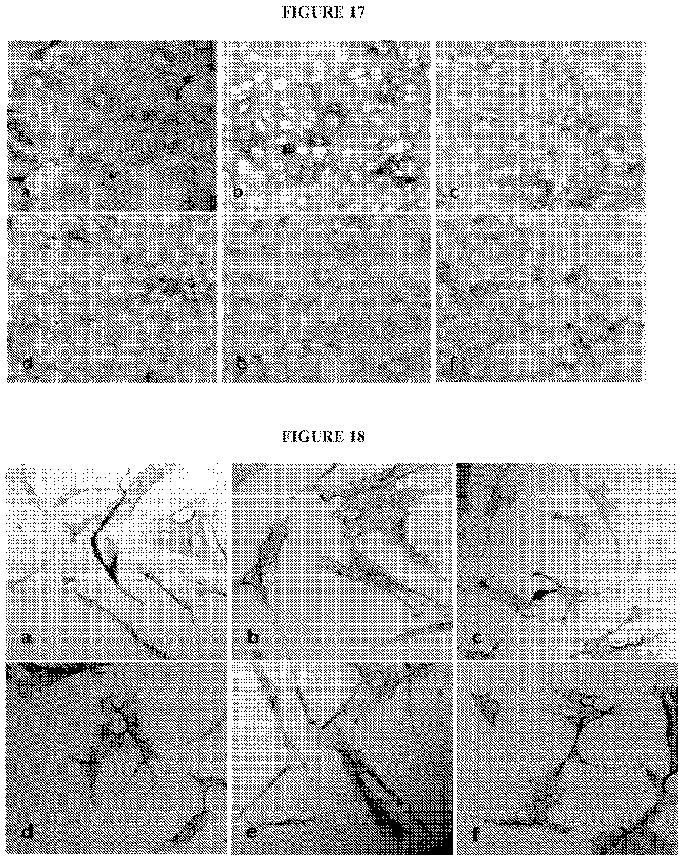

Figure 17 shows staining of actin protein in Vero 76 cells treated with antisense oligonucleotides complementary to the SCN1A natural antisense. Vero 76 cells were grown in 24 well plates and treated with antisense oligonucleotides complementary to the SCN1A natural antisense at 20nM (b: CUR-1740; c: CUR-1770; d: CUR-1916; e: CUR-1924; f: CUR-1945) and at 0 nM (a). The Vero 76 cells were stained for actin (b-f) by indirect immunohistochemistry using an anti-actin antibody (Abcam cat#ab1801) and secondary antibody staining/amplification with the avidin/biotin method (Vector Laboratories cat#SP-2001; Vector Laboratories cat# PK-6101; Vector Laboratories cat# SK-4105); panel a - negative control, a rabbit anti-mouse antibody was used as primary antibody followed by the same staining procedure as in panels b-g.
Figure 18 shows upregulation of actin protein in fibroblasts carrying a Dravet syndrome mutation treated with antisense oligonucleotides complementary to the SCN1A natural antisense. Fibroblasts were grown in 24 well plates and treated with antisense oligonucleotides complementary to the SCN1A natural antisense at 20nM (panel b: CUR-1740; c: CUR-1764; d: CUR-1770; e: CUR-1838 and f: CUR-1916) and at 0 nM (a). The cells were stained for actin (a-f) by indirect immunohistochemistry using an anti-actin antibody (Abcam cat#ab1801) and a secondary antibody staining/amplification with the avidin/biotin method (Vector Laboratories cat# SP-2001; Vector Laboratories cat# PK-6101; Vector Laboratories cat# SK-4105).

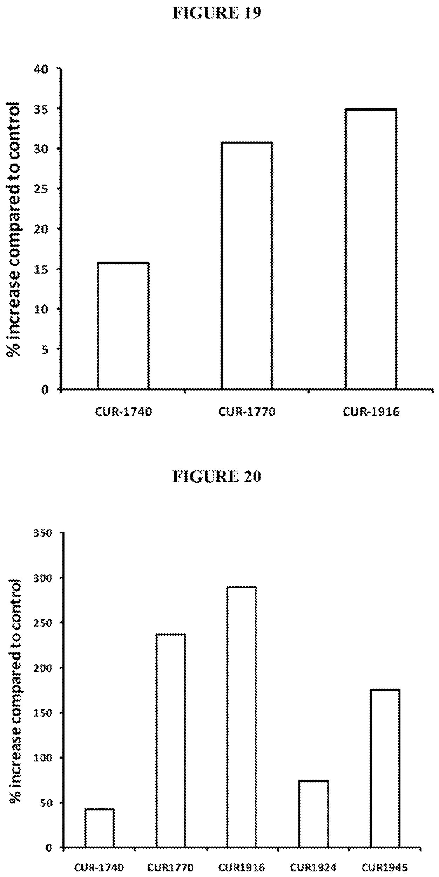

Figure 19 shows upregulation of SCN1A protein in fibroblasts carrying a Dravet syndrome mutation treated with antisense oligonucleotides complementary to the SCN1A natural antisense quantified using ELISA. Fibroblasts were treated with oligonucleotides complementary to the SCN1A natural antisense at 0 or 80nM. After 48h, the cells were transferred to a 96 well plate for 24 h, before being fixed and used for SCN1A and actin ELISAs. The OD readings for SCN1A signal were normalized to actin signal for the same experimental condition. The normalized SCN1A signal in cells dosed with 0 nM of oligonucleotide was used as baseline (100%). Bars denotes as CUR-1740, CUR-1770 and CUR-1916 correspond to samples treated with SEQ ID NOS: 45,57 and 70 respectively.
Figure 20 shows upregulation of SCN1A protein in Vero76 cells treated with antisense oligonucleotides complementary to the SCN1A natural antisense quantified using ELISA. Vero76 cells were treated with antisense oligonucleotides complementary to the SCNIA natural antisense at 0 or 80nM. After 48h, the cells were transferred to a 96 well plate for 24h before being fixed and used for SCN1A and actin ELISAs. The OD readings for SCN1A signal were normalized to actin signal for the same experimental condition. The normalized SCN1A signal in cells dosed with 0 nM of oligonucleotide was used as baseline (100%). Bars denoted as CUR-1740, CUR-1770, CUR-1916, CUR-1924, CUR-1945 correspond to samples treated with SEQ ID NOS: 45, 57, 70, 78 and 93 respectively.

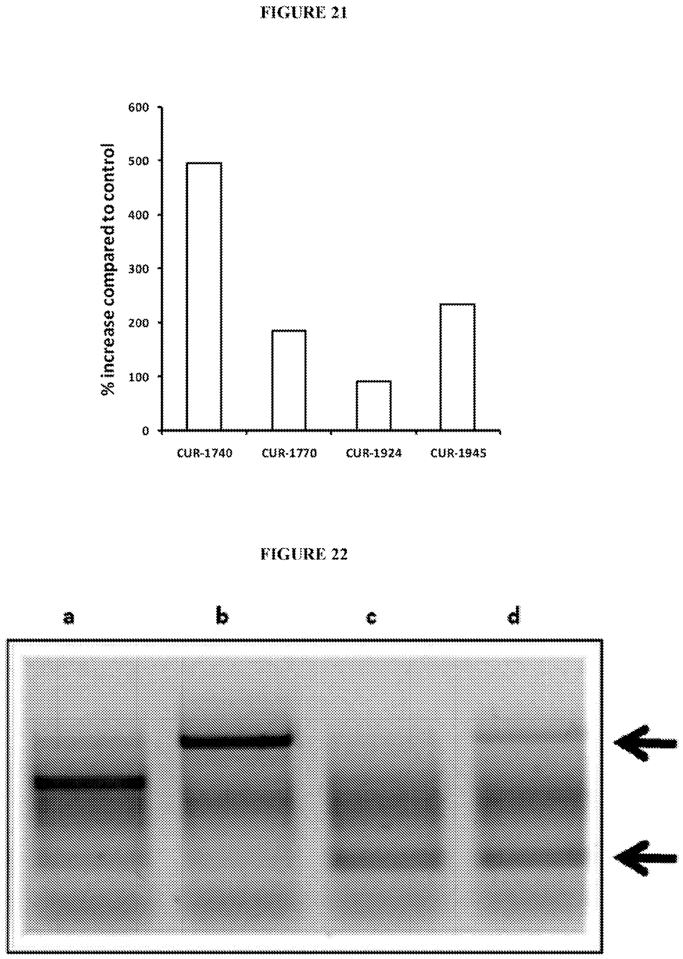

Figure 21 shows upregulation of SCN1A protein in SK-N-AS cells treated with oligonucleotides complementary to the SCN1A natural antisense quantified using ELISA. SK-N-AS cells were treated with antisense oligonucleotides complementary to the SCN1A natural antisense at 0 or 20nM. After 48h, the cells were transferred to a 96 well plate for 24 h, before being fixed and used for SCN1A and actin ELISAs. The OD readings for SCN1A signal were normalized to actin signal for the same experimental condition. The normalized SCN1A signal in cells dosed with 0 nM of oligonucleotide was used as baseline (100%). Bars denoted as CUR-1740, CUR-1770, CUR-1924, CUR-1945 correspond to samples treated with SEQ ID NOS: 45, 57, 78 and 93 respectively.
Figure 22 shows products from a second PCR round of a 3' RACE of the SCN1A natural antisense transcript B0724147. A 3' RACE was performed on: a) total RNA from HcpG2 cells with adenosine added; b - poly A RNA isolated from HcpG2 cells; c - on total RNA from primary human fibroblasts carrying a Dravet syndrome-associated mutation with adenosine added; d on poly A RNA isolated from primary human fibroblasts carrying a Dravet syndrome-associated mutation. Figure represents a negative of a 1% agarose gel/1xTAE stained with GelRed (GenScript, cat#M00120). Arrow shows a band common for HcpG2 cells and primary human fibroblasts carrying a Dravet syndrome-associated mutation, demonstrating the presence of BG724147 natural antisense transcript in these cells.

*Sequence Listing Description-* SEQ ID NO: 1: Homo sapiens sodium channel, voltage-gated, type 1, alpha subunit (SCN1A), transcript variant 1, mRNA (NCBI Accession No.: NM_001165963); SEQ ID NO: 2: Homo sapiens sodium channel, voltage-gated, type II, alpha subunit (SCN2A), transcript variant 1, mRNA (NCBI Accession No.: NM_021007); SEQ ID NO: 3: Homo sapiens sodium channel, voltage-gated, type III, alpha subunit (SCN3A), transcript variant 1, mRNA (NCBI Accession No.: NM_006922); SEQ ID NO: 4: Homo sapiens sodium channel, voltage-gated, type IV, alpha subunit (SCN4A), mRNA (NCBI Accession No.: NM_000334); SEQ ID NO: 5: Homo sapiens sodium channel, voltage-gated, type V, alpha subunit (SCN5A), transcript variant 1, mRNA (NCBI Accession No.: NM_198056); SEQ ID NO: 6: Homo sapiens sodium channel, voltage-gated, type VII, alpha (SCN7A), mRNA (NCBI Accession No.: NM_002976); SEQ ID NO: 7: Homo sapiens sodium channel, voltage gated, type VII, alpha subunit (SCN8A), transcript variant 1, mRNA (NCBI Accession No.: NM_014191); SEQ ID NO: 8: Homo sapiens sodium channel, voltage-gated, type IX, alpha subunit (SCN9A), mRNA (NCBI Accession No.: NM_002977); SEQ ID NO: 9: Homo sapiens sodium channel, voltage-gated, type X, alpha subunit (SCN10A), mRNA (NCBI Accession No.: NM_006514); SEQ ID NO: 10: Homo sapiens sodium channel, voltage-gated, type XI, alpha subunit (SCN11A), mRNA (NCBI Accession No.: NM_014139); SEQ ID NO: 11: Homo sapiens voltage-gated sodium channel alpha subunit SCN12A (SCN12A) mRNA, complete cds (NCBI Accession No.: AF109737); SEQ ID NO: 12: Natural SCN1A antisense sequence (BG724147 extended); SEQ ID NO: 13: Natural SCN1A antisense sequence (Hs,662210); SEQ ID NO: 14: Natural SCN1A antisense sequence (AA383040); SEQ ID NO: 15: Natural SCN1A antisense sequence (BC029452); SEQ ID NO; 16: Natural SCN1A antisense sequence (AA630035); SEQ ID NO: 17: Natural SCN1A antisense sequence (BE566126); SEQ ID NO: 18: Natural SCN1A antisense sequence (BF673100); SEQ ID NO: 19: Natural SCN1A antisense sequence (BG181807); SEQ ID NO: 20: Natural SCNIA antisense sequence (BG183871); SEQ ID NO; 21: Natural SCN1A antisense sequence (BG215777); SEQ ID NO: 22: Natural SCN1A antisense sequence (BG227970); SEQ ID NO: 23: Natural SCN1A antisense sequence (BM905527); SEQ ID NO: 24: Natural SCN1A antisense sequence (BU180772); SEQ ID NO: 25; Mouse Natural SCN1A antisense sequence (BG724147 ExtMouse); SEQ ID NO: 26: Mouse Natural SCN1A antisense sequence (Hs.662210mouseAS1); SEQ ID NO: 27: Mouse Natural SCN1A antisense sequence (Hs.662210mouseAS2); SEQ ID NO: 28: Mouse Natural SCN1A antisense sequence (Hs.662210mouseAS3); SEQ ID NOs: 29 to 94: Antisense oligonucleotides. SEQ ID NO: 95 and 96 are the reverse complements of the antisence oligonucleotides SEQ ID NO: 58 and 59 respectively, * indicates a phosphothioate bond, + indicates LNA, 'r' indicates RNA and 'm' indicates a methyl group on the 2' oxygen atom on the designated sugar moiety of the oligonucleotide.

### DETAILED DESCRIPTION

Several aspects of the disclosure are described below with reference to example applications for illustration. It should be understood that numerous specific details, relationships, and methods are set forth to provide a full understanding of the disclosure. One having ordinary skill in the relevant art, however, will readily recognize that the disclosure can be practiced without one or more of the specific details or with other methods. The present disclosure is not limited by the ordering of acts or events, as some acts may occur in different orders and/or concurrently with other acts or events. Furthermore, not all illustrated acts or events are required to implement a methodology in accordance with the present disclosure.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes disclosed herein, which in some instances relate to mammalian nucleic acid and amino acid sequences are intended to encompass homologous and/or orthologous genes and gene products from other animals including, but not limited to other mammals, fish, amphibians, reptiles, and birds. In an instance, the genes or nucleic acid sequences are human.

### Definitions

The terminology used herein is for the purpose of describing particular instances only and is not intended to be limiting of the disclosure. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "mRNA" means the presently known mRNA transcript(s) of a targeted gene, and any further transcripts which may be elucidated.

By "antisense oligonucleotides" or "antisense compound" is meant an RNA or DNA molecule that binds to another RNA or DNA (target RNA, DNA). For example, if it is an RNA oligonucleotide it binds to another RNA target by means of RNA-RNA interactions and alters the activity of the target RNA. An antisense oligonucleotide can upregulate or downregulate expression and/or function of a particular polynucleotide. The definition is meant to include any foreign RNA or DNA molecule which is useful from a therapeutic, diagnostic, or other viewpoint. Such molecules include, for example, antisense RNA or DNA molecules, interference RNA (RNAi), micro RNA, decoy RNA molecules, siRNA, enzymatic RNA, therapeutic editing RNA and agonist and antagonist RNA, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially single-stranded, or circular oligomeric compounds.

In the context of this disclosure, the term "oligonucleotide" refers to an oligomer or polymer of ribonucleic acid (RNA) or deoxyribonucleic acid (DNA) or mimetics thereof. The term "oligonucleotide", also includes linear or circular oligomers of natural and/or modified monomers or linkages, including deoxyribonucleosides, ribonucleosides, substituted and alpha-anomeric forms thereof, peptide nucleic acids (PNA), locked nucleic acids (LNA), phosphorothioate, methylphosphonate, and the like. Oligonucleotides are capable of specifically binding to a target polynucleotide by way of a regular pattern of monomer-to-monomer interactions, such as Watson-Crick type of base pairing, Hoögsteen or reverse Hoögsteen types of base pairing, or the like.

The oligonucleotide may be "chimeric", that is, composed of different regions. In the context of this disclosure "chimeric" compounds are oligonucleotides, which contain two or more chemical regions, for example, DNA region(s), RNA region(s), PNA region(s) etc. Each chemical region is made up of at least one monomer unit, i.e., a nucleotide in the case of an oligonucleotides compound. These oligonucleotides typically comprise at least one region wherein the oligonucleotide is modified in order to exhibit one or more desired properties. The desired properties of the oligonucleotide include, but are not limited, for example, to increased resistance to nuclease degradation, increased cellular uptake, and/or increased binding affinity for the target nucleic acid. Different regions of the oligonucleotide may therefore have different properties. The chimeric oligonucleotides of the present disclosure can be formed as mixed structures of two or more oligonucleotides, modified oligonucleotides, oligonucleosides and/or oligonucleotide analogs as described above.

The oligonucleotide can be composed of regions that can be linked in "register", that is, when the monomers are linked consecutively, as in native DNA, or linked via spacers. The spacers are intended to constitute a covalent "bridge" between the regions and have in preferred cases a length not exceeding about 100 carbon atoms. The spacers may carry different functionalities, for example, having positive or negative charge, carry special nucleic acid binding properties (intercalators, groove binders, toxins, fluorophors etc.), being lipophilic, inducing special secondary structures like, for example, alanine containing peptides that induce alpha-helices.

As used herein "SCN1A" is inclusive of all family members, mutants, alleles, fragments, species, coding and noncoding sequences, sense and antisense polynucleotide strands, etc. Similarly, SCN2A-SCN12A is inclusive of all mutants, alleles, fragments, etc.

As used herein, the words 'Sodium channel, voltage-gated, type I, alpha subunit', SCN1A, FEB3, FEB3A, GEFSP2, HBSCI, NACI, Nav1.1, SCN1, SME1, Sodium channel protein brain I subunit alpha, Sodium channel protein type I subunit alpha, Sodium channel protein type I subunit alpha and Voltage-gated sodium channel subunit alpha Nav1.1, are considered the same in the literature and are used interchangeably in the present application.

As used herein, the term "oligonucleotide specific for" or "oligonucleotide which targets" refers to an oligonucleotide having a sequence (i) capable of forming a stable complex with a portion of the targeted gene, or (ii) capable of forming a stable duplex with a portion of a mRNA transcript of the targeted gene. Stability of the complexes and duplexes can be determined by theoretical calculations and/or in vitro assays. Exemplary assays for determining stability of hybridization complexes and duplexes are described in the Examples below.

As used herein, the term "target nucleic acid" encompasses DNA, RNA (comprising premRNA and mRNA) transcribed from such DNA, and also cDNA derived from such RNA, coding, noncoding sequences, sense or antisense polynucleotides. The specific hybridization of an oligomeric compound with its target nucleic acid interferes with the normal function of the nucleic acid. This modulation of function of a target nucleic acid by compounds, which specifically hybridize to it, is generally referred to as "antisense". The functions of DNA to be interfered include, for example, replication and transcription. The functions of RNA to be interfered, include all vital functions such as, for example, translocation of the RNA to the site of protein translation, translation of protein from the RNA, splicing of the RNA to yield one or more mRNA species, and catalytic activity which may be engaged in or facilitated by the RNA. The overall effect of such interference with target nucleic acid function is modulation of the expression of an encoded product or oligonucleotides.

RNA interference "RNAi" is mediated by double stranded RNA (dsRNA) molecules that have sequence-specific homology to their "target" nucleic acid sequences. In certain instances of the present disclosure, the mediators are 5-25 nucleotide "small interfering" RNA duplexes (siRNAs). The siRNAs are derived from the processing of dsRNA by an RNase enzyme known as Dicer. siRNA duplex products are recruited into a multi-protein siRNA complex termed RISC (RNA Induced Silencing Complex). Without wishing to be bound by any particular theory, a RISC is then believed to be guided to a target nucleic acid (suitably mRNA), where the siRNA duplex interacts in a sequence-specific way to mediate cleavage in a catalytic fashion. Small interfering RNAs that can be used in accordance with the present disclosure can be synthesized and used according to procedures that are well known in the art and that will be familiar to the ordinarily skilled artisan. Small interfering RNAs for use in the methods of the present disclosure suitably comprise between about 1 to about 50 nucleotides (nt). In examples of non limiting instances, siRNAs can comprise about 5 to about 40 nt, about 5 to about 30 nt, about 10 to about 30 nt, about 15 to about 25 nt, or about 20-25 nucleotides.

Selection of appropriate oligonucleotides is facilitated by using computer programs that automatically align nucleic acid sequences and indicate regions of identity or homology. Such programs are used to compare nucleic acid sequences obtained, for example, by searching databases such as GenBank or by sequencing PCR products. Comparison of nucleic acid sequences from a range of species allows the selection of nucleic acid sequences that display an appropriate degree of identity between species. In the case of genes that have not been sequenced, Southern blots are performed to allow a determination of the degree of identity between genes in target species and other species. By performing Southern blots at varying degrees of stringency, as is well known in the art, it is possible to obtain an approximate measure of identity. These procedures allow the selection of oligonucleotides that exhibit a high degree of complementarity to target nucleic acid sequences in a subject to be controlled and a lower degree of complementarity to corresponding nucleic acid sequences in other species. One skilled in the art will realize that there is considerable latitude in selecting appropriate regions of genes for use in the present disclosure.

By "enzymatic RNA" is meant an RNA molecule with enzymatic activity (Cech, (1988) J. American. Med. Assoc. 260, 3030-3035), Enzymatic nucleic acids (ribozymes) act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through base pairing, and once bound to the correct site, acts enzymatically to cut the target RNA.

By "decoy RNA" is meant an RNA molecule that mimics the natural binding domain for a ligand. The decoy RNA therefore competes with natural binding target for the binding of a specific ligand. For example, it has been shown that over-expression of HIV trans-activation response (TAR) RNA can act as a "decoy" and efficiently binds HIV tat protein, thereby preventing it from binding to TAR sequences encoded in the HIV RNA. This is meant to be a specific example. Those in the art will recognize that this is but one example, and other instances can be readily generated using techniques generally known in the art.

As used herein, the term "monomers" typically indicates monomers linked by phosphodiester bonds or analogs thereof to form oligonucleotides ranging in size from a few monomeric units, e.g., from about 3-4, to about several hundreds of monomeric units. Analogs of phosphodiester linkages include: phosphorothioate, phosphorodithioate, methylphosphornates, phosphoroselenoate, phosphoramidate, and the like, as more fully described below.

The term "nucleotide" covers naturally occurring nucleotides as well as nonnaturally occurring nucleotides. It should be clear to the person skilled in the art that various nucleotides which previously have been considered "non-naturally occurring" have subsequently been found in nature. Thus, "nucleotides" includes not only the known purine and pyrimidine heterocycles-containing molecules, but also heterocyclic analogues and tautomers thereof. Illustrative examples of other types of nucleotides are molecules containing adenine, guanine, thymine, cytosine, uracil, purine, xanthine, diaminopurine, 8-oxo- N6-methyladenine, 7-deazaxanthine, 7-deazaguanine, N4,N4-ethanocytosin, N6,N6-ethano-2,6- diaminopurine, 5-methylcytosine, 5-(C3-C6)-alkynykytosine, 5-fluorouracil, 5-bromouracil, pseudoisocytosine, 2-hydroxy-5-methyl-4-triazolopyridin, isocytosine, isoguanin, inosine and the "non-naturally occurring" nucleotides described in Benner et al., U.S. Pat No. 5,432,272. The term "nucleotide" is intended to cover every and all of these examples as well as analogues and tautomers thereof. Especially interesting nucleotides are those containing adenine, guanine, thymine, cytosine, and uracil, which are considered as the naturally occurring nucleotides in relation to therapeutic and diagnostic application in humans. Nucleotides include the natural 2'-deoxy and 2'-hydroxyl sugars, e.g., as described in Kornberg and Baker, DNA Replication, 2nd Ed. (Freeman, San Francisco, 1992) as well as their analogs.

"Analogs" in reference to nucleotides includes synthetic nucleotides having modified base moieties and/or modified sugar moieties (see e.g., described generally by Scheit, Nucleotide Analogs, John Wiley, New York, 1980; Freier & Altmann, (1997) Nucl. Acid. Res., 25(22), 4429-4443, Toulmé, JJ. (2001) Nature Biotechnology 19:17-18; Manoharan M., (1999) Biochemica et Biophisica Acta 1489:117- 139; Freier S, M., (1997) Nucleic Acid Research, 25:4429-4443, Uhlman, E., (2000) Drug Discovery & Development, 3: 203-213, Herdewin P., (2000) Antisense & Nucleic Acid Drug Dev., 10:297-310); 2'-O, 3'-C-linked [3.2.0] bicycloarabinonucleosides. Such analogs include synthetic nucleotides designed to enhance binding properties, e.g., duplex or triplex stability, specificity, or the like.

As used herein, "hybridization" means the pairing of substantially complementary strands of oligomeric compounds. One mechanism of pairing involves hydrogen bonding, which may be Watson-Crick, Hoögsteen or reversed Hoögsteen hydrogen bonding, between complementary nucleoside or nucleotide bases (nucleotides) of the strands of oligomeric compounds. For example, adenine and thymine are complementary nucleotides which pair through the formation of hydrogen bonds. Hybridization can occur under varying circumstances.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of in vivo assays or therapeutic treatment, and under conditions in which assays are performed in the case of in vitro assays.

As used herein, the phrase "stringent hybridization conditions" or "stringent conditions" refers to conditions under which a compound of the disclosure will hybridize to its target sequence, but to a minimal number of other sequences. Stringent conditions are sequence-dependent and will be different in different circumstances and in the context of this disclosure, "stringent conditions" under which oligomeric compounds hybridize to a target sequence are determined by the nature and composition of the oligomeric compounds and the assays in which they are being investigated. In general, stringent hybridization conditions comprise low concentrations (<0.15M) of salts with inorganic cations such as Na++ or K++ (i.e., low ionic strength), temperature higher than 20°C ∼ 25°C. below the Tm of the oligomeric compound:target sequence complex, and the presence of denaturants such as formamide, dimethylformamide, dimethyl sulfoxide, or the detergent sodium dodecyl sulfate (SDS). For example, the hybridization rate decreases 1.1% for each 1% formamide. An example of a high stringency hybridization condition is 0.1X sodium chloride-sodium citrate buffer (SSC)/0.1% (w/v) SDS at 60° C. for 30 minutes.

"Complementary," as used herein, refers to the capacity for precise pairing between two nucleotides on one or two oligomeric strands. For example, if a nucleobase at a certain position of an antisense compound is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligomeric compound and the further DNA, RNA, or oligonucleotide molecule are complementary to each other when a sufficient number of complementary positions in each molecule are occupied by nucleotides which can hydrogen bond with each other. Thus, "specifically hybridizable" and "complementary" are terms which are used to indicate a sufficient degree of precise pairing or complementarity over a sufficient number of nucleotides such that stable and specific binding occurs between the oligomeric compound and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure, mismatch or hairpin structure). The oligomeric compounds of the present disclosure comprise at least about 70%, or at least about 75%, or at least about 80%, or at least about 85%, or at least about 90%, or at least about 95%, or at least about 99% sequence complementarity to a target region within the target nucleic acid sequence to which they are targeted. For example, an antisense compound in which 18 of 20 nucleotides of the antisense compound are complementary to a target region, and would therefore specifically hybridize, would represent 90 percent complementarity. In this example, the remaining non-complementary nucleotides may be clustered or interspersed with complementary nucleotides and need not be contiguous to each other or to complementary nucleotides. As such, an antisense compound which is 18 nucleotides in length having 4 (four) non-complementary nucleotides which are flanked by two regions of complete complementarity with the target nucleic acid would have 77.8% overall complementarity with the target nucleic acid and would thus fall within the scope of the present disclosure. Percent complementarity of an antisense compound with a region of a target nucleic acid can be determined routinely using BLAST programs (basic local alignment search tools) and PowerBLAST programs known in the art. Percent homology, sequence identity or complementarity, can be determined by, for example, the Gap program (Wisconsin Sequence Analysis Package. Version 8 for Unix, Genetics Computer Group, University Research Park, Madison Wis.), using default settings, which uses the algorithm of Smith and Waterman (Adv. Appl. Math., (1981) 2, 482-489).

As used herein, the term "Thermal Melting Point (Tm)" refers to the temperature, under defined ionic strength, pH, and nucleic acid concentration, at which 50% of the oligonucleotides complementary to the target sequence hybridize to the target sequence at equilibrium. Typically, stringent conditions will be those in which the salt concentration is at least about 0.01 to 1.0 M Na ion concentration (or other salts) at pH 7.0 to 8.3 and the temperature is at least about 30°C for short oligonucleotides (e.g., 10 to 50nucleotide). Stringent conditions may also be achieved with the addition of destabilizing agents such as formamide.

As used herein, "modulation" means either an increase (stimulation) or a decrease (inhibition) in the expression of a gene.

The term "variant", when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the disclosure are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs,) or single base mutations in which the polynucleotide sequence varies by one base. The presence of SNPs may be indicative of, for example, a certain population with a propensity for a disease state, that is susceptibility versus resistance.

Derivative polynucleotides include nucleic acids subjected to chemical modification, for example, replacement of hydrogen by an alkyl, acyl, or amino group. Derivatives, e.g., derivative oligonucleotides, may comprise non-naturally-occurring portions, such as altered sugar moieties or inter-sugar linkages. Exemplary among these are phosphorothioate and other sulfur containing species which are known in the art. Derivative nucleic acids may also contain labels, including radionucleotides, enzymes, fluorescent agents, chemiluminescent agents, chromogenic agents, substrates, cofactors, inhibitors, magnetic particles, and the like.

A "derivative" polypeptide or peptide is one that is modified, for example, by glycosylation, pegylation, phosphorylation, sulfation, reduction/alkylation, acylation, chemical coupling, or mild formalin treatment. A derivative may also be modified to contain a detectable label, either directly or indirectly, including, but not limited to, a radioisotope, fluorescent, and enzyme label.

As used herein, the term "animal" or "patient" is meant to include, for example, humans, sheep, elks, deer, mule deer, minks, mammals, monkeys, horses, cattle, pigs, goats, dogs, cats, rats, mice, birds, chicken, reptiles, fish, insects and arachnids.

"Mammal" covers warm blooded mammals that are typically under medical care (e.g., humans and domesticated animals). Examples include feline, canine, equine, bovine, and human, as well as just human.

"Treating" or "treatment" covers the treatment of a disease-state in a mammal, and includes: (a) preventing the disease-state from occurring in a mammal, in particular, when such mammal is predisposed to the disease-state but has not yet been diagnosed as having it; (b) inhibiting the disease-state, e.g., arresting it development; and/or (c) relieving the disease-state, e.g., causing regression of the disease state until a desired endpoint is reached. Treating also includes the amelioration of a symptom of a disease (e.g., lessen the pain or discomfort), wherein such amelioration may or may not be directly affecting the disease (e.g., cause, transmission, expression, etc.).

As used herein a "Neurological disease or disorder" refers to any disease or disorder of the nervous system and/or visual system. "Neurological disease or disorder" include disease or disorders that involve the central nervous system (brain, brainstem and cerebellum), the peripheral nervous system (including cranial nerves), and the autonomic nervous system (parts of which are located in both central and peripheral nervous system). A Neurological disease or disorder includes but is not limited to acquired epileptiform aphasia; acute disseminated encephalomyelitis; adrenoleukodystrophy; age-related macular degeneration; agenesis of the corpus callosum; agnosia; Aicardi syndrome; Alexander disease; Alpers' disease; alternating hemiplegia; Alzheimer's disease; Vascular dementia; amyotrophic lateral sclerosis; anencephaly; Angelman syndrome; angiomatosis; anoxia; aphasia; apraxia; arachnoid cysts; arachnoiditis; Anronl-Chiari malformation; arteriovenous malformation; Asperger syndrome; ataxia telegiectasia; attention deficit hyperactivity disorder; autism; autonomic dysfunction; back pain; Batten disease; Behcet's disease; Bell's palsy; benign essential blepharospasm; benign focal; amyotrophy; benign intracranial hypertension; Binswanger's disease; blepharospasm; Bloch Sulzberger syndrome; brachial plexus injury; brain abscess; brain injury; brain tumors (including glioblastoma multiforme); spinal tumor; Brown-Sequard syndrome; Canavan disease; carpal tunnel syndrome; causalgia; central pain syndrome; central pontine myelinolysis; cephalic disorder; cerebral aneurysm; cerebral arteriosclerosis; cerebral atrophy; cerebral gigantism; cerebral palsy; Charcot-Marie-Tooth disease; chemotherapy-induced neuropathy and neuropathic pain: Chiari malformation; chorea; chronic inflammatory demyelinating polyneuropathy; chronic pain; chronic regional pain syndrome; Coffin Lowry syndrome; coma, including persistent vegetative state; congenital facial diplegia; corticobasal degeneration; cranial arteritis; craniosynostosis; Creutzfeldt-Jakob disease; cumulative trauma disorders: Cushing's syndrome; cytomegalic inclusion body disease; cytomegalovirus infection; dancing eyes-dancing feet syndrome; DandyWalker syndrome; Dawson disease; De Morsier's syndrome; Dejerine-Klumke palsy; dementia; dermatomyositis; diabetic neuropathy; diffuse sclerosis; Dravet's, dysautonomia; dysgraphia; dyslexia; dystonias; early infantile epileptic encephalopathy; empty sella syndrome; encephalitis; encephaloceles; encephalotrigeminal angiomatosis; epilepsy; Erb's palsy; essential tremor; Fabry's disease; Fahr's syndrome; fainting; familial spastic paralysis; febrile seizures; Fisher syndrome; Friedreich's ataxia; fronto-temporal dementia and other "tanopathies"; Gaucher's disease; Gerstmann's syndrome; giant cell arteritis; giant cell inclusion disease; globoid cell leukodystrophy; Guillain-Barre syndrome; HTLV-1-associated myelopathy; Hallervorden-Spatz disease; head injury; headache; hemifacial spasm; hereditary spastic paraplegia; heredopathia atactic a polyneuritiformis; herpes zoster oticus; herpes zoster; Hirayama syndrome; HIVassociated dementia and neuropathy (also neurological manifestations of AIDS); holoprosencephaly; Huntington's disease and other polyglutamme repeat diseases; hydranencephaly; hydrocephalus; hypercortisolism; hypoxia; immune-mediated encephalomyelitis; inclusion body myositis; incontinentia pigmenti; infantile phytanic acid storage disease; infantile refsum disease; infantile spasms; inflammatory myopathy; intracranial cyst; intracranial hypertension; Joubert syndrome; Keams-Sayre syndrome; Kennedy disease Kinsboume syndrome; Klippel Feil syndrome; Krabbe disease; Kugelberg-Welander disease; kuru; Lafora disease; Lambert-Eaton myasthenic syndrome; Landau-Kleffner syndrome; lateral medullary (Wallenberg) syndrome; learning disabilities; Leigh's disease; Lennox-Gustaut syndrome; Lesch-Nyhan syndrome; leukodystrophy; Lewy body dementia; Lissencephaly; locked-in syndrome; Lou Gehrig's disease (i.e., motor neuron disease or amyotrophic lateral sclerosis); lumbar disc disease; Lyme disease-neurological sequelae; (i.e., motor neuron disease or amyotrophic lateral sclerosis); lumbar disc disease; Lyme disease-neurological sequelae; Machado-Joseph disease; macrencephaly; megalencephaly; Melkersson-Rosenthal syndrome; Menieres disease; meningitis; Menkes disease; metachromatic leukodystrophy; microcephaly; migraine; Miller Fisher syndrome; mini-strokes; mitochondrial myopathies; Mobius syndrome; monomelic amyotrophy; motor neuron disease; Moyamoya disease; mucopolysacchandoses; milti-infarct dementia; multifocal motor neuropathy; multiple sclerosis and other demyclinating disorders; multiple system atrophy with postural hypotension; muscular dystrophy; myasthenia gravis; myelinoelastic diffuse sclerosis; myoclonic encephalopathy of infants; myoclonus; myopathy; myotonia congenital; narcolepsy; neurofibromatosis; neuroleptic malignant syndrome; neurological manifestations of AIDS; neurological sequelae of lupus; neuromyotonia; neuronal ceroid lipofuscinosis; neuronal migration disorders; Niemann-Pick disease; O'Sullivan-McLeod syndrome; occipital neuralgia; occult spinal dysraphism sequence; Ohtahara syndrome; olivopontocerebellar atrophy; opsodonus myoclonus; optic neuritis; orthostatic hypotension; overuse syndrome; paresthesia; a neurodegenerative disease or disorder (Parkinson's disease, Huntington's disease, Alzheimer's disease, amyotrophic lateral sclerosis (ALS), dementia, multiple sclerosis and other diseases and disorders associated with neuronal cell death); paramyotonia congenital; paraneoplastic diseases; paroxysmal attacks; Parry Romberg syndrome: Pelixaens-Merzbacher disease; periodic paralyses; peripheral neuropathy; painful neuropathy and neuropathic pain; persistent vegetative state; pervasive developmental disorders; photic sneeze reflex; phytanic acid storage disease; Pick's disease; pinched nerve; pituitary tumors; polymyositis; porencephaly; post-polio syndrome; postherpetic neuralgia; postinfectious encephalomyelitis; postural hypotension; Prader- Willi syndrome; primary lateral sclerosis; prion diseases; progressive hemifacial atrophy; progressive multifocalleukoencephalopathy; progressive sclerosing poliodystrophy; progressive supranuclear palsy; pseudotumor cerebri; Ramsay-Hunt syndrome (types I and II); Rasmussen's encephalitis; reflex sympathetic dystrophy syndrome; Refsum disease; repetitive motion disorders; repetitive stress injuries; restless legs syndrome; retrovirus-associated myelopathy; Rett syndrome; Reye's syndrome; Saint Vitus dance; Sandhoff disease; Schilder's disease; schizencephaly; septo-optic dysplasia; shaken baby syndrome; shingles; Shy-Drager syndrome; Sjogren's syndrome; sleep apnea; Soto's syndrome; spasticity; spina bifida; spinal cord injury; spinal cord tumors; spinal muscular atrophy; Stiff-Person syndrome; stroke; Sturge-Weber syndrome; subacute sclerosing panencephalitis; subcortical arteriosclerotic encephalopathy; Sydenham chorea; syncope; syringomyelia; tardive dyskinesia; Tay-Sachs disease; temporal arteritis; tethered spinal cord syndrome; Thomsen disease; thoracic outlet syndrome; Tic Douloureux; Todd's paralysis; Tourette syndrome; transient ischemic attack; transmissible spongiform encephalopathies; transverse myelitis; traumatic brain injury; tremor: trigeminal, neuralgia; tropical spastic paraparesis; tuberous sclerosis; vascular dementia (multi-infarct dementia); vasculitis including temporal arteritis; Von Hippel-Lindau disease; Wallenberg's syndrome; Werdnig-Hoffman disease; West syndrome; whiplash; Williams syndrome; Wildon's disease; and Zellweger syndrome and other neurological disorders recited herein.

A cardiovascular disease or disorder includes those disorders that can either cause ischemia or are caused by reperfusion of the heart. Examples include, but are not limited to, atherosclerosis, coronary artery disease, granulomatous myocarditis, chronic myocarditis (non-granulomatous), primary hypertrophic cardiomyopathy, peripheral artery disease (PAD), peripheral vascular disease, venous thromboembolism, pulmonary embolism, stroke, angina pectoris, myocardial infarction, cardiovascular tissue damage caused by cardiac arrest, cardiovascular tissue damage caused by cardiac bypass, cardiogenic shock, and related conditions that would be known by those of ordinary skill in the art or which involve dysfunction; of or tissue damage to the heart or vasculature, especially, but not limited to, tissue damage related to SCNA activation. CVS diseases include, but are not limited to, atherosclerosis, granulomatous myocarditis, myocardial infarction, myocardial fibrosis secondary to valvular heart disease, myocardial fibrosis without infarction, primary hypertrophic cardiomyopathy, and chronic myocarditis (non-granulomatous).

Examples of diseases or disorders associated with sodium channel dysfunction include, but are not restricted to, malignant hyperthermia, myasthenia, episodic ataxia, neuropathic and inflammatory pain, Alzheimer's disease, Parkinson's disease, schizophrenia, hyperekplexia, myotonias such as hypo- and hyperkalaemic periodic paralysis, paramyotonia congenita and potassium aggravated myotonia as well as cardiac arrhythmias such as long QT syndrome.

### Polynucleotide ami Oligonucleotide Compositions and Molecules

*Targets:* In one instance, the targets comprise nucleic acid sequences of Sodium channel, voltage-gated, alpha subunit (SCNA), including without limitation sense and/or antisense noncoding and/or coding sequences associated with SCNA.

Voltage-sensitive ion channels are a class of transmembrane proteins that provide a basis for cellular excitability and the ability to transmit information via ion-generated membrane potentials. In response to changes in membrane potentials, these molecules mediate rapid ion flux through selective channels in a cell membrane. If channel density is high enough, a regenerative depolarization results, which is called an action potential.

The voltage-gated sodium channel is responsible for the generation and propagation of action potentials in most electrically excitable cells, including neurons, heart cells, and muscle. Electrical activity is triggered by depolarization of the membrane, which opens channels through the membrane that are highly selective for sodium ions. Ions are then driven intracellularly through open channels by an electrochemical gradient. Although sodium-based action potentials in different tissues are similar, electrophysiological studies have demonstrated that multiple structurally and functionally distinct sodium channels exist, and numerous genes encoding sodium channels have been cloned. The SCNA gene belongs to a gene family of voltage-gated sodium channels.

Voltage-gated sodium channels can be named according to a standardized form of nomenclature outlined in Goldin, et al, (2000) Neuron 28:365-368. According to that system, voltage-gated sodium channels are grouped into one family from which nine mammalian isoforms and have been identified and expressed. These nine isoforms are given the names Navl. 1. through Navl .9. Also, splice variants of the various isoforms are distinguished by the use of lower case letters following the numbers (e.g., "Navl. la")

Voltage-gated sodium channels play an important role in the generation of action potential in nerve cells and muscle. The alpha subunit (SCNA) is the main component of the channel, and would be sufficient to generate an efficient channel when expressed in cells in vitro. In turn, the beta-1 and 2 subunits need an alpha subunit to give an effective channel. The role of these subunits would be to modify the kinetic properties of the channel mainly by fast inactivation of the sodium currents. The mutation found in the GEFS syndrome on the SCN1B gene is shown to reduce the fast inactivation of the sodium channels as compared to a normal SCNB1, when co-expressed with an alpha subunit

In an instance, antisense oligonucleotides are used to prevent or treat diseases or disorders associated with SCNA family members. Exemplary Sodium channel, voltage-gated, alpha subunit (SCNA) mediated diseases and disorders which can be treated with cell/tissues regenerated from stem cells obtained using the antisense compounds comprise: a disease or disorder associated with abnormal function and/or expression of SCNA, a neurological disease or disorder, convulsion, pain (including chronic pain), impaired electrical excitability involving sodium channel dysfunction, a disease or disorder associated with sodium channel dysfunction, a disease or disorder associated with misregulation of voltage-gated sodium channel alpha subunit activity (e.g., paralysis, hyperkalemic periodic paralysis, paramyotonia congenita, potassium-aggravated myotonia, long Q-T syndrome 3, motor endplate disease, ataxia etc.), a gastrointestinal tract disease due to dysfunction of the enteric nervous system (e.g., colitis, ileitis, inflammatory bowel syndrome etc.), a cardiovascular disease or disorder (e.g., hypertension, congestive heart failure etc.); a disease or disorder of the genitourinary tract involving sympathetic and parasympathetic innervation (e.g., benign prostrate hyperplasia, impotence); a disease or disorder associated with neuromuscular system (e.g., muscular dystrophy, multiple sclerosis, epilepsy, autism, migraine (e.g., Sporadic and familial hemiplegic migraines etc.), Severe myoclonic epilepsy of infancy (SMEI or Dravet's syndrome), Generalised epilepsy with febrile seizure plus (GEFS+) etc.) and SCNA-related seizure disorders.

The present disclosure farther relates to a pharmaceutical composition comprising at least one of an oligonucleotide that targets a natural antisense transcript to at least one or more of a target selected from the group consisting of SCN1A to SCN12A genes or mRNAs or isoforms or variants thereof. The present disclosure further relates to a method of treating a neurological disease or disorder comprising administering an oligonucleotide that targets a natural antisense transcript of at least one or more of a target selected from the group consisting of mRNA SCN1A, SCN2A, SCN3A, SCN4A, SCN5A, SCN6A, SCN7A, SCN8A, SCN9A, SCN10A, SCN11A and SCN12A or variant thereof. In a preferred instance, oligos are selected to upregulate the expression of a fully functional expression product of said SCNA family. In a preferred instance, the oligos of the disclosure upregulate transcription and/or translation of any one of the mRNAs of an SCNXA family of genes to provide fully functional sodium channels in a patient in need of treatment thereof. In patients having a disease or disorder associated with a mutated version of a voltage gated sodium channel, in a preferred instance, administration or treatment with a pharmaceutical composition comprising an oligonucleotide that targets a natural antisense transcript of a voltage gated sodium channel alpha gene or mRNA of such a gene upregulates a fully functional expression product in a ratio that is greater than the upregulation of an expression product derived from a mutated form of the gene. In another instance, the present disclosure relates to a combination of oligonucleotides that target at least one natural antisense transcript of at least two SCNXA family members wherein X is selected from 1-12. For example, in the treatment of Dravett's Syndrome, a combination of oligonucleotides may be used to upregulate the expression products of, for example, SCN1A and SCN9A. In another instance, at least one oligonucleotide may be selected to target a natural antisense transcript of at least two genes selected from any one of SCN1A to SCN12A. Preferred oligonucleotides of the disclosure are between about 5 to about 30 nucleotides in length and are at least 50% complementary to a 5 to about 30 nucleotide segment of an NAT. Preferred NATs of any one of the SCNA genes or transcription products thereof are those which, when targeted by an oligonucleotide of the disclosure, interfere with and modulate the expression of mRNA and/or the translation product of said mRNA. In a preferred instance, the oligonucleotides upregulate the expression of the functional protein of the target to treat or mitigate an SCNA associated disease. In a preferred instance, this "upregulation" is not associated with a cause or promotion of a disease such as cancer.

Alterations in an SCNA gene may include or encompass many or all forms of gene mutations including insertions, deletions, rearrangements and/or point mutations in the coding and/or non-coding regions of a gene. Deletions may be of the entire gene or a portion of the gene. Point mutations may result in amino acid substitutions, frame shifts or stop codons. Point mutations may also occur in a regulatory region of an SCNA gene, such as a promoter, resulting in a loss or a decrease of expression of an mRNA or may result in improper processing of such mRNA leading to a decrease in stability or translation efficiency. Such alterations in humans may lead to various forms of disease and there are many publications which describe the association of an alteration in an SCNA gene with, for example, epilepsy or SMEI. Such alterations may be "de novo" or may be inherited. The present disclosure is not limited to treating diseases associated with alterations in an SCNA gene and also includes treatment of an SCNA associated disease or condition wherein a patient does not have or necessarily have an alteration or mutation in the SCNA gene. It is believed that any modulation or upregulation of functional voltage gated sodium channel expression products will result in mitigation or treatment of an associated SCNA disease or condition in a patient in need of treatment thereof. Such mitigation also may include at least one measurable indicia of clinical improvement including fewer seizures, less frequent seizures, less severe seizures, development of fewer seizure types, improvement in neurological development or any other treatment benefit.

In an instance, modulation of SCNA by one or more antisense oligonucleotides is administered to a patient in need thereof to prevent or treat any disease or disorder related to SCNA abnormal expression, function, activity as compared to a normal control.

In an instance, the oligonucleotides are specific for polynucleotides of SCNA, which includes, without limitation noncoding regions. The SCNA targets comprise variants of SCNA; mutants of SCNA, including SNPs; noncoding sequences of SCNA; alleles, fragments and the like. Preferably the oligonucleotide is an antisense RNA molecule.

In accordance with instances of the disclosure, the target nucleic acid molecule is not limited to SCNA polynucleotides alone but extends to any of the isoforms, receptors, homologs, non-coding regions and the like of SCNA.

In an instance, an oligonucleotide targets a natural antisense sequence (natural antisense to the coding and non-coding regions) of SCNA targets, including, without limitation, variants, alleles, homologs, mutants, derivatives, fragments and complementary sequences thereto. Preferably the oligonucleotide is an antisense RNA or DNA molecule.

In an instance, the oligomeric compounds of the present disclosure also include variants in which a different base is present at one or more of the nucleotide positions in the compound. For example, if the first nucleotide is an adenine, variants may be produced which contain thymidine, guanosine, cytidine or other natural or unnatural nucleotides at this position. This may be done at any of the positions of the antissense compound. These compounds are then tested using the methods described herein to determine their ability to inhibit expression of a target nucleic acid.

In some instances, homology, sequence identity or complementarity, between the antisense compound and target is from about 50% to about 60%. In some instances, homology, sequence identity or complementarity, is from about 60% to about 70%. In some instances, homology, sequence identity or complementarity, is from about 70% to about 80%. In some instances, homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%).

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired. Such conditions include, i.e., physiological conditions in the case of in vivo assays or therapeutic treatment, and conditions in which assays are performed in the case of in vitro assays.

An antisense compound, whether DNA, RNA, chimeric, substituted etc, is specifically hybridizable when binding of the compound to the target DNA or RNA molecule interferes with the normal function of the target DNA or RNA to cause a loss of utility, and there is a sufficient degree of complementarily to avoid non-specific binding of the antisense compound to non-target sequences under conditions in which specific binding is desired, i.e., under physiological conditions in the case of in vivo assays or therapeutic treatment, and in the case of in vitro assays, under conditions in which the assays are performed.

In an instance, targeting of SCNA including without limitation, antisense sequences which are identified and expanded, using for example, PCR, hybridization etc., one or more of the sequences set forth as SEQ ID NOS: 12 to 28, and the like, modulate the expression or function of SCNA. In one instance, expression or function is upregulated as compared to a control. In an instance, expression or function is down-regulated as compared to a control.

In an instance, oligonucleotides comprise nucleic acid sequences set forth as SEQ ID NOS: 29 to 94 including antisense sequences which are identified and expanded, using for example, PCR, hybridization etc. These oligonucleotides can comprise one or more modified nucleotides, shorter or longer fragments, modified bonds and the like. Examples of modified bonds or internucleotide linkages comprise phosphorothioate, phosphorodithioate or the like. In an instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, per se, is also known and need not be described here.

The specificity and sensitivity of antisense is also harnessed by those of skill in the art for therapeutic uses. Antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides can be useful therapeutic modalities that can be configured to be useful in treatment regimes for treatment of cells, tissues and animals, especially humans.

In instances of the present disclosure oligomeric antisense compounds, particularly oligonucleotides, bind to target nucleic acid molecules and modulate the expression and/or function of molecules encoded by a target gene. The functions of DNA to be interfered comprise, for example, replication and transcription. The functions of RNA to be interfered comprise all vital functions such as, for example, translocation of the RNA to the site of protein translation, translation of protein from the RNA, splicing of the RNA to yield one or more mRNA species, and catalytic activity which may be engaged in or facilitated by the RNA. The functions may be upregulated or inhibited depending on the functions desired.

The antisense compounds, include, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds tray be introduced in the form of single-stranded, double-stranded, partially single-stranded, or circular oligomeric compounds,

Targeting an antisense compound to a particular nucleic acid molecule, in the context of this disclosure, can be a multistep process. The process usually begins with the identification of a target nucleic acid whose function is to be modulated. This target nucleic acid may be, for example, a cellular gene (or mRNA transcribed from the gene) whose expression is associated with a particular disorder or disease state, or a nucleic acid molecule from an infectious agent. In the present disclosure, the target nucleic acid encodes Sodium channeL voltage-gated, alpha subunit (SCNA).

The targeting process usually also includes determination of at least one target region, segment, or site within the target nucleic acid for the antisense interaction to occur such that the desired effect e.g., modulation of expression, will result. Within the context of the present disclosure, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present disclosure, are defined as positions within a target nucleic acid.

In an instance, the antisense oligonucleotides bind to the natural antisense sequences of Sodium channel, voltage-gated, alpha subunit (SCNA) and modulate the expression and/or function of SCNA (SEQ ID NO: 1 to 11). Examples of natural antisense sequences include SEQ ID NOS: 12 to 28. Examples of antisense oligonucleotides include SEQ ID NOS. 29 to 94.

In an instance, the antisense oligonucleotides bind to one or more segments of Sodium channel, voltage-gated, alpha subunit (SCNA) polynucleotides and modulate the expression and/or function of SCNA, The segments comprise at least five consecutive nucleotides of the SCNA sense or antisense polynucleotides.

In an instance, the antisense oligonucleotides are specific for natural antisense sequences of SCNA wherein binding of the oligonucleotides to the natural antisense sequences of SCNA modulate expression and/or function of SCNA.

In an instance, oligonucleotide compounds comprise sequences set forth as SEQ ID NOS: 29 to 94 including antisense sequences which are identified and expanded, using for example, PCR, hybridization etc These oligonucleotides can comprise one or more modified nucleotides, shorter or longer fragments, modified bonds and the like. Examples of modified bonds or internucleotide linkages comprise phosphorothioate, phosphorodithioate or the like. In an instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, per se, is also known and need not be described here.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes has a translation initiation codon having the RNA sequence 5'-GUG, 5'-UUG or 5'-CUG; and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes), Eukaryotic and prokaryotic genes may have two or more alternative start codons, any one of which may be preferentially utilized for translation initiation in a particular cell type or tissue, or under a particular set of conditions. In the context of the disclosure, "start codon" and "translation initiation codon" refer to the codon or codons that are used in vivo to initiate translation of an mRNA transcribed from a gene encoding Sodium channel, voltage-gated, alpha subunit (SCNA), regardless of the sequence(s) of such codons. A translation termination codon (or "stop codon") of a gene may have one of three sequences, i.e., 5'-UAA, 5'-UAG and 5'-UGA (the corresponding DNA sequences are 5'-TAA, 5'-TAG and 5'- TGA, respectively).

The tenns "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from translation termination codon. Consequently, the "start codon region" (or "translation initiation codon region") and the "stop codon region" (or "translation termination codon region") are all regions that may be targeted effectively with the antisense compounds of the present disclosure.

The open reading frame (ORP) or "coding region," which is known in the art to refer to the region between the translation initiation codon and the translation termination codon, is also a region which may be targeted effectively. Within the context of the present disclosure, a targeted region is the intragenic region encompassing the translation initiation or termination codon of the open reading frame (ORF) of a gene.

Another target region includes the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene). Still another target region includes the 3 untranslated region (3'UTR), known in the art to refer to the portion of an mRNA in the 3' direction from the translation termination codon, and thus including nucleotides between the translation termination codon and 3' end of an mRNA or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. Another target region for this disclosure is the 5' cap region.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. In one instance, targeting splice sites, i.e., intron-exon junctions or exon-intron junctions, is particularly useful in situations where aberrant splicing is implicated in disease, or where an overproduction of a particular splice product is implicated in disease. An aberrant fusion junction due to rearrangement or deletion is another instance of a target site. mRNA transcripts produced via the process of splicing of two (or more) mRNAs from different gene sources are known as "fusion transcripts", Introns can be effectively targeted using antisense compounds targeted to, for example. DNA or pre-mRNA.

In an instance, the antisense oligonucleotides bind to coding and/or non-coding regions of a target polynucleotide and modulate the expression and/or function of the target molecule.

In an instance, the antisense oligonucleotides bind to natural antisense polynucleotides and modulate the expression and/or function of the target molecule.

In an instance, the antisense oligonucleotides bind to sense polynucleotides and modulate the expression and/or function of the target molecule,

Alternative RNA transcripts can be produced from the same genomic region of DNA. These alternative transcripts are generally known as "variants". More specifically, "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing, These mRNA variants are also known as "alternative splice variants". If no splicing of the pre-mRNA variant occurs then the pre-mRNA variant is identical to the mRNA variant.

Variants can be produced through the use of alternative signals to start or stop transcription. Pre-mRNAs and mRNAs can possess more than one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby produsing transcripts that terminate at unique polyA sites. Within the context of the disclosure, the types of variants described herein are also instances of target nucleic acids.

The locations on the target nucleic acid to which the antisense compounds hybridize are defined as at least a 5-nucleotide long portion of a target region to which an active antisense compound is targeted,

While the specific sequences of certain exemplary target segments are set forth herein, one of skill in the art will recognize that these serve to illustrate and describe particular instances within the scope of the present disclosure. Additional target segments are readily identifiable by one having ordinary skill in the art in view of this disclosure,

Target segments 5-100 nucleotides in length comprising a stretch of at least five (5) consecutive nucleotides selected from within the illustrative preferred target segments are considered to be suitable for targeting as well.

Target segments can include DNA or RNA sequences that comprise at least the 5 consecutive nucleotides from the 5'-terminus of one of the illustrative preferred target segments (the remaining nucleotides being a consecutive stretch of the same DNA or RNA beginning immediately upstream of the 5'-terminus of the target segment and continuing until the DNA or RNA contains about 5 to about 100 nucleotides). Similarly preferred target segments are represented by DNA or RNA sequences that comprise at least the 5 consecutive nucleotides from the 3'-terminus of one of the illustrative preferred target segments (the remaining nucleotides being a consecutive stretch of the same DNA or RNA beginning immediately downstream ,if the 3'-terminus of the target segment and continuing until the DNA or RNA contains about 5 to about 100 nucleotides). One having skill in the art armed with the target segments illustrated herein will be able, without undue experimentation, to identify further preferred target segments.

Once one or more target regions, segments or sites have been identified, antisense compounds are chosen which are sufficiently complementary to the target, i.e., hybridize sufficiently well and with sufficient specificity, to give the desired effect.

In instances of the disclosure the oligonucleotides bind to an antisense strand of a particular target. The oligonucleotides are at least nucleotides in length and can be synthesized so each oligonucleotide targets overlapping sequences such that oligonucleotides are synthesized to cover the entire length of the target polynucleotide, The targets also include coding as well as non coding regions.

In one instance, it is preferred to target specific nucleic acids by antisense oligonucleotides, Targeting an antisense compound to a particular nucleic acid is a multistep process. The process usually begins with the identification of a nucleic acid sequence whose function is to be modulated. This may be, for example, a cellular gene (or mRNA transcribed from the gene) whose expression is associated with a particular disorder or disease state, or a non coding polynucleotide such as for example, non coding RNA (ncRNA).

RNAs can be classified into (1) messenger RNAs (mRNAs), which are translated into proteins, and (2) non-protein-coding RNAs (ncRNAs). ncRNAs comprise micmRNAs, antisense transcripts and other Transcriptional Units (TU) containing a high density of stop codons and tacking any extensive "Open Reading Frame". Many ncRNAs appear to start from initiation sites in 3' untranslated regions (3'UTRs) of protein-coding loci. ncRNAs are often rare and at least half of the ncRNAs that have been sequenced by the FANTOM consortium seem not to be polyadenylated. Most researchers have for obvious reasons focused on polyadenylated mRNAs that are processed and exported to the cytoplasm. Recently, it was shown that the set of non-polyadenylated nuclear RNAs may be very large, and that many such transcripts arise from so-called intergenic regions. The mechanism by which ncRNAs may regulate gene expression is by base pairing with target transcripts. The RNAs that function by base pairing can be grouped into (1) cis encoded RNAs that are encoded at the same genetic location, but on the opposite strand to the RNAs they act upon and therefore display perfect complementarity to their target, and (2) trans-encoded RNAs that are encoded at a chromosomal location distinct from the RNAs they act upon and generally do not exhibit perfect base-pairing potential with their targets.

Without wishing to be bound by theory, perturbation of an antisense polynucleotide by the antisense oligonucleotides described herein can alter the expression of the corresponding sense messenger RNAs. However, this regulation can either be discordant (antisense knockdown results in messenger RNA elevation) or concordant (antisense knockdown results in concomitant messenger RNA reduction), In these cases, antisense oligonucleotides can be targeted to overlapping or non-overlapping parts of the antisense transcript resulting in its knockdown or sequestration. Coding as well as non-coding antisense can be targeted in an identical manner and that either category is capable of regulating the corresponding sense transcripts _ either in a concordant or disconcordant manner. The strategies that are employed in identifying new oligonucleotides for use against a target can be based on the knockdown of antisense RNA transcripts by antisense oligonucleotides or any other means of modulating the desired target.

*Strategy 1*: In the case of discordant regulation, knocking down the antisense transcript elevates the expression of the conventional (sense) gene. Should that latter gene encode for a known or putative drug target, then knockdown of its antisense counterpart could conceivably mimic the action of a receptor agonist or an enzyme stimulant.

*Strategy 2*: In the case of concordant regulation, one could concomitantly knock down both antisense and sense transcripts and thereby achieve synergistic reduction of the conventional (sense) gene expression. If for example, an antisense oligonucleotide is used to achieve knockdown, then this strategy can be used to apply one antisense oligonucleotide targeted to the sense transcript and another antisense oligonucleotide to the corresponding antisense transcript, or a single energetically symmetric antisense oligonucleotide that simultaneously targets overlapping sense and antisense transcripts.

According to the present disclosure, antisense compounds include antisense oligonucleotides, ribozymes, external guide sequence (EGS) oligonucleotides, siRNA compounds, single- or double-stranded RNA interference (RNAi) compounds such as siRNA compounds, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and or branched. Antisense compounds can include constructs such as, for example; two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double-stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines, however, in some instances, , the gene expression or function is up regulated. When formed from novo strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'- hydroxyl or 2'-modified sugars and, generally U rather than T bases). Nucleic acid helices can adopt more than one type of structure, most commonly the A- and β-forms. It is believed that, in general, oligonucleotides which have B-form-like structure are "DNA-like" and those which have A-formlike structure are "RNA-like." In some (chimeric) instances, an antisense compound may contain both A- and B-form regions.

In an instance, the desired oligonucleotides or antisense compounds, comprise at least one of: antisense RNA, antisense DNA, chimeric antisense oligonucleotides, antisense oligonucleotides comprising modified linkages, interference RNA (RNAi), short interfering RNA (siRNA); a micro, interfering RNA (miRNA); a small, temporal RNA (siRNA); or a short, hairpin RNA (shRNA); small RNA-induced gene activation (RNAa); small activating RNAs (saRNAs), or combinations thereof.

dsRNA can also activate gene expression, a mechanism that has been termed "small RNA-induced gene activation" or RNAa. dsRNAs targeting gene promoters induce potent transcriptional activation of associated genes. RNAa was demonstrated in human cells using synthetic dsRNAs, termed "small activating RNAs" (saRNAs), It is currently not known whence RNAa is conserved in other organisms.

Small double-stranded RNA (dsRNA), such as small interfering RNA (siRNA) and microRNA (miRNA), have been found to be the trigger of an evolutionary conserved mechanism known as RNA interference (RNAi). RNAi invariably leads to gene silencing via remodeling chromatin to thereby suppress transcription, degrading complementary mRNA, or blocking protein translation. However, in instances described in detail in the examples section which follows, oligonucleotides are shown to increase the expression and/or function of the Sodium channel voltage-gated, alpha subunit (SCNA) polynucleotides and encoded products thereof. dsRNAs may also act as small activating RNAs (saRNA). Without wishing to be bound by theory, by targeting sequences in gene promoters, saRNAs would induce target gene expression in a phenomenon referred to as dsRNA-induced transcriptional activation (RNAa).

In a further instance, the "preferred target segments" identified herein may be employed in a screen for additional compounds that modulate the expression of Sodium channel, voltage-gated, alpha subunit (SCNA) polynucleotides. "Modulators" are those compounds that decrease or increase the expression of a nucleic acid molecule encoding SCNA and which comprise at least a 5-nucleotide portion that, is complementary to a preferred target segment. The screening method comprises the steps of contacting a preferred target segment of a nucleic acid molecule encoding sense or natural antisense polynucleotides of SCNA with one or more candidate modulators, and selecting for one or more candidate modulators which decrease or increase the expression of a nucleic acid molecule encoding SCNA polynucleotides, e.g. SEQ ID NOS: 29 to 94. Once it is shown that the candidate modulator or modulators are capable of modulating (e.g. either decreasing or increasing) the expression of a nucleic acid molecule encoding SCNA. polynucleotides, the modulator may then be employed in further investigative studies of the function of SCNA polynucleotides, or for use as a research, diagnostic, or therapeutic agent in accordance with the present disclosure.

Targeting the natural antisense sequence preferably modulates the function of the target gene. For example, the SCNA gene (e.g. accession number NM_001165963, NM_021007, NM_006922, NM_000334, NM_198056, NM_002976, NM_014191, NM_002977, NM_006514, NM_014139, AF109737). In an instance, the target is an antisense polynucleotide of the SCNA gene, In an instance, an antisense oligonucleotide targets sense and/or natural antisense sequences of SCNA polynucleotides (e.g. accession number NM_{_}001165963, NM_021007, NM_006922, NM_000334, NM_198056, NM_002976, NM_014191, NM_002977. NM_006514, NM_014139, AF109737), variants, alleles, isoforms, homologs, mutants, derivatives, fragments and complementary sequences thereto. Preferably the oligonucleotide is an antisense molecule and the targets include coding and noncoding regions of antisense and/or sense SCNA polynucleotides.

The preferred target segments of the present disclosure may be also be combined with their respective complementary antisense compounds of the present disclosure to form stabilized double-stranded (duplexed) oligonucleotides,

Such double stranded oligonucleotide moieties have been shown in the art to modulate target expression and regulate translation as well as RNA processing via an antisense mechanism. Moreover, the double-stranded moieties may be subject to chemical modifications. For example, such double-stranded moieties have been shown to inhibit the target by the classical hybridization of antisense strand of the duplex to the target, thereby triggering enzymatic degradation of the target.

In an instance, an antisense oligonucleotide targets Sodium channel voltage-gated, alpha subunit (SCNA) polynucleotides (e,g. accession number NM_001165963, NM_021007, NM_006922, NM_000334, NM_198056, NM_002976, NM_014191, NM_002977, NM_006514, NM_014139, AF109737), variants, alleles, isoforms, homologs, mutants, derivatives, fragments and complementary sequences thereto. Preferably the oligonucleotide is an antisense molecule,

In accordance with instances of the disclosure, the target nucleic acid molecule is not limited to SCNA alone but extends to any of the isoforms, receptors, homologs and the like of SCNA molecules.

In an instance, an oligonucleotide targets a natural antisense sequence of SCNA polynucleotides, for example, polynucleotides set forth as SEQ ID NOS: 12 to 28, and any variants, alleles, homologs, mutants, derivatives, fragments and complementary sequences thereto. Examples of antisense oligonucleotides are set forth as SEQ ID NOS: 29 to 94.

In one instance, the oligonucleotides are complementary to or bind to nucleic acid sequences of SCNA antisense, including without limitation noncoding sense and/or antisense sequences associated with SCNA polynucleotides and modulate expression and/or function of SCNA molecules.

In an instance, the oligonucleotides are complementary to or bind to nucleic acid sequences of SCNA natural antisense, set forth as SEQ ID NOS: 12 to 28, and modulate expression and/or function of SCNA molecules.

In an instance, oligonucleotides comprise sequences of at least 5 consecutive nucleotides of SEQ ID NOS: 29 to 94 and modulate expression and/or function of SCNA molecules.

The polynucleotide targets comprise SCNA, including family members thereof, variants of SCNA; mutants of SCNA, including SNPs; noncoding sequences of SCNA, alleles of SCNA; species variants, fragments and the like. Preferably the oligonucleotide is an antisense molecule.

In an instance, the oligonucleotide targeting SCNA polynucleotides, comprise: antisense RNA, interference RNA (RNAi), short interfering RNA (siRNA); micro interfering RNA (miRNA); a small, temporal RNA (stRNA): or a short hairpin RNA (shRNA); small RNA-induced gene activation (RNAa); or, small activating RNA (saRNA).

In an instance, targeting of Sodium channel, voltage-gated, alpha subunit (SCNA) polynucleotides, e.g. SEQ ID NOS: 1 to11, modulate the expression or function of these targets. In one instance, expression or function is upregulated as compared to a control. In an instance, expression or function is down-regulated as compared to a control. In a further instance, targeting of the natural antisense transcripts (e.g. SEQ ID NOS. 12 to 28) as well as any other target NATs of such target polynucleotides results in the upregulation of said target mRNA and corresponding protein.

In an instance, antisense compounds comprise sequences set forth as SEQ ID NOS: 29 to 94. These oligonucleotides can comprise one or more modified nucleotides, shorter or longer fragments, modified bonds and the like.

In an instance, SEQ ID NOS: 29 to 94 comprise one or more LNA nucleotides. Table 1 shows exemplary antisense oligonucleotides useful in the methods of the disclosure.

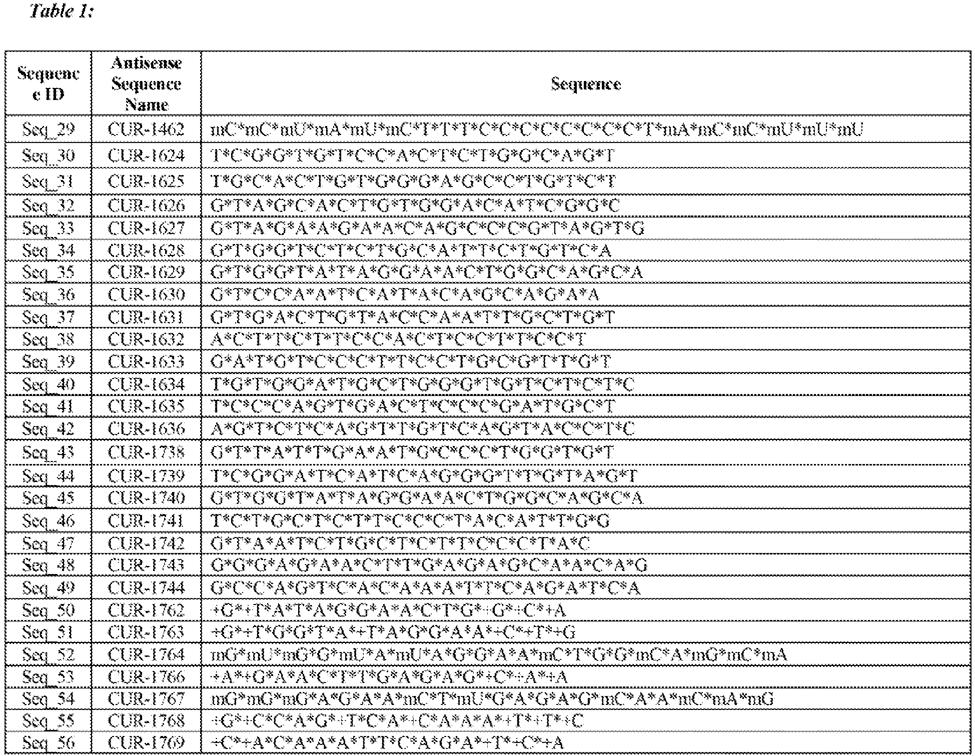

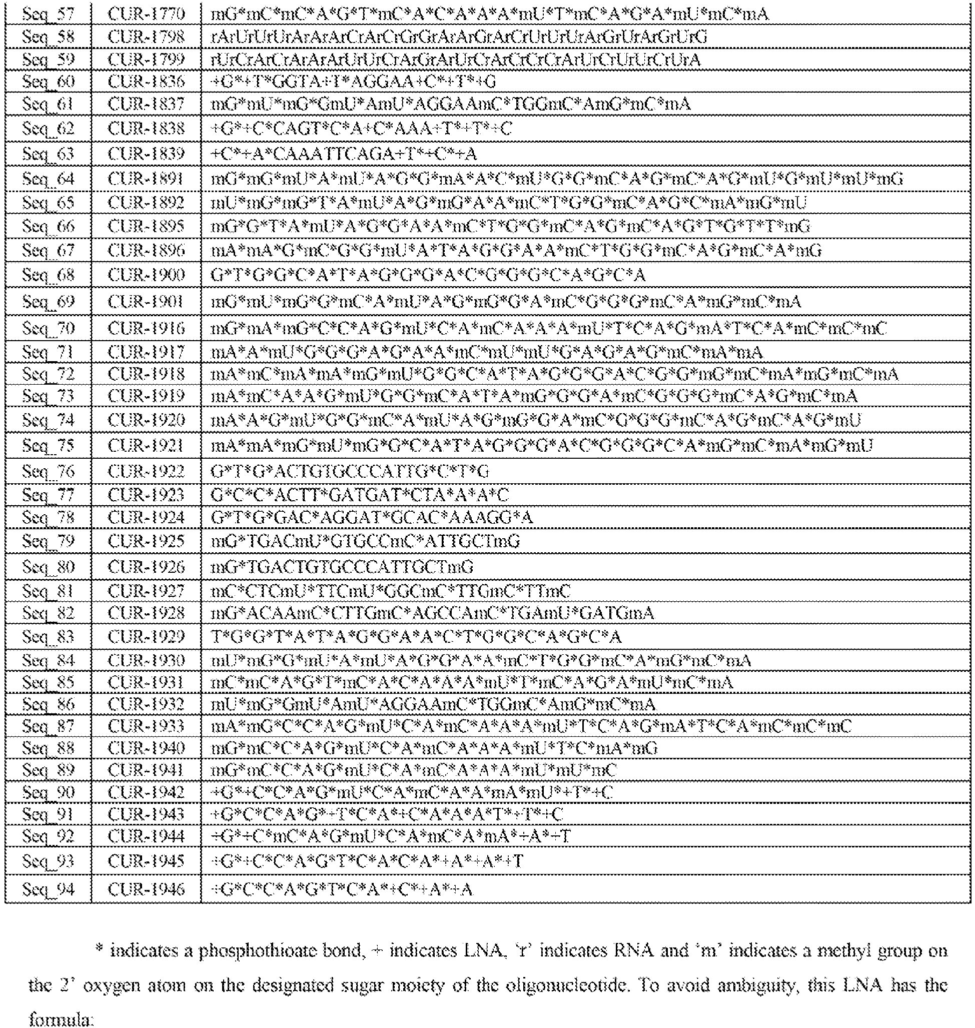

wherein B is the particular designated base.

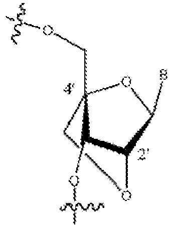

***Table 2:*** Relative expression of SCN1A mRNA in cells treated with antisense oligonucleotides targeting SCN1A-specific natural antisense transcript. Avg - average fold difference in SCN1A expression compared to mock transfected control; Std - standard deviation. P - probability that the treated samples are not different from mock control. N - total number of replicates

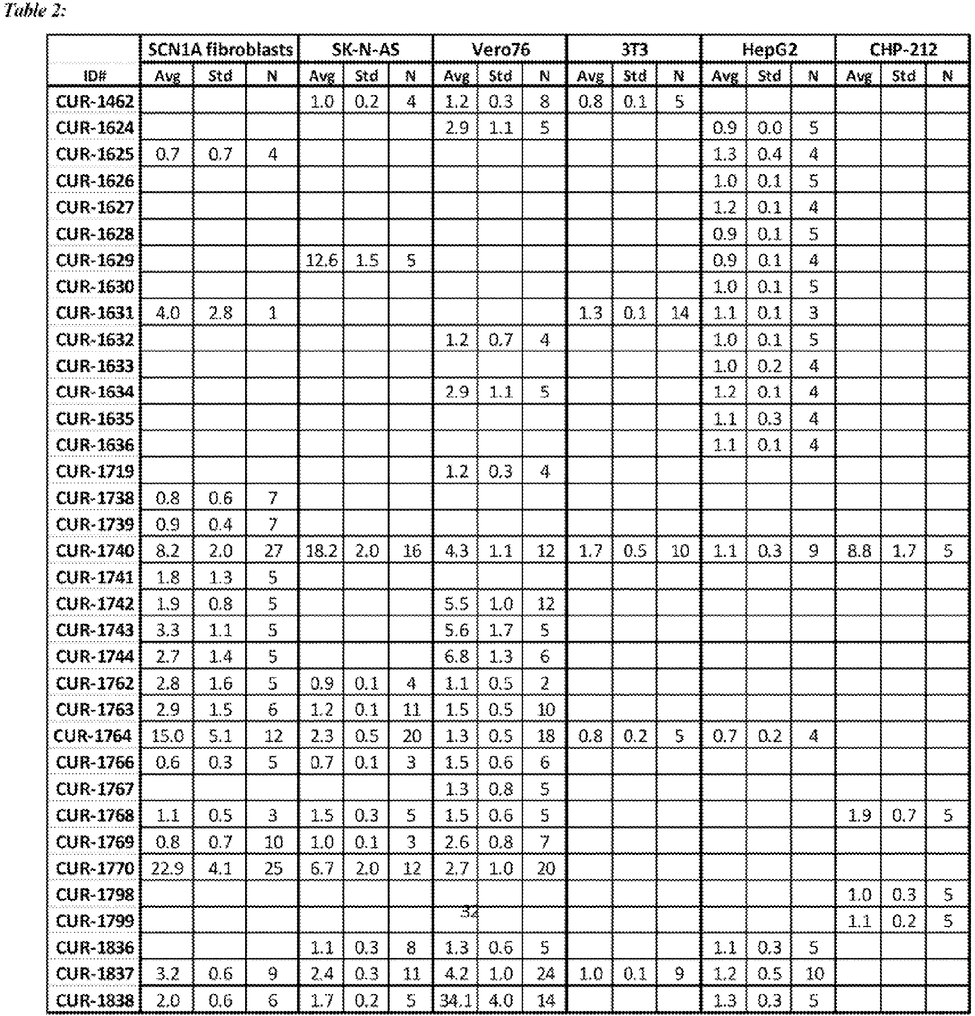

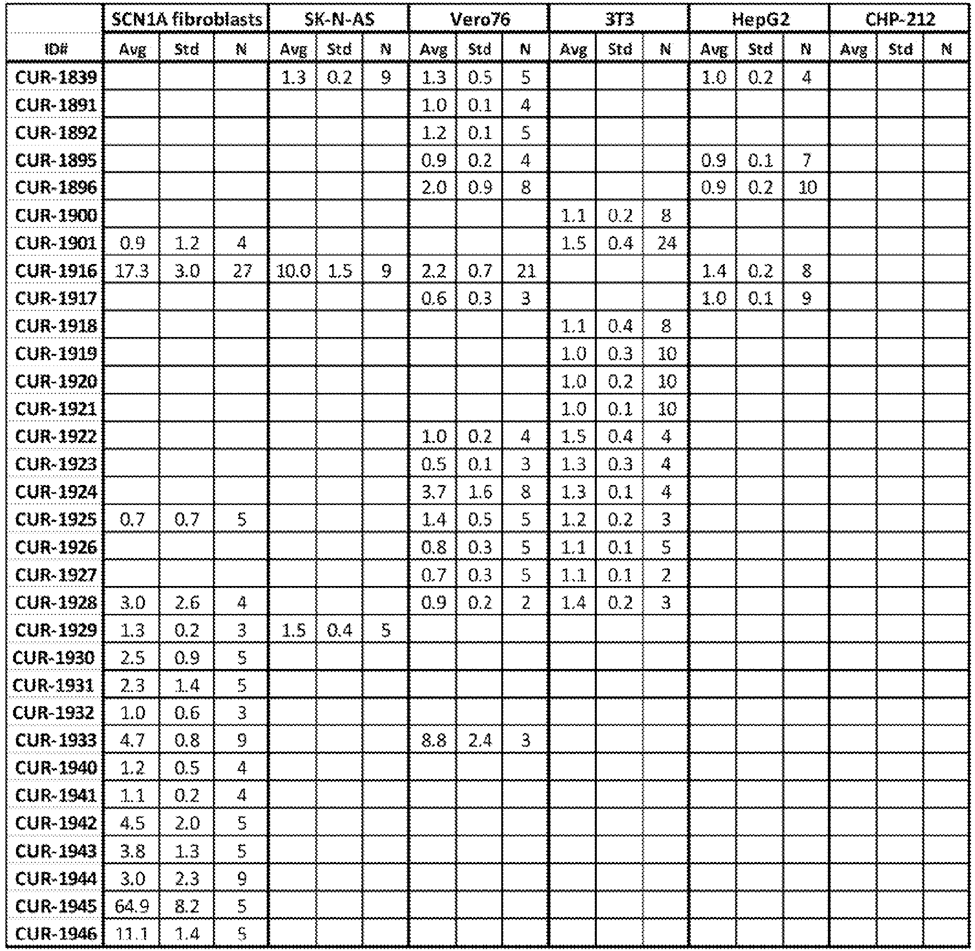

The modulation of a desired target nucleic acid can be carried out in several ways known in the art. For example, antiense oligonucleotides, siRNA etc. Enzymatic nucleic acid molecules (e.g., ribozymes) are nucleic acid molecules capable of catalyzing one or more of a variety of reactions, including the ability to repeatedly cleave other separate nucleic acid molecules in a nucleotide base sequence-specific manner. Such enzymatic nucleic acid molecules can be used, for example, to target virtually any RNA transcript.

Because of their sequence-specificity, trans-cleaving enzymatic nucleic acid molecules show promise as therapeutic agents for human disease. Enzymatic nucleic acid molecules can be designed to cleave specific RNA targets within the background of cellular RNA. Such a cleavage event renders the mRNA non-functional and abrogates protein expression from that RNA. In this manner, synthesis of a protein associated with a disease state can be selectively inhibited.

In general, enzymatic nucleic acids with RNA cleaving activity act by first binding to a target RNA, Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target it is released from that RNA to search for another target and can repeatedly bind and cleave new targets.

Several approaches such as in vitro selection (evolution) strategies (Orgel, (1979) Proc. R. Soc. London, B 205, 435) have been used to evolve new nucleic acid catalysts capable of catalyzing a variety of reactions, such as cleavage and ligation of phosphodiester linkages and amide linkages.

The development of ribozymes that are optimal for catalytic activity would contribute significantly to any strategy that employs RNA-cleaving ribozymes for the purpose of regulating gene expression. The hammerhead ribozyme, for example, functions with a catalytic rate (kcat) of about 1 min-1 in the presence of saturating (10 mM) concentrations of Mg2+ cofactor. An artificial "RNA ligase" ribozyme has been shown to catalyze the corresponding self-modification reaction with a rate of about 100 min-1. In addition, it is known that certain modified hammerhead ribozymes that have substrate binding arms made of DNA catalyze RNA cleavage with multiple turn-over rates that approach 100 min-1. Finally, replacement of a specific residue within the catalytic core of the hammerhead with certain nucleotide analogues gives modified ribozymes that show as much as a 10-fold improvement in catalytic rate. These findings demonstrate that ribozymes can promote chemical transformations with catalytic rates that are significantly greater than those displayed in vitro by most natural self-cleaving ribozymes. It is then possible that the structures of certain selfcleaving ribozymes may be optimized to give maximal catalytic activity, or that entirely new RNA motifs can be made that display significantly faster rates for RNA phosphodiester cleavage.

Intermolecular cleavage of an RNA substrate by an RNA catalyst that fits the "hammerhead" model was first shown in 1987 (Uhlenbeck, O. C. (1987) Nature, 328: 596-600). The RNA catalyst was recovered and reacted with multiple RNA molecules, demonstrating that it was truly catalytic.

Catalytic RNAs designed based on the "hammerhead" motif have been used to cleave specific target sequences by making appropriate base changes in the catalytic RNA to maintain necessary base pairing with the target sequences. This has allowed use of the catalytic RNA to cleave specific target sequences and indicates that catalytic RNAs designed according to the "hammerhead" model may possibly cleave specific substrate RNAs in vivo.

RNA interference (RNAi) has become a powerful tool for modulating gene expression in mammals and mammalian cells. This approach requires the delivery of small interfering RNA (siRNA) either as RNA itself or as DNA, using an expression plasmid or virus and the coding sequence for small hairpin RNAs that arc processed to siRNAs. This system enables efficient transport of the pre-siRNAs to the cytoplasm where they are active and permit the use of regulated and tissue specific promoters for gene expression.

In an instance, an oligonucleotide or antisense compound comprises an oligomer or polymer of ribonucleic acid (RNA) and/or deoxyribonucleic acid (DNA), or a mimetic, chimera, analog or homolog thereof. This term includes oligonucleotides composed of naturally occurring nucleotides, sugars and covalent internucleoside (backbone) linkages as well as oligonucleotides having non-naturally occurring portions which function similarly. Such modified or substituted oligonucleotides are often desired over native forms because of desirable properties such as, for example, enhanced cellular uptake, enhanced affinity for a target nucleic acid and increased stability in the presence of nucleases.

According to the present disclosure, the oligonucleotides or "antisense compounds" include antisense oligonucleotides (e.g. RNA, DNA, mimetic, chimera, analog or homolog thereof), ribozymes, external guide sequence (EGS) oligonucleotides, siRNA compounds, single- or double-stranded RNA interference (RNAi) compounds such as siRNA compounds, saRNA, aRNA, and other oligomeric compounds which hybridizeto at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines. When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'- hydroxyl or 2'-modified sugars and, generally U rather than T bases). Nucleic acid helices can adopt more than one type of structure, most commonly the A- and B-forms. It is believed that, in general, oligonucleotides which have B-form-like structure are "DNA-like" and those which have A-formlike structure are "RNA-like." In some (chimeric) embodiments, an antisense compound may contain both A- and B-form regions.

The antisense compounds in accordance with this disclosure can comprise an antisense portion from about 5 to about 80 nucleotides (i.e. from about 5 to about 80 linked nucleosides) in length. This refers to the length of the antisense strand or portion of the antisense compound. In other words, a single-stranded antisense compound of the disclosure comprises from 5 to about 80 nucleotides, and a double-stranded antisense compound of the disclosure (such as a dsRNA, for example) comprises a sense and an antisense strand or portion of 5 to about 80 nucleotides in length. One of ordinary skill in the art will appreciate that this comprehends antisense portions of 5, 6, 7,8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44,45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides in length, or any range therewithin.

In one instance, the antisense compounds of the disclosure have antisense portions of 10 to 50 nucleotides in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length, or any range therewithin. In some instances, the oligonucleotides are 15 nucleotides in length.

In one instance, the antisense or oligonucleotide compounds of the disclosure have antisense portions of 12 or 13 to 30 nucleotides in length. One having ordinary skill in the art will appreciate that this embodies antisense compounds having antisense portions of 12, 13, 14, 15, 16, 17, 18, 19, 20, 21, 22, 23, 24, 25, 26, 27, 28, 29 or 30 nucleotides in length, or any range therewithin.

In an instance, the oligomeric compounds of the present disclosure also include variants in which a different base is present at one or more of the nucleotide positions in the compound. For example, if the first nucleotide is an adenosine, variants may be produced which contain thymidine, guanosine or cytidine at this position. This may be done at any of the positions of the antisense or dsRNA compounds. These compounds are then tested using the methods described herein to determine their ability to inhibit expression of a target nucleic acid.

In some instances, homology, sequence identity or complementarity, between the antisense compound and target is from about 40% to about 60%. In some instances, homology, sequence identity or complementarity, is from about 60% to about 70%. In some instances, homology, sequence identity or complementarity, is from about 70% to about 80%. In some instances, homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%.

In an instance, the antisense oligonucleotides, such as for example, nucleic acid molecules set forth in SEQ ID NOS: 29 to 94 comprise one or more substitutions or modifications. In one instance, the nucleotides are substituted with locked nucleic acids (LNA).

In an instance, the oligonucleotides target one or more regions of the nucleic acid molecules sense and/or antisense of coding and/or non-coding sequences associated with SCNA and the sequences set forth as SEQ ID NOS: 1 to 28. The oligonucleotides are also targeted to overlapping regions of SEQ ID NOS: 1 to 28.

Certain preferred oligonucleotides of this disclosure are chimeric oligonucleotides. "Chimeric oligonucleotides" or "chimeras," in the context of this disclosure, are oligonucleotides which contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of antisense modulation of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art. In one an instance, a chimeric oligonucleotide comprises at least one region modified to increase target binding affinity, and, usually, a region that acts as a substrate for RNAse H. Affinity of an oligonucleotide for its target (in this case, a nucleic acid encoding ras) is routinely determined by measuring the Tm of an oligonucleotide/target pair, which is the temperature at which the oligonucleotide and target dissociate; dissociation is detected spectrophotometrically. The higher the Tm, the greater is the affinity of the oligonucleotide for the target.

Chimeric antisense compounds of the disclosure may be formed as composite structures of two or more oligonucleotides, modified oligonucleotides, oligonucleosides and or oligonucleotides mimetics as described above. Such; compounds have also been referred to in the art as hybrids or gapmers. Representative United States patents that teach the preparation of such hybrid structures comprise, but are not limited to, US patent nos. 5,013,830; 5,149,797; 5, 220,007; 5,256,775; 5,366,878; 5,403,711; 5,491,133; 5,565,350; 5,623,065; 5,652,355; 5,652,356; and 5,700,900.

In an instance, the region of the oligonucleotide which is modified comprises at least one nucleotide modified at the 2' position of the sugar, most preferably a 2'-Oalkyl, 2'-O-alkyl-O-alkyl or 2'-fluoro-modified nucleotide. In other an instance, RNA modifications include 2'-fluoro, 2'-amino and 2' O-methyl modifications on the ribose of pyrimidines, abasic residues or an inverted base at the 3' end of the RNA. Such modifications are routinely incorporated into oligonucleotides and these oligonucleotides have been shown to have a higher Tm (i.e., higher target binding affinity) than; 2'-deoxyoligonucleotides against a given target. The effect of such increased affinity is to greatly enhance RNAi oligonucleotide inhibition of gene expression. RNAse H is a cellular endonuclease that cleaves the RNA strand of RNA:DNA duplexes; activation of this enzyme therefore results in cleavage of the RNA target, and thus can greatly enhance the efficiency of RNAi inhibition. Cleavage of the RNA target can he routinely demonstrated by gel electrophoresis. In an instance, the chimeric oligonucleotide is also modified to enhance nuclease resistance, Cells contain a variety of exo- and endo-nucleases which can degrade nucleic acids. A number of nucleotide and nucleoside modifications have been shown to make the oligonucleotide into which they are incorporated more resistant to nuclease digestion than the native oligodeoxynudeotide; Nuclease resistance is routinely measured by incubating oligonucleotides with cellular extracts or isolated nuclease solutions and measuring the extent of intact oligonucleotide remaining over time, usually by gel electrophoresis. Oligonucleotides which have been modified to enhance their nuclease resistance survive intact for a longer time than unmodified oligonucleotides. A variety of oligonucleotide modifications have been demonstrated to enhance or confer nuclease resistance. Oligonucleotides which contain at least one phosphorothioate modification are presently more preferred. In some cases, oligonucleotide modications which enhance target binding affinity are also, independently, able to enhance nuclease resistance.

Specific examples of some preferred oligonucleotides envisioned for this disclosure include those comprising modified backbones, for example, phosphorothioates, phosphotriesters, methyl phosphonates, short chain alkyl or cycloalkyl intersugar linkages or short chain heteroatomic or heterocyclic intersugar linkages. Most preferred are oligonucleotides with phosphorothioate backbones and those with heteroatom backbones, particularly CH2--NH--O-CH2, CH,--N(CH3)--O--CH2 [known as a methylene(methylimino) or MMI backbone], CH2--O--N(CH3)--CH2, CH2-N(CH3)-N(CH3)-CH2 and O--N*(CH3)--CH2--CH2 backbones, wherein the native phosphodiester backbone is represented as O--P-O--CH₂). The amide backbones disclosed by De Mesmaeker et al. (1995) Acc. Chem. Res. 28:366-374 are also preferred. Also preferred are oligonucleotides having morpholino backbone structures (Summerton and Weller, U.S. Pat No. 5,034,506). In other an instance, such as the peptide nucleic acid (PNA) backbone, the phosphodiester backbone of the oligonucleotide is replaced with a polyamide backbone, the nucleotides being bound directly or indirectly to the aza nitrogen atoms of the polyamide backbone. Oligonucleotides may also comprise one or more substituted sugar moieties. Preferred oligonucleotides comprise one of the following at the 2' position: OH, SH, SCH3, F, OCN, OCH3 OCH3, OCH3 O(CH2)n CH3, O(CH2)n NH2 or O(CH2)n CH3 where n is from 1 to about 10; C1 to C10 lower alkyl, alkoxyalkoxy, substituted lower alkyl, alkaryl or aralkyl; Cl; Br; CN; CF3 ; OCF3; O--, S--, or N-alkyl; O-, S--, or N-alkenyl; SOCH3; SO2 CH3, ONO2; NO2; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. A preferred modification includes 2'-methoxyethoxy [2'-O-CH2 CH2 OCH3, also known as 2'-O-(2-methoxyethyl)]. Other preferred modifications include 2'-methoxy (2'-O--CH3), 2'-propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

Oligonucleotides may also include, additionally or alternatively, nucleobase (often referred to in the art simply as "base") modifications or substitutions. As used herein, "unmodified" or "natural" nucleotides include adenine (A), guanine (G), thymine (T), cytosine (C) and uracil (U). Modified nucleotides include nucleotides found only infrequently or transiently in natural nucleic acids, e.g., hypoxanthine, 6-methyladenine, 5-Me pyrimidines, particularly 5-methylcytosine (also referred to as 5-methyl-2' deoxycytosine and often referred to in the art as 3-Me-C), 5-hydroxymethylcytosine (HMC), glycosyl HMC and gentobiosyl HMC, as well as synthetic nucleotides, e.g., 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5- bromouracil, 5-hydroxymethyluracil, 8-azaguanine, 7-deazaguanine, N6 (6-aminohexyl)adenine and 2,6-diaminopurine. A "universal" base known in the art, e.g., inosine, may be included. 5-Me-C substitutions have been shown to increase nucleic acid duplex stability by 0.6-1.2°C, and are presently preferred base substitutions.

Another modification of the oligonucleotides of the disclosure involves chemically linking to the oligonucleotide one or more moieties or conjugates which enhance the activity or cellular uptake of the oligonucleotide. Such moieties include but are not limited to lipid moieties such as a cholesterol moiety, a cholesteryl moiety, an aliphatic chain, e.g., dodecandiol or undecyl residues, a polyamine or a polyethylene glycol chain, or Adamantane acetic acid. Oligonucleotides comprising lipophilic moieties, and methods for preparing such oligonucleotides are known in the art, for example, U.S. Pat. Nos, 5,138,045, 5,118,105 and 5,459,255.

It is not necessary for all positions in a given oligonucleotide to be uniformly modified, and in fact more than one of the aforementioned modifications may be incorporated in a single oligonucleotide or even at within a single nucleoside within an oligonucleotide. The present disclosure also includes oligonucleotides which are chimeric oligonucleotides as hereinbefore defined.

In another instance, the nucleic acid molecule of the present disclosure is conjugated with another moiety including but not limited to abasic nucleotides, polyether, polyamine, polyamides, peptides, carbohydrates, lipid, or polyhydrocarbon compounds. Those skilled in the art will recognize that these molecules can be linked to one or more of any nucleotides comprising the nucleic acid molecule at several positions on the sugar, base or phosphate group.

The oligonucleotides used in accordance with this disclosure may be conveniently and routinely made through the well-known technique of phase synthesis. Equipment for such synthesis is sold by several vendors including Applied Biosystems. Any other means tor such synthesis may also be employed; the actual synthesis of the oligonucleotides is well within the talents of one of ordinary skill in the art. It is also well known to use similar techniques to prepare other oligonucleotides such as the phosphorothioates and alkylated derivatives. It is also well known to use similar techniques and commercially available modified amidites and controlled-pore glass (CPG) products such as biotin, fluorescein, acridine or psoralen-modified amidites and/or CPG (available from Glen Research, Sterling VA) to synthesize fluorescently labeled, biotinylated or other modified oligonucleotides such as cholsterol-modified oligonucleotides.

In accordance with the disclosure, use of modifications such as the use of LNA monomers to enhance the potency, specificity and duration of action and broaden the routes of administration of oligonucleotides comprised of current chemistries such as MOE, ANA, FANA, PS etc. This can be achieved by substituting some of the monomers in the current oligonucleotides by LNA monomers. The LNA modified oligonucleotide may have a size similar to the parent compound or may be larger or preferably smaller. It is preferred that such LNA-modified oligonucleotides contain less than about 70%, more preferably less than about 60%, most preferably less than about 50% LNA monomers and that their sizes are between about 5 and 25 nucleotides, more preferably between about 12 and 20 nucleotides.

Preferred modified oligonucleotide backbones comprise, but not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative United States patents that teach the preparation of the above phosphorus containing linkages comprise, but are not limited to, US patent nos. 3,687,808; 4,469,863; 4,476,301; 5,023,243; 5, 177,196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts.

Representative United States patents that teach the preparation of the above oligonucleosides comprise, but are not limited to, US patent nos. 5,034,506; 5,166,315; 5,185,444; 5,214,134; 5,216,141; 5,235,033; 5,264, 562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704, 5,623,070; 5,663,312; 5,633,360; 5,677,437; and 5,677,439.

In other preferred oligonucleotide mimetics, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds comprise, but are not limited to, US patent nos, 5,539,082; 5,714,331; and 5,719,262.

Further teaching of PNA compounds can be found in Nielsen, et al. (1991) Science 254, 1497-1500.

In an instance of the disclosure the oligonucleotides with phosphorothioate backbones and oligonucleosides with heteroatom backbones, and in particular- CH2-NH-O-CH2-,-CH2-N (CH3)-O-CH2-known as a methylene (methylimino) or MMl backbone,- CH2-O-N (CH3)-CH2-,-CH2N(CH3)-N(CH3) CH2-and-O-N(CH3)-CH2-CH2- wherein the native phosphodiester backbone is represented as-O-P-O-CH2- of the above referenced US patent no, 5,489,677, and the amide backbones of the above referenced US patent no, 5,602,240. Also preferred are oligonucleotides having morpholino backbone structures of the above-referenced US patent no. 5,034,506.

Modified oligonucleotides may also contain one or more substituted sugar moieties. Preferred oligonucleotides comprise one of the following at the 2' posinon: OH; F; O-, S-, or N-alkyl; O-, S-, or N-alkenyl; O-, S-or N-alkynyl; or O alkyl-O-alkyl, wherein the alkyl, alkenyl and alkynyl may be substituted or unsubstituted C to CO alkyl or C2 to CO alkenyl and alkynyl. Particularly preferred are O (CH2)n OmCH3, O(CH2)n,OCH3, O(CH2)nNH2, O(CH2)nCH3, O(CH2)nONH2, and O(CH2nON(CH2)nCH3)2 where n and m can be from 1 to about 10. Other preferred oligonucleotides comprise one of the following at the 2' position: C to CO, (lower alkyl, substituted lower alkyl, alkaryl, aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification comprises 2'-methoxyethoxy (2'-O-CH2CH2OCH3, also known as 2-O(2-methoxyethyl) or 2'-MOE) i.e., an alkoxyalkoxy group. A further preferred modification comprises 2'-dimethylaminooxyethoxy, i.e., a O(CH2)2ON(CH3)2 group, also known as 2'-DMAOE, as described in examples herein below, and 2'-dimethylaminoethoxyethoxy (also known in the art as 2'-O-dimethylaminoethoxyethyl or 2'-DMAEOE), i.e., 2'-O-CH2-O-CH2-N (CH2)2.

Other preferred modifications comprise 2'-methoxy (2'-OCH3), 2'-aminopropoxy (2'-O CH2CH2CH2NH2) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures comprise, but are not limited to, US patent nos. 4,981,957; 5,118,800; 5,319,080; 5,359,044; 5,393,878; 5,446,137; 5,466,786; 5,514, 785; 5,519,134; 5,567,811; 5,576,427; 5,591,722; 5,597,909; 5,610,300; 5,627,053; 5,639,873; 5,646, 265; 5,658,873; 5,670,633; and 5,700,920.

Oligonucleotides may also comprise nucleobase (often referred to in the art simply as "base") modifications or substitutions. As used herein, "unmodified" or "natural" nucleotides comprise the purine bases adenine (A) and guanine (G), and the pyrimidine bases thymine (T), cytosine (C) and uracil (U). Modified nucleotides comprise other synthetic and natural nucleotides such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other 8-substituted adenines and guanines, 5-halo particularly 5-hromo, 5-trifluoromethyl and other 5-substituted uracils and cytosines, 7-methylquanine and 7-methyladenine, 8-azaguanine and 8-azaadenine, 7-deazaguanine and 7-deazaadenine and 3-deazaguanine and 3-deazaadenine.

Further, nucleotides comprise those disclosed in United States Patent No. 3,687,808, those disclosed in The Concise Encyclopedia of Polymer Science And Engineering', pages 858-859, Kroschwitz, J.I., ed. John Wiley & Sons, 1990, those disclosed by Englisch et al., 'Angewandle Chemie, International Edition', 1991, 30, page 613, and those disclosed by Sanghvi, Y.S., Chapter 15, 'Antisense Research and Applications', pages 289-302, Crooke, S.T. and Lebleu, B. ea., CRC Press, 1993. Certain of these nucleotides are particularly useful increasing the binding affinity of the oligomeric compounds of the disclosure. These comprise 5-substituted pyrimidines, 6- azapyrimidines and N-2, N-6 and 0-6 substituted purines, comprising 2-aminopropyladenine, 5-propynyluracil and 5-propynylcytosine, 5-methylcytosine substitutions have been shown to increase nucleic acid duplex stability by 0.6-1.2°C (Sanghvi, Y.S., Crooke, S.T. and Lebleu, B., eds, 'Antisense Research and Applications', CRC Press, Boca Raton, 1993, pp. 276-278) and are presently preferred base substitutions, even more particularly when combined with 2'-Omethoxyethyl sugar modifications.

Representative United States patents that teach the preparation of the above noted modified nucleotides as well as other modified nucleotides comprise, but are not limited to, US patent nos. 3,687,808, as well as 4,845,205; 5,130,302; 5,134,066; 5,175, 273; 5, 367,066; 5,433,272. 5,457,187; 5,459,255; 5,484,908; 5,502,177; 5,525,711; 5,552,540; 5,587,469; 5,596,091; 5,614,617; 5,750,692, and 5,681,941.

Another modification of the oligonucleotides of the disclosure involves chemically linking to the oligonucleotide one or more moieties or conjugates, which enhance the activity, cellular distribution, or cellular uptake of the oligonucleotide.

Such moieties comprise but are not limited to, lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or Adamantane acetic acid, a palmityl moiety, or an octadecylamine or hexylamino-carbonyl-toxycholeslerol moiety.

Representative United States patents that teach the preparation of such oligonucleotides conjugates comprise, but are not limited to, US patent nos. 4,828,979; 4,948,882; 5,218,105; 5,525,465; 5,541,313; 5,545,730; 5,552, 538; 5,578,717, 5,580,731; 5,580,731; 5,591,584; 5,108,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5, 245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5, 565,552; 5,567,810; 5,574,142; 5,585,481; 5,587,371; 5,595,726; 5,597,696; 5,599,923; 5,599,928 and 5,688,941.

*Drug discovery:* The compounds of the present disclosure can also be applied in the areas of drug discovery and target validation. The present disclosure comprehends the use of the compounds and preferred target segments identified herein in drug discovery efforts to elucidate relationships that exist between Sodium channel, voltage-gated, alpha subunit (SCNA) polynucleotides and a disease state, phenotype, or condition. These methods include detecting or modulating SCNA polynucleotides comprising contacting a sample, tissue, cell, or organism with the compounds of the present disclosure, measuring the nucleic acid or protein level of SCNA polynucleotides and/or a related phenotypic or chemical endpoint at some time after treatment, and optionally comparing the measured value to a non-treated sample or sample treated with a further compound of the disclosure. These methods can also be performed in parallel or in combination with other experiments to determine the function of unknown genes for the process of target validation or to determine the validity of a particular gene product as a target for treatment or prevention of a particular disease, condition, or phenotype.

### Assessing Upreguknion or Inhibition of Gene Expression:

Transfer of an exogenous nucleic acid into a host cell or organism can be assessed by directly detecting the presence of the nucleic acid in the cell or organism. Such detection can be achieved by several methods well known in the art. For example, the presence of the exogenous nucleic acid can be detected by Southern blot or by a polymerase chain reaction (PCR) technique using primers that specifically amplify nucleotide sequences associated with the nucleic acid. Expression of the exogenous nucleic acids can also be measured using conventional methods including gene expression analysis. For instance, mRNA produced from an exogenous nucleic acid can be detected and quantified using a Northern blot and reverse transcription PCR (RT-PCR).

Expression of RNA from the exogenous nucleic acid can also be detected by measuring an enzymatic activity or a reporter protein activity. For example, antisense modulatory activity can be measured indirectly as a decrease or increase in target nucleic acid expression as an indication that the exogenous nucleic acid is producing the effector RNA. Based on sequence conservation, primers can be designed and used to amplify coding regions of the target genes. Initially, the most highly expressed coding region from each gene can be used to build a model control gene, although any coding or non coding region can be used. Each control gene is assembled by inserting each coding region between a reporter coding region and its poly(A) signal. These plasmids would produce an mRNA with a reporter gene in the upstream portion of the gene and a potential RNAi target in the 3' non-coding region. The effectiveness of individual antisense oligonucleotides would be assayed by modulation of the reporter gene. Reporter genes useful in the methods of the present disclosure include acetohydroxyacid synthase (AHAS), alkaline phosphatase (AP), beta galactosidase (LacZ), beta glucoronidase (GUS), chloramphenicol acetyltransterase (CAT), green fluorescent protein (GFP), red fluorescent protein (RFP), yellow fluorescent protein (YFP), cyan fluorescent protein (CFP), horseradish peroxidase (MRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracycline. Methods to determine modulation of a reporter gene are well known in the art, and include, but are not limited to, fluorometric methods (e.g. fluorescence spectroscopy, Fluorescence Activated Cell Sorting (FACS), fluorescence microscopy), antibiotic resistance determination.

Target nucleic acid segments can also be detected in the cell based assays. Experiments are conducted to detect the Senla natural antisense BG724147 in HepG2, in Primary human fibroblasts carrying a Dravet syndrome-associated mutation and also in human Testis. For HepG2 as well as Primary human fibroblasts carrying a Dravet syndrome-associated mutation the cells are grown and RNA is extracted For the human Testis, polyA isolated RNA is purchased and utilized. This experiment is called a RACE (Rapid Amplification of cDNA Ends) and specific primers for the BG724147 RNA transcript are used.

A PCR product very similar in polyA isolated RNA from HepG2 and polyA isolated RNA from Primary human fibroblasts carrying a Dravet syndrome-associated mutation was detected but this product was not detected in poly A isolated RNA from human Testis. Furthermore, that PCR product was not detected (or in very very low amounts) in the total RNA from HepG2 cells and total RNA from Primary human fibroblasts carrying a Dravet syndrome-associated mutation. The results suggest that the natural antisense for Scn1a called BG724147 is present in HepG2 cells and Primary human fibroblasts carrying a Dravet syndrome-associated mutation but not in human Testis.

SCNA protein and mRNA expression can be assayed using methods known to those of skill in the art and described elsewhere herein. For example, immunoassays such as the ELISA can be used to measure protein levels. SCNA ELISA assay kits are available commercially, e.g., from R&D Systems (Minneapolis, MN).

In instances, SCNA expression (e.g., mRNA or protein) in a sample (e.g., cells or tissues in vivo or in vitro) treated using an antisense oligonucleotide of the disclosure is evaluated by comparison with SCNA expression in a control sample. For example, expression of the protein or nucleic acid can be compared using methods known to those of skill in the art with that in a mock-treated or untreated sample. Alternatively, comparison with a sample treated with a control antisense oligonucleotide (e.g., one having an altered or different sequence) can be made depending on the information desired. In another instance, a difference in the expression of the SCNA protein or nucleic acid in a treated vs. an untreated sample can be compared with the difference in expression of a different nucleic acid (including any standard deemed appropriate by the researcher, e.g., a housekeeping gene) in a treated sample vs. an untreated sample.

Observed differences can be expressed as desired, e.g., in the form of a ratio or fraction, for use in a comparison with control. In instances, the level of SCNA mRNA or protein, in a sample treated with an antisense oligonucleotide of the present disclosure, is increased or decreased by about 1.25-fold to about 10-fold or more relative to an untreated sample or a sample treated with a control nucleic acid. In instances, the level of SCNA mRNA or protein is increased or decreased by at least about 1.25-fold, at least about 1.3-fold, at least about 1.4-fold, at least about 1.5-fold, at least about 1.6-fold, at least about 1.7-fold, at least about 1.8-fold, at least about 2-fold, at least about 2.5-fold, at least about 3-fold, at least about 3.5-fold, at least about 4-fold, at least about 4.5-fold, at least about 5-fold, at least about 5.5-fold, at least about 6-fold, at least about 6.5-fold, at least about 7-fold, at least about 7.5-fold, at least about 8-fold, at least about 8.5-fold, at least about 9-fold, at least about 9.5-fold, or at least about 10-fold or more.

### Kits, Research Reagents, Diagnostics, and Therapeutics

The compounds of the present disclosure can be utilized for diagnostics, therapeutics, and prophylaxis, and as research reagents and components of kits. Furthermore, antisense oligonucleotides, which are able to inhibit gene expression with exquisite specificity, are often used by those of ordinary skill to elucidate the function of particular genes or to distinguish between functions of various members of a biological pathway.

For use in kits and diagnostics and in various biological systems, the compounds of the present disclosure, either alone or in combination with other compounds or therapeutics, are useful as tools in differential and/or combinatorial analyses to elucidate expression patterns of a portion or the entire complement of genes expressed within cells and tissues.

As used herein the term "biological system" or "system" is defined as any organism, cell, cell culture or tissue that expresses, or is made competent to express products of the Sodium channel, voltage-gated, alpha subunit (SCNA) genes. These include, but are not limited to, humans, transgenic animals, cells, cell cultures, tissues, xenografts, transplants and combinations thereof.

As one non limiting example, expression patterns within cells or tissues treated with one or more antisense compounds are compared to control cells or tissues not treated with antisense compounds and the patterns produced are analyzed for differential levels of gene expression as they pertain, for example, to disease association, signaling pathway, cellular localization, expression level size, structure or function of the genes examined. These analyses can be performed on stimulated or unstimulated cells and in the presence or absence of other compounds that affect expression patterns.

Examples of methods of gene expression analysis known in the art include DNA arrays or microarrays, SAGE (serial analysis of gene expression), READS (restriction enzyme amplification of digested cDNAs), TOGA (total gene expression analysis), protein arrays and proteomics, expressed sequence tag (EST) sequencing subtractive RNA fingerprinting (SuRF), subtractive cloning, differential display (DD), comparative genomic hybridization, FISH (fluorescent in situ hybridization) techniques and mass spectrometry methods.

The compounds of the disclosure are useful for research and diagnostics, because these compounds hybridize to nucleic acids encoding Sodium channel, voltage-gated, alpha subunit (SCNA). For example, oligonucleotides that hybridize with such efficiency and under such conditions as disclosed herein as to be effective SCNA modulators are effective primers or probes under conditions favoring gene amplification or detection, respectively. These primers and probes are useful in methods requiring the specific detection of nucleic acid molecules encoding SCNA and in the amplification of said nucleic acid molecules for detection or for use in further studies of SCNA. Hybridization of the antisense oligonucleotides, particularly the primers and probes, of the disclosure with a nucleic acid encoding SCNA can be detected by means known in the art. Such means may include conjugation of an enzyme to the oligonucleotide, radiolabeling of the oligonucleotide, or any other suitable detection means. Kits using such detection means for detecting the level of SCNA in a sample may also be prepared.

The specificity and sensitivity of antisense are also harnessed by those of skill in the art for therapeutic uses. Antisense compounds have been employed as therapeutic moieties in the treatment of disease states in animals, including humans. Antisense oligonucleotide drugs have been safely and effectively administered to humans and numerous clinical trials are presently underway. It is thus established that antisense compounds can be useful therapeutic modalities that can be configured to be useful in treatment regimes for the treatment of cells, tissues and animals, especially humans.

For therapeutics, an animal, preferably a human, suspected of having a disease or disorder which can be treated by modulating the expression of SCNA polynucleotides is treated by administering antisense compounds in accordance with this disclosure. For example, in one non-limiting instance, the methods comprise the step of administering to the animal in need of treatment, a therapeutically effective amount of SCNA modulator. The SCNA modulators of the present disclosure effectively modulate the activity of the SCNA or modulate the expression of the SCNA protein. In one instance, the activity or expression of SCNA in an animal is inhibited by about 10% as compared to a control. Preferably, the activity or expression of SCNA in an animal is inhibited by about 30%. More preferably, the activity or expression of SCNA in an animal is inhibited by 50% or more. Thus, the oligomeric compounds modulate expression of Sodium channel voltage-gated, alpha subunit (SCNA) mRNA by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

In one instance, the activity or expression of Sodium channel, voltage-gated, alpha subunit (SCNA) and/or in an animal is increased by about 10% as compared to a control. Preferably, the activity or expression of SCNA in an animal is increased by about 30%. More preferably, the activity or expression of SCNA in an animal is increased by 50% or more. Thus, the oligomeric compounds modulate expression of SCNA mRN A by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

For example, the reduction of the expression of Sodium channel, voltage-gated, alpha subunit (SCNA) may be measured in serum, blood, adipose tissue, liver or any other body fluid, tissue or organ of the animal Preferably, the cells contained within said fluids, tissues or organs being analyzed contain a nucleic acid molecule encoding SCNA peptides and/or the SCNA protein itself

The compounds of the disclosure can be utilized in pharmaceutical compositions by adding an effective amount of a compound to a suitable pharmaceutically acceptable diluent or carrier. Use of the compounds and methods of the invention may also be useful prophylactically.

### Conjugates

Another modification of the oligonucleotides of the disclosure involves chemically linking to the oligonucleotide one or more moieties or conjugates that enhance the activity, cellular distribution or cellular uptake of the oligonucleotide. These moieties or conjugates can include conjugate groups covalently bound to functional groups such as primary or secondary hydroxyl groups. Conjugate groups of the disclosure include intercalators, reporter molecules, polyamines, polyamides, polyethylene glycols, polyethers, groups that enhance the pharmacodynamic properties of oligomers, and groups that enhance the pharmacokinetic properties of oligomers. Typicalconjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this disclosure, include groups that improve uptake, enhance resistance to degradation, and/or strengthen sequence-specific hybridization with the target nucleic acid. Groups that enhance the pharmacokinetic properties, in the context of this disclosure, include groups that improve uptake, distribution, metabolism or excretion of the compounds of the present disclosure. Representative conjugate groups are disclosed in international Patent Application No. PCT/US92/09196, filed Oct. 23, 1992, and U.S. Pat. No. 6,287,860.
Conjugate moieties include, but are not limited to, lipid moieties such as a cholesterol moiety, cholic acid, a thioether, e.g., hexyl-5- tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-Hphosphonate, a polyamine or a polyethylene glycol chain, or Adamantane acetic acid, a palmityl moiety, or an octadecylamine or hexylamino-carbonyl-oxycholesterol moiety. Oligonucleotides of the disclosure may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic.

Representative United States patents that teach the preparation of such oligonucleotides conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

### Formulations

The compounds of the disclosure may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption, Representative United States patents that teach the preparation of such uptake, distribution and/or absorption-assisting formulations include, but are not limited to, U.S. Pat. Nos. 5,108,921; 5,354,844; 5,416,016; 5,459,127; 5,521,291; 5,543,165; 5,547,932; 5,583,020; 5,591,721; 4,426,330; 4,534,899; 5,013,556; 5,108,921: 5,213,804; 5,227,170; 5,264,221; 5,356,633; 5,395,619; 5,416,016; 5,417,978; 5,462,854; 5,468,854; 5,512,295; 5,527,528; 5,534,259; 5,543,152; 5,556,948; 5,580,575; and 5,595,756.

Although, the antisense oligonucleotides do not need to be administered in the context of a vector in order to modulate a target expression and/or function, instances of the disclosure relates to expression vector constructs for the expression of antisense oligonucleotides, comprising promoters, hybrid promoter gene sequences and possess a strong constitutive promoter activity, or a promoter activity which can be induced in the desired case.

In an instance, disclosure practice involves administering at least one of the foregoing antisense oligonucleotides with a suitable nucleic acid delivery system. In one instance, that system includes a non-viral vector operably linked to the polynucleotide. Examples of such nonviral vectors include the oligonucleotide alone (e.g. any one or more of SEQ ID NOS: 29 to 94) or in combination with a suitable protein, polysaccharide or lipid formulation.

Additionally suitable nucleic acid delivery systems include viral vector, typically sequence from at least one of an adenovirus, adenovirus-associated virus (AAV), herper-dependent adenovirus, retrovirus, or hemagglutinatin virus of Japan-liposome (HVJ) complex. Preferably, the viral vector comprises a strong eukaryotic promoter operably linked to the polynucleotide e.g., a cytomegalovirus (CMV) promoter.

Additionally preferred vectors include viral vectors, fusion proteins and chemical conjugates. Retroviral vectors include Moloney murine leukemia viruses and HIV-based viruses. One preferred HIV-based viral vector comprises at least two vectors wherein the gag and pol genes are from an HIV genome and the env gene is from another virus. DNA viral vectors are preferred. These vectors include pox vectors such as orthopox or avipox vectors, herpesvirus vectors such as a herpes simplex I virus (HSV) vector, Adenovirus Vectors and Adeno-associated Virus Vectors.

The antisense compounds of the disclosure encompass any pharmaceutically acceptable salts, esters, or salts of such esters, or any other compound which, upon administration to an animal, including a human, is capable of providing (directly or indirectly) the biologically active metabolite or residue thereof.

The term "pharmaceutically acceptable salts" refers to physiologically and pharmaceutically acceptable salts of the compounds of the disclosure: i.e., salts that retain the desired biological activity of the parent compound and do not impart undesired toxicological effects thereto. For oligonucleotides, preferred examples of pharmaceutically acceptable salts and their uses are further described in U.S. Pat. No. 6,287,860.

The present disclosure also includes pharmaceutical compositions and formulations that include the antisense compounds of the disclosure. The pharmaceutical compositions of the present disclosure may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

For treating tissues in the central nervous system, administration can be made by, e.g., injection or infusion into the cerebrospinal fluid. Administration of antisense RNA into cerebrospinal fluid is described, e.g., in U.S. Pat. ADD. Pub. No. 2007/0117772, "Methods for slowing familial ALS disease progression,".

When it is intended that the antisense oligonucleotide of the present disclosure be administered to cells in the central nervous system, administration can be with one or more agents capable of promoting penetration of the subject antisense oligonucleotide across the blood-brain barrier. Injection can be made, e.g., in the entorhinal cortex or hippocampus. Delivery of neurotrophic factors by administration of an adenovirus vector to motor neurons in muscle tissue is described in, e.g., U.S. Pat. No. 6,632,427, "Adenoviral-vector-mediated gene transfer into medullary motor neurons,". Delivery of vectors directly to the brain, e.g., the striatum, the thalamus, the hippocampus, or the substantia nigra, is known in the art and described, e.g., in U.S. Pat. No. 6,756,523, "Adenovirus vectors for the transfer of foreign genes into cells of the central nervous system particularly in brain,".

Administration can be rapid as by injection or made over a period of time as by slow infusion or administration of slow release formulations.

The subject antisense oligonucleotides can also be linked or conjugated with agents that provide desirable pharmaceutical or pharmacodynamic properties. For example, the antisense oligonucleotide can be coupled to any substance, known in the art to promote penetration or transport across the blood-brain barrier, such as an antibody to the transferrin receptor, and administered by intravenous injection. The antisense compound can be linked with a viral vector, for example, that makes the antisense compound more effective and/or increases the transport of the antisense compound across the blood-brain barrier. Osmotic blood brain barrier disruption can also be accomplished by, e.g., infusion of sugars including, but not limited to, meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(-) raffinose, L(+) rhamnose, D(+) melibiose, D(-) ribose, adonitol, D(+) arabitol, L(-) arabitol, D(+) fucose, L(-) fucose_{.} D(-) lyxose, L(+) lyxose, and L(-) lyxose, or amino acids including, but not limited to, glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U. S. Patent No. 4,866,042, "Method for the delivery of genetic material across the blood brain barrier," 6,294,520, "Material for passage through the blood-brain barrier," and 6,936,589, "Parenteral delivery systems,".

The subject antisense compounds may be admixed, encapsulated, conjugated or otherwise associated with other molecules, molecule structures or mixtures of compounds, for example, liposomes, receptor-targeted molecules, oral, rectal, topical or other formulations, for assisting in uptake, distribution and/or absorption. For example, cationic lipids may be included in the formulation to facilitate oligonucleotide uptake. One such composition shown to facilitate uptake is LIPOFECTIN (available from GIBCO-BRL, Bethesda, MD).

Oligonucleotides with at least one 2'-O-methoxyethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s), In general, the formulations are prepared by uniformly and intimately bringing into association the active ingredients with liquid carriers or finely divided solid carriers or both, and then, if necessary, shaping the product.

The compositions of the present disclosure may be formulated into any of many possible dosage forms such as, but not limited to, tablets, capsules, gel capsules, liquid syrups, soft gels, suppositories, and enemas. The compositions of the present disclosure may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

Pharmaceutical compositions of the present disclosure include, but are not limited to, solutions, emulsions, foams and liposome-containing formulations. The pharmaceutical compositions and formulations of the present disclosure may comprise one or more penetration enhancers, carriers, excipients or other active or inactive ingredients.

Emulsions are typically heterogeneous systems of one liquid dispersed in another in the form of droplets usually exceeding 0.1 µm in diameter. Emulsions may contain additional components in addition to the dispersed phases, and the active drug that may be present as a solution in either the aqueous phase, oily phase or itself as a separate phase. Microemulsions are included as an instance of the present disclosure. Emulsions and their uses are well known in the art and are further described in U.S. Pat. No. 6,287,860.

Formulations of the present disclosure include liposomal formulations. As used in the present disclosure, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes that are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids. When incorporated into liposomes, these specialized lipids result in liposomes with enhanced circulation lifetimes relative to liposomeslacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860.

The pharmaceutical formulations and compositions of the present disclosure may also include surfactants. The use of surfactants in drug products, formulations and in emulsions is well known in the art. Surfactants and their uses are further described in U.S. Pat. No. 6,287,860.

In one instance, the present disclosure employs various penetration enhancers to effect the efficient delivery of nucleic acids, particularly oligonucleotides. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating nonsurfactants. Penetration enhancers and their uses are further described in U.S. Pat. No. 6,287,860.

One of skill in the art will recognize that formulations are routinely designed according to their intended use, i.e. route of administration.

Preferred formulations for topical administration include those in which the oligonucleotides of the disclosure are in admixture with a topical delivery agent such as lipids, liposomes, fatty acids, fatty acid esters, steroids, chelating agents and surfactants. Preferred lipids and liposomes include neutral (e.g. dioleoyl-phosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoyl-phosphatidyl ethanolamine DOTMA).

For topical or other administration, oligonucleotides of the disclosure may be encapsulated within liposomes or may form complexes thereto, in particular to cationic liposomes. Alternatively, oligonucleotides may be complexed to lipids, in particular to cationic lipids. Preferred fatty acids and esters, pharmaceutically acceptable salts thereof, and their uses are further described in U.S. Pat. No. 6,287,860.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Preferred oral formulations are those in which oligonucleotides of the disclosure are administered in conjunction with one or more penetration enhancers surfactants and chelators. Preferred surfactants include fatty acids and/or esters or salts thereof, bile acids and/or salts thereof. Preferred bile acids/salts and fatty acids and their uses are further described in U.S. Pat. No. 6,287,860. Also preferred are combinations of penetration enhancers, for example, fatty acids/salts in combination with bile acids/salts. A particularly preferred combination is the sodium salt of lauric acid, capric acid and UDCA. Further penetration enhancers include polyoxyethylene-9-lauryl ether, polyoxyethylene-20-cetyl ether. Oligonucleotides of the disclosure may be delivered orally, in granular form including sprayed dried particles, or complexed to form micro or nanoparticles. Oligonucleotide complexing agents and their uses are further described in U.S. Pat. No. 6,287,860.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Certain instances of the disclosure provide pharmaceutical compositions containing one or more oligomeric compounds and one or more other chemotherapeutic agents that function by a non-antisense mechanism. Examples of such chemotherapeutic agents include but are not limited to cancer chemotherapeutic drugs such as daunorubicin, daunomycin, dactinomycin, doxorubicin, epirubicin, idarubicin, esorubicin, bleomycin, mafosfamide, ifosfamide, cytosine arabinoside, bischloroethyl- nitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcydohexylnitrosurea, nitrogen mustards, melphalan, cyclophosphamide, 6-mercaptopurine, 6-thioguanine, cytarabine, 5- azacytidine, hydroxyurea, deoxycoformycin, 4-hydroxyperoxycyclo-phosphoramide, 5-fluorouracil (5-FU), S-fluorodeoxyuridine (5-FUdR), methotrexate (MTX), colchicine, taxol, vincristine, vinblastine, etoposide (VP-16), trimetrexate, irinotecan, topotecan, gemeitabine, teniposide, cisplatin and diethylstilbestrol (DES). When used with the compounds of the disclosure, such chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (e.g., 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the disclosure. Combinations of antisense compounds and other non-antisense drugs are also within the scope of this disclosure. Two or more combined compounds may be used together or sequentially.

In another related instance, compositions of the disclosure may contain one or more antisense compounds, particularly oligonucleotides, targeted to a first nucleic acid and one or more additional antisense compounds targeted to a second nucleic acid target. For example, the first target may be a particular antisense sequence of Sodium channel, voltage-gated, alpha subunit (SCNA), and the second target may be a region from another nucleotide sequence. Alternatively, compositions of the disclosure may contain two or more antisense compounds targeted to different regions of the same Sodium channel, voltage-gated, alpha submit (SCNA) nucleic acid target. Numerous examples of antisense compounds are illustrated herein and others may be selected from among suitable compounds known in the art. Two or more combined compounds may be used together or sequentially.

### Dosing:

The formulation of therapeutic compositions and their subsequent administration (dosing) is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligonucleotides, and can generally be estimated based on EC50s found to be effective *in vitro* and *in vivo* animal models. In general, dosage is from 0.01 µg to 100 mg per kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligonucleotide is administered in maintenance doses, ranging from 0.01 µg to 100 mg per kg of body weight, once or more daily, to once every 20 years.

In instances, a patient is treated with a dosage of drug that is at least about 1, at least about 2, at least about 3, at least about 4, at least about 5, at least about 6, at least about 7, at least about 8, at least about 9, at least about 10, at least about 15, at least about 20, at least about 25, at least about 30, at least about 35, at least about 40, at least about 45, at least about 50, at least about 60, at least about 70, at least about 80, at least about 90, or at least about 100 mg/kg body weight. Certain injected dosages of antisense oligonucleotides are described, e.g., in U.S. Pat. No. 7,563,884, "Antisense modulation of PTPIB expression,".

While various instances of the present disclosure have been described above, it should be understood that they have been presented by way of example only, and not limitation. Numerous changes to the disclosed instances can be made in accordance with the disclosure herein without departing from the spirit or scope of the disclosure. Thus, the breadth and scope of the present disclosure should not be limited by any of the above described instances.

By their citation of various references in this document, Applicants do not admit any particular reference is "prior art" to their invention. Embodiments of inventive compositions and methods are illustrated in the following examples.

### EXAMPLES

The following non-limiting Examples serve to illustrate selected embodiments of the invention. It will be appreciated that variations in proportions and alternatives in elements of the components shown will be apparent to those skilled in the art and are within the scope of instances of the present disclosure.

### Example 1: Design of antisense oligonucleotides specific for a nucleic acid molecule antisense to a Sodium channel, voltage-gated, alpha submit (SCNA) and/or a sense strand of SCNA polynucleotide

As indicated above the term "oligonucleotide specific for" or "oligonucleotide targets" refers to an oligonucleotide having a sequence (i) capable of forming a stable complex with a portion of the targeted gene, or (ii) capable of forming a stable duplex with a portion of an mRNA transcript of the targeted gene.

Selection of appropriate oligonucleotides is facilitated by using computer programs (e.g. IDT AntiSense Design, IDT OligoAnalyzer) that automatically identify in each given sequence subsequences of 19-25 nucleotides that will form hybrids with a target polynucleotide sequence with a desired melting temperature (usually 50-60°C) and will not form self-dimers or other complex secondary structures.

Selection of appropriate oligonucleotides is further facilitated by using computer programs that automatically align nucleic acid sequences and indicate regions of identity or homology. Such programs are used to compare nucleic acid sequences obtained, for example, by searching databases such as GenBank or by sequencing PCR products. Comparison of nucleic acid sequences from a range of genes and intergenic regions of a given genome allows the selection of nucleic acid sequences that display an appropriate degree of specificity to the gene of interest These procedures allow the selection of oligonucleotides that exhibit a high degree of complementarity to target nucleic acid sequences and a lower degree of complementarity to other nucleic acid sequences in a given genome. One skilled in the art will realize that there is considerable latitude in selecting appropriate regions of genes for use in the present disclosure.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of in vitro assays.

The hybridization properties of the oligonucleotides described herein can be determined by one or more *in vitro* assays as known in the art. For example, the properties of the oligonucleotides described herein can be obtained by determination of binding strength between the target natural antisense and a potential drug molecules using melting curve assay.

The binding strength between the target natural antisense and a potential drug molecule (Molecule) can be estimated using any of the established methods of measuring the strength of intermolecular interactions, for example, a melting curve assay.

Melting curve assay determines the temperature at which a rapid transition from double-stranded to single-stranded conformation occurs for the natural antisense/Molecule complex. This temperature is widely accepted as a reliable measure of the interaction strength between the two molecules.

A melting curve assay can be performed using a cDNA copy of the actual natural antisense RNA molecule or a synthetic DNA or RNA nucleotide corresponding to the binding site of the Molecule. Multiple kits containing all necessary reagents to perform this assay are available (e.g. Applied Biosystems inc. MeltDoctor kit). These kits include a suitable buffer solution containing one of the double strand DNA (dsDNA) binding dyes (such as ABI HRM dyes, SYBR Green, SYTO, etc.). The properties of the dsDNA dyes are such that they emit almost no fluorescence in free form, but are highly fluorescent when bound to dsDNA,

To perform the assay the cDNA or a corresponding oligonucleotide are mixed with Molecule in concentrations defined by the particular manufacturer's protocols. The mixture is heated to 95 °C to dissociate all preformed dsDNA complexes, then slowly cooled to room temperature or other lower temperature defined by the kit manufacturer to allow the DNA molecules to anneal. The newly formed complexes are then slowly heated to 95 °C with simultaneous continuous collection of data on the amount of fluorescence that is produced by the reaction. The fluorescence intensity is inversely proportional to the amounts of dsDNA present in the reaction. The data can be collected using a real time PCR instrument compatible with the kit (e.g.ABI's StepOne Plus Real Time PCR System or lightTyper instrument, Roche Diagnostics, Lewes, UK),

Melting peaks are constructed by plotting the negative derivative of fluorescence with respect to temperature (-d(Fluorescence)/dT) on the y-axis) against temperature (x-axis) using appropriate software (for example lightTyper (Roche) or SDS Dissociation Curve, ABI). The data is analyzed to identify the temperature of the rapid transition from dsDNA complex to single strand molecules. This temperature is called Tm and is directly proportional to the strength of interaction between the two molecules. Typically, Tm will exceed 40 °C.

### Example 2: Modulation of SCNA polynucleotides

### Treatment of HepG2 cells with antisense oligonucleotides

HepG2 cells from ATCC (cat# HB-8065) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM, Two µl of this solution was incubated with 400 µl of Opti-MEM media (Gibco cat#311985-070) and 4 µl of Lipofectamine 2000 (Invitrogen cat# 11668019) at room temperature for 20 min and applied to each well of the 6 well plates with HepG2 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4168813) as described in the manufacturer's protocoL The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00374696_ml, Hs00897350_ml or Hs00897341_ml for human SCNA) by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples. *Results*: Real Time PCR results show that levels of SCN1A mRNA in HepG2 cells are significantly increased 48h after treatment with antisense oligonucleotides to SCN1A antisense B0724147 (Fig 1,4). Other oligonucleotides designed to SCN1A antisense BG724147 and Hs.662210 did not elevate SCN1A levels. (Fig 2, 3)

### Example 3: Upregulation of SCNA mRNA in different cell lines by treatment with antisense oligonucleotides targeting SCNA-specific natural antisense transcript

In Example 3 antisense oligonucleotides of different chemistries targeting SCN1A-specific natural antisense transcript were screened in a panel of various cell lines at a final concentration of 20 nM. The cell lines used originate from different organs and different animal species. The data below confirms that upregulation of SCN1A mRNA/protein through modulation of the function of the SCN1A-specific natural antisense transcript is not limited to a single oligonucleotide, tissue or species and thus represents a general phenomenon.

### Materials and Methods

*Primary human fibroblasts carrying a Dravet syndrome-associated mutation.* Primary human skin fibroblasts carrying a Dravet syndrome-associated mutation E1099X introduced into culture by Dr. N.Kenyon (University of Miami) were grown in Growth Media consisting of a-MEM (Gibco, cat: 12561-056)+10% FBS (Mediatech, cat: 35-015 CV) + 1% Antimycotic-Antibiotic (Gibco, cat: 15240-062) at 37°C and 5% CO₂. The cells were treated with antisense oligonucleotides using one of the following methods. For the Next Day Method, one day before the experiment the cells were replated at the density of approximately 2x105/well into 6 well plates in Growth Media and incubated at 37°C and 5% CO₂ overnight. Next day, the media in the 6 well plates was changed to fresh Growth Media (1.5 ml/well) and the cells were dosed with antisense oligonucleotides. All antisense oligonucleotides were manufactured by IDT Inc. (Coralville, 1A) or Exiqon (Vedback, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019) at room temperature for 20 min and applied dropwise to one well of a 6 well plate with cells. Similar mixture including 2 µl of water instead of the oligonucleotide solution was used for the mock-transfected controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA. was extracted from the cells using SV Total RNA Isolation System from Promega (cat # Z3105) following the manufacturers' instructions. Six hundred nanograms of purified total RNA was added to the reverse transcription reaction performed using Superscript VILO cDNA Synthesis Kit from Invitrogen (cat# 11754-250) as described in the manufacturer's protocol. The cDNA from this reverse transcription reaction was used to monitor gene expression by real time PCR using ABI Taqman Gene Expression Mix (cat#4369510) and primers/probes designed by ABI (assays Hs00374696_ml, Hs00897350_ml or Hs00897341_ml for human SCN1A). Results obtained using all three assays were very similar. The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat# 4319413E). Fold change in gene expression after treatment with antisense oligonucleotides was calculated based on the difference in 18S-normalized dCt values between treated and mock-transfected samples. For the alternative Same Day Method all procedures were performed similarly, but cells were dosed with antisense oligonucleotides on the first day, immediately after they were distributed into 6-well plates,

*SK-N-AS cell line.* SK-N-AS human neuroblastoma cells from ATCC (cat# CRL-2137) were grown in Growth Media (DMEM (Mediatech cat# 10-013-CV) + 10% FBS (Mediatech cat# MT35-011-CV)+ penicillin/streptomycin (Mediatech cat# MT30-002-CI)+ Non-Essential Amino Acids (NEAA)(MyClone SH30238.01)) at 37°C and 5% CO₂. The cells were treated with antisense oligonucleotides using one of the following methods. For the Next Day Method, one day before the experiment the cells were replated at the density of approximately 3x105/well into 6 well plates in Growth Media and incubated at 37°C and 5% CO₂ overnight. Next day, the media in the 6 well plates was changed to fresh Growth Media (1.5 ml/well) and the cells were dosed with antisense oligonucleotides. All antisense oligonucleotides were manufactured by IDT Inc. (Coralville, IA) or Exiqon (Vedbaek, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019) at room temperature for 20 min and applied dropwise to one well of a 6 well plate with cells. Similar mixture including 2 µl of water instead of the oligonucleotide solution was used for the mock-transfected controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA was extracted from the cells using SV Total RNA Isolation System from Promega (cat # Z3105) following the manufacturers' instructions. Six hundred nanograms of purified total RNAwas added to the reverse transcription reaction performed using SuperScript VILO cDNA Synthesis Kit from Invitrogen (cat#11754-250) as described in the manufacturer's protocol. The cDNA from this reverse transcription reaction was used to monitor gene expression by real time PCR using ABI Taqman Gene Expression Mix (cat#4369510) and primers/probes designed by ABI (assays Hs00374696_ml, Hs00897350_ml or Hs0089734I_m1 for human SCN1A). Results obtained using all three assays were very similar. The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat#4319413E). Fold change in gene expression after treatment with antisense oligonucleotides was calculated based on the difference in 18S-normalized dCt values between treated and mock-transfeeted samples. For the alternative Same Day Method all procedures were performed similarly, but cells were dosed with antisense oligonucleotides on the first day, immediately after they were distributed into 6-well plates.

*CHP-212 cell line.* CHP-212 human neuroblastoma cells from ATCC (cat# CRL-2273) were grown in growth media (1: mixture of MEM and F12 (ATCC cat 30-2003 and Mediatech cat# CV respectively) + 10% FBS (Mediatech cat# MT35-011-CV) + penicillin/streptomycin (Mediated) cat# MT30-002-CI)) at 37°C and 5% CO₂. The cells were treated with antisense oligonucleotides using one of the following methods. For the Next Day Method, one day before the experiment the cells were replated at the density of approximately 2x105/well into 6 well plates in Growth Media and incubated at 37°C and 5% CO₂ overnight. Next day, the media in the 6 well plates was changed to fresh Growth Media (1.5 ml/well) and the cells were dosed with antisense oligonucleotides. All antisense oligonucleotides were manufactured by IDT Inc. (Coralville, 1A) or Exiqon (Vedbaek, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019 at room temperature for 20 min and applied dropwise to one well of a 6 well plate with cells, Similar mixture including 2 µl of water instead of the oligonucleotide solution was used for the mock-transfected controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA was extracted from the cells using SV Total RNA Isolation System from Promega (cat # Z3105) following the manufacturers' instructions. Six hundred nanograms of purified total RNA was added to the reverse transcription reaction performed using SuperScript VILO cDNA Synthesis Kit from Invitrogen (cat#11754-250) as described in the manufacturer's protocol. The cDNA from this reverse transcription reaction was used to monitor gene expression by real time PCR using ABI Taqman Gene Expression Mix (cat#4369510) and primers/probes designed by AB1 (assays Hs00374696_m1, Hs00897350_ml or Hs00897341_ml for human SCNIA). Results obtained using all three assays were very similar. The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat# 4319413E). Fold change in gene expression after treatment with antisense oligonucleotides was calculated based on the difference in 18S-normalized dCt values between treated and mock-transfected samples. For the alternative Same Day Method all procedures were performed similarly, but cells were dosed with antisense oligonucleotides on the first day, immediately after they were distributed into 6-well plates.

*Vero76 cell line.* Vero76 African green monkey embryonic kidney cells from ATCC (cat# CRL-1587) were grown in growth media (Dulbecco's Modified Eagle's Medium (Cellgrow 10-013-CV)+5%, FBS (Mediatech cat# MT35- 011-CV)+ penicillin/streptomycin (Mediatech cat# MT30-002-CI)) at 37°C and 5% CO₂. The cells were treated with antisense oligonucleotides using one of the following methods. For the Next Day Method, one day before the experiment the cells were replated at the density of approximately 105/well into 6 well plates in Growth Media and incubated at 37°C and 5% CO₂ overnight. Next day, the media in the 6 well plates was changed to fresh Growth Media (1.5 ml/well) and the cells were dosed with antisense oligonucleotides. All antisense oligonucleotides were manufactured by IDT Inc. (Coralville, 1A) or Exiqon (Vedbaek, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019) at room temperature for 20 min and applied dropwise to one well of a 6 well plate with cells. Similar mixture including 2 µl of water instead of the oligonucleotide solution was used for the mock-transfected controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA was extracted from the cells using SV Total RNA Isolation System from Promega (cat # Z3105) following the manufacturers' instructions. Six hundred nanograms of purified total RNA was added to the reverse transcription reaction performed using SuperScript VILO cDNA Synthesis Kit from Invitrogen (cat# 11754-250) as described in the manufacturer's protocol. The cDNA from this reverse transcription reaction was used to monitor gene expression by real time PCR using ABI Taqman Gene Expression Mix (cat#4369510) and primers/probes designed by ABI (assays Hs00374696_m1, Hs00897350_m1 or Hs00897341_m1 for human SCNIA). The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat# 4319413E). Fold change in gene expression after treatment with antisense oligonucleotides was calculated based on the difference in 18S-normalized dCt values between treated and mock-transfected samples. For the alternative Same Day Method all procedures were performed similarly, but cells were dosed with antisense oligonucleotides on the first day, immediately after they were distributed into 6-well plates,

*3T3 cell line.* 3T3 mouse embryonic fibroblast cells from ATCC (cat# CRL-1658) were grown in Growth Media (Dulbecco's Modified Eagle's Medium (Cellgrow 10-013-CV) + 10% Fetal Calf Serum (Cellgrow 35-22-CV)+ penicillin/streptomycin (Mediatech catt# MT30-002-CI)) at 37°C and 5% CO₂. The cells were treated with antisense oligonucleotides using one of the following methods. For the Next Day Method, one day before the experiment the cells were replated at the density of approximately 105/well into 6 well plates in Growth Media and incubated at 37°C and 5% CO₂ overnight. Next day, the media in the 6 well plates was changed to fresh. Growth Media (1.5 ml/well) and the cells were dosed with antisense oligonucleotides. All antisense oligonucleotides were manufactured by IDT Inc. (Coralville, 1A) or Exiqon (Vedbaek, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019) at room temperature for 20 min and applied dropwise to one well of a 6 well plate with cells. Similar mixture including 2 µl of water instead of the oligonucleotide solution was used for the mock-transfected controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA was extracted from the cells using SV Total RNA Isolation System from Promega (cat # Z3105) following the manufacturers' instructions. Six hundred nanograms of purified total RNA was added to the reverse transcription reaction performed using SuperScript VILO cDNA Synthesis Kit from Invitrogen (cat#11754-250) as described in the manufacturer's protocol. The cDNA from this reverse transcription reaction was used to monitor gene expression by real time PCR using ABI Taqman Gene Expression Mix (cat#4369510) and primers/probes designed by ABI (assays Hs00374696_m1, Hs00897350_m1 or Hs00897341_ml for human SCNIA). Results obtained using all three assays were very similar. The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat# 4319413E). Fold change in gene expression after treatment with antisense oligonucleotides was calculated based on the difference in 18S-normalized dCt values between treated and mock-transfected samples. For the alternative Same Day Method all procedures were performed similarly, but cells were dosed with antisense oligonucleotides on the first day, immediately after they were distributed into 6-well plates.

*HepG2 cell line.* HepG2 human hepatocellular carcinoma cells from ATCC (cat# HB-8065) were grown in growth media (MEM/EBSS (Hyclone cat #SH30024, or Mediatech cat # MT-10-010-CV)+10% FBS (Mediatech cat# MT35- 011-CV)+ penicillin/streptomycin (Mediatech cat# MT30-002-CI)) at 31°C and 5% CO₂. The cells were treated with antisense oligonucleotides using one of the following methods. For the Next Day Method, one day before the experiment the cells were replated at the density of approximately 3x105/well into 6 well plates in Growth Madia and incubated at 37°C and 5% CO₂ overnight. Next day, the media in the 6 well plates was changed to fresh Growth Media (1.5 ml/well) and the cells were dosed with antisense oligonucleotides. All antisense oligonucleotides were manufactured by IDT Inc. (Coralville, IA) or Exiqon (Vedbaek, Denmark). The sequences for all oligonucleotides are listed in Table 1. Stock solutions of oligonucleotides were diluted to the concentration of 20 µM in DNAse/RNAse-free sterile water. To dose one well, 2 µl of this solution was incubated with 400 µl of Opti-MEM media (Gibco cat#31985-070) and 4 µl of Lipofectamine 2000 (Invitrogen cat# 11668019) at room temperature for 20 min and applied dropwise to one well of a 6 well plate with cells. Similar mixture including 2 µl of water instead of the oligonucleotide solution was used for the mock-transfeeted controls. Additionally an inactive oligonucleotide CUR-1462 at the same concentration was used as control. After about 18 h of incubation at 37°C and 5% CO₂ the media was changed to fresh Growth Media. Forty eight hours after addition of antisense oligonucleotides the media was removed and RNA was extracted from the cells using SV Total RNA Isolation System from Promega (cat # Z3105) following the manufacturers' instructions. Six hundred nanograms of purified total RNA was added to the reverse transcription reaction performed using SuperScript VILO cDNA Synthesis Kit from invitrogen (cat#11754-250) as described in the manufacturer's protocol. The cDNA from this reverse transcription reaction was used to monitor gene expression by real time PCR using ABI Taqman Gene Expression Mix (cat#4369510) and primers/probes designed by ABI (assays Hs00374696_ml, Hs00897350_ml or Hs00897341_ml for human SCN1A). Results obtained using all three assays were very similar. The following PCR cycle was used: 50°C for 2 min, 95°C for 10 min, 40 cycles of (95°C for 15 seconds, 60°C for 1 min) using StepOne Plus Real Time PCR system (Applied Biosystems). The assay for 18S was manufactured by ABI (cat# 4319413E). Fold change in gene expression after treatment with antisense oligonucleotides was calculated based on the difference in 18S-normalized dCt values between treated and mock-transfected samples. For the alternative Same Day Method all procedures were performed similarly, but cells were dosed with antisense oligonucleotides on the first day, immediately after they were distributed into 6-well plates.

*Results*. SCN1A mRNA levels in different cell lines after treatment with 20 nM of antisense oligonucleotides compared to mock-transfected control are shown in Table 2. As seen from the data some of the oligonucleotides when applied at 20 nM were highly active at upregulating the levels of SCN1A mRNA and showed upregulation consistently in several species (human, African green monkey and mouse), in cell lines derived from different organs/cell types (liver, kidney, brain, embryonic fibroblasts) and primary skin fibroblasts carrying the SCN1A mutation. Upregulation of SCN1A protein in cells carrying the Draver mutation supports the suitability of the method for the treatment of diseases associated with mutations in SCN1A gene. Some of the oligonucleotides designed against the natural antisense sequence did not affect or only marginally affected the SCN1A mRNA levels in all, or some, of the cell lines tested. These differences are in agreement with literature data which indicates that binding of oligonucleotides may depend on the secondary and tertiary structures of the oligonuclotide's target sequence. Notably the SCN1A levels in cells treated with an oligonucleotide with no homology to the SCN1A natural antisense sequence but of similar chemistry (CUR-1462) are not significantly different from mock transacted control which confirms that the effects of the targeted oligonucleotides do not depend on the non-specific toxicity of these molecules.

### Example 4: Dose-dependency of SCY4 mRNA upregulation in different cell lines by treatment with antisense oligonucleotides targeting SCNA-specific natural antisense transcript

In Example 4 antisense oligonucleotides of different chemistries targeting SCNA-specific natural antisense transcript were screened in a panel of various cell lines at final concentrations ranging from 5 to 80 nM. The cell lines used originated from different organs and different animal species. The data below confirms that the degree of upregulation of SCNA mRNA through modulation of the function of the SCNA-specific natural antisense transcript can be varied by applying varying amounts of active oligonucleotides.

*Materials and Methods*. SK-N-AS, Vero 76 and primary human fobroblasts canying a Dravet mutation were treated with antisense oligonucleotides as described in Example 2 with the exception of oligonucleotide and Lipofectamine 2000 concentrations used to treat each well The oligonucleotide and Lipofectamine 2000 concentrations were adjusted so as to assure the final oligonucleotide concentrations of 5, 10, 20, 40 and 80 nM and the ratio of Lipofectamine 2000 to 20 µM oligonucleotide stock solution of 2:1 (v:v).

*Results.* The results of dose response experiments have confirmed that the antisense oligonucleotides targeted against SCNIA-specific natural antisense RNA can induce dose-dependent upregulation of SCNIA mRNA (Fig 1-3). In some cases this upregulation was very potent (up to 60-fold) at higher doses (Fig 1-3). The degree of upregulation induced by the same nucleotide in different cell lines appeared to be different, for example upregulation achieved, in primary fibroblasts at 40 nM was at the level of 10-40 fold, while upregulation in Vero 76 cells by the same oligonucleotides at the same concentration was 2-6 fold (Fig.1 vs Fig.3). These differences could be due to different transfection efficiency of different cell lines and/or various feedback pathways expressed by them. The effect of most oligonucleotides reached plateau at about 40 nM, with the exception of CUR-1764 and CUR-1770 in SCN1A fibroblasts and all oligonucleotides tested in Vero 76 cells where the plateau was not reached at the highest concentration tested (Fig. 1-3).

### Example 5: Sequence specificity of the SCNA mRNA upregulation by antisense oligonucleotides targeting SCNA-specific natural antisense transcript

In Example 5 antisense oligonucleotides targeting SCN1A-specific natural antisense transcript were tested in experiments designed to confirm the independence of the SCN1A upregulation caused by the oligonucleotides from the non-specific toxicity associated with the oligonucleotide chemistry used. The data below confirms that the degree of upregulation of SCN1A mRNA through modulation of the function of the SCN1A-specific natural antisense transcript only depends on the amounts of active oligonucleotides, and not on the total amount of molecules of similar chemistry.

*Materials and Methods:* Veto 76 and primary human fibroblasts carrying a Dravet mutation were treated with antisense oligonucleotides as described in Example 2 with the exception of oligonucleotide concentrations used to treat each well. The active oligonucleotide was co-administerd with an inactive oligonucleotide of similar chemistry but with no known target in the human genome (CUR-1462) and no effect on the expression of multiple genes tested (data not shown). The total amount of oligonucleotides as well as the amount of Lipofectamine 2000 were kept constant while the proportion of the active oligonucleotide in the mix was varied. The oligonucleotide concentrations were adjusted so as to ensure the final active oligonucleotide concentrations of 5, 10, 20 and 40 nM and the total oligonucleotide concentration (active+inactive) of 40 nM.

As seen from the data (Fig. 7), the dose-dependent effect of oligonucleotides targeted against SCN1A natural antisense did not result from the non-specific toxicity potentially associated with such molecules. The SCN1A mRNA levels depended on the dose of the active oligonucleotide used to treat them (Fig. 7).

### Example 6: Target specificity of the SCNA mRNA upregulation by antisense oligonucleotides targeting SCNA-specific natural antisense transcript

In Example 6 antisense oligonucleotides targeting SCN1A-specific natural antisense transcript were tested in experiments designed to confirm the specificity of their target, i.e. SCN1A. The data below confirms that the upregulation of SCN1A mRNA through modulation of the function of the SCN1A-specific natural antisense transcript was limited to the SCN1A mRNA and did not affect the related sodium channels SCN9A, SCN8A, SCN7A, SCN3A and SCN2A.

*Materials and Methods*. Vero 76 and primary human fibroblasts carrying a Dravet mutation were treated with antisense oligonucleotides as described in Example 3. Post-treatment the isolated RNA was analyzed as described in Example 2 with the exception that the Taqman gene expression assays used to run the real time PCR detected mRNA for SCN9A, SCN8A, SCN7A, SCN3A and SCN2A channels. The assays for alpha subunits of human SCN9A, SCN8A, SCN7A, SCN3A and SCN2A channels were obtained from AB1 Inc. (cat# Hs00161567_mL, Hs00274075_ml, Hs00161546_ml, Hs00366902_ml, and Hs00221379_ml respectively).

*Results.* As shown in Fig. 8, treatment with oligonucleotides CUR-1916 and CUR-1770 did not significantly affect the expression of SCN8A and SCN9A channels in human fibroblasts carrying Dravet mutation. Expression of SCN7A, SCN3A and SCN2A channels was undetectable in these cells before or after treatment (data not shown). The data confirms the specificity of the gene expression modulation using oligonucleotides directed against the natural antisense RNA for a given gene.

### Example 7: Stability of antisense oligonucleotides targeting SCNA-specific natural antisense transcript

In Example 7 two batches of an antisense oligonucleotide targeting SCN1A-specific natural antisense transcript were tested in experiments designed to check its stability after storage in a dilute (1 mM) aqueous solution at 4°C. The data below shows that the oligonucleotides can be stable in these conditions for periods of at least 6 months without significant loss of activity.

*Materials and Methods.* Vero 76 cells were treated with two different batches of an antisense oligonucleotide as described in Example 2. The batches were synthesized in August 2010 and March 2011, The oligonucleotide synthesized in August 2010 was stored as a 1 mM aqueous solution at 4°C. The oligonucleotide synthesized in March 2011 was shipped in lyophilized form within 3 days after synthesis and tested immediately upon arrival.

*Results:* As shown in Fig. 9 there was no significant loss of biological activity after a 6 month long storage of the oligonucleotides in aqueous solution at 4°C.

### Example 8: SCNA protein upregulation in primary human fibroblasts carrying a Dravet syndrome-associated mutation treated with antisense oligonucleotides targeting SCNA-specific natural antisense transcript

The purpose of this experiment was to rank the antisense oligonucleotides CUR-1740, CUR-1770 and CUR-1916 according to their ability to upregulate the SCNA protein expression in fibroblast cells carrying a Dravet syndrome-associated mutation.

*Materials and Methods.* Fibroblasts carrying a Dravet syndrome-associated mutation introduced into culture by Dr. N.Kenyon (University of Miami) were grown in Growth Media consisting of a-MEM (Gibco, cat: 12561-056)+10% FBS (Mediatech, cat: 35-015 CV) + 1% Antimycotic-Antibiotic (Gibco, cat: 15240-062) at 37°C and 5% CO₂. The cells were treated with antisense oligonucleotides using one of the following methods. For the Next Day Method, one day before the experiment the cells were replated at the density of approximately 4x10⁴/well into 24 well plates in Growth Media and incubated at 37°C and 5% CO₂ overnight. Next day, the media in the 24 well plates was changed to fresh Growth Media (1 ml/well) and the cells were dosed with antisense oligonucleotides CUR-1740, CUR-1770 and CUR-1916, All antisense oligonucleotides were manufactured by IDT Inc. (Coralville, 1A) or Exiqon (Vedbaek, Denmark). The sequences for oligonucleotides CUR-1740, CUR-1770 and CUR-1916 listed in Table 1. Stock solutions of oligonucleotides were diluted to the concentration of 20 µM in DNAse/RNAse-free sterile water. To dose one well at a final concentration of 20 nM, 1 µl of the 20 µM oligonucleotide stock solution was incubated with 200 µl of Opti-MEM media (Gibco cat#31985-070) and 2 µl of Lipofectamine 2000 (Invitrogen ca# 11668019) at room temperature for 20 min and applied dropwise to one well of a 24 well plate with cells. To achieve final concentrations of 5, 10, 40 and 80 nM the volumes of the 20 µM oligonucleotide stock used were adjusted accordingly. The ratio of the 20 µM oligonucleotide stock solution to Lipofectamine 2000 was 1:2 (v:v). Similar mixture including 8 µl of water instead of the oligonucleotide solution and the corresponding volume of Lipofectamine 2000 was used for the mock-transfected controls. After about 18 h of incubation at 37°C and 5% CO₂ the media was changed to fresh Growth Media. Forty eight hours after addition of antisense oligonucleotides the media was removed and cells were washed 3 times with Dulbecco's phosphate-buffered saline without calcium and magnesium (PBS) (Mediatech cat# 21-031-CV). Then PBS was discarded and the cells were fixed in the 24 well plate using 300 µl of 100% methanol for 15 min at ∼20°C. After removing the methanol and washing with PBS, the cells were incubated with 3% hydrogen peroxide (Fisher Chemical cat#H325-100) for 5 min at 21°C. The cells were washed three times for 5 min with PBS, then incubated with 300 µl of bovine serum albumin (BSA) (Sigma cat# A-9647) at 0.1% in PBS for 30 min at 21°C. The cells were washed three times for 5 min with PBS then incubated with 300 µl of avidin solution (Vector Laboratories cat# SP-2001) for 30 min at 21°C. The cells were briefly rinsed three times with PBS then incubated with biotin solution (Vector Laboratories cat# SP-2001) for 30 min at 21°C. The cells were washed three times with PBS and then incubated overnight at 4°C with 300 µl per well of rabbit antibody against human SCN1A (Abcam cat# ab24820) diluted at 1:250 in PBS/BSA 0.1%. After equilibrating the plate for 5 min at 21°C, the cells were washed three times 5 min each with PBS then incubated with goat anti-rabbit antibody diluted 1:200 in PBS/BSA 0.1% for 30 min at 21°C. The cells were washed three times 5 min with PBS and then incubated with 300 µl of Vectastain Elite ABC reagent A+B solution (Vector Laboratories cat# PK-6101) for 30 min; the Vectastain Elite ABC reagent A+B solution was prepared at 21°C 30 min before incubation with the cells by adding and mixing successively 2 drops of reagent A to 5 ml of PBS and then 2 drops of reagent B. The cells were washed 3 times 5 min each with PBS at 21°C and then incubated with Diaminobenzidine (DAB) peroxidase substrate solution (Vector Laboratories cat# SK-4105) until cells are stained; the DAB peroxidase substrate solution is reconstituted before being added to the cells by mixing 1 ml of ImmPACT™DAB Diluent with 30 µl of ImmPACT™ DAB Chromogen concentrate. At this time, the cells are briefly washed three times with PBS and 300 µl of PBS is left in each well. The staining of the cells was analyzed directly inside the wells of the 24-well plate using an inverted Nikon Eclipse TS100 microscope equipped with a Nikon DS-Ri1 camera coupled with Nikon Digital-Sight equipment on the screen of a Dell Latitude D630 laptop. Photos of individual wells were made using the software provided with the Nikon camera, the NIS-Elements D 3.0.

*Results.* All antisense oligonucleotides tested efficiently upregulated SCN1A protein, CUR-1770 and CUR-1916 being the two best (Fig. 10).

### Example 9: SCM protein upregulation in SK-N-AS cells treated with antisense oligonucleotides targeting SCNA-specific natural antisense transcript

The purpose of this experiment was to rank the antisense oligonucleotides CUR-1740, CUR-1764, CUR-1770 and CUR-1916 according to their ability to upregulate the SCN1A protein expression in SK-N-AS cells. SK-N-AS is a human neuroblastoma cell line.

*Materials and Methods*: SK-N-AS human neuroblastoma cells from ATCC (cat# CRL-2137) were grown in Growth Media (DMEM (Mediatech cat# 10-013-CV) +10% FBS (Mediatech cat# MT35-011-CV)+ penicillin/streptomycin (Mediatech cat# MT30-002-CI) + Non-Essential Amino Acids (NEAA)(HyClone SH30238.01)) at 37°C and 5% CO₂. The cells were treated with antisense oligonucleotides using one of the following methods. For the Next Day Method, one day before the experiment the cells were replated at the density of approximately 5x10⁴/well into 24 well plates in Growth Media and incubated at 37°C and 5% CO₂ overnight. Next day, the media in the 24 well plates was changed to fresh Growth Media (1 ml/well) and the cells were dosed with antisense oligonucleotides CUR-1740, CUR-1764, CUR-1770 and CUR-1916. All antisense oligonucleotides were manufactured by IDT Inc. (Coralville, IA) or Exiqon (Vedbaek, Denmark). The sequences for oligonucleotides CUR-1740, CUR-1764, CUR-1770 and CUR-1916 are listed in Table 1. Stock solutions of oligonucleotides were diluted to the concentration of 20 µM in DNAse/RNAse-free sterile water. To dose one well at a final concentration of 20 nM, 1 µl of the 20 µM oligonucleotide stock solution was incubated with µl of Opti-MEM media (Gibco cat#31985-070) and 2 µl of Lipofectamine 2000 (Invitrogen cat# 11668019) at room temperature for 20 min and applied dropwise to one well of a 24 well plate with cells. To achieve final concentrations of 5, 10, 40 and 80 nM the volumes of the 20 µM oligonucleotide stock used were adjusted accordingly. The ratio of the 20 µM oligonucleotide stock solution to Lipofectamine 2000 was 1:2 (v:v). Similar mixture including 8 µl of water instead of the oligonucleotide solution and the corresponding volume of Lipofectamine 2000 was used for the mock-transfected controls. After about 18 h of incubation at 37°C and 5% CO₂ the media was changed to fresh Growth Media. Forty eight hours after addition of antisense oligonucleotides the media was removed and cells were washed 3 times with Dulbecco's phosphate-buffered saline without calcium and magnesium (PBS) (Mediatech cat# 21-031-CV). Then PBS was discarded and the cells were fixed in the 24 well plate using 300 µl of 100% methanol for 15 min at -20°C. After removing the methanol and washing with PBS, the cells were incubated with 3% hydrogen peroxide (Fisher Chemical cat#H325∼100) for 5 min at 21°C. The cells were washed three times for 5 min with PBS then incubated with 300 µl of bovine serum albumin (BSA) (Sigma cat# A-9647) at 0.1% in PBS for 30 min at 21°C. The cells were washed three times for 5 min with PBS then incubated with 300 µl of avidin solution (Vector Laboratories cat# SP-2001) for 30 min at 21°C. The cells were briefly rinsed three times with PBS then incubated with biotin solution (Vector Laboratories cat# SP-2001) for 30 min at 21°C. The cells were washed three times with PBS and then incubated overnight at 4°C with 300 µl per well of rabbit antibody against human SCN1A (Abcam cat# ab24820) diluted at 1:250 in PBS/BSA 0.1%. After equilibrating the plate for 5 min at 21°C, the cells were washed three times for 5 min each with PBS then incubated with goat anti-rabbit antibody diluted 1:200 in PBS/BSA 0.1% for 30 min at 21°C. The cells were washed three times for 5 min with PBS and then incubated with 300 µl of Vectastain Elite ABC reagent A+B solution (Vector Laboratories cat# PK-6101) for 30 min; the Vectastain Elite ABC reagent A+B solution was prepared at 21°C 30 min before incubation with the cells by adding and mixing successively 2 drops of reagent A to 5 ml of PBS and then 2 drops of reagent B. The cells were washed 3 times for 5 min each with PBS at 21°C and then incubated with Diaminobenzidine (DAB) peroxidase substrate solution (Vector Laboratories cat# SK-4105) until cells are stained; the DAB peroxidase substrate solution is reconstituted before being added to the cells by mixing 1ml of ImmPACT™DAB Diluent with 30 µl of ImmPACT™ DAB Chromogen concentrate. At this time, the cells are briefly washed three times with PBS and 300 µl of PBS is left in each well. The staining of the cells was analyzed directly inside the wells of the 24-well plate using an inverted Nikon Eclipse TS100 Microscope equipped with a Nikon DS-RiI camera coupled with Nikon Digital-Sight equipment on the screen of a Dell Latitude D630 laptop. Photos of individual wells were made using the software provided with the Nikon camera, the NIS-Ekments D 3.0.

*Results:* All antisense oligonucleotide tested upregulated SCNIA protein, CUR-1764 and CUR-1770 being the two best (Fig. 11).

### Example 10: SCNA protein upregulation in Vero 76 cells treated with antisense oligonucleotides targeting SCNA-specific natural antisense transcript

The purpose of this experiment was to rank the antisense oligonucleotides CUR-1740, CUR-1770, CUR-1916, CUR-1924 and CUR-1945 according to their ability to upregulate SCNIA protein expression in Vero 76 cells. The Vero76 is a *Cercopithecus aethiops* (vervet or African green monkey) kidney cell line.

*Materials and Methods:* Vero76 African green monkey embryonic kidney cells from ATCC (cat# CRL-1587) were grown in growth media (Dulbecco's Modified Eagle's Medium (Cellgrow 10-013-CV) + 5% FBS (Mediatech cat# MI35-011-CV) penicillin/streptomycin (Mediatech cat# MT30-002-CI)) at 37°C and 5% CO₂. The cells were treated with antisense oligonucleotides using one of the following methods. For the Next Day Method, one day before the experiment the cells were replated at the density of approximately 4x10⁴/well into 24 well plates in Growth Media and incubated at 37°C and 5% CO₂ overnight. Next day, the media in the well plates was changed to fresh Growth Media (1 ml/well) and the cells were dosed with antisense oligonucleotides CUR-1740, CUR-1770 and CUR-1916. All antisense oligonucleotides were manufactured by IDT Inc. (Coralville, IA) or Exiqon (Vedback, Denmark). The sequences for oligonucleotide CUR-1740, CUR-1770, CUR-1916, CUR-1924 and CUR-1945 are listed in Table 1. Stock solutions of oligonucleotides were diluted to the concentration of 20 µM in DNAse/RNAse-free sterile water. To dose one well at a final concentration of 20 NM, 1 µl of the 20 µM oligonucleotide solution was incubated with 200 µl of Opti-MEM media (Gibco cat#31985-070) and 2 µl of Lipofectamine 2000 (Invitrogen cat# 11668019) at room temperature for 20 min and applied dropwise to one well of a 24 well plate with cells. To achieve final concentrations of 5, 10, 40 and 80 nM the volumes of the 20 µM oligonucleotide stock used were adjusted accordingly. The ratio of the 20 µM oligonucleotide stock solution to Lipofectamine 2000 was 1:2 (v:v). Similar mixture including 8 µl of water instead of the oligonucleotide solution and the corresponding volume of Lipofectamine 2000 was used for the mock-transfected controls. After about 18 h of incubation at 37°C and 5% CO₂ the media was changed to fresh Growth Media. Forty eight hours after addition of antisense oligonucleotides the media was removed and cells were washed 3 times with Dulbecco's phosphate-buffered saline without calcium and magnesium (PBS) (Mediatech cat# 21-031-CV). The PBS was discarded and the Vero 76 cells were fixed in the 24 well plate using 300 µl methanol 100% for 15 min at -20°C. After removing the methanol and washing the cells with PBS, the cells were incubated with 3% hydrogen peroxide (Fisher Chemical cat#H325-100) for 5 min at 21°C. The cells were washed three times for 5 min with PBS then incubated with 300 µl of bovine serum albumin (BSA) (Sigma cat# A-9647) at 0.1% in PBS for 30 min at 21°C. The cells were washed three times for 5 min with PBS then incubated with 300 µl of avidin solution (Vector Laboratories cat# SP-2001) for 30 min at 21°C. The cells were briefly rinsed three times with PBS then incubated with biotin solution (Vector Laboratories cat# SP-2001) for 30 min. at 21°C. The cells were washed three times with PBS and then incubated overnight at 4°C with 300 µl per well of rabbit antibody against human SCNIA (Abcam cat# ab24820) diluted at 1:250 in PBS/BSA 0.1%. After equilibrating the plate for 5 min at 21°C, the cells were washed three times 5 min each with PBS then incubated with goat anti-rabbit antibody diluted 1:200 in PBS/BSA 0.1% for 30 min at 21°C. The cells were washed three times 5 min with PBS and then incubated with 300 µl of Vectastain Elite ABC reagent A+B solution (Vector Laboratories cat# PK-6101) for 30 min; the Vectastain Elite ABC reagent A+B solution was prepared at 21°C 30 min before incubation with the cells by adding and mixing successively 2 drops of reagent A to 5 ml of PBS and then 2 drops of reagent B. The cells were washed 3 times 5 min each with PBS at 21°C and then incubated with Diaminobenzidine (DAB) peroxidase substrate solution (Vector Laboratories cat# SK-4105) until cells are stained; the DAB peroxidase substrate solution is reconstituted before being added to the cells by mixing 1ml of ImmPACT™DAB Diluent with 30 µl of ImmPACT™ DAB Chromogen concentrate. At this time, the cells are briefly washed three times with PBS and 300 µl of PBS is left in each well. The staining of the cells was analyzed directly inside the wells of the 24-well plate using an inverted Nikon Eclipse TS100 microscope equipped with a Nikon DS-Ri1 camera coupled with Nikon Digital-Sight equipment on the screen of a Dell Latitude D630 laptop. Photos of individual wells were made using the software provided with the Nikon camera, the NIS-Elements D 3.0.

*Results.* All antisense oligonucleotides tested upregulated SCNIA protein, CUR-1764 and CUR-1770 producing the highest upregulation (Fig 12).

### Example 11: Oligonucleotides targeting SCNA-specific antisense transcript powerful at upregulating SCNA mRNA do not upregalate actin mRNA in Vero76 cells

The purpose of this experiment was to check whether antisense oligonucleotides (CUR-1924, CUR-1740, CUR-1838) targeting SCNIA-specific natural antisense transcript that were shown to upregulate SCNIA mRNA and protein are able to regulate the mRNA of other non-related genes such as actin in Vero76 African green monkey embryonic kidney cells.

*Materials and Methods.* Vero76 African green monkey embryonic kidney cell line from ATCC (cat# CRL-1587) was dosed in the same conditions as described in Example 2. The actin mRNA was quantified by real-time PCR as described in Example 2 except that this time primers/probes designed by ABI were specific for actin (cat# Hs99999903_m1). The data is presented in figure 13.

*Results.* As shown in figure 13, oligonucleotide targeting SCNIA-specific natural antisense transcript (CUR-1924, CUR-1740, CUR-1838) that were shown in Examples 3 and 10 to upregulate SCNIA mRNA and protein in Vero76 cells were tested for their effect on actin mRNA expression in Vero 76 cells. The data in figure 13 confirms that the oligonucleotides targeting SCNIA-specific natural antisense transcript do not upregulate a non related gene such as actin. Thus these oligonucleotides are specific in upregulating SCN1A.

### Example 12: Oligonucleotides targeting SCNA-specific natural antisense transcript shown to upregulate SCNA mRNA and protein do not upregulate actin mRNA in primary fibroblasts carrying a Dravet-associated mutation

The purpose of this experiment was to check whether antisense oligonucleotides (CUR-1916, CUR-1945) targeting SCN1A-specific natural antisense transcript that were shown to upregulate SCNIA mRNA and protein are able to regulate the mRNA of other non-related genes such as actin in primary human skin fibroblasts carrying a Dravet syndrome-associated mutation E1099X.

*Materials and Methods*. Primary human skin fibroblasts carrying a Dravet syndrome-associated mutation E1099X introduced into culture by Dr. N.Kenyon (University of Miami) were dosed in the same conditions as described in Example 3. The actin mRNA was quantified by real-time PCR as described in Example 3 except that this time primers/probes designed by ABI were specific for actin (cat# Hs99999903_m1). The data is presented in figure 14.

*Results:* As shown in figure 14 oligonucleotides targeting SCN1A-specific natural antisense transcript do not upregulate a non-related gene such as actin. Thus these oligonucleotides are specific in upregulating SCN1A.

### Example 13: Oligonucleotides targeting SCNA-specific natural antisense transcript shown to upregulate SCNA mRNA and protein do not upregulate actin mRNA in SK-N-AS cells

The purpose of this experiment was to check whether antisense oligonucleotides (CUR-1740, CUR-1764, CUR-1770, CUR-1838, CUR-1916) targeting SCNIA-specific natural antisense transcript that were shown to upregulate SCN1A mRNA and protein are able to regulate the mRNA of other non-related genes such as actin in SK-N-AS human neuroblastoma cells.

*Materials and Methods.* SK-N-AS human neuroblastoma cells from ATCC (cat# CRL-2137) were dosed in the same conditions as described in Example 2. The actin mRNA was quantified by real-time PCR as described in Example 2 except that this time primers/probes designed by ABI were specific for actin (cat# Hs99999903_ml). The data is presented in Figure 15.

*Results.* As shown in figure 15 oligonucleotides targeting SCN1A-specific natural antisense transcript do not upregulate a non-related gene such as actin, Thus these oligonucleotides are specific in upregulating SCN1A.

### Example 14: Actin protein is not upregulated in SK-N-AS cells treated with antisense oligonucleotides targeting SCNA-specific natural antisense transcript

The purpose of this experiment was to determine whether oligonucleotides targeting SCN1A-specific natural antisense transcript (CUR-1740, CUR-1764, CUR-1770 and CUR-1916) and able to upregulate SCN1A protein are also able to regulate the expression of non-relevant proteins such as actin in SK-N-AS cells. SK-N-AS is a human neuroblastoma cell line.

*Materials and Methods:* SK-N-AS human neuroblastoma cells from ATCC (cat# CRL-2137) were grown in the same conditions as described in Example 9. The cells were fixed and stained exactly in the same conditions as described in Example 8, except that the first antibody was a rabbit anti-actin (Abcam cat#ab1801) used at a dilution of 1:500. The staining of the cells was analyzed directly inside the wells of the 24-well plate using the same process as described in Example 9.

*Results:* As shown in figure 16, none of the antisense oligonucleotides tested upregulated actin protein. Thus, these oligonucleotides are specific at upregulating SCN1A protein.

### Example 15: Actin protein is not upregulated in Vero 76 cells treated with antisense oligonucleotides targeting SCNA-specific natural antisense transcript

The purpose of this experiment was to determine whether specific antisense oligonucleotides targeting SCN1A-speciftc natural antisense transcript (CUR-1740, CUR-1770, CUR-1916, CUR-1924 and CUR-1945) and able to upregulate SCN1A protein are also able to regulate the protein expression of non-relevant genes such as actin in Vero76 cells. The Vero76 is a *Cercopithecus aethiops* (vervet or African green monkey) kidney cell line.

*Materials and Methods*. Vero76 African green monkey embryonic kidney cell line from ATCC (cat# CRL-1587) was grown in the same conditions that described in Example 10. The cells were fixed and stained exactly in the same conditions as described in Example 10, except that the first antibody was a rabbit anti-actin (Abcam cat#ab1801) used at a dilution of 1:500. The staining of the cells was analyzed directly inside the wells of the 24-well plate using the same process as described in Example 10.

*Results*. As shown in figure 17, none of the antisense oligonucleotides tested upregulated actin protein. Thus, these oligonucleotides are specific at upregulating SCN1A protein.

### Example 16: Actin protein is not upregulated in primary human fibroblasts carrying a Dravet syndrome-associated mutation treated with antisense oligonucleotides targeting SCNA-specific natural antisense transcript

The purpose of this experiment was to determine whether oligonucleotides targeting SCN1A-speciftc natural antisense transcript (CUR-1740, CUR-1764, CUR-1770, CUR-1838 and CUR-1916) and able to upregulate SCNA protein are also able to regulate the protein expression of non-relevant genes such as actin in primary human fibroblasts carrying a Dravet syndrome-associated mutation.

*Materials and Methods.* Fibroblasts carrying a Dravet syndrome-associated mutation introduced into culture by Dr. N.Kenyon (University of Miami) were grown in the same conditions as described in Example 8. The cells were fixed and stained exactly in the same conditions as described in Example 8, except that the first antibody was a rabbit anti-actin (Abcam cat#ab1801) used at a dilution of 1:500. The staining of the cells was analyzed directly inside the wells of the 24-well plate using the same process as described in Example 8.

*Results:* As shown in figure 18, none of the antisense oligonucleotides tested upregulated actin protein. Thus, these oligonucleotides are specific at upregulating SCN1A protein.

### Example 17: Quantification of SCNA protein using ELISA in primary human fibrolasts carrying a Dravet syndrome-associated mutation treated with oligonucleotides targeting SCNA-specific natural antisense transcript

The purpose of this experiment was to quantify using ELISA the level of SCN1A protein upregulation due to the treatment with oligonucleotides targeting SCN1A-specific natural antisense transcript (CUR-1740, CUR-1770 and CUR-1916) in primary human fibroblasts carrying a Dravet syndrome-associated imitation.

*Materials and Methods:* Fibroblasts carrying a Dravet syndrome-associated mutation introduced into culture by Dr. N.Kenyon (University of Miami) were grown in the same conditions as described in Example 8 but only 0 and 80nM concentrations of oligonucleotides were used for dosing. The cells were then counted and re-plated in 96 well plates. After 24 hours, the cells were fixed exactly in the same conditions as described in Example 8 and 16 except all 300 µl volumes were reduced to 100µl. Replicate wells were stained with actin and SCN1A antibodies as described in Example 8, except that all reaction were performed in 100 µl volumes. Anti-actin antibody dilution was 1:500, anti-SCN1A dilution was 1:250 and anti-mouse dilution was 1:250. In addition, instead of diaminobenzidine (DAB) peroxidase substrate solution tetrarnethylbenzidine (TMB) peroxidase substrate solution was used (Thermo Scientific cat#N301). After the supernatant turned blue, it was transferred to a new 96 well plate (Greiner bio one cat#65121) and 1 M sulfuric acid was added. The absorbance was read at 450nm using a Muitiskan Spectrum spectrophotometer (Thermo Scientific). The background signal (read in the wells stained with an anti- mouse as primary antibody) was subtracted from all SCN1A and actin readings. Then SCN1A signal was normalized to actin signal for each condition.

*Resulis:* Figure 19 shows that all antisense oligonucleotides tested (CUR-1740, CUR-1770 and CUR-1916) were efficient at upregulating SCN1A protein up to 40%.

### Example 18: Quantification of the SCNA protein using ELISA in Vero 76 cells treated with oligonucleotides targeting SCNA-specific natural antisense transcript

The purpose of this experiment was to quantify using ELISA the level of SCN1A protein upregulation due to the treatment with oligonucleotides targeting SCN1A-specific natural antisense transcript (CUR-1740, CUR-1770, CUR-1916, CUR-1924, CUR-1945) in primary human fibroblasts carrying a Dravet syndrome-associated mutation.

*Materials and Methods:* Vero76 African green monkey embryonic kidney cell were grown in the same conditions that described in Example 10 but only 0 and 80nM concentrations of oligonucleotides were used for dosing. The cells were then counted and re-plated in 96 well plates. After 24 hours, the cells were fixed exactly in the same conditions as described in Example 8 except all 300 µl volumes were reduced to 100µl. Replicate wells were stained with actin and SCN1A antibodies as described in Examples 10 and 15, except that all reactions were performed at 100 µl, the anti-actin antibody dilution was 1:500, anti-SCN1A dilution was 1:250 and anti-mouse dilution was 1:250. In addition, instead of using diaminobenzidine (DAB) peroxidase substrate solution tetramethylbenzidine (TMB) peroxidase substrate solution was used (Thermo Scientific cat#N301). After the supernatant turned blue, it was transferred to a new 96 well plate (Greiner bio one cat#651201) and 1 M sulfuric acid was added. The absorbance was read at 450nm using a Multiskan Spectrum spectrophotometer (Thermo Scientific). The background signal (read in the wells stained with an anti- mouse as primary antibody) was subtracted from all SCN1A and actin readings. Then SCN1A signal was normalized to actin signal for each condition.

*Results:* Figure 20 shows that all of the antisense oligonucleotides tested (CUR-1740, CUR-1770, CUR-1916, CUR-1924, CUR-1945) were efficient at upregulating SCN1A protein up to 300%.

### Example 19: Quantification of the SCNA protein using ELiSA in SK-N-AS cells treated with oligonucleotides targeting SCNA-specific natural antisense transcript

The purpose of this experiment was to quatitfy the level of SCN1A protein upregalation due to the treatment with oligonucleotides targeting SCN1A-specific natural antisense transcript (CUR-1740, CUR-1770, CUR-1924 and CUR-1945) in SK-N-AS cells.

*Materials and Methods:* SK-N-AS human neuroblastoma cells from ATCC (cat# CRL-2137) were grown the same conditions as described in Example 10 but only 0 and 20nM concentrations of oligonucleotides were used for dosing. The cells were then counted and re-plated in 96 well plates. After 24 hours, the cells were fixed exactly in the same conditions as described in Example 9 except all 300 µl volumes were reduced to 100µl. Replicate wells were stained with actin and SCN1A antibodies as described in Examples 9 and 13, except that all reactions were performed at 100 µl, the anti-actin antibody dilution was 1:500, anti-SCNIA dilution was 1:250 and anti-mouse dilution was 1:250. In addition, instead of diaminobenzidine (DAB) peroxidase substrate solution tetramethylbenzidine (TMB) peroxidase substrate solution was used spectrophotometer (Thermo Scientific cat#N301). After the supernatant turned blue, it was transferred to a new 96 well plate (Greiner bio one cat#651201) and 1 M sulfuric acid was added. The absorbance was read at 450nm using a Multiskan Spectrum (Thermo Scientific). The background signal (read in the wells stained with an anti- mouse as primary antibody) was subtracted from all SCN1A and actin readings. Then SCN1A signal was normalized to actin signal for each condition.

*Results:* Figure 21 shows that all antisense oligonucleotides tested (CUR-1740, CUR-1770, CUR-1924 and [00312] *Results :* Figure 21 shows that all antisense oligonucleotides tested (CUR-1740, CUR-1770, CUR-1945) were efficient at upregulating SCN1A protein in SK-N-AS cells up to 500%.

### Example 20: Detection of the natural antisense BG724147 in HepG2 cells and primary human fibroblasts carrying a Dravet syndrome-associated mutation.

The purpose of this experiment was to determine whether the natural antisense BG724147 is present in human hepatocellular carcinoma HepG2 cell line and primary human fibroblasts carrying a Dravet syndrome-associated mutation*.* To achieve this, two different kinds of RNA (poly A RNA and total RNA) isolated from each cell type were used. The PCR products obtained after two successive rounds of PCR using both cell types were analyzed on a gel. Amplification of bands of similar size using BG724147-specific primer confirmed the presence of BG724147 in both cell types.

### Materials and Methods

*Isolation of total RNA*. HepG2 cells or primary human fibroblasts carrying a Dravet syndrome-associated mutation at 80% confluence grown in 75 cm² culture flasks were washed twice with PBS AccuGENE 1X (Lonza Rockeland Inc., Rockeland, ME). After discarding PBS, 5 ml of RLT buffer with b-mercaptoethanol (QIAGEN Inc.-USA, Valencia, CA) were added to these cells and the cell lysate was stored in 1 ml aliquots in microcentrifuge tubes at -80°C until isolation of total RNA. The total RNA isolation from these cells was done using the RNeasy midi kit (QIAGEN Inc.-USA, Valencia, CA) following the manufacturer's protocol. Briefly, the cell lysate was centrifuged at 3000xg for 5 min to clear the lysate and discard any pellet The cleared cell lysate was centrifuged through QIAshredder columns (inside 2 ml microcentrifuge tubes) at 14800xg and the resulting homogenized lysate was mixed with an equal volume of 70% ethanol. The cell lysate mixed with ethanol was applied to RNeasy midi columns (inside a 15 ml conical tube) and centrifuged for 5 min at 3000xg, The column was washed once with 4 ml of RW1 buffer and then subjected to a 15 min on-column DNase digestion with 140 µl of RNase-free DNase in RDD buffer. The DNase digestion was stopped by adding 4 ml of RW1 buffer and centrifuging the column at 3000xg. The column was washed twice with RPE buffer and total RNA binding to the filter was eluted with 150 µl of DNase and RNAse free water. The total RNA was stored at -80°C until the next step.

*Isolation of poly-A RNA* from *total RNA of HepG2 cells.* The isolation of poly A RNA from total RNA of HepG2 cells and primary human fibroblasts carrying a Dravet syndrome-associated mutation was done using a poly-A isolation with magnetic beads kit from Ambion (Applied Biosystems/Ambion, Austin, TX) following the manufacturer protocol. Basically, 100 µg of total RNA was resuspended to a final concentration of 600 µg/ml in DNase/ RNAse free water and an equal volume of 2X binding solution was added. During that time, 10 µl of Oligo(dT) magnetic beads were placed in a microcentrifuge tube, captured by placing this tube on the magnetic stand and the storage buffer was discarded. 50 µl of Wash solution 1 was added to the beads and the tube was removed from the magnetic stand and the wash solution was discarded. At this time, the total RNA from HepG2 cells in 1X Binding buffer was mixed with the magnetic beads and heated at 70 °C for 5 min, then incubated for 60 min at room temperature with gentle agitation. The poly A RNA bound to the magnetic beads was captured by using the magnetic stand for 5 min. The supernatant was discarded. The Oligo(dT) magnetic beads were washed twice with Wash solution 1 and once with Wash solution 2 to remove the non-specifically bound RNA. The magnetic beads were captured with the magnetic stand and 200 µl of warm RNA storage solution (preheated at 70 °C for 5 min) was added to the beads. The magnetic beads were captured by the magnetic stand, the supernatant was stored (first elution of poly A RNA). Then a second 200 µl of warm RNA storage solution (preheated at 70°C for 5 min) was added to the beads. The second elution of polyA RNA was added to the first elution. At this time, the eluted RNA was precipitated using 5 M ammonium acetate, glycogen and 100% ethanol at -20°C overnight. The poly RNA was centrifuged at 14800xg for 30 min at 4°C. The supernatant was discarded and the RNA pellet was washed three times with 1 ml of 70% ethanol, the RNA pellet was recovered each time by centrifuging for 10 min at 4°C. Finally, the poly A. RNA pellet was resuspended in RNA storage solution heated to 70°C to dissolve RNA better. The poly A RNA was stored at -80 °C.

Addition of adenosines to the 3' end of an RNA transcript. Total RNA (40 µg) from HepG2 cells or primary human fibroblasts carrying a Dravet syndrome-associated mutation was mixed with 2 units of RNA Poly (A) Polymerase using a final reaction volume of 100 µl (Ambion, Applied Biosystems, St. Austin TX). The ATP used in the polyadenylation reaction was from Invitrogen. After polyadenylation, the RNA was purified using the phenol/chloroform technique followed by glycogen/sodium acetate precipitation. This RNA was resuspended in 40 µl of DNAse/RNAse free water and was used in a 3'RACE reaction (FirstChoice RLM-RACE kit from Ambion, Applied Biosystems, St. Austin TX).

3' extension of the BG724147 natural antisense transcript of SCN1A. Two different sets of 3' Rapid Amplification of cDNA Ends (RACE) reactions were performed using FirstChoice RLM-RACE kit from Ambion, Applied Biosystems (St. Austin, TX). One set used poly A RNA and the other used total RNA with one adenosine added, from HepG2 cells or primary human fibroblasts carrying a Dravet syndrome-associated mutation. Two consecutive rounds of PCR were performed. The first PCR was done using the 3' outer primer supplied in the kit and a 5' primer specific for BG724147 designed by OPKO CURNA (5' GATTCTCCTACAGCAATTGGTA 3'). The second PCR round was conducted using the 3' outer primer supplied in the kit and a different 5' primer specific for BG724147 designed by OPKO CURNA (5' GACATGTAATCACTTTCATCAA 3'). The products of the second PCR reaction were run on a 1% agarose -1xTAE gel.

*Results:* Figure 22 shows the products from the second round of PCR reactions from a 3' RACE experiments using poly A RNA and total RNA with adenosine added from HepG2 cells and poly A RNA and total RNA with adenosine added from primary human fibroblasts carrying a Dravet syndrome-associated mutation. An identical band is observed in the poly A RNA from HepG2 cells and primary human fibroblasts carrying a Dravet syndrome-associated mutation.

*Conclusion:* PCR amplification using primers specific for the BG724147 natural antisense transcript of SCN1A produced a common PCR band in two different cells (HepG2 cells and primary human fibroblasts carrying a Dravet syndrome-associated mutation). In addition, antisense oligonucleotides targeting the SCN1A natural antisense BG724147 have been shown to upregulate SCN1A mRNA and protein in these cells as shown in Examples 2, 7 and 16. This data indicates that the BG724147 is indeed present in these two kinds of cells (HepG2 cells and primary human fibroblasts carrying a Dravet syndrome-associated mutation).

### Example 21: Extension of the SCNA natural antisense sequence BG724147

The purpose of this experiment is to extend the known sequence of the SCN1A natural antisense BG724147 by sequencing all its sequence. The original BG724147 RNA transcript was obtained from human testis procured by Miklos Palkovits. The cDNA library was prepared in a pBluescriptR vector by Michael J. Brownstein (at NHGRI), Shiraki Toshiyuki and Piero Carninci (at RIKEN). The cDNA library was arrayed by the I.M.A.G.E Consortium, (or LLNL) and the clones were sequenced by Incyte Genomics, Inc. in May 2001. The BG724147 clone is available at Open Biosystems (Open Biosystems Products, Huntsville, AL). In 2001 the cDNA insert in the BG724147 clone was not sequenced completely. OPKO-CURNA obtained the BG724147 clone and sequenced the full insert. To achieve this, a bacterial clone containing a plasmid with the BG724147 insert was acquired from Open Biosystems and plated in a Luria Bertani (LB)-agar plate with ampicillin to isolate individual colonies. Then colonies were amplified in 5 ml of LB broth. The plasmid containing the BG724147 insert was then isolated from these bacteria and sent for sequencing to Davis Sequencing (Davis, CA).

### Material and Methods:

*Isolation and sequencing of the plasmid containing the cDNA for the SCNA natural antisense BG724147.* Suspension of frozen bacteria containing the BG724147 plasmid was purchased from Open Biosystems (Open Biosystems Products, cat# 4829512), diluted 1:10, 1:100, 1:1000, 1:10000, 1:100000 times, then plated on Luria Bertani (LB) (BD, cat# 244520)-agar plate (Falcon, cat#351005) with 100 µg/ml of ampicillin (Calbiochem, cat#171254). After 15h, 20 individual colonies of bacteria were isolated from the plate with the 1:100000 dilution and grown separately in 5 ml of LB broth (Fisher Scientific, cat# BP 1426-2) for 15h-24h. At this time, the bacteria were pelleted and the plasmid (containing the cDNA from the BG724147 RNA transcript) was isolated using the PureYieldTM Plasmid Miniprep System kit from Promega (Promega, cat#A1222) following the manufacturer's protocol. The isolated DNA was diluted to 200 ng/ml and 12 µl of plasmid from each colony was sent for sequencing to Davis sequencing (Davis, CA).

*Results:* The sequences obtained from Davis sequencing provide BG724147 extended (SEQ ID NO: 12)

*Conclusion:* The successful extension of the known BG724147 sequence by 403 nucleotides served as a basis to design antisense oligonucleotides against the SCN1A natural antisense transcript BG724147.

In addition, while a particular feature of the invention may have been disclosed with respect to only one of several implementations, such feature may be combined with one or more other features of the other implementations as may be desired and advantageous for any given or particular application.

The Abstract of the disclosure will allow the reader to quickly ascertain the nature of the technical disclosure. It is submitted with the understanding that it will not be used to interpret or limit the scope or meaning of the following claims.

### SEQUENCE LISTING

<110> OPKO CURNA LLC
<120> TREATMENT OF SODIUM CHANNEL, VOLTAGE-GATED, TYPE I, ALPHA SUBUNIT (SCN1A) RELATED DISEASES BY INHIBITION OF NATURAL ANTISENSE TRANSCRIPT TO SCN1A
<130> SCN1A
<150> US61/357,774
   <151> 2010-06-23
<160> 96
<170> Patent In version 3.5
<210> 1
   <211> 8133
   <212> DNA
   <213> Homo sapiens
<400> 1 
aatgtgcagg atgacaagat ggagcaaaca gtgcttgtac caccaggacc tgacagcttc 60

aacttcttca ccagagaatc tcttgcggct attgaaagac gcattgcaga agaaaaggca 120

aagaatccca aaccagacaa aaaagatgac gacgaaaatg gcccaaagcc aaatagtgac 180

ttggaagctg gaaagaacct tccatttatt tatggagaca ttcctccaga gatggtgtca 240

gagcccctgg aggacctgga cccctactat atcaataaga aaacttttat agtattgaat 300

aaagggaagg ccatcttccg gttcagtgcc acctctgccc tgtacatttt aactcccttc 360

aatcctctta ggaaaatagc tattaagatt ttggtacatt cattattcag catgctaatt 420

atgtgcacta ttttgacaaa ctgtgtgttt atgacaatga gtaaccctcc tgattggaca 480

aagaatgtag aatacacctt cacaggaata tatacttttg aatcacttat aaaaattatt 540

gcaaggggat tctgtttaga agattttact ttccttcggg atccatggaa ctggctcgat 600

ttcactgtca ttacatttgc gtacgtcaca gagtttgtgg acctgggcaa tgtctcggca 660

ttgagaacat tcagagttct ccgagcattg aagacgattt cagtcattcc aggcctgaaa 720

accattgtgg gagccctgat ccagtctgtg aagaagctct cagatgtaat gatcctgact 780

gtgttctgtc tgagcgtatt tgctctaatt gggctgcagc tgttcatggg caacctgagg 840

aataaatgta tacaatggcc tcccaccaat gcttccttgg aggaacatag tatagaaaag 900

aatataactg tgaattataa tggtacactt ataaatgaaa ctgtctttga gtttgactgg 960

aagtcatata ttcaagattc aagatatcat tatttcctgg agggtttttt agatgcacta 1020

ctatgtggaa atagctctga tgcaggccaa tgtccagagg gatatatgtg tgtgaaagct 1080

ggtagaaatc ccaattatgg ctacacaagc tttgatacct tcagttgggc ttttttgtcc 1140

ttgtttcgac taatgactca ggacttctgg gaaaatcttt atcaactgac attacgtgct 1200

gctgggaaaa cgtacatgat attttttgta ttggtcattt tcttgggctc attctaccta 1260
 
ataaatttga tcctggctgt ggtggccatg gcctacgagg aacagaatca ggccaccttg 1320

gaagaagcag aacagaaaga ggccgaattt cagcagatga ttgaacagct taaaaagcaa 1380

caggaggcag ctcagcaggc agcaacggca actgcctcag aacattccag agagcccagt 1440

gcagcaggca ggctctcaga cagctcatct gaagcctcta agttgagttc caagagtgct 1500

aaggaaagaa gaaatcggag gaagaaaaga aaacagaaag agcagtctgg tggggaagag 1560

aaagatgagg atgaattcca aaaatctgaa tctgaggaca gcatcaggag gaaaggtttt 1620

cgcttctcca ttgaagggaa ccgattgaca tatgaaaaga ggtactcctc cccacaccag 1680

tctttgttga gcatccgtgg ctccctattt tcaccaaggc gaaatagcag aacaagcctt 1740

ttcagcttta gagggcgagc aaaggatgtg ggatctgaga acgacttcgc agatgatgag 1800

cacagcacct ttgaggataa cgagagccgt agagattcct tgtttgtgcc ccgacgacac 1860

ggagagagac gcaacagcaa cctgagtcag accagtaggt catcccggat gctggcagtg 1920

tttccagcga atgggaagat gcacagcact gtggattgca atggtgtggt ttccttggtt 1980

ggtggacctt cagttcctac atcgcctgtt ggacagcttc tgccagaggt gataatagat 2040

aagccagcta ctgatgacaa tggaacaacc actgaaactg aaatgagaaa gagaaggtca 2100

agttctttcc acgtttccat ggactttcta gaagatcctt cccaaaggca acgagcaatg 2160

agtatagcca gcattctaac aaatacagta gaagaacttg aagaatccag gcagaaatgc 2220

ccaccctgtt ggtataaatt ttccaacata ttcttaatct gggactgttc tccatattgg 2280

ttaaaagtga aacatgttgt caacctggtt gtgatggacc catttgttga cctggccatc 2340

accatctgta ttgtcttaaa tactcttttc atggccatgg agcactatcc aatgacggac 2400

catttcaata atgtgcttac agtaggaaac ttggttttca ctgggatctt tacagcagaa 2460

atgtttctga aaattattgc catggatcct tactattatt tccaagaagg ctggaatatc 2520

tttgacggtt ttattgtgac gcttagcctg gtagaacttg gactcgccaa tgtggaagga 2580

ttatctgttc tccgttcatt tcgattgctg cgagttttca agttggcaaa atcttggcca 2640

acgttaaata tgctaataaa gatcatcggc aattccgtgg gggctctggg aaatttaacc 2700

ctcgtcttgg ccatcatcgt cttcattttt gccgtggtcg gcatgcagct ctttggtaaa 2760

agctacaaag attgtgtctg caagatcgcc agtgattgtc aactcccacg ctggcacatg 2820

aatgacttct tccactcctt cctgattgtg ttccgcgtgc tgtgtgggga gtggatagag 2880

accatgtggg actgtatgga ggttgctggt caagccatgt gccttactgt cttcatgatg 2940

gtcatggtga ttggaaacct agtggtcctg aatctctttc tggccttgct tctgagctca 3000

tttagtgcag acaaccttgc agccactgat gatgataatg aaatgaataa tctccaaatt 3060

gctgtggata ggatgcacaa aggagtagct tatgtgaaaa gaaaaatata tgaatttatt 3120

caacagtcct tcattaggaa acaaaagatt ttagatgaaa ttaaaccact tgatgatcta 3180

aacaacaaga aagacagttg tatgtccaat catacagcag aaattgggaa agatcttgac 3240
 
tatcttaaag atgtaaatgg aactacaagt ggtataggaa ctggcagcag tgttgaaaaa 3300

tacattattg atgaaagtga ttacatgtca ttcataaaca accccagtct tactgtgact 3360

gtaccaattg ctgtaggaga atctgacttt gaaaatttaa acacggaaga ctttagtagt 3420

gaatcggatc tggaagaaag caaagagaaa ctgaatgaaa gcagtagctc atcagaaggt 3480

agcactgtgg acatcggcgc acctgtagaa gaacagcccg tagtggaacc tgaagaaact 3540

cttgaaccag aagcttgttt cactgaaggc tgtgtacaaa gattcaagtg ttgtcaaatc 3600

aatgtggaag aaggcagagg aaaacaatgg tggaacctga gaaggacgtg tttccgaata 3660

gttgaacata actggtttga gaccttcatt gttttcatga ttctccttag tagtggtgct 3720

ctggcatttg aagatatata tattgatcag cgaaagacga ttaagacgat gttggaatat 3780

gctgacaagg ttttcactta cattttcatt ctggaaatgc ttctaaaatg ggtggcatat 3840

ggctatcaaa catatttcac caatgcctgg tgttggctgg acttcttaat tgttgatgtt 3900

tcattggtca gtttaacagc aaatgccttg ggttactcag aacttggagc catcaaatct 3960

ctcaggacac taagagctct gagacctcta agagccttat ctcgatttga agggatgagg 4020

gtggttgtga atgccctttt aggagcaatt ccatccatca tgaatgtgct tctggtttgt 4080

cttatattct ggctaatttt cagcatcatg ggcgtaaatt tgtttgctgg caaattctac 4140

cactgtatta acaccacaac tggtgacagg tttgacatcg aagacgtgaa taatcatact 4200

gattgcctaa aactaataga aagaaatgag actgctcgat ggaaaaatgt gaaagtaaac 4260

tttgataatg taggatttgg gtatctctct ttgcttcaag ttgccacatt caaaggatgg 4320

atggatataa tgtatgcagc agttgattcc agaaatgtgg aactccagcc taagtatgaa 4380

gaaagtctgt acatgtatct ttactttgtt attttcatca tctttgggtc cttcttcacc 4440

ttgaacctgt ttattggtgt catcatagat aatttcaacc agcagaaaaa gaagtttgga 4500

ggtcaagaca tctttatgac agaagaacag aagaaatact ataatgcaat gaaaaaatta 4560

ggatcgaaaa aaccgcaaaa gcctatacct cgaccaggaa acaaatttca aggaatggtc 4620

tttgacttcg taaccagaca agtttttgac ataagcatca tgattctcat ctgtcttaac 4680

atggtcacaa tgatggtgga aacagatgac cagagtgaat atgtgactac cattttgtca 4740

cgcatcaatc tggtgttcat tgtgctattt actggagagt gtgtactgaa actcatctct 4800

ctacgccatt attattttac cattggatgg aatatttttg attttgtggt tgtcattctc 4860

tccattgtag gtatgtttct tgccgagctg atagaaaagt atttcgtgtc ccctaccctg 4920

ttccgagtga tccgtcttgc taggattggc cgaatcctac gtctgatcaa aggagcaaag 4980

gggatccgca cgctgctctt tgctttgatg atgtcccttc ctgcgttgtt taacatcggc 5040

ctcctactct tcctagtcat gttcatctac gccatctttg ggatgtccaa ctttgcctat 5100

gttaagaggg aagttgggat cgatgacatg ttcaactttg agacctttgg caacagcatg 5160
 
atctgcctat tccaaattac aacctctgct ggctgggatg gattgctagc acccattctc 5220

aacagtaagc cacccgactg tgaccctaat aaagttaacc ctggaagctc agttaaggga 5280

gactgtggga acccatctgt tggaattttc ttttttgtca gttacatcat catatccttc 5340

ctggttgtgg tgaacatgta catcgcggtc atcctggaga acttcagtgt tgctactgaa 5400

gaaagtgcag agcctctgag tgaggatgac tttgagatgt tctatgaggt ttgggagaag 5460

tttgatcccg atgcaactca gttcatggaa tttgaaaaat tatctcagtt tgcagctgcg 5520

cttgaaccgc ctctcaatct gccacaacca aacaaactcc agctcattgc catggatttg 5580

cccatggtga gtggtgaccg gatccactgt cttgatatct tatttgcttt tacaaagcgg 5640

gttctaggag agagtggaga gatggatgct ctacgaatac agatggaaga gcgattcatg 5700

gcttccaatc cttccaaggt ctcctatcag ccaatcacta ctactttaaa acgaaaacaa 5760

gaggaagtat ctgctgtcat tattcagcgt gcttacagac gccacctttt aaagcgaact 5820

gtaaaacaag cttcctttac gtacaataaa aacaaaatca aaggtggggc taatcttctt 5880

ataaaagaag acatgataat tgacagaata aatgaaaact ctattacaga aaaaactgat 5940

ctgaccatgt ccactgcagc ttgtccacct tcctatgacc gggtgacaaa gccaattgtg 6000

gaaaaacatg agcaagaagg caaagatgaa aaagccaaag ggaaataaat gaaaataaat 6060

aaaaataatt gggtgacaaa ttgtttacag cctgtgaagg tgatgtattt ttatcaacag 6120

gactccttta ggaggtcaat gccaaactga ctgtttttac acaaatctcc ttaaggtcag 6180

tgcctacaat aagacagtga ccccttgtca gcaaactgtg actctgtgta aaggggagat 6240

gaccttgaca ggaggttact gttctcacta ccagctgaca ctgctgaaga taagatgcac 6300

aatggctagt cagactgtag ggaccagttt caaggggtgc aaacctgtga ttttggggtt 6360

gtttaacatg aaacacttta gtgtagtaat tgtatccact gtttgcattt caactgccac 6420

atttgtcaca tttttatgga atctgttagt ggattcatct ttttgttaat ccatgtgttt 6480

attatatgtg actatttttg taaacgaagt ttctgttgag aaataggcta aggacctcta 6540

taacaggtat gccacctggg gggtatggca accacatggc cctcccagct acacaaagtc 6600

gtggtttgca tgagggcatg ctgcacttag agatcatgca tgagaaaaag tcacaagaaa 6660

aacaaattct taaatttcac catatttctg ggaggggtaa ttgggtgata agtggaggtg 6720

ctttgttgat cttgttttgc gaaatccagc ccctagacca agtagattat ttgtgggtag 6780

gccagtaaat cttagcaggt gcaaacttca ttcaaatgtt tggagtcata aatgttatgt 6840

ttctttttgt tgtattaaaa aaaaaacctg aatagtgaat attgcccctc accctccacc 6900

gccagaagac tgaattgacc aaaattactc tttataaatt tctgcttttt cctgcacttt 6960

gtttagccat cttcggctct cagcaaggtt gacactgtat atgttaatga aatgctattt 7020

attatgtaaa tagtcatttt accctgtggt gcacgtttga gcaaacaaat aatgacctaa 7080

gcacagtatt tattgcatca aatatgtacc acaagaaatg tagagtgcaa gctttacaca 7140
 
ggtaataaaa tgtattctgt accatttata gatagtttgg atgctatcaa tgcatgttta 7200

tattaccatg ctgctgtatc tggtttctct cactgctcag aatctcattt atgagaaacc 7260

atatgtcagt ggtaaagtca aggaaattgt tcaacagatc tcatttattt aagtcattaa 7320

gcaatagttt gcagcacttt aacagctttt tggttatttt tacattttaa gtggataaca 7380

tatggtatat agccagactg tacagacatg tttaaaaaaa cacactgctt aacctattaa 7440

atatgtgttt agaattttat aagcaaatat aaatactgta aaaagtcact ttattttatt 7500

tttcagcatt atgtacataa atatgaagag gaaattatct tcaggttgat atcacaatca 7560

cttttcttac tttctgtcca tagtactttt tcatgaaaga aatttgctaa ataagacatg 7620

aaaacaagac tgggtagttg tagatttctg ctttttaaat tacatttgct aattttagat 7680

tatttcacaa ttttaaggag caaaataggt tcacgattca tatccaaatt atgctttgca 7740

attggaaaag ggtttaaaat tttatttata tttctggtag tacctgcact aactgaattg 7800

aaggtagtgc ttatgttatt tttgttcttt ttttctgact tcggtttatg ttttcatttc 7860

tttggagtaa tgctgctcta gattgttcta aatagaatgt gggcttcata attttttttt 7920

ccacaaaaac agagtagtca acttatatag tcaattacat caggacattt tgtgtttctt 7980

acagaagcaa accataggct cctcttttcc ttaaaactac ttagataaac tgtattcgtg 8040

aactgcatgc tggaaaatgc tactattatg ctaaataatg ctaaccaaca tttaaaatgt 8100

gcaaaactaa taaagattac atttttattt tta 8133
<210> 2
   <211> 8876
   <212> DNA
   <213> Homo sapiens
<400> 2 
ggctgcttca gacatatgtc tgtgtgtacg ctgtgaaggt gtttctcttc acagttcccc 60

gccctctagt ggtagttaca ataatgccat tttgtagtcc ctgtacagga aatgcctctt 120

cttacttcag ttaccagaat ccttttacag gaagttaggt gtggtctttg aaggagaatt 180

aaaaaaaaaa aaaaaaaaaa aaaaaaaaga tttttttttt tttaaagcat gatggaattt 240

tagctgcagt cttcttggtg ccagcttatc aatcccaaac tctgggtgta aaagattcta 300

cagggcactt tcttatgcaa ggagctaaac agtgattaaa ggagcaggat gaaaagatgg 360

cacagtcagt gctggtaccg ccaggacctg acagcttccg cttctttacc agggaatccc 420

ttgctgctat tgaacaacgc attgcagaag agaaagctaa gagacccaaa caggaacgca 480

aggatgagga tgatgaaaat ggcccaaagc caaacagtga cttggaagca ggaaaatctc 540

ttccatttat ttatggagac attcctccag agatggtgtc agtgcccctg gaggatctgg 600

acccctacta tatcaataag aaaacgttta tagtattgaa taaaggaaaa gcaatctctc 660

gattcagtgc cacccctgcc ctttacattt taactccctt caaccctatt agaaaattag 720
 
ctattaagat tttggtacat tctttattca atatgctcat tatgtgcacg attcttacca 780
actgtgtatt tatgaccatg agtaaccctc cagactggac aaagaatgtg gagtatacct 840
ttacaggaat ttatactttt gaatcactta ttaaaatact tgcaaggggc ttttgtttag 900
aagatttcac atttttacgg gatccatgga attggttgga tttcacagtc attacttttg 960
catatgtgac agagtttgtg gacctgggca atgtctcagc gttgagaaca ttcagagttc 1020
tccgagcatt gaaaacaatt tcagtcattc caggcctgaa gaccattgtg ggggccctga 1080
tccagtcagt gaagaagctt tctgatgtca tgatcttgac tgtgttctgt ctaagcgtgt 1140
ttgcgctaat aggattgcag ttgttcatgg gcaacctacg aaataaatgt ttgcaatggc 1200
ctccagataa ttcttccttt gaaataaata tcacttcctt ctttaacaat tcattggatg 1260
ggaatggtac tactttcaat aggacagtga gcatatttaa ctgggatgaa tatattgagg 1320
ataaaagtca cttttatttt ttagaggggc aaaatgatgc tctgctttgt ggcaacagct 1380
cagatgcagg ccagtgtcct gaaggataca tctgtgtgaa ggctggtaga aaccccaact 1440
atggctacac gagctttgac acctttagtt gggccttttt gtccttattt cgtctcatga 1500
ctcaagactt ctgggaaaac ctttatcaac tgacactacg tgctgctggg aaaacgtaca 1560
tgatattttt tgtgctggtc attttcttgg gctcattcta tctaataaat ttgatcttgg 1620
ctgtggtggc catggcctat gaggaacaga atcaggccac attggaagag gctgaacaga 1680
aggaagctga atttcagcag atgctcgaac agttgaaaaa gcaacaagaa gaagctcagg 1740
cggcagctgc agccgcatct gctgaatcaa gagacttcag tggtgctggt gggataggag 1800
ttttttcaga gagttcttca gtagcatcta agttgagctc caaaagtgaa aaagagctga 1860
aaaacagaag aaagaaaaag aaacagaaag aacagtctgg agaagaagag aaaaatgaca 1920
gagtccgaaa atcggaatct gaagacagca taagaagaaa aggtttccgt ttttccttgg 1980
aaggaagtag gctgacatat gaaaagagat tttcttctcc acaccagtcc ttactgagca 2040
tccgtggctc ccttttctct ccaagacgca acagtagggc gagccttttc agcttcagag 2100
gtcgagcaaa ggacattggc tctgagaatg actttgctga tgatgagcac agcacctttg 2160
aggacaatga cagccgaaga gactctctgt tcgtgccgca cagacatgga gaacggcgcc 2220
acagcaatgt cagccaggcc agccgtgcct ccagggtgct ccccatcctg cccatgaatg 2280
ggaagatgca tagcgctgtg gactgcaatg gtgtggtctc cctggtcggg ggcccttcta 2340
ccctcacatc tgctgggcag ctcctaccag agggcacaac tactgaaaca gaaataagaa 2400
agagacggtc cagttcttat catgtttcca tggatttatt ggaagatcct acatcaaggc 2460
aaagagcaat gagtatagcc agtattttga ccaacaccat ggaagaactt gaagaatcca 2520
gacagaaatg cccaccatgc tggtataaat ttgctaatat gtgtttgatt tgggactgtt 2580
gtaaaccatg gttaaaggtg aaacaccttg tcaacctggt tgtaatggac ccatttgttg 2640
 
acctggccat caccatctgc attgtcttaa atacactctt catggctatg gagcactatc 2700

ccatgacgga gcagttcagc agtgtactgt ctgttggaaa cctggtcttc acagggatct 2760

tcacagcaga aatgtttctc aagataattg ccatggatcc atattattac tttcaagaag 2820

gctggaatat ttttgatggt tttattgtga gccttagttt aatggaactt ggtttggcaa 2880

atgtggaagg attgtcagtt ctccgatcat tccggctgct ccgagttttc aagttggcaa 2940

aatcttggcc aactctaaat atgctaatta agatcattgg caattctgtg ggggctctag 3000

gaaacctcac cttggtattg gccatcatcg tcttcatttt tgctgtggtc ggcatgcagc 3060

tctttggtaa gagctacaaa gaatgtgtct gcaagatttc caatgattgt gaactcccac 3120

gctggcacat gcatgacttt ttccactcct tcctgatcgt gttccgcgtg ctgtgtggag 3180

agtggataga gaccatgtgg gactgtatgg aggtcgctgg ccaaaccatg tgccttactg 3240

tcttcatgat ggtcatggtg attggaaatc tagtggttct gaacctcttc ttggccttgc 3300

ttttgagttc cttcagttct gacaatcttg ctgccactga tgatgataac gaaatgaata 3360

atctccagat tgctgtggga aggatgcaga aaggaatcga ttttgttaaa agaaaaatac 3420

gtgaatttat tcagaaagcc tttgttagga agcagaaagc tttagatgaa attaaaccgc 3480

ttgaagatct aaataataaa aaagacagct gtatttccaa ccataccacc atagaaatag 3540

gcaaagacct caattatctc aaagacggaa atggaactac tagtggcata ggcagcagtg 3600

tagaaaaata tgtcgtggat gaaagtgatt acatgtcatt tataaacaac cctagcctca 3660

ctgtgacagt accaattgct gttggagaat ctgactttga aaatttaaat actgaagaat 3720

tcagcagcga gtcagatatg gaggaaagca aagagaagct aaatgcaact agttcatctg 3780

aaggcagcac ggttgatatt ggagctcccg ccgagggaga acagcctgag gttgaacctg 3840

aggaatccct tgaacctgaa gcctgtttta cagaagactg tgtacggaag ttcaagtgtt 3900

gtcagataag catagaagaa ggcaaaggga aactctggtg gaatttgagg aaaacatgct 3960

ataagatagt ggagcacaat tggttcgaaa ccttcattgt cttcatgatt ctgctgagca 4020

gtggggctct ggcctttgaa gatatataca ttgagcagcg aaaaaccatt aagaccatgt 4080

tagaatatgc tgacaaggtt ttcacttaca tattcattct ggaaatgctg ctaaagtggg 4140

ttgcatatgg ttttcaagtg tattttacca atgcctggtg ctggctagac ttcctgattg 4200

ttgatgtctc actggttagc ttaactgcaa atgccttggg ttactcagaa cttggtgcca 4260

tcaaatccct cagaacacta agagctctga ggccactgag agctttgtcc cggtttgaag 4320

gaatgagggt tgttgtaaat gctcttttag gagccattcc atctatcatg aatgtacttc 4380

tggtttgtct gatcttttgg ctaatattca gtatcatggg agtgaatctc tttgctggca 4440

agttttacca ttgtattaat tacaccactg gagagatgtt tgatgtaagc gtggtcaaca 4500

actacagtga gtgcaaagct ctcattgaga gcaatcaaac tgccaggtgg aaaaatgtga 4560

aagtaaactt tgataacgta ggacttggat atctgtctct acttcaagta gccacgttta 4620
 
agggatggat ggatattatg tatgcagctg ttgattcacg aaatgtagaa ttacaaccca 4680
agtatgaaga caacctgtac atgtatcttt attttgtcat ctttattatt tttggttcat 4740
tctttacctt gaatcttttc attggtgtca tcatagataa cttcaaccaa cagaaaaaga 4800
agtttggagg tcaagacatt tttatgacag aagaacagaa gaaatactac aatgcaatga 4860
aaaaactggg ttcaaagaaa ccacaaaaac ccatacctcg acctgctaac aaattccaag 4920
gaatggtctt tgattttgta accaaacaag tctttgatat cagcatcatg atcctcatct 4980
gccttaacat ggtcaccatg atggtggaaa ccgatgacca gagtcaagaa atgacaaaca 5040
ttctgtactg gattaatctg gtgtttattg ttctgttcac tggagaatgt gtgctgaaac 5100
tgatctctct tcgttactac tatttcacta ttggatggaa tatttttgat tttgtggtgg 5160
tcattctctc cattgtagga atgtttctgg ctgaactgat agaaaagtat tttgtgtccc 5220
ctaccctgtt ccgagtgatc cgtcttgcca ggattggccg aatcctacgt ctgatcaaag 5280
gagcaaaggg gatccgcacg ctgctctttg ctttgatgat gtcccttcct gcgttgttta 5340
acatcggcct ccttcttttc ctggtcatgt tcatctacgc catctttggg atgtccaatt 5400
ttgcctatgt taagagggaa gttgggatcg atgacatgtt caactttgag acctttggca 5460
acagcatgat ctgcctgttc caaattacaa cctctgctgg ctgggatgga ttgctagcac 5520
ctattcttaa tagtggacct ccagactgtg accctgacaa agatcaccct ggaagctcag 5580
ttaaaggaga ctgtgggaac ccatctgttg ggattttctt ttttgtcagt tacatcatca 5640
tatccttcct ggttgtggtg aacatgtaca tcgcggtcat cctggagaac ttcagtgttg 5700
ctactgaaga aagtgcagag cctctgagtg aggatgactt tgagatgttc tatgaggttt 5760
gggagaagtt tgatcccgat gcgacccagt ttatagagtt tgccaaactt tctgattttg 5820
cagatgccct ggatcctcct cttctcatag caaaacccaa caaagtccag ctcattgcca 5880
tggatctgcc catggtgagt ggtgaccgga tccactgtct tgacatctta tttgctttta 5940
caaagcgtgt tttgggtgag agtggagaga tggatgccct tcgaatacag atggaagagc 6000
gattcatggc atcaaacccc tccaaagtct cttatgagcc cattacgacc acgttgaaac 6060
gcaaacaaga ggaggtgtct gctattatta tccagagggc ttacagacgc tacctcttga 6120
agcaaaaagt taaaaaggta tcaagtatat acaagaaaga caaaggcaaa gaatgtgatg 6180
gaacacccat caaagaagat actctcattg ataaactgaa tgagaattca actccagaga 6240
aaaccgatat gacgccttcc accacgtctc caccctcgta tgatagtgtg accaaaccag 6300
aaaaagaaaa atttgaaaaa gacaaatcag aaaaggaaga caaagggaaa gatatcaggg 6360
aaagtaaaaa gtaaaaagaa accaagaatt ttccattttg tgatcaattg tttacagccc 6420
gtgatggtga tgtgtttgtg tcaacaggac tcccacagga ggtctatgcc aaactgactg 6480
tttttacaaa tgtatactta aggtcagtgc ctataacaag acagagacct ctggtcagca 6540
 
aactggaact cagtaaactg gagaaatagt atcgatggga ggtttctatt ttcacaacca 6600
gctgacactg ctgaagagca gaggcgtaat ggctactcag acgataggaa ccaatttaaa 6660
gggggggagg aagttaaatt tttatgtaaa ttcaacatgt gacacttgat aatagtaatt 6720
gtcaccagtg tttatgtttt aactgccaca cctgccatat ttttacaaaa cgtgtgctgt 6780
gaatttatca cttttctttt taattcacag gttgtttact attatatgtg actatttttg 6840
taaatgggtt tgtgtttggg gagagggatt aaagggaggg aattctacat ttctctattg 6900
tattgtataa ctggatatat tttaaatgga ggcatgctgc aattctcatt cacacataaa 6960
aaaatcacat cacaaaaggg aagagtttac ttcttgtttc aggatgtttt tagatttttg 7020
aggtgcttaa atagctattc gtatttttaa ggtgtctcat ccagaaaaaa tttaatgtgc 7080
ctgtaaatgt tccatagaat cacaagcatt aaagagttgt tttattttta cataacccat 7140
taaatgtaca tgtatatatg tatatatgta tatgtgcgtg tatatacata tatatgtata 7200
cacacatgca cacacagaga tatacacata ccattacatt gtcattcaca gtcccagcag 7260
catgactatc acatttttga taagtgtcct ttggcataaa ataaaaatat cctatcagtc 7320
ctttctaaga agcctgaatt gaccaaaaaa catccccacc accactttat aaagttgatt 7380
ctgctttatc ctgcagtatt gtttagccat cttctgctct tggtaaggtt gacatagtat 7440
atgtcaattt aaaaaataaa agtctgcttt gtaaatagta attttaccca gtggtgcatg 7500
tttgagcaaa caaaaatgat gatttaagca cactacttat tgcatcaaat atgtaccaca 7560
gtaagtatag tttgcaagct ttcaacaggt aatatgatgt aattggttcc attatagttt 7620
gaagctgtca ctgctgcatg tttatcttgc ctatgctgct gtatcttatt ccttccactg 7680
ttcagaagtc taatatggga agccatatat cagtggtaaa gtgaagcaaa ttgttctacc 7740
aagacctcat tcttcatgtc attaagcaat aggttgcagc aaacaaggaa gagcttcttg 7800
ctttttattc ttccaacctt aattgaacac tcaatgatga aaagcccgac tgtacaaaca 7860
tgttgcaagc tgcttaaatc tgtttaaaat atatggttag agttttctaa gaaaatataa 7920
atactgtaaa aagttcattt tattttattt ttcagccttt tgtacgtaaa atgagaaatt 7980
aaaagtatct tcaggtggat gtcacagtca ctattgttag tttctgttcc tagcactttt 8040
aaattgaagc acttcacaaa ataagaagca aggactagga tgcagtgtag gtttctgctt 8100
ttttattagt actgtaaact tgcacacatt tcaatgtgaa acaaatctca aactgagttc 8160
aatgtttatt tgctttcaat agtaatgcct tatcattgaa agaggcttaa agaaaaaaaa 8220
aatcagctga tactcttggc attgcttgaa tccaatgttt ccacctagtc tttttattca 8280
gtaatcatca gtcttttcca atgtttgttt acacagatag atcttattga cccatatggc 8340
actagaactg tatcagatat aatatgggat cccagctttt tttcctctcc cacaaaacca 8400
ggtagtgaag ttatattacc agttacagca aaatactttg tgtttcacaa gcaacaataa 8460
atgtagattc tttatactga agctattgac ttgtagtgtg ttggtgaaat gcatgcagga 8520
 
aaatgctgtt accataaaga acggtaaacc acattacaat caagccaaaa gaataaaggt 8580

ttcgcttttg tttttgtatt taattgttgt ctttgtttct atctttgaaa tgccatttaa 8640

aggtagattt ctatcatgta aaaataatct atctgaaaaa caaatgtaaa gaacacacat 8700

taattactat aattcatctt tcaatttttt catggaatgg aagttaatta agaagagtgt 8760

attggataac tactttaata ttggccaaaa agctagatat ggcatcaggt agactagtgg 8820

aaagttacaa aaattaataa aaaattgact aacattttaa aaaaaaaaaa aaaaaa 8876
<210> 3
   <211> 9141
   <212> DNA
   <213> Homo sapiens
<400> 3 
accatagagt gaatctcaga acaggaagcg gaggcataag cagagaggat tctggaaagg 60

tctctttgtt ttcttatcca cagagaaaga aagaaaaaaa attgtaacta atttgtaaac 120

ctctgtggtc aaaaaaaaaa aaaaaaaaaa aagctgaaca gctgcagagg aagacacgtt 180

ataccctaac catcttggat gctgggcttt gttatgctgt aattcataag gctctgtttt 240

atcagagatt atggagcaag aaaactgaag ccaagccaca tcaaggtttg acagggatga 300

gatacctgtc aaggattcat agtagagtgg cttactggga aaggagcaaa gaatctcttc 360

tagggatatt gtaagaataa atgagataat tcacagaagg gacctggagc ttttccggaa 420

aaaggtgctg tgactatcta aggtaattcg tatgcaagaa gctacacgta attaaatgtg 480

caggatgaaa agatggcaca ggcactgttg gtacccccag gacctgaaag cttccgcctt 540

tttactagag aatctcttgc tgctatcgaa aaacgtgctg cagaagagaa agccaagaag 600

cccaaaaagg aacaagataa tgatgatgag aacaaaccaa agccaaatag tgacttggaa 660

gctggaaaga accttccatt tatttatgga gacattcctc cagagatggt gtcagagccc 720

ctggaggacc tggatcccta ctatatcaat aagaaaactt ttatagtaat gaataaagga 780

aaggcaattt tccgattcag tgccacctct gccttgtata ttttaactcc actaaaccct 840

gttaggaaaa ttgctatcaa gattttggta cattctttat tcagcatgct tatcatgtgc 900

actattttga ccaactgtgt atttatgacc ttgagcaacc ctcctgactg gacaaagaat 960

gtagagtaca cattcactgg aatctatacc tttgagtcac ttataaaaat cttggcaaga 1020

gggttttgct tagaagattt tacgtttctt cgtgatccat ggaactggct ggatttcagt 1080

gtcattgtga tggcatatgt gacagagttt gtggacctgg gcaatgtctc agcgttgaga 1140

acattcagag ttctccgagc actgaaaaca atttcagtca ttccaggttt aaagaccatt 1200

gtgggggccc tgatccagtc ggtaaagaag ctttctgatg tgatgatcct gactgtgttc 1260

tgtctgagcg tgtttgctct cattgggctg cagctgttca tgggcaatct gaggaataaa 1320

tgtttgcagt ggcccccaag cgattctgct tttgaaacca acaccacttc ctactttaat 1380
 
ggcacaatgg attcaaatgg gacatttgtt aatgtaacaa tgagcacatt taactggaag 1440

gattacattg gagatgacag tcacttttat gttttggatg ggcaaaaaga ccctttactc 1500

tgtggaaatg gctcagatgc aggccagtgt ccagaaggat acatctgtgt gaaggctggt 1560

cgaaacccca actatggcta cacaagcttt gacaccttta gctgggcttt cctgtctcta 1620

tttcgactca tgactcaaga ctactgggaa aatctttacc agttgacatt acgtgctgct 1680

gggaaaacat acatgatatt ttttgtcctg gtcattttct tgggctcatt ttatttggtg 1740

aatttgatcc tggctgtggt ggccatggcc tatgaggagc agaatcaggc caccttggaa 1800

gaagcagaac aaaaagaggc cgaatttcag cagatgctcg aacagcttaa aaagcaacag 1860

gaagaagctc aggcagttgc ggcagcatca gctgcttcaa gagatttcag tggaataggt 1920

gggttaggag agctgttgga aagttcttca gaagcatcaa agttgagttc caaaagtgct 1980

aaagaatgga ggaaccgaag gaagaaaaga agacagagag agcaccttga aggaaacaac 2040

aaaggagaga gagacagctt tcccaaatcc gaatctgaag acagcgtcaa aagaagcagc 2100

ttccttttct ccatggatgg aaacagactg accagtgaca aaaaattctg ctcccctcat 2160

cagtctctct tgagtatccg tggctccctg ttttccccaa gacgcaatag caaaacaagc 2220

attttcagtt tcagaggtcg ggcaaaggat gttggatctg aaaatgactt tgctgatgat 2280

gaacacagca catttgaaga cagcgaaagc aggagagact cactgtttgt gccgcacaga 2340

catggagagc gacgcaacag taacgttagt caggccagta tgtcatccag gatggtgcca 2400

gggcttccag caaatgggaa gatgcacagc actgtggatt gcaatggtgt ggtttccttg 2460

gtgggtggac cttcagctct aacgtcacct actggacaac ttcccccaga gggcaccacc 2520

acagaaacgg aagtcagaaa gagaaggtta agctcttacc agatttcaat ggagatgctg 2580

gaggattcct ctggaaggca aagagccgtg agcatagcca gcattctgac caacacaatg 2640

gaagaacttg aagaatctag acagaaatgt ccgccatgct ggtatagatt tgccaatgtg 2700

ttcttgatct gggactgctg tgatgcatgg ttaaaagtaa aacatcttgt gaatttaatt 2760

gttatggatc catttgttga tcttgccatc actatttgca ttgtcttaaa taccctcttt 2820

atggccatgg agcactaccc catgactgag caattcagta gtgtgttgac tgtaggaaac 2880

ctggtcttta ctgggatttt cacagcagaa atggttctca agatcattgc catggatcct 2940

tattactatt tccaagaagg ctggaatatc tttgatggaa ttattgtcag cctcagttta 3000

atggagcttg gtctgtcaaa tgtggaggga ttgtctgtac tgcgatcatt cagactgctt 3060

agagttttca agttggcaaa atcctggccc acactaaata tgctaattaa gatcattggc 3120

aattctgtgg gggctctagg aaacctcacc ttggtgttgg ccatcatcgt cttcattttt 3180

gctgtggtcg gcatgcagct ctttggtaag agctacaaag aatgtgtctg caagatcaat 3240

gatgactgta cgctcccacg gtggcacatg aacgacttct tccactcctt cctgattgtg 3300
 
ttccgcgtgc tgtgtggaga gtggatagag accatgtggg actgtatgga ggtcgctggc 3360

caaaccatgt gccttattgt tttcatgttg gtcatggtca ttggaaacct tgtggttctg 3420

aacctctttc tggccttatt gttgagttca tttagctcag acaaccttgc tgctactgat 3480

gatgacaatg aaatgaataa tctgcagatt gcagtaggaa gaatgcaaaa gggaattgat 3540

tatgtgaaaa ataagatgcg ggagtgtttc caaaaagcct tttttagaaa gccaaaagtt 3600

atagaaatcc atgaaggcaa taagatagac agctgcatgt ccaataatac tggaattgaa 3660

ataagcaaag agcttaatta tcttagagat gggaatggaa ccaccagtgg tgtaggtact 3720

ggaagcagtg ttgaaaaata cgtaatcgat gaaaatgatt atatgtcatt cataaacaac 3780

cccagcctca ccgtcacagt gccaattgct gttggagagt ctgactttga aaacttaaat 3840

actgaagagt tcagcagtga gtcagaacta gaagaaagca aagagaaatt aaatgcaacc 3900

agctcatctg aaggaagcac agttgatgtt gttctacccc gagaaggtga acaagctgaa 3960

actgaacccg aagaagacct taaaccggaa gcttgtttta ctgaaggatg tattaaaaag 4020

tttccattct gtcaagtaag tacagaagaa ggcaaaggga agatctggtg gaatcttcga 4080

aaaacctgct acagtattgt tgagcacaac tggtttgaga ctttcattgt gttcatgatc 4140

cttctcagta gtggtgcatt ggcctttgaa gatatataca ttgaacagcg aaagactatc 4200

aaaaccatgc tagaatatgc tgacaaagtc tttacctata tattcattct ggaaatgctt 4260

ctcaaatggg ttgcttatgg atttcaaaca tatttcacta atgcctggtg ctggctagat 4320

ttcttgatcg ttgatgtttc tttggttagc ctggtagcca atgctcttgg ctactcagaa 4380

ctcggtgcca tcaaatcatt acggacatta agagctttaa gacctctaag agccttatcc 4440

cggtttgaag gcatgagggt ggttgtgaat gctcttgttg gagcaattcc ctctatcatg 4500

aatgtgctgt tggtctgtct catcttctgg ttgatcttta gcatcatggg tgtgaatttg 4560

tttgctggca agttctacca ctgtgttaac atgacaacgg gtaacatgtt tgacattagt 4620

gatgttaaca atttgagtga ctgtcaggct cttggcaagc aagctcggtg gaaaaacgtg 4680

aaagtaaact ttgataatgt tggcgctggc tatcttgcac tgcttcaagt ggccacattt 4740

aaaggctgga tggatattat gtatgcagct gttgattcac gagatgttaa acttcagcct 4800

gtatatgaag aaaatctgta catgtattta tactttgtca tctttatcat ctttgggtca 4860

ttcttcactc tgaatctatt cattggtgtc atcatagata acttcaacca gcagaaaaag 4920

aagtttggag gtcaagacat ctttatgaca gaggaacaga aaaaatatta caatgcaatg 4980

aagaaacttg gatccaagaa acctcagaaa cccatacctc gcccagcaaa caaattccaa 5040

ggaatggtct ttgattttgt aaccagacaa gtctttgata tcagcatcat gatcctcatc 5100

tgcctcaaca tggtcaccat gatggtggaa acggatgacc agggcaaata catgacccta 5160

gttttgtccc ggatcaacct agtgttcatt gttctgttca ctggagaatt tgtgctgaag 5220

ctcgtctccc tcagacacta ctacttcact ataggctgga acatctttga ctttgtggtg 5280
 
gtgattctct ccattgtagg tatgtttctg gctgagatga tagaaaagta ttttgtgtcc 5340
cctaccttgt tccgagtgat ccgtcttgcc aggattggcc gaatcctacg tctgatcaaa 5400
ggagcaaagg ggatccgcac gctgctcttt gctttgatga tgtcccttcc tgcgttgttt 5460
aacatcggcc tcctgctctt cctggtcatg tttatctatg ccatctttgg gatgtccaac 5520
tttgcctatg ttaaaaagga agctggaatt gatgacatgt tcaactttga gacctttggc 5580
aacagcatga tctgcttgtt ccaaattaca acctctgctg gctgggatgg attgctagca 5640
cctattctta atagtgcacc acccgactgt gaccctgaca caattcaccc tggcagctca 5700
gttaagggag actgtgggaa cccatctgtt gggatttctt tttttgtcag ttacatcatc 5760
atatccttcc tggttgtggt gaacatgtac atcgcggtca tcctggagaa cttcagtgtt 5820
gctactgaag aaagtgcaga gcccctgagt gaggatgact ttgagatgtt ctatgaggtt 5880
tgggaaaagt ttgatcccga tgcgacccag tttatagagt tctctaaact ctctgatttt 5940
gcagctgccc tggatcctcc tcttctcata gcaaaaccca acaaagtcca gcttattgcc 6000
atggatctgc ccatggtcag tggtgaccgg atccactgtc ttgatatttt atttgccttt 6060
acaaagcgtg ttttgggtga gagtggagag atggatgccc ttcgaataca gatggaagac 6120
aggtttatgg catcaaaccc ctccaaagtc tcttatgagc ctattacaac cactttgaaa 6180
cgtaaacaag aggaggtgtc tgccgctatc attcagcgta atttcagatg ttatctttta 6240
aagcaaaggt taaaaaatat atcaagtaac tataacaaag aggcaattaa agggaggatt 6300
gacttaccta taaaacaaga catgattatt gacaaactaa atgggaactc cactccagaa 6360
aaaacagatg ggagttcctc taccacctct cctccttcct atgatagtgt aacaaaacca 6420
gacaaggaaa agtttgagaa agacaaacca gaaaaagaaa gcaaaggaaa agaggtcaga 6480
gaaaatcaaa agtaaaaaga aacaaagaat tatctttgtg atcaattgtt tacagcctat 6540
gaaggtaaag tatatgtgtc aactggactt caagaggagg tccatgccaa actgactgtt 6600
ttaacaaata ctcatagtca gtgcctatac aagacagtga agtgacctct ctgtcactgc 6660
aactctgtga agcagggtat caacattgac aagaggttgc tgtttttatt accagctgac 6720
actgctgagg agaaacccaa tggctaccta gactataggg atagttgtgc aaagtgaaca 6780
ttgtaactac accaaacacc tttagtacag tccttgcatc cattctattt ttaacttcca 6840
tatctgccat atttttacaa aatttgttct agtgcatttc catggtcccc aattcatagt 6900
ttattcataa tgctatgtca ctatttttgt aaatgaggtt tacgttgaag aaacagtata 6960
caagaaccct gtctctcaaa tgatcagaca aaggtgtttt gccagagaga taaaattttt 7020
gctcaaaacc agaaaaagaa ttgtaatggc tacagtttca gttacttcca ttttctagat 7080
ggctttaatt ttgaaagtat tttagtctgt tatgtttgtt tctatctgaa cagttatgtg 7140
cctgtaaagt ctcctctaat atttaaagga ttatttttat gcaaagtatt ctgtttcagc 7200
 
aagtgcaaat tttattctaa gtttcagagc tctatattta atttaggtca aatgctttcc 7260

aaaaagtaat ctaataaatc cattctagaa aaatatatct aaagtattgc tttagaatag 7320

ttgttccact ttctgctgca gtattgcttt gccatcttct gctctcagca aagctgatag 7380

tctatgtcaa ttaaataccc tatgttatgt aaatagttat tttatcctgt ggtgcatgtt 7440

tgggcaaata tatatatagc ctgataaaca acttctatta aatcaaatat gtaccacagt 7500

gtatgtgtct tttgcaagct tccaacaggg atgtatcctg tatcattcat taaacatagt 7560

ttaaaggcta tcactaatgc atgttaatat tgcctatgct gctctatttt actcaatcca 7620

ttcttcacaa gtcttggtta aagaatgtca catattggtg atagaatgaa ttcaacctgc 7680

tctgtccatt atgtcaagca gaataatttg aagctattta caaacacctt tacttttgca 7740

cttttaattc aacatgagta tcatatggta tctctctaga tttcaaggaa acacactgga 7800

tactgcctac tgacaaaacc tattcttcat attttgctaa aaatatgtct aaaacttgtt 7860

taaatataaa taatgtaaaa atataatcaa ctttatttgt cagcattttg tacataagaa 7920

aattattttc aggttgatga catcacaatt tattttactt tatgcttttg cttttgattt 7980

ttaatcacaa ttccaaactt ttgaatccat aagatttttc aatggataat ttcctaaaat 8040

aaaagttaga taatgggttt tatggatttc tttgttataa tatattttct accattccaa 8100

taggagatac attggtcaaa cactcaaacc tagatcattt tctaccaact atggttgcct 8160

caatataacc ttttattcat agatgttttt ttttattcaa cttttgtagt atttacgtat 8220

gcagactagt cttatttttt taattcctgc tgcactaaag ctattacaaa tataacatgg 8280

actttgttct ttttagccat gaacaaagtg gcaaagttgt gcaattacct aacatgatat 8340

aaatttttgt tttttgcaca aaccaaaagt ttaatgttaa ttctttttac aaaactattt 8400

actgtagtgt attgaagaac tgcatgcagg gaattgctat tgctaaaaag aatggtgagc 8460

tacgtcatta ttgagccaaa agaataaatt tcatttttta ttgcatttca cttattggcc 8520

tctggggttt tttgtttttg ttttttgctg ttggcagttt aaaatatata taattaataa 8580

aacctgtgct tgatctgaca tttgtataca taaaagttta catgaatttt acaacaaact 8640

agtgcatgat tcaccaagca gtactacaga acaaaggcaa attaaaagca gctttgtgaa 8700

cttttatgtg tgcaaaggat caagttcaca tgttccaact ttcaggtttg ataataatag 8760

tagtaaccac ctacaatagc tttcaatttc aattaactcc cttggctata agcatctaaa 8820

ctcatcttct ttcaatataa ttgatgctat ctcctaatta cttggtggct aataaatgtt 8880

acattctttg ttacttaaat gcattatata aactcctatg tatacataag gtattaatga 8940

tatagttatt gagaatttat attaactttt ttttcaagaa cccttggatt tatgtgaggt 9000

caaaaccaaa ctcttattct cagtggaaaa ctccagttgt aatgcatatt tttaaagaca 9060

atttggatct aaatatgtat ttcataattc tcccataata aattatataa ggtggctaat 9120

tggaaaaaaa aaaaaaaaaa a 9141
<210> 4
   <211> 7805
   <212> DNA
   <213> Homo sapiens
<400> 4 
ccagcacccc ggggctgcgc actgcagctc cccaggccac ccaccaccct tctggtctct 60

gagcccagga tgcgaggatg gccagaccat ctctgtgcac cctggtgcct ctgggccctg 120

agtgcttgcg ccccttcacc cgggagtcac tggcagccat agaacagcgg gcggtggagg 180

aggaggcccg gctgcagcgg aataagcaga tggagattga ggagcccgaa cggaagccac 240

gaagtgactt ggaggctggc aagaacctac ccatgatcta cggagacccc ccgccggagg 300

tcatcggcat ccccctggag gacctggatc cctactacag caataagaag accttcatcg 360

tactcaacaa gggcaaggcc atcttccgct tctccgccac acctgctctc tacctgctga 420

gccccttcag cgtagtcagg cgcggggcca tcaaggtgct catccatgcg ctgttcagca 480

tgttcatcat gatcaccatc ttgaccaact gcgtattcat gaccatgagt gacccgcctc 540

cctggtccaa gaatgtggag tacaccttca cagggatcta cacctttgag tccctcatca 600

agatactggc ccgaggcttc tgtgtcgacg acttcacatt cctccgggac ccctggaact 660

ggctggactt cagtgtcatc atgatggcgt acctgacaga gtttgtggac ttgggcaaca 720

tctcagccct gaggaccttc cgggtgctgc gggccctcaa aaccatcacg gtcatcccag 780

ggctgaagac gatcgtgggg gccctgatcc agtcggtgaa aaagctgtcg gatgtgatga 840

tcctcactgt cttctgcctg agcgtctttg cgctggtagg actgcagctc ttcatgggaa 900

acctgaggca gaagtgtgtg cgctggcccc cgccgttcaa cgacaccaac accacgtggt 960

acagcaatga cacgtggtac ggcaatgaca catggtatgg caatgagatg tggtacggca 1020

atgactcatg gtatgccaac gacacgtgga acagccatgc aagctgggcc accaacgata 1080

cctttgattg ggacgcctac atcagtgatg aagggaactt ctacttcctg gagggctcca 1140

acgatgccct gctctgtggg aacagcagtg atgctgggca ctgccctgag ggttatgagt 1200

gcatcaagac cgggcggaac cccaactatg gctacaccag ctatgacacc ttcagctggg 1260

ccttcttggc tctcttccgc ctcatgacac aggactattg ggagaacctc ttccagctga 1320

cccttcgagc agctggcaag acctacatga tcttcttcgt ggtcatcatc ttcctgggct 1380

ctttctacct catcaatctg atcctggccg tggtggccat ggcatatgcc gagcagaatg 1440

aggccaccct ggccgaggat aaggagaaag aggaggagtt tcagcagatg cttgagaagt 1500

tcaaaaagca ccaggaggag ctggagaagg ccaaggccgc ccaagctctg gaaggtgggg 1560

aggcagatgg ggacccagcc catggcaaag actgcaatgg cagcctggac acatcgcaag 1620

gggagaaggg agccccgagg cagagcagca gcggagacag cggcatctcc gacgccatgg 1680

aagaactgga agaggcccac caaaagtgcc caccatggtg gtacaagtgc gcccacaaag 1740
 
tgctcatatg gaactgctgc gccccgtggc tgaagttcaa gaacatcatc cacctgatcg 1800

tcatggaccc gttcgtggac ctgggcatca ccatctgcat cgtgctcaac accctcttca 1860

tggccatgga acattacccc atgacggagc actttgacaa cgtgctcact gtgggcaacc 1920

tggtcttcac aggcatcttc acagcagaga tggttctgaa gctgattgcc atggacccct 1980

acgagtattt ccagcagggt tggaatatct tcgacagcat catcgtcacc ctcagcctgg 2040

tagagctagg cctggccaac gtacagggac tgtctgtgct acgctccttc cgtctgctgc 2100

gggtcttcaa gctggccaag tcgtggccaa cgctgaacat gctcatcaag atcattggca 2160

attcagtggg ggcgctgggt aacctgacgc tggtgctggc tatcatcgtg ttcatcttcg 2220

ccgtggtggg catgcagctg tttggcaaga gctacaagga gtgcgtgtgc aagattgcct 2280

tggactgcaa cctgccgcgc tggcacatgc atgatttctt ccactccttc ctcatcgtct 2340

tccgcatcct gtgcggggag tggatcgaga ccatgtggga ctgcatggag gtggccggcc 2400

aagccatgtg cctcaccgtc ttcctcatgg tcatggtcat cggcaatctt gtggtcctga 2460

acctgttcct ggctctgctg ctgagctcct tcagcgccga cagtctggca gcctcggatg 2520

aggatggcga gatgaacaac ctgcagattg ccatcgggcg catcaagttg ggcatcggct 2580

ttgccaaggc cttcctcctg gggctgctgc atggcaagat cctgagcccc aaggacatca 2640

tgctcagcct cggggaggct gacggggccg gggaggctgg agaggcgggg gagactgccc 2700

ccgaggatga gaagaaggag ccgcccgagg aggacctgaa gaaggacaat cacatcctga 2760

accacatggg cctggctgac ggccccccat ccagcctcga gctggaccac cttaacttca 2820

tcaacaaccc ctacctgacc atacaggtgc ccatcgcctc cgaggagtcc gacctggaga 2880

tgcccaccga ggaggaaacc gacactttct cagagcctga ggatagcaag aagccgccgc 2940

agcctctcta tgatgggaac tcgtccgtct gcagcacagc tgactacaag ccccccgagg 3000

aggaccctga ggagcaggca gaggagaacc ccgaggggga gcagcctgag gagtgcttca 3060

ctgaggcctg cgtgcagcgc tggccctgcc tctacgtgga catctcccag ggccgtggga 3120

agaagtggtg gactctgcgc agggcctgct tcaagattgt cgagcacaac tggttcgaga 3180

ccttcattgt cttcatgatc ctgctcagca gtggggctct ggccttcgag gacatctaca 3240

ttgagcagcg gcgagtcatt cgcaccatcc tagaatatgc cgacaaggtc ttcacctaca 3300

tcttcatcat ggagatgctg ctcaaatggg tggcctacgg ctttaaggtg tacttcacca 3360

acgcctggtg ctggctcgac ttcctcatcg tggatgtctc catcatcagc ttggtggcca 3420

actggctggg ctactcggag ctgggaccca tcaaatccct gcggacactg cgggccctgc 3480

gtcccctgag ggcactgtcc cgattcgagg gcatgagggt ggtggtgaac gccctcctag 3540

gcgccatccc ctccatcatg aatgtgctgc ttgtctgcct catcttctgg ctgatcttca 3600

gcatcatggg tgtcaacctg tttgccggca agttctacta ctgcatcaac accaccacct 3660
 
ctgagaggtt cgacatctcc gaggtcaaca acaagtctga gtgcgagagc ctcatgcaca 3720

caggccaggt ccgctggctc aatgtcaagg tcaactacga caacgtgggt ctgggctacc 3780

tctccctcct gcaggtggcc accttcaagg gttggatgga catcatgtat gcagccgtgg 3840

actcccggga gaaggaggag cagccgcagt acgaggtgaa cctctacatg tacctctact 3900

ttgtcatctt catcatcttt ggctccttct tcaccctcaa cctcttcatt ggcgtcatca 3960

ttgacaactt caaccagcag aagaagaagt taggggggaa agacatcttt atgacggagg 4020

aacagaagaa atactataac gccatgaaga agcttggctc caagaagcct cagaagccaa 4080

ttccccggcc ccagaacaag atccagggca tggtgtatga cctcgtgacg aagcaggcct 4140

tcgacatcac catcatgatc ctcatctgcc tcaacatggt caccatgatg gtggagacag 4200

acaaccagag ccagctcaag gtggacatcc tgtacaacat caacatgatc ttcatcatca 4260

tcttcacagg ggagtgcgtg ctcaagatgc tcgccctgcg ccagtactac ttcaccgttg 4320

gctggaacat ctttgacttc gtggtcgtca tcctgtccat tgtgggcctt gccctctctg 4380

acctgatcca gaagtacttc gtgtcaccca cgctgttccg tgtgatccgc ctggcgcgga 4440

ttgggcgtgt cctgcggctg atccgcgggg ccaagggcat ccggacgctg ctgttcgccc 4500

tcatgatgtc gctgcctgcc ctcttcaaca tcggcctcct cctcttcctg gtcatgttca 4560

tctactccat cttcggcatg tccaactttg cctacgtcaa gaaggagtcg ggcatcgatg 4620

atatgttcaa cttcgagacc ttcggcaaca gcatcatctg cctgttcgag atcaccacgt 4680

cggccggctg ggacgggctc ctcaacccca tcctcaacag cgggccccca gactgtgacc 4740

ccaacctgga gaacccgggc accagtgtca agggtgactg cggcaacccc tccatcggca 4800

tctgcttctt ctgcagctat atcatcatct ccttcctcat cgtggtcaac atgtacatcg 4860

ccatcatcct ggagaacttc aatgtggcca cagaggagag cagcgagccc cttggtgaag 4920

atgactttga gatgttctac gagacatggg agaagttcga ccccgacgcc acccagttca 4980

tcgcctacag ccgcctctca gacttcgtgg acaccctgca ggaaccgctg aggattgcca 5040

agcccaacaa gatcaagctc atcacactgg acttgcccat ggtgccaggg gacaagatcc 5100

actgcctgga catcctcttt gccctgacca aagaggtcct gggtgactct ggggaaatgg 5160

acgccctcaa gcagaccatg gaggagaagt tcatggcagc caacccctcc aaggtgtcct 5220

acgagcccat caccaccacc ctcaagagga agcacgagga ggtgtgcgcc atcaagatcc 5280

agagggccta ccgccggcac ctgctacagc gctccatgaa gcaggcatcc tacatgtacc 5340

gccacagcca cgacggcagc ggggatgacg cccctgagaa ggaggggctg cttgccaaca 5400

ccatgagcaa gatgtatggc cacgagaatg ggaacagcag ctcgccaagc ccggaggaga 5460

agggcgaggc aggggacgcc ggacccacta tggggctgat gcccatcagc ccctcagaca 5520

ctgcctggcc tcccgcccct cccccagggc agactgtgcg cccaggtgtc aaggagtctc 5580

ttgtctagca ggcagcatcg ggtggcccca ctgagtctcg gcatagtccc cagagctccc 5640
 
ccgtggtgcc tgcacacaga gtgagggagg agggctttga atctgggact gtgcctggct 5700

ccctgatggg ggacaggatt tggccacact ggggctgaca cccaggcccg agcgcctgcg 5760

ttcccagacc atgggaaatg ggaattgcgc tcaggggctc catgctgggt ctgaggcccc 5820

tgcctccaag atttaacctg caagttgctc tgacctcctc tgggccctgt cgcccctcct 5880

tttggcctgg gggaggtcag aacattcgaa tctctgcccc tcacttgagg aggagctggc 5940

ctgcggtgga gggatcagtt gccccccatc accagagtct taagggtcac tggcctctcc 6000

ccaggaagtg gctcagaccc ctcagcccca gcccagacaa agatgtctta acctcaggga 6060

gtgcagacac ctaaccccag ggcactgcca gcccaccccc tttgactctg gggtgcagct 6120

tcacccacca ggccagctca ggaattccct ggaaaaggga aatgtgactg gttcagaaat 6180

agctcctcaa agcctcaaaa cctgattggc cactggatcc tgctgctttg ggctgggatg 6240

gtgactcctg aaacctcttc ctaggccacg tccaggtccg tagctcccct ggctggctcc 6300

taggggaaga gcagaaggaa ggatgccact tgggaatgaa ttgtcctttt ctaggaagca 6360

cgggggagtg agacaggctg ggtcctgcca gctggatcgc tgcacatggc ctgagcatcc 6420

agacctgagc gggagtcagg gacctgctgc tcagtaagaa gattctcgcc ccttccctct 6480

ctccctgcct cactcctccg tgagcaccac cagggctcca ggagcctcat ccagcctcag 6540

agatctccct tctcatctcc ccacgcccgt ctctttctca cctttcccac ctctctcccc 6600

aaagtgatcc taagaatgta cagttgagct caggttagat atttcgaccc tggggcgtgc 6660

agcagggaag gcccaactgg ttcaggctca accttccaac ttcctgtggc ctgaagaagc 6720

acttctgctg catcgctgtt ctgggcatgg cagggccagg cctctgctgg ctcaggagga 6780

ggggtgagag acctgctcag gcgtcgctgg atttattcac ttgtgtgtgt acctgtggct 6840

gtgtgtctgc ttgtatgctt ttataggcct gtgtgtatag ctgtgtgtgt gttcaagtgc 6900

gtgactgtat gtgtgtgtgt gaaccactgt gtactggagc ctgcattatg cacgtgtctg 6960

ggtatctttg tatatatgtg tatatatgtg tgccctggac tgtttcaagg tccatggagt 7020

acggctggtg tgtcatactg tgcaggcctg tccctgggag tgttcccgtg cctgggagag 7080

tggacctgtg ctgtgagtgt gtggatgcgt gtgaacgcat gtggtaaggt gtgtactcag 7140

ggcattctgt tggcctaagt gcctcttctt tttcttcttg tttctcatga aaagtttgat 7200

taaaattcag gaagcagcaa aaccttcaaa acaagacatg tatgtgtgct tgagtgtgtg 7260

aacacgtgtg tgtgtgtgca catctacatg ccatgcctat gggccagagt tgtctttatt 7320

gtccaccatg ctctctcacc tgctcccagt cctgcctgaa cagccctctc tctcactccc 7380

ctctcctccc cttcctgttt ctcgttgtca cacccatggc ctcagccctg ctccctgcct 7440

cctgcctatg tctcctctat ggaaggaggc ctccactcct tccatctctt ccttcagaag 7500

tttcgtctaa tgggggcagt ctccccttcc tggcacattg cccctctgcc ttgccctcct 7560
 
gggccctggg ctggcacagc ccctggagcc tcagaaatct gtttgattgg atattctcct 7620

cggactgtgt gcaggttgca gaggaagagt agatgagccg ggtccggcct ctccctgcct 7680

gtggcccctc ccctgcagac ggatgcccat tcctgcctgg tccagtgggg aacaggtccc 7740

acgccaggcc agcaggcggg ctcctttgta cagttcttac aataaaccct ccttggtgcc 7800

tctgg 7805
<210> 5
   <211> 8504
   <212> DNA
   <213> Homo sapiens
<400> 5 
agacggcggc ggcgcccgta ggatgcaggg atcgctcccc cggggccgct gagcctgcgc 60

ccagtgcccc gagccccgcg ccgagccgag tccgcgccaa gcagcagccg cccaccccgg 120

ggcccggccg ggggaccagc agcttcccca caggcaacgt gaggagagcc tgtgcccaga 180

agcaggatga gaagatggca aacttcctat tacctcgggg caccagcagc ttccgcaggt 240

tcacacggga gtccctggca gccatcgaga agcgcatggc agagaagcaa gcccgcggct 300

caaccacctt gcaggagagc cgagaggggc tgcccgagga ggaggctccc cggccccagc 360

tggacctgca ggcctccaaa aagctgccag atctctatgg caatccaccc caagagctca 420

tcggagagcc cctggaggac ctggacccct tctatagcac ccaaaagact ttcatcgtac 480

tgaataaagg caagaccatc ttccggttca gtgccaccaa cgccttgtat gtcctcagtc 540

ccttccaccc catccggaga gcggctgtga agattctggt tcactcgctc ttcaacatgc 600

tcatcatgtg caccatcctc accaactgcg tgttcatggc ccagcacgac cctccaccct 660

ggaccaagta tgtcgagtac accttcaccg ccatttacac ctttgagtct ctggtcaaga 720

ttctggctcg aggcttctgc ctgcacgcgt tcactttcct tcgggaccca tggaactggc 780

tggactttag tgtgattatc atggcataca caactgaatt tgtggacctg ggcaatgtct 840

cagccttacg caccttccga gtcctccggg ccctgaaaac tatatcagtc atttcagggc 900

tgaagaccat cgtgggggcc ctgatccagt ctgtgaagaa gctggctgat gtgatggtcc 960

tcacagtctt ctgcctcagc gtctttgccc tcatcggcct gcagctcttc atgggcaacc 1020

taaggcacaa gtgcgtgcgc aacttcacag cgctcaacgg caccaacggc tccgtggagg 1080

ccgacggctt ggtctgggaa tccctggacc tttacctcag tgatccagaa aattacctgc 1140

tcaagaacgg cacctctgat gtgttactgt gtgggaacag ctctgacgct gggacatgtc 1200

cggagggcta ccggtgccta aaggcaggcg agaaccccga ccacggctac accagcttcg 1260

attcctttgc ctgggccttt cttgcactct tccgcctgat gacgcaggac tgctgggagc 1320

gcctctatca gcagaccctc aggtccgcag ggaagatcta catgatcttc ttcatgcttg 1380

tcatcttcct ggggtccttc tacctggtga acctgatcct ggccgtggtc gcaatggcct 1440
 
atgaggagca aaaccaagcc accatcgctg agaccgagga gaaggaaaag cgcttccagg 1500

aggccatgga aatgctcaag aaagaacacg aggccctcac catcaggggt gtggataccg 1560

tgtcccgtag ctccttggag atgtcccctt tggccccagt aaacagccat gagagaagaa 1620

gcaagaggag aaaacggatg tcttcaggaa ctgaggagtg tggggaggac aggctcccca 1680

agtctgactc agaagatggt cccagagcaa tgaatcatct cagcctcacc cgtggcctca 1740

gcaggacttc tatgaagcca cgttccagcc gcgggagcat tttcaccttt cgcaggcgag 1800

acctgggttc tgaagcagat tttgcagatg atgaaaacag cacagcgggg gagagcgaga 1860

gccaccacac atcactgctg gtgccctggc ccctgcgccg gaccagtgcc cagggacagc 1920

ccagtcccgg aacctcggct cctggccacg ccctccatgg caaaaagaac agcactgtgg 1980

actgcaatgg ggtggtctca ttactggggg caggcgaccc agaggccaca tccccaggaa 2040

gccacctcct ccgccctgtg atgctagagc acccgccaga cacgaccacg ccatcggagg 2100

agccaggcgg gccccagatg ctgacctccc aggctccgtg tgtagatggc ttcgaggagc 2160

caggagcacg gcagcgggcc ctcagcgcag tcagcgtcct caccagcgca ctggaagagt 2220

tagaggagtc tcgccacaag tgtccaccat gctggaaccg tctcgcccag cgctacctga 2280

tctgggagtg ctgcccgctg tggatgtcca tcaagcaggg agtgaagttg gtggtcatgg 2340

acccgtttac tgacctcacc atcactatgt gcatcgtact caacacactc ttcatggcgc 2400

tggagcacta caacatgaca agtgaattcg aggagatgct gcaggtcgga aacctggtct 2460

tcacagggat tttcacagca gagatgacct tcaagatcat tgccctcgac ccctactact 2520

acttccaaca gggctggaac atcttcgaca gcatcatcgt catccttagc ctcatggagc 2580

tgggcctgtc ccgcatgagc aacttgtcgg tgctgcgctc cttccgcctg ctgcgggtct 2640

tcaagctggc caaatcatgg cccaccctga acacactcat caagatcatc gggaactcag 2700

tgggggcact ggggaacctg acactggtgc tagccatcat cgtgttcatc tttgctgtgg 2760

tgggcatgca gctctttggc aagaactact cggagctgag ggacagcgac tcaggcctgc 2820

tgcctcgctg gcacatgatg gacttctttc atgccttcct catcatcttc cgcatcctct 2880

gtggagagtg gatcgagacc atgtgggact gcatggaggt gtcggggcag tcattatgcc 2940

tgctggtctt cttgcttgtt atggtcattg gcaaccttgt ggtcctgaat ctcttcctgg 3000

ccttgctgct cagctccttc agtgcagaca acctcacagc ccctgatgag gacagagaga 3060

tgaacaacct ccagctggcc ctggcccgca tccagagggg cctgcgcttt gtcaagcgga 3120

ccacctggga tttctgctgt ggtctcctgc ggcagcggcc tcagaagccc gcagcccttg 3180

ccgcccaggg ccagctgccc agctgcattg ccacccccta ctccccgcca cccccagaga 3240

cggagaaggt gcctcccacc cgcaaggaaa cacggtttga ggaaggcgag caaccaggcc 3300

agggcacccc cggggatcca gagcccgtgt gtgtgcccat cgctgtggcc gagtcagaca 3360

cagatgacca agaagaagat gaggagaaca gcctgggcac ggaggaggag tccagcaagc 3420
 
agcaggaatc ccagcctgtg tccggtggcc cagaggcccc tccggattcc aggacctgga 3480

gccaggtgtc agcgactgcc tcctctgagg ccgaggccag tgcatctcag gccgactggc 3540

ggcagcagtg gaaagcggaa ccccaggccc cagggtgcgg tgagacccca gaggacagtt 3600

gctccgaggg cagcacagca gacatgacca acaccgctga gctcctggag cagatccctg 3660

acctcggcca ggatgtcaag gacccagagg actgcttcac tgaaggctgt gtccggcgct 3720

gtccctgctg tgcggtggac accacacagg ccccagggaa ggtctggtgg cggttgcgca 3780

agacctgcta ccacatcgtg gagcacagct ggttcgagac attcatcatc ttcatgatcc 3840

tactcagcag tggagcgctg gccttcgagg acatctacct agaggagcgg aagaccatca 3900

aggttctgct tgagtatgcc gacaagatgt tcacatatgt cttcgtgctg gagatgctgc 3960

tcaagtgggt ggcctacggc ttcaagaagt acttcaccaa tgcctggtgc tggctcgact 4020

tcctcatcgt agacgtctct ctggtcagcc tggtggccaa caccctgggc tttgccgaga 4080

tgggccccat caagtcactg cggacgctgc gtgcactccg tcctctgaga gctctgtcac 4140

gatttgaggg catgagggtg gtggtcaatg ccctggtggg cgccatcccg tccatcatga 4200

acgtcctcct cgtctgcctc atcttctggc tcatcttcag catcatgggc gtgaacctct 4260

ttgcggggaa gtttgggagg tgcatcaacc agacagaggg agacttgcct ttgaactaca 4320

ccatcgtgaa caacaagagc cagtgtgagt ccttgaactt gaccggagaa ttgtactgga 4380

ccaaggtgaa agtcaacttt gacaacgtgg gggccgggta cctggccctt ctgcaggtgg 4440

caacatttaa aggctggatg gacattatgt atgcagctgt ggactccagg gggtatgaag 4500

agcagcctca gtgggaatac aacctctaca tgtacatcta ttttgtcatt ttcatcatct 4560

ttgggtcttt cttcaccctg aacctcttta ttggtgtcat cattgacaac ttcaaccaac 4620

agaagaaaaa gttagggggc caggacatct tcatgacaga ggagcagaag aagtactaca 4680

atgccatgaa gaagctgggc tccaagaagc cccagaagcc catcccacgg cccctgaaca 4740

agtaccaggg cttcatattc gacattgtga ccaagcaggc ctttgacgtc accatcatgt 4800

ttctgatctg cttgaatatg gtgaccatga tggtggagac agatgaccaa agtcctgaga 4860

aaatcaacat cttggccaag atcaacctgc tctttgtggc catcttcaca ggcgagtgta 4920

ttgtcaagct ggctgccctg cgccactact acttcaccaa cagctggaat atcttcgact 4980

tcgtggttgt catcctctcc atcgtgggca ctgtgctctc ggacatcatc cagaagtact 5040

tcttctcccc gacgctcttc cgagtcatcc gcctggcccg aataggccgc atcctcagac 5100

tgatccgagg ggccaagggg atccgcacgc tgctctttgc cctcatgatg tccctgcctg 5160

ccctcttcaa catcgggctg ctgctcttcc tcgtcatgtt catctactcc atctttggca 5220

tggccaactt cgcttatgtc aagtgggagg ctggcatcga cgacatgttc aacttccaga 5280

ccttcgccaa cagcatgctg tgcctcttcc agatcaccac gtcggccggc tgggatggcc 5340
 
tcctcagccc catcctcaac actgggccgc cctactgcga ccccactctg cccaacagca 5400

atggctctcg gggggactgc gggagcccag ccgtgggcat cctcttcttc accacctaca 5460

tcatcatctc cttcctcatc gtggtcaaca tgtacattgc catcatcctg gagaacttca 5520

gcgtggccac ggaggagagc accgagcccc tgagtgagga cgacttcgat atgttctatg 5580

agatctggga gaaatttgac ccagaggcca ctcagtttat tgagtattcg gtcctgtctg 5640

actttgccga tgccctgtct gagccactcc gtatcgccaa gcccaaccag ataagcctca 5700

tcaacatgga cctgcccatg gtgagtgggg accgcatcca ttgcatggac attctctttg 5760

ccttcaccaa aagggtcctg ggggagtctg gggagatgga cgccctgaag atccagatgg 5820

aggagaagtt catggcagcc aacccatcca agatctccta cgagcccatc accaccacac 5880

tccggcgcaa gcacgaagag gtgtcggcca tggttatcca gagagccttc cgcaggcacc 5940

tgctgcaacg ctctttgaag catgcctcct tcctcttccg tcagcaggcg ggcagcggcc 6000

tctccgaaga ggatgcccct gagcgagagg gcctcatcgc ctacgtgatg agtgagaact 6060

tctcccgacc ccttggccca ccctccagct cctccatctc ctccacttcc ttcccaccct 6120

cctatgacag tgtcactaga gccaccagcg ataacctcca ggtgcggggg tctgactaca 6180

gccacagtga agatctcgcc gacttccccc cttctccgga cagggaccgt gagtccatcg 6240

tgtgagcctc ggcctggctg gccaggacac actgaaaagc agcctttttc accatggcaa 6300

acctaaatgc agtcagtcac aaaccagcct ggggccttcc tggctttggg agtaagaaat 6360

gggcctcagc cccgcggatc aaccaggcag agttctgtgg cgccgcgtgg acagccggag 6420

cagttggcct gtgcttggag gcctcagata gacctgtgac ctggtctggt caggcaatgc 6480

cctgcggctc tggaaagcaa cttcatccca gctgctgagg cgaaatataa aactgagact 6540

gtatatgttg tgaatgggct ttcataaatt tattatattt gatatttttt tacttgagca 6600

aagaactaag gatttttcca tggacatggg cagcaattca cgctgtctct tcttaaccct 6660

gaacaagagt gtctatggag cagccggaag tctgttctca aagcagaagt ggaatccagt 6720

gtggctccca caggtcttca ctgcccaggg gtcgaatggg gtccccctcc cacttgacct 6780

gagatgctgg gagggctgaa cccccactca cacaagcaca cacacacagt cctcacacac 6840

ggaggccaga cacaggccgt gggacccagg ctcccagcct aagggagaca ggcctttccc 6900

tgccggcccc ccaaggatgg ggttcttgtc cacggggctc actctggccc cctattgtct 6960

ccaaggtccc attttccccc tgtgttttca cgcaggtcat attgtcagtc ctacaaaaat 7020

aaaaggcttc cagaggagag tggcctgggt cccagggctg gccctaggca ctgatagttg 7080

ccttttcttc ccctcctgta agagtattaa caaaaccaaa ggacacaagg gtgcaagccc 7140

cattcacggc ctggcatgca gcttgtcctt gctcctggaa cctggcaggc cctgcccagc 7200

cagccatcgg aagagagggc tgagccatgg gggtttgggg ctaagaagtt caccagccct 7260

gagccatggc ggcccctcag cctgcctgaa gagaggaaac tggcgatctc ccagggctct 7320
 
ctggaccata cgcggaggag ttttctgtgt ggtctccagc tcctctccag acacagagac 7380

atgggagtgg ggagcggagc ttggccctgc gccctgtgca gggaaaggga tggtcaggcc 7440

cagttctcgt gcccttagag gggaatgaac catggcacct ttgagagagg gggcactgtg 7500

gtcaggccca gcctctctgg ctcagcccgg gatcctgatg gcacccacac agaggacctc 7560

tttggggcaa gatccaggtg gtcccatagg tcttgtgaaa aggctttttc agggaaaaat 7620

attttactag tccaatcacc cccaggacct cttcagctgc tgacaatcct atttagcata 7680

tgcaaatctt ttaacataga gaactgtcac cctgaggtaa cagggtcaac tggcgaagcc 7740

tgagcaggca ggggcttggc tgccccattc cagctctccc atggagcccc tccaccgggc 7800

gcatgcctcc caggccacct cagtctcacc tgccggctct gggctggctg ctcctaacct 7860

acctcgccga gctgtcggag ggctggacat ttgtggcagt gctgaagggg gcattgccgg 7920

cgagtaaagt attatgtttc ttcttgtcac cccagttccc ttggtggcaa ccccagaccc 7980

aacccatgcc cctgacagat ctagttctct tctcctgtgt tccctttgag tccagtgtgg 8040

gacacggttt aactgtccca gcgacatttc tccaagtgga aatcctattt ttgtagatct 8100

ccatgctttg ctctcaaggc ttggagaggt atgtgcccct cctgggtgct caccgcctgc 8160

tacacaggca ggaatgcggt tgggaggcag gtcgggctgc cagcccagct ggccggaagg 8220

agactgtggt ttttgtgtgt gtggacagcc cgggagcttt gagacaggtg cctggggctg 8280

gctgcagacg gtgtggttgg gggtgggagg tgagctagac ccaaccctta gcttttagcc 8340

tggctgtcac ctttttaatt tccagaactg cacaatgacc agcaggaggg aaggacagac 8400

atcaagtgcc agatgttgtc tgaactaatc gagcacttct caccaaactt catgtataaa 8460

taaaatacat atttttaaaa caaaccaata aatggcttac atga 8504
<210> 6
   <211> 7186
   <212> DNA
   <213> Homo sapiens
<400> 6 
caggtcctcc accatgtgaa tgccaacatg gccaggtcat tagagctgag ggaaaactag 60

tgcccaaaga tatgaaaaga gtgtggatct tctggagaag tgctgttgtt caacaggtac 120

aaaattggaa atgttggctt caccagaacc taagggcctt gttcccttca ctaaagagtc 180

ttttgaactt ataaaacagc atattgctaa aacacataat gaagaccatg aagaagaaga 240

cttaaagcca actcctgatt tggaagttgg caaaaagctt ccatttattt atggaaacct 300

ttctcaagga atggtgtcag agcccttgga agatgtggac ccatattact acaagaaaaa 360

aaatactttc atagtattaa ataaaaatag aacaatcttc agattcaatg cggcttccat 420

cttgtgtaca ttgtctcctt tcaattgtat tagaagaaca actatcaagg ttttggtaca 480

tccctttttc caactgttta ttctaattag tgtcctgatt gattgcgtat tcatgtccct 540
 
gactaatttg ccaaaatgga gaccagtatt agagaatact ttgcttggaa tttacacatt 600
tgaaatactt gtaaaactct ttgcaagagg tgtctgggca ggatcatttt ccttcctcgg 660
tgatccatgg aactggctcg atttcagcgt aactgtgttt gaggttatta taagatactc 720
acctctggac ttcattccaa cgcttcaaac tgcaagaact ttgagaattt taaaaattat 780
tcctttaaat caaggtctga aatcccttgt aggggtcctg atccactgct tgaagcagct 840
tattggtgtc attatcctaa ctctgttttt tctgagcata ttttctctaa ttgggatggg 900
gctcttcatg ggcaacttga aacataaatg ttttcgatgg ccccaagaga atgaaaatga 960
aaccctgcac aacagaactg gaaacccata ttatattcga gaaacagaaa acttttatta 1020
tttggaagga gaaagatatg ctctcctttg tggcaacagg acagatgctg gtcagtgtcc 1080
tgaaggatat gtgtgtgtaa aagctggcat aaatcctgat caaggcttca caaattttga 1140
cagttttggc tgggccttat ttgccctatt tcggttaatg gctcaggatt accctgaagt 1200
actttatcac cagatacttt atgcttctgg gaaggtctac atgatatttt ttgtggtggt 1260
aagttttttg ttttcctttt atatggcaag tttgttctta ggcatacttg ccatggccta 1320
tgaagaagaa aagcagagag ttggtgaaat atctaagaag attgaaccaa aatttcaaca 1380
gactggaaaa gaacttcaag aaggaaatga aacagatgag gccaagacca tacaaataga 1440
aatgaagaaa aggtcaccaa tttccacaga cacatcattg gatgtgttgg aagatgctac 1500
tctcagacat aaggaagaac ttgaaaaatc caagaagata tgcccattat actggtataa 1560
gtttgctaaa actttcttga tctggaattg ttctccctgt tggttaaaat tgaaagagtt 1620
tgtccatagg attataatgg caccatttac tgatcttttc cttatcatat gcataatttt 1680
aaacgtatgt tttctgacct tggagcatta tccaatgagt aaacaaacta acactcttct 1740
caacattgga aacctggttt tcattggaat tttcacagca gaaatgattt ttaaaataat 1800
tgcaatgcat ccatatgggt atttccaagt aggttggaac atttttgata gcatgatagt 1860
gttccatggt ttaatagaac tttgtctagc aaatgttgca ggaatggctc ttcttcgatt 1920
attcaggatg ttaagaattt tcaagttggg aaagtattgg ccaacattcc agattttgat 1980
gtggtctctt agtaactcat gggtggccct gaaagacttg gtcctgttgt tgttcacatt 2040
catcttcttt tctgctgcat tcggcatgaa gctgtttggt aagaattatg aagaatttgt 2100
ctgccacata gacaaagact gtcaactccc acgctggcac atgcatgact ttttccactc 2160
cttcctgaat gtgttccgaa ttctctgtgg agagtgggta gagaccttgt gggactgtat 2220
ggaggttgca ggccaatcct ggtgtattcc tttttacctg atggtcattt taattggaaa 2280
tttactggta ctttacctgt ttctggcatt ggtgagctca tttagttcat gcaaggatgt 2340
aacagctgaa gagaataatg aagcaaaaaa tctccagctt gcagtggcaa gaattaaaaa 2400
aggaataaac tatgtgcttc ttaaaatact atgcaaaaca caaaatgtcc caaaggacac 2460
 
aatggaccat gtaaatgagg tatatgttaa agaagatatt tctgaccata ccctttctga 2520
attgagcaac acccaagatt ttctcaaaga taaggaaaaa agcagtggca cagagaaaaa 2580
cgctactgaa aatgagagcc aatcacttat ccccagtcct agtgtctcag aaactgtacc 2640
aattgcttca ggagaatctg atatagaaaa tctggataat aaggagattc agagtaagtc 2700
tggtgatgga ggcagcaaag agaaaataaa gcaatctagc tcatctgaat gcagtactgt 2760
tgatattgct atctctgaag aagaagaaat gttctatgga ggtgaaagat caaagcatct 2820
gaaaaatggt tgcagacgcg gatcttcact tggtcaaatc agtggagcat ccaagaaagg 2880
aaaaatctgg cagaacatca ggaaaacctg ctgcaagatt gtagagaaca attggtttaa 2940
gtgttttatt gggcttgtta ctctgctcag cactggcact ctggcttttg aagatatata 3000
tatggatcag agaaagacaa ttaaaatttt attagaatat gctgacatga tctttactta 3060
tatcttcatt ctggaaatgc ttctaaaatg gatggcatat ggttttaagg cctatttctc 3120
taatggctgg tacaggctgg acttcgtggt tgttattgtg ttttgtctta gcttaatagg 3180
caaaactcgg gaagaactaa aacctcttat ttccatgaaa ttccttcggc ccctcagagt 3240
tctatctcaa tttgaaagaa tgaaggtggt tgtgagagct ttgatcaaaa caaccttacc 3300
cactttgaat gtgtttcttg tctgcctgat gatctggctg atttttagta tcatgggagt 3360
agacttattt gctggcagat tctatgaatg cattgaccca acaagtggag aaaggtttcc 3420
ttcatctgaa gtcatgaata agagtcggtg tgaaagcctt ctgtttaacg aatccatgct 3480
atgggaaaat gcaaaaatga actttgataa tgttggaaat ggtttccttt ctctgcttca 3540
agtagcaaca tttaatggat ggatcactat tatgaattca gcaattgatt ctgttgctgt 3600
taatatacag cctcattttg aagtcaacat ctacatgtat tgttacttta tcaactttat 3660
tatatttgga gtatttctcc ctctgagtat gctgattact gttattattg ataatttcaa 3720
caagcataaa ataaagctgg gaggctcaaa tatctttata acggttaaac agagaaaaca 3780
gtaccgcagg ctgaagaagc taatgtatga ggattctcaa agaccagtac ctcgcccatt 3840
aaacaagctc caaggattca tctttgatgt ggtaacaagc caagctttta atgtcattgt 3900
tatggttctt atatgtttcc aagcaatagc catgatgata gacactgatg ttcagagtct 3960
acaaatgtcc attgctctct actggattaa ctcaattttt gttatgctat atactatgga 4020
atgtatactg aagctcatcg ctttccgttg tttttatttc accattgcgt ggaacatttt 4080
tgattttatg gtggttattt tctccatcac aggactatgt ctgcctatga cagtaggatc 4140
ctaccttgtg cctccttcac ttgtgcaact gatacttctc tcacggatca ttcacatgct 4200
gcgtcttgga aaaggaccaa aggtgtttca taatctgatg cttcctttga tgctgtccct 4260
cccagcatta ttgaacatca ttcttctcat cttcctggtc atgttcatct atgccgtatt 4320
tggaatgtat aattttgcct atgttaaaaa agaagctgga attaatgatg tgtctaattt 4380
tgaaaccttt ggcaacagta tgctctgtct ttttcaagtt gcaatatttg ctggttggga 4440
 
tgggatgctt gatgcaattt tcaacagtaa atggtctgac tgtgatcctg ataaaattaa 4500

ccctgggact caagttagag gagattgtgg gaacccctct gttgggattt tttattttgt 4560

cagttatatc ctcatatcat ggctgatcat tgtaaatatg tacattgttg ttgtcatgga 4620

gtttttaaat attgcttcta agaagaaaaa caagaccttg agtgaagatg attttaggaa 4680

attctttcag gtatggaaaa ggtttgatcc tgataggacc cagtacatag actctagcaa 4740

gctttcagat tttgcagctg ctcttgatcc tcctcttttc atggcaaaac caaacaaggg 4800

ccagctcatt gctttggacc tccccatggc tgttggggac agaattcatt gcctcgatat 4860

cttacttgct tttacaaaga gagttatggg tcaagatgtg aggatggaga aagttgtttc 4920

agaaatagaa tcagggtttt tgttagccaa cccttttaag atcacatgtg agccaattac 4980

gactactttg aaacgaaaac aagaggcagt ttcagcaacc atcattcaac gtgcttataa 5040

aaattaccgc ttgaggcgaa atgacaaaaa tacatcagat attcatatga tagatggtga 5100

cagagatgtt catgctacta aagaaggtgc ctattttgac aaagctaagg aaaagtcacc 5160

tattcaaagc cagatctaat accacttacc acctcttttc atatttcttc acatatctga 5220

aaaatgttga aagcctaagc caggaataaa agaaaagtag agataataat cagttcttta 5280

caaccgatgg taattaagct tgtattcaca agacttcatg ccaaattcac tttttagcat 5340

tatatctaac aaatcaagag aatccttaat attgctgcag tgagtttaaa gtgggttaaa 5400

gtggccattt gacaatctca tatttgtttt ctctacatgg cttatatgat gtgtgccttc 5460

tagggaatga agggaagtgg tgatagagat cagcagcagc aggggctttc ttttatattt 5520

tatgtataat ttaatgggct ttaagtcacc actattaaga cttacaaata agcaaatact 5580

ttcctgatgt gggatggtga aatgctaatg gccattaaat cataaacttg cctagacaaa 5640

agccaattgg aagaagggag agagcagttc tttagaaagt gcctttgaga tcaacctcag 5700

agattcttgg gctgattaaa actgcatttg aaaaagattg gttgaagctc tgtgtttatt 5760

tttgtatgtt cttgttttca tttggaactg ggaatgaata ggatttcatt gtgctcaagc 5820

tcctggtttc tcatctctgg atagtttcac ctaagctctg gctcttaagc aggacagatt 5880

cgtaaaacaa gaagcataaa ggagaggtat agcctttttt tttttttttt ttttcatttt 5940

cttctcattt acacctattt ttttaaaaag tatacattta ctaaaatgat gtaataaata 6000

acatgttaat agactcaagc tttaccttat gaaattgatg tatttttacc agttatttct 6060

aatgtaacat tgaatatata agatctgaca aatgtatgtt taaacatgaa ttagaagagt 6120

tgagaactac cattatgtat agggattctc atagtgtctt ggcccttaat tggaaagttg 6180

tggcaacttt aaagtacttt ttactgtatg ttataattct ttataactta gagagagaca 6240

atggtcactc aaactatgag aactatgaat taggagataa aagtttaaat ttgttgttgt 6300

tttataacag tatgtacaag ttagttttcc cttatatatt tacgttttca agttttttaa 6360
 
tctcatcata tacatccata ctctataaaa tgttttatat tcaaagaact gtaaaatcct 6420

aaacattagt tttcactatt gaaattgttt tttaaagata ggcataaata gttgtcctta 6480

gacttattca tacaaatata gtcatttact tctatgtagt ttgagattct gagagttatt 6540

ccaactttat gaagattgat ttcaatgtgc ctgctaagtc ctaaaagatt cagaaagaaa 6600

atttatatat tattgattta aatatcatcc tttaaatatg ttgtataaca ttcaatatag 6660

tttatgtatc agtgattgta ttttattctg aatgcatgat ctcaagcctt aactactata 6720

atctttttct gcccctcaga aattgaataa cctaaccaag atgcctttag gggatgccct 6780

aagtaaatgt aatttcagat ttcagggttt tttttttttc ctctctaagt gttccttccc 6840

tttcttctcc tgctctccat catgttatgg agaccagtga ggaaccagtg ttaacttggt 6900

gacaatgtga cagctggtgc tttatctaag ctccgttttc tatttcttgg gaatgcttta 6960

ttgtggaaac tgcttcagat acttaaattg aatcataact tgcttctgta aattgcgtaa 7020

agacaacaaa ctgattttag tttgaaaagt ttatctttta cttgtaaacc ttgtttgcca 7080

gttaccttcc gaaagctgtg taaagagtta tttttaacaa agtcttaaca atatatgtta 7140

ctttttagat actatagaaa ataataaata taacctgtaa accaca 7186
<210> 7
   <211> 7215
   <212> DNA
   <213> Homo sapiens
<400> 7 
gagcgctcca agatggcgcc caccgcagtc ccgcccgccg catcctcggc gcctttgcag 60

tccggccgcg cctcccgggc cccgcgttag ggccgccgct gcctccctcg ccgccgccgc 120

tgccagctga cctgtcctgg acgcagcata actaacgaag ctgctgcagg atgagaagat 180

ggcagcgcgg ctgcttgcac caccaggccc tgatagtttc aagcctttca cccctgagtc 240

actggcaaac attgagaggc gcattgctga gagcaagctc aagaaaccac caaaggccga 300

tggcagtcat cgggaggacg atgaggacag caagcccaag ccaaacagcg acctggaagc 360

agggaagagt ttgcctttca tctacgggga catcccccaa ggcctggttg cagttcccct 420

ggaggacttt gacccatact atttgacgca gaaaaccttt gtagtattaa acagagggaa 480

aactctcttc agatttagtg ccacgcctgc cttgtacatt ttaagtcctt ttaacctgat 540

aagaagaata gctattaaaa ttttgataca ttcagtattt agcatgatca ttatgtgcac 600

tattttgacc aactgtgtat tcatgacttt tagtaaccct cctgactggt cgaagaatgt 660

ggagtacacg ttcacaggga tttatacatt tgaatcacta gtgaaaatca ttgcaagagg 720

tttctgcata gatggcttta cctttttacg ggacccatgg aactggttag atttcagtgt 780

catcatgatg gcgtatataa cagagtttgt aaacctaggc aatgtttcag ctctacgcac 840

tttcagggta ctgagggctt tgaaaactat ttcggtaatc ccaggcctga agacaattgt 900
 
gggtgccctg attcagtctg tgaagaaact gtcagatgtg atgatcctga cagtgttctg 960
cctgagtgtt tttgccttga tcggactgca gctgttcatg gggaaccttc gaaacaagtg 1020
tgttgtgtgg cccataaact tcaacgagag ctatcttgaa aatggcacca aaggctttga 1080
ttgggaagag tatatcaaca ataaaacaaa tttctacaca gttcctggca tgctggaacc 1140
tttactctgt gggaacagtt ctgatgctgg gcaatgccca gagggatacc agtgtatgaa 1200
agcaggaagg aaccccaact atggttacac aagttttgac acttttagct gggccttctt 1260
ggcattattt cgccttatga cccaggacta ttgggaaaac ttgtatcaat tgactttacg 1320
agcagccggg aaaacataca tgatcttctt cgtcttggtc atctttgtgg gttctttcta 1380
tctggtgaac ttgatcttgg ctgtggtggc catggcttat gaagaacaga atcaggcaac 1440
actggaggag gcagaacaaa aagaggctga atttaaagca atgttggagc aacttaagaa 1500
gcaacaggaa gaggcacagg ctgctgcgat ggccacttca gcaggaactg tctcagaaga 1560
tgccatagag gaagaaggtg aagaaggagg gggctcccct cggagctctt ctgaaatctc 1620
taaactcagc tcaaagagtg caaaggaaag acgtaacagg agaaagaaga ggaagcaaaa 1680
ggaactctct gaaggagagg agaaagggga tcccgagaag gtgtttaagt cagagtcaga 1740
agatggcatg agaaggaagg cctttcggct gccagacaac agaataggga ggaaattttc 1800
catcatgaat cagtcactgc tcagcatccc aggctcgccc ttcctctccc gccacaacag 1860
caagagcagc atcttcagtt tcaggggacc tgggcggttc cgagacccgg gctccgagaa 1920
tgagttcgcg gatgacgagc acagcacggt ggaggagagc gagggccgcc gggactccct 1980
cttcatcccc atccgggccc gcgagcgccg gagcagctac agcggctaca gcggctacag 2040
ccagggcagc cgctcctcgc gcatcttccc cagcctgcgg cgcagcgtga agcgcaacag 2100
cacggtggac tgcaacggcg tggtgtccct catcggcggc cccggctccc acatcggcgg 2160
gcgtctcctg ccagaggcta caactgaggt ggaaattaag aagaaaggcc ctggatctct 2220
tttagtttcc atggaccaat tagcctccta cgggcggaag gacagaatca acagtataat 2280
gagtgttgtt acaaatacac tagtagaaga actggaagag tctcagagaa agtgcccgcc 2340
atgctggtat aaatttgcca acactttcct catctgggag tgccacccct actggataaa 2400
actgaaagag attgtgaact tgatagttat ggaccctttt gtggatttag ccatcaccat 2460
ctgcatcgtc ctgaatacac tgtttatggc aatggagcac catcctatga caccacaatt 2520
tgaacatgtc ttggctgtag gaaatctggt tttcactgga attttcacag cggaaatgtt 2580
cctgaagctc atagccatgg atccctacta ttatttccaa gaaggttgga acatttttga 2640
cggatttatt gtctccctca gtttaatgga actgagtcta gcagacgtgg aggggctttc 2700
agtgctgcga tctttccgat tgctccgagt cttcaaattg gccaaatcct ggcccaccct 2760
gaacatgcta atcaagatta ttggaaattc agtgggtgcc ctgggcaacc tgacactggt 2820
gctggccatt attgtcttca tctttgccgt ggtggggatg caactctttg gaaaaagcta 2880
 
caaagagtgt gtctgcaaga tcaaccagga ctgtgaactc cctcgctggc atatgcatga 2940
ctttttccat tccttcctca ttgtctttcg agtgttgtgc ggggagtgga ttgagaccat 3000
gtgggactgc atggaagtgg caggccaggc catgtgcctc attgtcttta tgatggtcat 3060
ggtgattggc aacttggtgg tgctgaacct gtttctggcc ttgctcctga gctccttcag 3120
tgcagacaac ctggctgcca cagatgacga tggggaaatg aacaacctcc agatctcagt 3180
gatccgtatc aagaagggtg tggcctggac caaactaaag gtgcacgcct tcatgcaggc 3240
ccactttaag cagcgtgagg ctgatgaggt gaagcctctg gatgagttgt atgaaaagaa 3300
ggccaactgt atcgccaatc acaccggtgc agacatccac cggaatggtg acttccagaa 3360
gaatggcaat ggcacaacca gcggcattgg cagcagcgtg gagaagtaca tcattgatga 3420
ggaccacatg tccttcatca acaaccccaa cttgactgta cgggtaccca ttgctgtggg 3480
cgagtctgac tttgagaacc tcaacacaga ggatgttagc agcgagtcgg atcctgaagg 3540
cagcaaagat aaactagatg acaccagctc ctctgaagga agcaccattg atatcaaacc 3600
agaagtagaa gaggtccctg tggaacagcc tgaggaatac ttggatccag atgcctgctt 3660
cacagaaggt tgtgtccagc ggttcaagtg ctgccaggtc aacatcgagg aagggctagg 3720
caagtcttgg tggatcctgc ggaaaacctg cttcctcatc gtggagcaca actggtttga 3780
gaccttcatc atcttcatga ttctgctgag cagtggcgcc ctggccttcg aggacatcta 3840
cattgagcag agaaagacca tccgcaccat cctggaatat gctgacaaag tcttcaccta 3900
tatcttcatc ctggagatgt tgctcaagtg gacagcctat ggcttcgtca agttcttcac 3960
caatgcctgg tgttggctgg acttcctcat tgtggctgtc tctttagtca gccttatagc 4020
taatgccctg ggctactcgg aactaggtgc cataaagtcc cttaggaccc taagagcttt 4080
gagaccctta agagccttat cacgatttga aggatgaggg tggtggtgaa atgccttggt 4140
gggcgccatc ccctccatca tgaatgtgct gctggtgtgt ctcatcttct ggctgatttt 4200
cagcatcatg ggagttaact tgtttgcggg aaagtaccac tactgcttta atgagacttc 4260
tgaaatccga tttgaaattg aagatgtcaa caataaaact gaatgtgaaa agcttatgga 4320
ggggaacaat acagagatca gatggaagaa cgtgaagatc aactttgaca atgttggggc 4380
aggatacctg gcccttcttc aagtagcaac cttcaaaggc tggatggaca tcatgtatgc 4440
agctgtagat tcccggaagc ctgatgagca gcctaagtat gaggacaata tctacatgta 4500
catctatttt gtcatcttca tcatcttcgg ctccttcttc accctgaacc tgttcattgg 4560
tgtcatcatt gataacttca atcaacaaaa gaaaaagttc ggaggtcagg acatcttcat 4620
gaccgaagaa cagaagaagt actacaatgc catgaaaaag ctgggctcaa agaagccaca 4680
gaaacctatt ccccgcccct tgaacaaaat ccaaggaatc gtctttgatt ttgtcactca 4740
gcaagccttt gacattgtta tcatgatgct catctgcctt aacatggtga caatgatggt 4800
 
ggagacagac actcaaagca agcagatgga gaacatcctc tactggatta acctggtgtt 4860

tgttatcttc ttcacctgtg agtgtgtgct caaaatgttt gcgttgaggc actactactt 4920

caccattggc tggaacatct tcgacttcgt ggtagtcatc ctctccattg tgggaatgtt 4980

cctggcagat ataattgaga aatactttgt ttccccaacc ctattccgag tcatccgatt 5040

ggcccgtatt gggcgcatct tgcgtctgat caaaggcgcc aaagggattc gtaccctgct 5100

ctttgcctta atgatgtcct tgcctgccct gttcaacatc ggccttctgc tcttcctggt 5160

catgttcatc ttctccattt ttgggatgtc caattttgca tatgtgaagc acgaggctgg 5220

tatcgatgac atgttcaact ttgagacatt tggcaacagc atgatctgcc tgtttcaaat 5280

cacaacctca gctggttggg atggcctgct gctgcccatc ctaaaccgcc cccctgactg 5340

cagcctagat aaggaacacc cagggagtgg ctttaaggga gattgtggga acccctcagt 5400

gggcatcttc ttctttgtaa gctacatcat catctctttc ctaattgtcg tgaacatgta 5460

cattgccatc atcctggaga acttcagtgt agccacagag gaaagtgcag accctctgag 5520

tgaggatgac tttgagacct tctatgagat ctgggagaag ttcgaccccg atgccaccca 5580

gttcattgag tactgtaagc tggcagactt tgcagatgcc ttggagcatc ctctccgagt 5640

gcccaagccc aataccattg agctcatcgc tatggatctg ccaatggtga gcggggatcg 5700

catccactgc ttggacatcc tttttgcctt caccaagcgg gtcctgggag atagcgggga 5760

gttggacatc ctgcggcagc agatggaaga gcggttcgtg gcatccaatc cttccaaagt 5820

gtcttacgag ccaatcacaa ccacactgcg tcgcaagcag gaggaggtat ctgcagtggt 5880

cctgcagcgt gcctaccggg gacatttggc aaggcggggc ttcatctgca aaaagacaac 5940

ttctaataag ctggagaatg gaggcacaca ccgggagaaa aaagagagca ccccatctac 6000

agcctccctc ccgtcctatg acagtgtaac taaacctgaa aaggagaaac agcagcgggc 6060

agaggaagga agaagggaaa gagccaaaag acaaaaagag gtcagagaat ccaagtgtta 6120

gaggagaaca aaaattcagt attatacaga tctaaaactc gcaagtgaaa gattgtttac 6180

aaacttcctg aatattatca atgcagaaca gctgtggaga ctctaacctg aagatctata 6240

ccaaacgtcg tctgcttacc acgtaacaca gctgcatctt gagcagtgac ctgccaaggg 6300

caaaggaccc cgctccctag acttacagat tttctaatgc ttgggcaggt ggttactgca 6360

tgttccacat cagtcaatgc aacttaggac aaaactaacc agatacagaa acagaagaga 6420

ggctgccggg accagcatat ttccgttgca gccaaatgga ttttattttt tcattttatt 6480

gattctcaga agcagaaagc atcactttaa aagttcgttt gttcatgcaa actatatttg 6540

cattcttaca ttagttaagc taagcagcaa aaagaaaaca cacacacaca ctcacattta 6600

gcccatgtca tttaattgtc agtttctttg acataaagcg catcttctcc acatgggctt 6660

cacgtggttt ggagatgggt gggggaaaac aatcaggttt cttcaggctg aggaggactt 6720

gctcaggccg attccaaaca ttgtgctcgt tcaatgcgta gaaatgattt gcatgatggc 6780
 
atgccgtgat cagaagtcat gcatgagatc catacaccac aggacactac taatctagtc 6840

ccttgcactg ggtcagcctt tggacaggac ccagccctgc accgttcact gtatttggag 6900

aaaatggtaa gagttccata ccggctacaa ttctttgagt tcttaaaagt ccttcataca 6960

ccttctgggt agggaaacaa ccaactaatt gactaacacc accaacaaca aaaaacaaac 7020

ccaatccaac aagcagatgg atccgttgcg tgtatatgtt taacagacat ctctaacata 7080

cagccattgt tgcacatttt gcaagatgaa ctatttaatg ctgctctgtg tccagtacat 7140

gggggagact ttgatcccaa atggcttgta ctatttatgt cactgtaaaa ccaaatccta 7200

gggctaaaaa aaaaa 7215
<210> 8
   <211> 9771
   <212> DNA
   <213> Homo sapiens
<400> 8 
cggggctgct acctccacgg gcgcgccctg gcaggagggg cgcagtctgc ttgcaggcgg 60

tcgccagcgc tccagcggcg gctgtcggct ttccaattcc gccagctcgg ctgaggctgg 120

gctagcctgg gtgccagtgg ctgctagcgg caggcgtccc ctgagcaaca ggagcccaga 180

gaaaaagaag cagccctgag agagcgccgg ggaaggagag gcccgcgccc tctcctggag 240

ccagattctg caggtgcact gggtggggat gatcggcggg ctaggttgca agcctcttat 300

gtgaggagct gaagaggaat taaaatatac aggatgaaaa gatggcaatg ttgcctcccc 360

caggacctca gagctttgtc catttcacaa aacagtctct tgccctcatt gaacaacgca 420

ttgctgaaag aaaatcaaag gaacccaaag aagaaaagaa agatgatgat gaagaagccc 480

caaagccaag cagtgacttg gaagctggca aacagctgcc cttcatctat ggggacattc 540

ctcccggcat ggtgtcagag cccctggagg acttggaccc ctactatgca gacaaaaaga 600

ctttcatagt attgaacaaa gggaaaacaa tcttccgttt caatgccaca cctgctttat 660

atatgctttc tcctttcagt cctctaagaa gaatatctat taagatttta gtacactcct 720

tattcagcat gctcatcatg tgcactattc tgacaaactg catatttatg accatgaata 780

acccaccgga ctggaccaaa aatgtcgagt acacttttac tggaatatat acttttgaat 840

cacttgtaaa aatccttgca agaggcttct gtgtaggaga attcactttt cttcgtgacc 900

cgtggaactg gctggatttt gtcgtcattg tttttgcgta tttaacagaa tttgtaaacc 960

taggcaatgt ttcagctctt cgaactttca gagtattgag agctttgaaa actatttctg 1020

taatcccagg cctgaagaca attgtagggg ctttgatcca gtcagtgaag aagctttctg 1080

atgtcatgat cctgactgtg ttctgtctga gtgtgtttgc actaattgga ctacagctgt 1140

tcatgggaaa cctgaagcat aaatgttttc gaaattcact tgaaaataat gaaacattag 1200

aaagcataat gaatacccta gagagtgaag aagactttag aaaatatttt tattacttgg 1260
 
aaggatccaa agatgctctc ctttgtggtt tcagcacaga ttcaggtcag tgtccagagg 1320

ggtacacctg tgtgaaaatt ggcagaaacc ctgattatgg ctacacgagc tttgacactt 1380

tcagctgggc cttcttagcc ttgtttaggc taatgaccca agattactgg gaaaaccttt 1440

accaacagac gctgcgtgct gctggcaaaa cctacatgat cttctttgtc gtagtgattt 1500

tcctgggctc cttttatcta ataaacttga tcctggctgt ggttgccatg gcatatgaag 1560

aacagaacca ggcaaacatt gaagaagcta aacagaaaga attagaattt caacagatgt 1620

tagaccgtct taaaaaagag caagaagaag ctgaggcaat tgcagcggca gcggctgaat 1680

atacaagtat taggagaagc agaattatgg gcctctcaga gagttcttct gaaacatcca 1740

aactgagctc taaaagtgct aaagaaagaa gaaacagaag aaagaaaaag aatcaaaaga 1800

agctctccag tggagaggaa aagggagatg ctgagaaatt gtcgaaatca gaatcagagg 1860

acagcatcag aagaaaaagt ttccaccttg gtgtcgaagg gcataggcga gcacatgaaa 1920

agaggttgtc tacccccaat cagtcaccac tcagcattcg tggctccttg ttttctgcaa 1980

ggcgaagcag cagaacaagt ctttttagtt tcaaaggcag aggaagagat ataggatctg 2040

agactgaatt tgccgatgat gagcacagca tttttggaga caatgagagc agaaggggct 2100

cactgtttgt gccccacaga ccccaggagc gacgcagcag taacatcagc caagccagta 2160

ggtccccacc aatgctgccg gtgaacggga aaatgcacag tgctgtggac tgcaacggtg 2220

tggtctccct ggttgatgga cgctcagccc tcatgctccc caatggacag cttctgccag 2280

agggcacgac caatcaaata cacaagaaaa ggcgttgtag ttcctatctc ctttcagagg 2340

atatgctgaa tgatcccaac ctcagacaga gagcaatgag tagagcaagc atattaacaa 2400

acactgtgga agaacttgaa gagtccagac aaaaatgtcc accttggtgg tacagatttg 2460

cacacaaatt cttgatctgg aattgctctc catattggat aaaattcaaa aagtgtatct 2520

attttattgt aatggatcct tttgtagatc ttgcaattac catttgcata gttttaaaca 2580

cattatttat ggctatggaa caccacccaa tgactgagga attcaaaaat gtacttgcta 2640

taggaaattt ggtctttact ggaatctttg cagctgaaat ggtattaaaa ctgattgcca 2700

tggatccata tgagtatttc caagtaggct ggaatatttt tgacagcctt attgtgactt 2760

taagtttagt ggagctcttt ctagcagatg tggaaggatt gtcagttctg cgatcattca 2820

gactgctccg agtcttcaag ttggcaaaat cctggccaac attgaacatg ctgattaaga 2880

tcattggtaa ctcagtaggg gctctaggta acctcacctt agtgttggcc atcatcgtct 2940

tcatttttgc tgtggtcggc atgcagctct ttggtaagag ctacaaagaa tgtgtctgca 3000

agatcaatga tgactgtacg ctcccacggt ggcacatgaa cgacttcttc cactccttcc 3060

tgattgtgtt ccgcgtgctg tgtggagagt ggatagagac catgtgggac tgtatggagg 3120

tcgctggtca agctatgtgc cttattgttt acatgatggt catggtcatt ggaaacctgg 3180
 
tggtcctaaa cctatttctg gccttattat tgagctcatt tagttcagac aatcttacag 3240

caattgaaga agaccctgat gcaaacaacc tccagattgc agtgactaga attaaaaagg 3300

gaataaatta tgtgaaacaa accttacgtg aatttattct aaaagcattt tccaaaaagc 3360

caaagatttc cagggagata agacaagcag aagatctgaa tactaagaag gaaaactata 3420

tttctaacca tacacttgct gaaatgagca aaggtcacaa tttcctcaag gaaaaagata 3480

aaatcagtgg ttttggaagc agcgtggaca aacacttgat ggaagacagt gatggtcaat 3540

catttattca caatcccagc ctcacagtga cagtgccaat tgcacctggg gaatccgatt 3600

tggaaaatat gaatgctgag gaacttagca gtgattcgga tagtgaatac agcaaagtga 3660

gattaaaccg gtcaagctcc tcagagtgca gcacagttga taaccctttg cctggagaag 3720

gagaagaagc agaggctgaa cctatgaatt ccgatgagcc agaggcctgt ttcacagatg 3780

gttgtgtacg gaggttctca tgctgccaag ttaacataga gtcagggaaa ggaaaaatct 3840

ggtggaacat caggaaaacc tgctacaaga ttgttgaaca cagttggttt gaaagcttca 3900

ttgtcctcat gatcctgctc agcagtggtg ccctggcttt tgaagatatt tatattgaaa 3960

ggaaaaagac cattaagatt atcctggagt atgcagacaa gatcttcact tacatcttca 4020

ttctggaaat gcttctaaaa tggatagcat atggttataa aacatatttc accaatgcct 4080

ggtgttggct ggatttccta attgttgatg tttctttggt tactttagtg gcaaacactc 4140

ttggctactc agatcttggc cccattaaat cccttcggac actgagagct ttaagacctc 4200

taagagcctt atctagattt gaaggaatga gggtcgttgt gaatgcactc ataggagcaa 4260

ttccttccat catgaatgtg ctacttgtgt gtcttatatt ctggctgata ttcagcatca 4320

tgggagtaaa tttgtttgct ggcaagttct atgagtgtat taacaccaca gatgggtcac 4380

ggtttcctgc aagtcaagtt ccaaatcgtt ccgaatgttt tgcccttatg aatgttagtc 4440

aaaatgtgcg atggaaaaac ctgaaagtga actttgataa tgtcggactt ggttacctat 4500

ctctgcttca agttgcaact tttaagggat ggacgattat tatgtatgca gcagtggatt 4560

ctgttaatgt agacaagcag cccaaatatg aatatagcct ctacatgtat atttattttg 4620

tcgtctttat catctttggg tcattcttca ctttgaactt gttcattggt gtcatcatag 4680

ataatttcaa ccaacagaaa aagaagcttg gaggtcaaga catctttatg acagaagaac 4740

agaagaaata ctataatgca atgaaaaagc tggggtccaa gaagccacaa aagccaattc 4800

ctcgaccagg gaacaaaatc caaggatgta tatttgacct agtgacaaat caagcctttg 4860

atattagtat catggttctt atctgtctca acatggtaac catgatggta gaaaaggagg 4920

gtcaaagtca acatatgact gaagttttat attggataaa tgtggttttt ataatccttt 4980

tcactggaga atgtgtgcta aaactgatct ccctcagaca ctactacttc actgtaggat 5040

ggaatatttt tgattttgtg gttgtgatta tctccattgt aggtatgttt ctagctgatt 5100

tgattgaaac gtattttgtg tcccctaccc tgttccgagt gatccgtctt gccaggattg 5160
 
gccgaatcct acgtctagtc aaaggagcaa aggggatccg cacgctgctc tttgctttga 5220

tgatgtccct tcctgcgttg tttaacatcg gcctcctgct cttcctggtc atgttcatct 5280

acgccatctt tggaatgtcc aactttgcct atgttaaaaa ggaagatgga attaatgaca 5340

tgttcaattt tgagaccttt ggcaacagta tgatttgcct gttccaaatt acaacctctg 5400

ctggctggga tggattgcta gcacctattc ttaacagtaa gccacccgac tgtgacccaa 5460

aaaaagttca tcctggaagt tcagttgaag gagactgtgg taacccatct gttggaatat 5520

tctactttgt tagttatatc atcatatcct tcctggttgt ggtgaacatg tacattgcag 5580

tcatactgga gaattttagt gttgccactg aagaaagtac tgaacctctg agtgaggatg 5640

actttgagat gttctatgag gtttgggaga agtttgatcc cgatgcgacc cagtttatag 5700

agttctctaa actctctgat tttgcagctg ccctggatcc tcctcttctc atagcaaaac 5760

ccaacaaagt ccagctcatt gccatggatc tgcccatggt tagtggtgac cggatccatt 5820

gtcttgacat cttatttgct tttacaaagc gtgttttggg tgagagtggg gagatggatt 5880

ctcttcgttc acagatggaa gaaaggttca tgtctgcaaa tccttccaaa gtgtcctatg 5940

aacccatcac aaccacacta aaacggaaac aagaggatgt gtctgctact gtcattcagc 6000

gtgcttatag acgttaccgc ttaaggcaaa atgtcaaaaa tatatcaagt atatacataa 6060

aagatggaga cagagatgat gatttactca ataaaaaaga tatggctttt gataatgtta 6120

atgagaactc aagtccagaa aaaacagatg ccacttcatc caccacctct ccaccttcat 6180

atgatagtgt aacaaagcca gacaaagaga aatatgaaca agacagaaca gaaaaggaag 6240

acaaagggaa agacagcaag gaaagcaaaa aatagagctt catttttgat atattgttta 6300

cagcctgtga aagtgattta tttgtgttaa taaaactctt ttgaggaagt ctatgccaaa 6360

atccttttta tcaaaatatt ctcgaaggca gtgcagtcac taactctgat ttcctaagaa 6420

aggtgggcag cattagcaga tggttatttt tgcactgatg attctttaag aatcgtaaga 6480

gaactctgta ggaattattg attatagcat acaaaagtga ttcagttttt tggtttttaa 6540

taaatcagaa gaccatgtag aaaactttta catctgcctt gtcatctttt cacaggattg 6600

taattagtct tgtttcccat gtaaataaac aacacacgca tacagaaaaa tctattattt 6660

atctattatt tggaaatcaa caaaagtatt tgccttggct ttgcaatgaa atgcttgata 6720

gaagtaatgg acattagtta tgaatgttta gttaaaatgc attattaggg agcttgactt 6780

tttatcaatg tacagaggtt attctatatt ttgaggtgct taaatttatt ctacattgca 6840

tcagaaccaa tttatatgtg cctataaaat gccatgggat taaaaatata tgtaggctat 6900

tcatttctac aaatgttttt cattcatctt gactcacatg ccaacaagga taagacttac 6960

ctttagagta ttgtgtttca tagcctttct tctttcatat ccctttttgt tcatagaata 7020

accacagaac ttgaaaaatt attctaagta catattacac tcctcaaaaa aaacaaagat 7080
 
aactgagaaa aaagttattg acagaagttc tatttgctat tatttacata gcctaacatt 7140

tgactgtgct gcccaaaata ctgataatag tctcttaaac tcttttgtca aattttcctg 7200

ctttcttatg cagtattgtt tagtcatcct ttcgctgtaa gcaaagttga tgaaatcctt 7260

cctgatatgc agttagttgt ttgaccacgg tacatacttg agcagataat aacttgggca 7320

cagtatttat tgcatcactt gtatacaatc ccgtgtttgg caagctttca aatcatgtaa 7380

tatgacagac tttacacaga tatgtgttta gtatgaataa aaaagcattg aaatagggat 7440

tcttgccaac ttgctctctt gccaccaact tactttccta aattatggaa gtaatctttt 7500

ttggatatac ttcaatgtat acaatgagga agatgtcacc ttctccttaa aattctatga 7560

tgtgaaatat attttgcctc aatcaacaca gtaccatggg cttctaattt atcaagcaca 7620

tattcatttt gcattagctg tagacatcta gttttttgaa aacacctatt aatagtaatt 7680

tgaaaagaaa taaccataat gctttttttc gtgagtttat ttcaggaata tgagatcttt 7740

cttctataaa gttattcatg cacaggcaaa aattgagcta cacaggtaga atgtagtttt 7800

acttagaaga tttttgtggg aggttttgaa gcaaatatat aaaacaactt tcactaattt 7860

gctttccata tttaaaaaat aataaattac atttatataa taaatgttta aagcacatat 7920

tttttgttgt tctggcaatt taaaaagaaa gaggatttaa acgtacctat agaaacaaag 7980

atttatggtt aaagaatgag atcagaagtc tagaatgttt ttaaattgtg atatatttta 8040

caacatccgt tattactttg agacatttgt cctaatctac gtataaaact caatctaggg 8100

ctaaagattc tttataccat cttaggttca ttcatcttag gctatttgaa ccacttttta 8160

atttaatatg aaagacacca tgcagtgttt tccgagacta catagatcat tttatcacat 8220

acctaccaag cctgttggaa ataggttttg ataatttaag tagggaccta tacaaaatat 8280

attacattta tcagattttt aaatacattc aattaagaat ttaacatcac cttaaatttg 8340

aattcaatct accgttattt caaactcaca aatataactg cattatgaat acttacataa 8400

tgtagtaaga caagatgttt gacaggttcg tgtgtaattt tctattaatg tttttacatt 8460

gccttgtttt tatgtaaaat aaaaaatatg ggcaactggt ttgttaacaa cacaatttct 8520

tcttagcatt tcaaaaatat atataaagtt gttctttttc ctatttcatg aactatgttt 8580

ttttttaaaa taacatggtt aagttttata tatatttacg tttgtttcag gaatgtctac 8640

ttgtgacttt ttatcaatta aaaataatat ttggaagaaa gagcttatta agtataagct 8700

tgaagtaaaa ttagacctct ctttccatgt agattactgt ttgtactgat ggtttcaccc 8760

ttcagaaggc actgtcatat taatatttaa attttataat cgctgaactt attacaccca 8820

acaatacaga aaggcagtta cactgaagaa cttaacttag aataaaatgg aagcaaacag 8880

gttttctaaa aactttttta agtgaccagg tctcgctctg tcacccaggc tagagtgcaa 8940

tggcatgatc atagctctct gcagcctcaa ctctgggctc aagcaaccct cctgcctcag 9000

cctcccaagt agctaagact acaggtacat gccaccatgc ctggctaata tttaaatttt 9060
 
tgtagataag gggtcttgct atgttgccca ggctagtctc aaactcctgg cttcaagtgt 9120

tcctactgtc atgacctgcc aacatgctgg ggttacaggc atgagccacc atgccccaaa 9180

caggtttgaa cacaaatctt tcggatgaaa attagagaac ctaattttag ctttttgata 9240

gttacctagt ttgcaaaaga tttgggtgac ttgtgagctg tttttaaatg ctgattgttg 9300

aacatcacaa cccaaaatac ttagcatgat tttatagagt tttgatagct ttattaaaaa 9360

gagtgaaaat aaaatgcata tgtaaataaa gcagttctaa atagctattt cagagaaatg 9420

ttaatagaag tgctgaaaga agggccaact aaattaggat ggccagggaa ttggcctggg 9480

tttaggacct atgtatgaag gccaccaatt ttttaaaaat atctgtggtt tattatgtta 9540

ttatcttctt gaggaaaaca atcaagaatt gcttcatgaa aataaataaa tagccatgaa 9600

tatcataaag ctgtttacat aggattcttt acaaatttca tagatctatg aatgctcaaa 9660

atgtttgagt ttgccataaa ttatattgta gttatattgt agttatactt gagactgaca 9720

cattgtaata taatctaaga ataaaagtta tacaaaataa aaaaaaaaaa a 9771
<210> 9
   <211> 5874
   <212> DNA
   <213> Homo sapiens
<400> 9 
atggaattcc ccattggatc cctcgaaact aacaacttcc gtcgctttac tccggagtca 60

ctggtggaga tagagaagca aattgctgcc aagcagggaa caaagaaagc cagagagaag 120

catagggagc agaaggacca agaagagaag cctcggcccc agctggactt gaaagcctgc 180

aaccagctgc ccaagttcta tggtgagctc ccagcagaac tgatcgggga gcccctggag 240

gatctagatc cgttctacag cacacaccgg acatttatgg tgctgaacaa agggaggacc 300

atttcccggt ttagtgccac tcgggccctg tggctattca gtcctttcaa cctgatcaga 360

agaacggcca tcaaagtgtc tgtccactcg tggttcagtt tatttattac ggtcactatt 420

ttggttaatt gtgtgtgcat gacccgaact gaccttccag agaaaattga atatgtcttc 480

actgtcattt acacctttga agccttgata aagatactgg caagaggatt ttgtctaaat 540

gagttcacgt acctgagaga tccttggaac tggctggatt ttagcgtcat taccctggca 600

tatgttggca cagcaataga tctccgtggg atctcaggcc tgcggacatt cagagttctt 660

agagcattaa aaacagtttc tgtgatccca ggcctgaagg tcattgtggg ggccctgatt 720

cactcagtga agaaactggc tgatgtgacc atcctcacca tcttctgcct aagtgttttt 780

gccttggtgg ggctgcaact cttcaagggc aacctcaaaa ataaatgtgt caagaatgac 840

atggctgtca atgagacaac caactactca tctcacagaa aaccagatat ctacataaat 900

aagcgaggca cttctgaccc cttactgtgt ggcaatggat ctgactcagg ccactgccct 960

gatggttata tctgccttaa aacttctgac aacccggatt ttaactacac cagctttgat 1020
 
tcctttgctt gggctttcct ctcactgttc cgcctcatga cacaggattc ctgggaacgc 1080
ctctaccagc agaccctgag gacttctggg aaaatctata tgatcttttt tgtgctcgta 1140
atcttcctgg gatctttcta cctggtcaac ttgatcttgg ctgtagtcac catggcgtat 1200
gaggagcaga accaggcaac cactgatgaa attgaagcaa aggagaagaa gttccaggag 1260
gccctcgaga tgctccggaa ggagcaggag gtgctagcag cactagggat tgacacaacc 1320
tctctccact cccacaatgg atcaccttta acctccaaaa atgccagtga gagaaggcat 1380
agaataaagc caagagtgtc agagggctcc acagaagaca acaaatcacc ccgctctgat 1440
ccttacaacc agcgcaggat gtcttttcta ggcctcgcct ctggaaaacg ccgggctagt 1500
catggcagtg tgttccattt ccggtcccct ggccgagata tctcactccc tgagggagtc 1560
acagatgatg gagtctttcc tggagaccac gaaagccatc ggggctctct gctgctgggt 1620
gggggtgctg gccagcaagg ccccctccct agaagccctc ttcctcaacc cagcaaccct 1680
gactccaggc atggagaaga tgaacaccaa ccgccgccca ctagtgagct tgcccctgga 1740
gctgtcgatg tctcggcatt cgatgcagga caaaagaaga ctttcttgtc agcagaatac 1800
ttagatgaac ctttccgggc ccaaaggcaa atgagtgttg tcagtatcat aacctccgtc 1860
cttgaggaac tcgaggagtc tgaacagaag tgcccaccct gcttgaccag cttgtctcag 1920
aagtatctga tctgggattg ctgccccatg tgggtgaagc tcaagacaat tctctttggg 1980
cttgtgacgg atccctttgc agagctcacc atcaccttgt gcatcgtggt gaacaccatc 2040
ttcatggcca tggagcacca tggcatgagc cctaccttcg aagccatgct ccagataggc 2100
aacatcgtct ttaccatatt ttttactgct gaaatggtct tcaaaatcat tgccttcgac 2160
ccatactatt atttccagaa gaagtggaat atctttgact gcatcatcgt cactgtgagt 2220
ctgctagagc tgggcgtggc caagaaggga agcctgtctg tgctgcggag cttccgcttg 2280
ctgcgcgtat tcaagctggc caaatcctgg cccaccttaa acacactcat caagatcatc 2340
ggaaactcag tgggggcact ggggaacctc accatcatcc tggccatcat tgtctttgtc 2400
tttgctctgg ttggcaagca gctcctaggg gaaaactacc gtaacaaccg aaaaaatatc 2460
tccgcgcccc atgaagactg gccccgctgg cacatgcacg acttcttcca ctctttcctc 2520
attgtcttcc gtatcctctg tggagagtgg attgagaaca tgtgggcctg catggaagtt 2580
ggccaaaaat ccatatgcct catccttttc ttgacggtga tggtgctagg gaacctggtg 2640
gtgcttaacc tgttcatcgc cctgctattg aactctttca gtgctgacaa cctcacagcc 2700
ccggaggacg atggggaggt gaacaacctg caggtggccc tggcacggat ccaggtcttt 2760
ggccatcgta ccaaacaggc tctttgcagc ttcttcagca ggtcctgccc attcccccag 2820
cccaaggcag agcctgagct ggtggtgaaa ctcccactct ccagctccaa ggctgagaac 2880
cacattgctg ccaacactgc cagggggagc tctggagggc tccaagctcc cagaggcccc 2940
 
agggatgagc acagtgactt catcgctaat ccgactgtgt gggtctctgt gcccattgct 3000

gagggtgaat ctgatcttga tgacttggag gatgatggtg gggaagatgc tcagagcttc 3060

cagcaggaag tgatccccaa aggacagcag gagcagctgc agcaagtcga gaggtgtggg 3120

gaccacctga cacccaggag cccaggcact ggaacatctt ctgaggacct ggctccatcc 3180

ctgggtgaga cgtggaaaga tgagtctgtt cctcaggtcc ctgctgaggg agtggacgac 3240

acaagctcct ctgagggcag cacggtggac tgcctagatc ctgaggaaat cctgaggaag 3300

atccctgagc tggcagatga cctggaagaa ccagatgact gcttcacaga aggatgcatt 3360

cgccactgtc cctgctgcaa actggatacc accaagagtc catggatgtg ggctggcag 3420

gtgcgcaaga cttgctaccg tatcgtggag cacagctggt ttgagagctt catcatcttc 3480

atgatcctgc tcagcagtgg atctctggcc tttgaagact attacctgga ccagaagccc 3540

acggtgaaag ctttgctgga gtacactgac agggtcttca cctttatctt tgtgttcgag 3600

atgctgctta agtgggtggc ctatggcttc aaaaagtact tcaccaatgc ctggtgctgg 3660

ctggacttcc tcattgtgaa tatctcactg ataagtctca cagcgaagat tctggaatat 3720

tctgaagtgg ctcccatcaa agcccttcga acccttcgcg ctctgcggcc actgcgggct 3780

ctttctcgat ttgaaggcat gcgggtggtg gtggatgccc tggtgggcgc catcccatcc 3840

atcatgaatg tcctcctcgt ctgcctcatc ttctggctca tcttcagcat catgggtgtg 3900

aacctcttcg cagggaagtt ttggaggtgc atcaactata ccgatggaga gttttccctt 3960

gtacctttgt cgattgtgaa taacaagtct gactgcaaga ttcaaaactc cactggcagc 4020

ttcttctggg tcaatgtgaa agtcaacttt gataatgttg caatgggtta ccttgcactt 4080

ctgcaggtgg caacctttaa aggctggatg gacattatgt atgcagctgt tgattcccgg 4140

gaggtcaaca tgcaacccaa gtgggaggac aacgtgtaca tgtatttgta ctttgtcatc 4200

ttcatcattt ttggaggctt cttcacactg aatctctttg ttggggtcat aattgacaac 4260

ttcaatcaac agaaaaaaaa gttagggggc caggacatct tcatgacaga ggagcagaag 4320

aaatactaca atgccatgaa gaagttgggc tccaagaagc cccagaagcc catcccacgg 4380

cccctgaaca agttccaggg ttttgtcttt gacatcgtga ccagacaagc ttttgacatc 4440

accatcatgg tcctcatctg cctcaacatg atcaccatga tggtggagac tgatgaccaa 4500

agtgaagaaa agacgaaaat tctgggcaaa atcaaccagt tctttgtggc cgtcttcaca 4560

ggcgaatgtg tcatgaagat gttcgctttg aggcagtact acttcacaaa tggctggaat 4620

gtgtttgact tcattgtggt ggttctctcc attgcgagcc tgattttttc tgcaattctt 4680

aagtcacttc aaagttactt ctccccaacg ctcttcagag tcatccgcct ggcccgaatt 4740

ggccgcatcc tcagactgat ccgagcggcc aaggggatcc gcacactgct ctttgccctc 4800

atgatgtccc tgcctgccct cttcaacatc gggctgttgc tattccttgt catgttcatc 4860

tactctatct tcggtatgtc cagctttccc catgtgaggt gggaggctgg catcgacgac 4920
 
atgttcaact tccagacctt cgccaacagc atgctgtgcc tcttccagat taccacgtcg 4980

gccggctggg atggcctcct cagccccatc ctcaacacag ggccccccta ctgtgacccc 5040

aatctgccca acagcaatgg caccagaggg gactgtggga gcccagccgt aggcatcatc 5100

ttcttcacca cctacatcat catctccttc ctcatcatgg tcaacatgta cattgcagtg 5160

attctggaga acttcaatgt ggccacggag gagagcactg agcccctgag tgaggacgac 5220

tttgacatgt tctatgagac ctgggagaag tttgacccag aggccactca gtttattacc 5280

ttttctgctc tctcggactt tgcagacact ctctctggtc ccctgagaat cccaaaaccc 5340

aatcgaaata tactgatcca gatggacctg cctttggtcc ctggagataa gatccactgc 5400

ttggacatcc tttttgcttt caccaagaat gtcctaggag aatccgggga gttggattct 5460

ctgaaggcaa atatggagga gaagtttatg gcaactaatc tttcaaaatc atcctatgaa 5520

ccaatagcaa ccactctccg atggaagcaa gaagacattt cagccactgt cattcaaaag 5580

gcctatcgga gctatgtgct gcaccgctcc atggcactct ctaacacccc atgtgtgccc 5640

agagctgagg aggaggctgc atcactccca gatgaaggtt ttgttgcatt cacagcaaat 5700

gaaaattgtg tactcccaga caaatctgaa actgcttctg ccacatcatt cccaccgtcc 5760

tatgagagtg tcactagagg ccttagtgat agagtcaaca tgaggacatc tagctcaata 5820

caaaatgaag atgaagccac cagtatggag ctgattgccc ctgggcccta gtga 5874
<210> 10
   <211> 6500
   <212> DNA
   <213> Homo sapiens
<400> 10 
tcctgctata cccacagtgg tggtcatctc ttctgatctt cacagccaat cagctcccaa 60

ggcccctgac ctcagctcag cttttgtaga tccttatgac accatccttt aagactggaa 120

tcctagggca ggctgtttta ttcccgcctc ctgaggcctt tctgaggatc tgtggcttgt 180

ctctgtcctg agggtgaaga tggatgacag atgctaccca gtaatctttc cagatgagcg 240

gaatttccgc cccttcactt ccgactctct ggctgcaatt gagaagcgga ttgccatcca 300

aaaggagaaa aagaagtcta aagaccagac aggagaagta ccccagcctc ggcctcagct 360

tgacctaaag gcctccagga agttgcccaa gctctatggc gacattcctc gtgagctcat 420

aggaaagcct ctggaagact tggacccatt ctaccgaaat cataagacat ttatggtgtt 480

aaacagaaag aggacaatct accgcttcag tgccaagcat gccttgttca tttttgggcc 540

tttcaattca atcagaagtt tagccattag agtctcagtc cattcattgt tcagcatgtt 600

cattatcggc accgttatca tcaactgcgt gttcatggct acagggcctg ctaaaaacag 660

caacagtaac aatactgaca ttgcagagtg tgtcttcact gggatttata tttttgaagc 720

tttgattaaa atattggcaa gaggtttcat tctggatgag ttttctttcc ttcgagatcc 780
 
atggaactgg ctggactcca ttgtcattgg aatagcgatt gtgtcatata ttccaggaat 840
caccatcaaa ctattgcccc tgcgtacctt ccgtgtgttc agagctttga aagcaatttc 900
agtagtttca cgtctgaagg tcatcgtggg ggccttgcta cgctctgtga agaagctggt 960
caacgtgatt atcctcacct tcttttgcct cagcatcttt gccctggtag gtcagcagct 1020
cttcatggga agtctgaacc tgaaatgcat ctcgagggac tgtaaaaata tcagtaaccc 1080
ggaagcttat gaccattgct ttgaaaagaa agaaaattca cctgaattca aaatgtgtgg 1140
catctggatg ggtaacagtg cctgttccat acaatatgaa tgtaagcaca ccaaaattaa 1200
tcctgactat aattatacga attttgacaa ctttggctgg tcttttcttg ccatgttccg 1260
gctgatgacc caagattcct gggagaagct ttatcaacag accctgcgta ctactgggct 1320
ctactcagtc ttcttcttca ttgtggtcat tttcctgggc tccttctacc tgattaactt 1380
aaccctggct gttgttacca tggcatatga ggagcagaac aagaatgtag ctgcagagat 1440
agaggccaag gaaaagatgt ttcaggaagc ccagcagctg ttaaaggagg aaaaggaggc 1500
tctggttgcc atgggaattg acagaagttc acttacttcc cttgaaacat catattttac 1560
cccaaaaaag agaaagctct ttggtaataa gaaaaggaag tccttctttt tgagagagtc 1620
tgggaaagac cagcctcctg ggtcagattc tgatgaagat tgccaaaaaa agccacagct 1680
cctagagcaa accaaacgac tgtcccagaa tctatcactg gaccactttg atgagcatgg 1740
agatcctctc caaaggcaga gagcactgag tgctgtcagc atcctcacca tcaccatgaa 1800
ggaacaagaa aaatcacaag agccttgtct cccttgtgga gaaaacctgg catccaagta 1860
cctcgtgtgg aactgttgcc cccagtggct gtgcgttaag aaggtcctga gaactgtgat 1920
gactgacccg tttactgagc tggccatcac catctgcatc atcatcaaca ctgtcttctt 1980
ggccatggag catcacaaga tggaggccag ttttgagaag atgttgaata tagggaattt 2040
ggttttcact agcattttta tagcagaaat gtgcctaaaa atcattgcgc tcgatcccta 2100
ccactacttt cgccgaggct ggaacatttt tgacagcatt gttgctcttc tgagttttgc 2160
agatgtaatg aactgtgtac ttcaaaagag aagctggcca ttcttgcgtt ccttcagagt 2220
gctcagggtc ttcaagttag ccaaatcctg gccaactttg aacacactaa ttaagataat 2280
cggcaactct gtcggagccc ttggaagcct gactgtggtc ctggtcattg tgatctttat 2340
tttctcagta gttggcatgc agctttttgg ccgtagcttc aattcccaaa agagtccaaa 2400
actctgtaac ccgacaggcc cgacagtctc atgtttacgg cactggcaca tgggggattt 2460
ctggcactcc ttcctagtgg tattccgcat cctctgcggg gaatggatcg aaaatatgtg 2520
ggaatgtatg caagaagcga atgcatcatc atcattgtgt gttattgtct tcatattgat 2580
cacggtgata ggaaaacttg tggtgctcaa cctcttcatt gccttactgc tcaattcctt 2640
tagcaatgag gaaagaaatg gaaacttaga aggagaggcc aggaaaacta aagtccagtt 2700
 
agcactggat cgattccgcc gggctttttg ttttgtgaga cacactcttg agcatttctg 2760

tcacaagtgg tgcaggaagc aaaacttacc acagcaaaaa gaggtggcag gaggctgtgc 2820

tgcacaaagc aaagacatca ttcccctggt catggagatg aaaaggggct cagagaccca 2880

ggaggagctt ggtatactaa cctctgtacc aaagaccctg ggcgtcaggc atgattggac 2940

ttggttggca ccacttgcgg aggaggaaga tgacgttgaa ttttctggtg aagataatgc 3000

acagcgcatc acacaacctg agcctgaaca acaggcctat gagctccatc aggagaacaa 3060

gaagcccacg agccagagag ttcaaagtgt ggaaattgac atgttctctg aagatgagcc 3120

tcatctgacc atacaggatc cccgaaagaa gtctgatgtt accagtatac tatcagaatg 3180

tagcaccatt gatcttcagg atggctttgg atggttacct gagatggttc ccaaaaagca 3240

accagagaga tgtttgccca aaggctttgg ttgctgcttt ccatgctgta gcgtggacaa 3300

gagaaagcct ccctgggtca tttggtggaa cctgcggaaa acctgctacc aaatagtgaa 3360

acacagctgg tttgagagct ttattatctt tgtgattctg ctgagcagtg gggcactgat 3420

atttgaagat gttcaccttg agaaccaacc caaaatccaa gaattactaa attgtactga 3480

cattattttt acacatattt ttatcctgga gatggtacta aaatgggtag ccttcggatt 3540

tggaaagtat ttcaccagtg cctggtgctg ccttgatttc atcattgtga ttgtctctgt 3600

gaccaccctc attaacttaa tggaattgaa gtccttccgg actctacgag cactgaggcc 3660

tcttcgtgcg ctgtcccagt ttgaaggaat gaaggtggtg gtcaatgctc tcataggtgc 3720

catacctgcc attctgaatg ttttgcttgt ctgcctcatt ttctggctcg tattttgtat 3780

tctgggagta tacttctttt ctggaaaatt tgggaaatgc attaatggaa cagactcagt 3840

tataaattat accatcatta caaataaaag tcaatgtgaa agtggcaatt tctcttggat 3900

caaccagaaa gtcaactttg acaatgtggg aaatgcttac ctcgctctgc tgcaagtggc 3960

aacatttaag ggctggatgg atattatata tgcagctgtt gattccacag agaaagaaca 4020

acagccagag tttgagagca attcactcgg ttacatttac ttcgtagtct ttatcatctt 4080

tggctcattc ttcactctga atctcttcat tggcgttatc attgacaact tcaaccaaca 4140

gcagaaaaag ttaggtggcc aagacatttt tatgacagaa gaacagaaga aatactataa 4200

tgcaatgaaa aaattaggat ccaaaaaacc tcaaaaaccc attccacggc ctctgaacaa 4260

atgtcaaggt ctcgtgttcg acatagtcac aagccagatc tttgacatca tcatcataag 4320

tctcattatc ctaaacatga ttagcatgat ggctgaatca tacaaccaac ccaaagccat 4380

gaaatccatc cttgaccatc tcaactgggt ctttgtggtc atctttacgt tagaatgtct 4440

catcaaaatc tttgctttga ggcaatacta cttcaccaat ggctggaatt tatttgactg 4500

tgtggtcgtg cttctttcca ttgttagtac aatgatttct accttggaaa atcaggagca 4560

cattcctttc cctccgacgc tcttcagaat tgtccgcttg gctcggattg gccgaatcct 4620

gaggcttgtc cgggctgcac gaggaatcag gactctcctc tttgctctga tgatgtcgct 4680
 
tccttctctg ttcaacattg gtcttctact ctttctgatt atgtttatct atgccattct 4740

gggtatgaac tggttttcca aagtgaatcc agagtctgga atcgatgaca tattcaactt 4800

caagactttt gccagcagca tgctctgtct cttccagata agcacatcag caggttggga 4860

ttccctgctc agccccatgc tgcgatcaaa agaatcatgt aactcttcct cagaaaactg 4920

ccacctccct ggcatagcca catcctactt tgtcagttac attatcatct cctttctcat 4980

tgttgtcaac atgtacattg ctgtgatttt agagaacttc aatacagcca ctgaagaaag 5040

tgaggaccct ttgggtgaag atgactttga catattttat gaagtgtggg aaaagtttga 5100

cccagaagca acacaattta tcaaatattc tgccctttct gactttgctg atgccttgcc 5160

tgagcctttg cgtgtcgcaa agccaaataa atatcaattt ctagtaatgg acttgcccat 5220

ggtgagtgaa gatcgcctcc actgcatgga tattcttttc gccttcaccg ctagggtact 5280

cggtggctct gatggcctag atagtatgaa agcaatgatg gaagagaagt tcatggaagc 5340

caatcctctc aagaagttgt atgaacccat agtcaccacc accaagagaa aggaagagga 5400

aagaggtgct gctattattc aaaaggcctt tcgaaagtac atgatgaagg tgaccaaggg 5460

tgaccaaggt gaccaaaatg acttggaaaa cgggcctcat tcaccactcc agactctttg 5520

caatggagac ttgtctagct ttggggtggc caagggcaag gtccactgtg actgagccct 5580

cacctccacg cctacctcat agcttcacag ccttgccttc agcctctgag ctccaggggt 5640

cagcagctta gtgtatcaac agggagtgga ttcaccaaat tagccattcc atttcttttt 5700

ctggctaaaa taaatgatat ttcaatttca ttttaaataa tacttacaga gatataagat 5760

aaggctactt gacaaccagt ggtactatta taataaggaa gaagacacca ggaaggactg 5820

taaaaggaca taccaatttt aggattgaaa tagttcaggc cgggcgcagt ggctcatgcc 5880

tgtaatccca gcactttgag aggccaaggc aggtggatca cgaggtcaag agatcgagac 5940

catcctggcc aacatgatga aactccgtct ctactaaaaa tacaaaaatt agctgggcat 6000

ggtggcgtgc gcctgtagtc ccagctactt gggaggctga ggcaggagaa tcgcttaaac 6060

ctgggagacg gaggttgcag tgagccaaga tcgtgccact gcactccagc ctggtgacag 6120

agtgagactc tgtttcaaaa aagaaaagaa aagaaacatg gttcaaatta tatctaaaca 6180

aaaaagaata agaaacaaaa aacacattaa aattttaagt tgtattttct atgtttctag 6240

atacatcatt tttgtttgat attttcctga tgcaagtatg tggtttatca catgtagctc 6300

ttttgcatgc taaatgaaaa ttcaaaactt gccaataaat gaatagctta ttgcagacat 6360

tttttaccaa cattaattat tttgggtttg tttaaaacct agaggcacaa tcttgacttg 6420

tcaattacta ccctttcaca agctaccatc tcagatatat atatatatat aaattcaata 6480

aagctttctg tttgtgttcc 6500
<210> 11
   <211> 6528
   <212> DNA
   <213> Homo sapiens
<400> 11 
tcctgctata cccacagtgg tggtcatctc ttctgatctt cacagccaat cagctcccaa 60

ggcccctgac ctcagctcag cttttgtaga tccttatgac accatccttt aagactggaa 120

tcctagggca ggctgtttta ttcccgcctc ctgaggcctt tctgaggatc tgtggcttgt 180

ctctgtcctg agggtgaaga tggatgacag atgctaccca gtaatctttc cagatgagcg 240

gaatttccgc cccttcactt ccgactctct ggctgcaatt gagaagcgga ttgccatcca 300

aaaggagaaa aagaagtcta aagaccagac aggagaagta ccccagcctc ggcctcagct 360

tgacctaaag gcctccagga agttgcccaa gctctatggc gacattcctc gtgagctcat 420

aggaaagcct ctggaagact tggacccatt ctaccgaaat cataagacat ttatggtgtt 480

aaacagaaag aggacaatct accgcttcag tgccaagcat gccttgttca tttttgggcc 540

tttcaattca atcagaagtt tagccattag agtctcagtc cattcattgt tcagcatgtt 600

cattatcggc accgttatca tcaactgcgt gttcatggct acagggcctg ctaaaaacag 660

caacagtaac aatactgaca ttgcagagtg tgtcttcact gggatttata tttttgaagc 720

tttgattaaa atattggcaa gaggtttcat tctggatgag ttttctttcc ttcgagatcc 780

atggaactgg ctggactcca ttgtcattgg aatagcgatt gtgtcatata ttccaggaat 840

caccatcaaa ctattgcccc tgcgtacctt ccgtgtgttc agagctttga aagcaatttc 900

agtagtttca cgtctgaagg tcatcgtggg ggccttgcta cgctctgtga agaagctggt 960

caacgtgatt atcctcacct tcttttgcct cagcatcttt gccctggtag gtcagcagct 1020

cttcatggga agtctgaacc tgaaatgcat ctcgagggac tgtaaaaata tcagtaaccc 1080

ggaagcttat gaccattgct ttgaaaagaa agaaaattca cctgaattca aaatgtgtgg 1140

catctggatg ggtaacagtg cctgttccat acaatatgaa tgtaagcaca ccaaaattaa 1200

tcctgactat aattatacga attttgacaa ctttggctgg tcttttcttg ccatgttccg 1260

gctgatgacc caagattcct gggagaagct ttatcaacag accctgcgta ctactgggct 1320

ctactcagtc ttcttcttca ttgtggtcat tttcctgggc tccttctacc tgattaactt 1380

aaccctggct gttgttacca tggcatatga ggagcagaac aagaatgtag ctgcagagat 1440

agaggccaag gaaaagatgt ttcaggaagc ccagcagctg ttaaaggagg aaaaggaggc 1500

tctggttgcc atgggaattg acagaagttc acttacttcc cttgaaacat catattttac 1560

cccaaaaaag agaaagctct ttggtaataa gaaaaggaag tccttctttt tgagagagtc 1620

tgggaaagac cagcctcctg ggtcagattc tgatgaagat tgccaaaaaa agccacagct 1680

cctagagcaa accaaacgac tgtcccagaa tctatcactg gaccactttg atgagcatgg 1740

agatcctctc caaaggcaga gagcactgag tgccgtcagc atcctcacca tcaccatgaa 1800
 
ggaacaagaa aaatcacaag agccttgtct cccttgcgga gaaaacctgg catccaagta 1860

cctcgtgtgg aactgttgcc cccagtggct gtgcgttaag aaggtcctga gaactgtgat 1920

gactgacccg tttactgagc tggccatcac catctgcatc atcatcaaca ctgtcttctt 1980

ggccatggag catcacaaga tggaggccag ttttgagaag atgttgaata tagggaattt 2040

ggttttcact agcattttta tagcagaaat gtgcctaaaa atcattgcgc tcgatcccta 2100

ccactacttt cgccgaggct ggaacatttt tgacagcatt gttgctcttc tgagttttgc 2160

agatgtaatg aactgtgtac ttcaaaagag aagctggcca ttcttgcgtt ccttcagggt 2220

gctcagggtc ttcaagttag ccaaatcctg gccaactttg aacacactaa ttaagataat 2280

cggcaactct gtcggagccc ttggaaacct gactgtggtc ctggtcattg tgatctttat 2340

tttctcagta gttggcatgc agctttttgg ccgtagcttc aattcccaaa agagtccaaa 2400

actctgtaac ccgacaggcc cgacagtctc atgtttacgg cactggcaca tgggggattt 2460

ctggcactcc ttcctagtgg tattccgcat cctctgcggg gaatggatcg aaaatatgtg 2520

ggaatgtatg caagaagcga atgcatcatc atcattgtgt gttattgtct tcatattgat 2580

cacggtgata ggaaaacttg tggtgctcaa cctcttcatt gccttactgc tcaattcctt 2640

tagcaatgag gaaagaaatg gaaacttaga aggagaggcc aggaaaacta aagtccagtt 2700

agcactggat cgattccgcc gggctttttg ttttgtgaga cacactcttg agcatttctg 2760

tcacaagtgg tgcaggaagc aaaacttacc acagcaaaaa gaggtggcag gaggctgtgc 2820

tgcacaaagc aaagacatca ttcccctggt catggagatg aaaaggggct cagagaccca 2880

ggaggagctt ggtatactaa cctctgtacc aaagaccctg ggcgtcaggc atgattggac 2940

ttggttggca ccacttgcgg aggaggaaga tgacgttgaa ttttctggtg aagataatgc 3000

acagcgcatc acacaacctg agcctgaaca acaggcctat gagctccatc aggagaacaa 3060

gaagcccacg agccagagag ttcaaagtgt ggaaattgac atgttctctg aagatgagcc 3120

tcatctgacc atacaggatc cccgaaagaa gtctgatgtt accagtatac tatcagaatg 3180

tagcaccatt gatcttcagg atggctttgg atggttacct gagatggttc ccaaaaagca 3240

accagagaga tgtttgccca aaggctttgg ttgctgcttt ccatgctgta gcgtggacaa 3300

gagaaagcct ccctgggtca tttggtggaa cctgcggaaa acctgctacc aaatagtgaa 3360

acacagctgg tttgagagct ttattatctt tgtgattctg ctgagcagtg gggcactgat 3420

atttgaagat gttcaccttg agaaccaacc caaaatccaa gaattactaa attgtactga 3480

cattattttt acacatattt ttatcctgga gatggtacta aaatgggtag ccttcggatt 3540

tggaaagtat ttcaccagtg cctggtgctg ccttgatttc atcattgtga ttgtctctgt 3600

gaccaccctc attaacttaa tggaattgaa gtccttccgg actctacgag cactgaggcc 3660

tcttcgtgcg ctgtcccagt ttgaaggaat gaaggtggtg gtcaatgctc tcataggtgc 3720

catacctgcc attctgaatg ttttgcttgt ctgcctcatt ttctggctcg tattttgtat 3780
 
tctgggagta tacttctttt ctggaaaatt tgggaaatgc attaatggaa cagactcagt 3840
tataaattat accatcatta caaataaaag tcaatgtgaa agtggcaatt tctcttggat 3900
caaccagaaa gtcaactttg acaatgtggg aaatgcttac ctcgctctgc tgcaagtggc 3960
aacatttaag ggctggatgg atattatata tgcagctgtt gattccacag agaaagaaca 4020
acagccagag tttgagagca attcactcgg ttacatttac ttcgtagtct ttatcatctt 4080
tggctcattc ttcactctga atctcttcat tggcgttatc attgacaact tcaaccaaca 4140
gcagaaaaag ttaggtggcc aagacatttt tatgacagaa gaacagaaga aatactataa 4200
tgcaatgaaa aaattaggat ccaaaaaacc tcaaaaaccc attccacggc ctctgaacaa 4260
atgtcaaggt ctcgtgttcg acatagtcac aagccagatc tttgacatca tcatcataag 4320
tctcattatc ctaaacatga ttagcatgat ggctgaatca tacaaccaac ccaaagccat 4380
gaaatccatc cttgaccatc tcaactgggt ctttgtggtc atctttacgt tagaatgtct 4440
catcaaaatc tttgctttga ggcaatacta cttcaccaat ggctggaatt tatttgactg 4500
tgtggtcgtg cttctttcca ttgttagtac aatgatttct accttggaaa atcaggagca 4560
cattcctttc cctccgacgc tcttcagaat tgtccgcttg gctcggattg gccgaatcct 4620
gaggcttgtc cgggctgcac gaggaatcag gactctcctc tttgctctga tgatgtcgct 4680
tccttctctg ttcaacattg gtcttctact ctttctgatt atgtttatct atgccattct 4740
gggtatgaac tggttttcca aagtgaatcc agagtctgga atcgatgaca tattcaactt 4800
caagactttt gccagcagca tgctctgtct cttccagata agcacatcag caggttggga 4860
ttccctgctc agccccatgc tgcgatcaaa agaatcatgt aactcttcct cagaaaactg 4920
ccacctccct ggcatagcca catcctactt tgtcagttac attatcatct cctttctcat 4980
tgttgtcaac atgtacattg ctgtgatttt agagaacttc aatacagcca ctgaagaaag 5040
tgaggaccct ttgggtgaag atgactttga catattttat gaagtgtggg aaaagtttga 5100
cccagaagca acacaattta tcaaatattc tgccctttct gactttgctg atgccttgcc 5160
tgagcctttg cgtgtcgcaa agccaaataa atatcaattt ctagtaatgg acttgcccat 5220
ggtgagtgaa gatcgcctcc actgcatgga tattcttttc gccttcaccg ctagggtact 5280
cggtggctct gatggcctag atagtatgaa agcaatgatg gaagagaagt tcatggaagc 5340
caatcctctc aagaagttgt atgaacccat agtcaccacc accaagagaa aggaagagga 5400
aagaggtgct gctattattc aaaaggcctt tcgaaagtac atgatgaagg tgaccaaggg 5460
tgaccaaggt gaccaaaatg acttggaaaa cgggcctcat tcaccactcc agactctttg 5520
caatggagac ttgtctagct ttggggtggc caagggcaag gtccactgtg actgagccct 5580
cacctccacg cctacctcat agcttcacag ccttgccctc agcctctgag ctccaggggt 5640
cagcagctta gtgtatcaac agggagtgga ttcaccaaat tagccattcc attttctttt 5700
 
ctggctaaat aaatgatatt tcaatttcat tttaaatgat acttacagag atataagata 5760

aggctacttg acaaccagcg gtactatttt aataaggaag aagacaccag gaaggactgt 5820

aaaaggacat tccaatttta ggattggaat agttcaagcc gggcgcagtg gctcatgcct 5880

ggaatcccag cactttgaga ggccaaggca ggtggatcac gaggtcaaga gatcgagacc 5940

atcctggcca accatgatga aactccgtct ctactaaaat acaaaaatta gctgggcatg 6000

gttgcgtgcg cctgtagtcc cagctacttg ggaggctgag gcaggagaat cgcttaaacc 6060

tgggagacgg aggttgcagt gagccaagat cgtgtcactg cactccagcc tggtgacaga 6120

gtgagactct gtttcaaaaa agaaaagaaa agaaacatgg ttcaaattat atctaaacaa 6180

aaaagaataa gaaacaaaaa acacattaaa attttaagtt gtattttcta tgtttctaga 6240

tacatcattt ttgtttgata ttttcctgat gcaagtatgt ggtttatcac atgtagctct 6300

tttgcatgct aaatgaaaat tcaagacttg ccaataaatg aatagcttat tgcagacatt 6360

ttttaccaac attaattatt ttgggtttgt ttaaaaccta gaggcacaat cttgacttgt 6420

caattactac cctttcacaa gctaccatct cagatatata tatatatata aattcaataa 6480

agctttctgt ttgtgttcca taaaaaaaaa aaaaaaaaaa aaaaaaaa 6528
<210> 12
   <211> 1123
   <212> DNA
   <213> Homo sapiens
<400> 12 
agcggggggt tcccgcccgc gcctctccct ccacacctcc ccgcaagcag agggagccgg 60

ctccggcctt ggccagccca gagacaggct cccacagtgc agcggcgggc tgaagggctc 120

ctcaagcact gccagagtgg acaccgaggc cgaggagacg ccaagagcga tgaaacaagc 180

ttctggttca agagtttctt caggttccac tacgggctgt tcttctacag gtgcgccgat 240

gtccacagtg ctaccttctg atgagctact gctttcattc agtttctgct gacttaatat 300

gtgagctgac agaatgcaga gaccacgttg tatatgaagc atccactttg ccttgtacac 360

cagggcattc aataaccact taataactac aaccctgatg atccgattca ctactaaagt 420

cttccgtgtt taaattttca aagtcagatt ctcctacagc aattggtaca gtcacagtaa 480

gactggggtt gtttatgaat gacatgtaat cactttcatc aataatgtat ttttcaacac 540

tgctgccagt tcctatacca cttgtagttc catttacatc tttaagatag tcaagatctt 600

tcccaatttc tgctgtatga ttggacatac aactgtcttt cttgttgttt agatcatcaa 660

gtggtttaat ttcatctaaa atcttttgtt tcctaatgaa ggactgttga ataaattcat 720

atatttttct tttcacataa gctactcctt tgtgcatcct atccacagca atttggagat 780

tattcatttc attatcatca tcagtggctg caaggttgtc tgcactaaat gagctcagaa 840

gcaaggccag aaagagattc aggaccttaa aaacaacaaa aacatgatta taattttaca 900
 
ccaatgtagg gaagagcaga ttacaatcac ttattctttc ttttaagtgt ggaaaaaact 960

ctaagttcta aaacttgatg agaaggaaac accacagcat agtgattaga agatgggtga 1020

tctgaatttg tgactggctc aatagcacat ccttggacaa agacatgatt tctgttgctc 1080

tcaagttctc ccattcgtaa agtgaaattg aatgagctaa tct 1123
<210> 13
   <211> 2352
   <212> DNA
   <213> Homo sapiens
<400> 13 
gtcttagtcc tctgaatatt ttttctttgt tcagaacctg agaaagatga atgaaattta 60

gtgtttccca tccccgagag acacccagca tccacaactt cacactctga tgccattgtg 120

tatttatgca agaaatacca taactaagat gaggtctcgc tatgttgcct aggctagtct 180

tgaactcctg gcctcaagtg atccttctgc ctcagcatcg ggagtcactg ggattaaagg 240

catgagccac catgctcagg aacttgacat catcagacag taaatcaaga ggtactgaca 300

actgagactg agtgatggtc cctgcctagg atttggaatc tttgcatgaa atagggactc 360

tagccaccag cgacaaagaa gtcagggaga ctgcggtctc taggaagcac ttccaggaaa 420

caggaattca ggcaaagttg gacattccaa agatggcgta gatgaacatg accaggaaga 480

gcaggaggcc gatgttaaac aacgcaggaa gggacatcat caaagcaaag agcagcgtgc 540

ggatcccctt tgctcctttg actagacgta ggattcggcc aatcctggca agacggatca 600

ctcggaacag ggtaacagag atctctggag gtgaaacatt gctctgaact ggcattgatt 660

ctagcccagc tcttgtagac caattaccaa cttgtccatc tccctccagt gcctggaact 720

ggacctggca cacaggcctc tggctcatcg gaattcatag gttcagcctc tgcttcttct 780

ccttctccag gcaaagggtt atcaactgtg ctgcactctg aggagcttga ccggtttaat 840

ctctagaaag gaattcacca ccccaccagc acgcggaaca caatcaggaa ggagtggaag 900

aagtcgttca tgtgccaccg tgggagcgta cagtcatcat tgatcttgca gacacattct 960

ttgtagctct taccaaagag ctgcatgccg accacagcaa aaatgaagac gatgatggcc 1020

aacactaagc ctttagacta aaaagaaaac aaatattaca taccctgaat ctgtgctgaa 1080

accacaaagg agagcatctt tggatccttc caagtaataa aaatattctg ttgaagaaga 1140

atttgaacag ttataacatc acagacttta atctgtgatt gtgataaagg aggtcaaatt 1200

aaaaaatctg attattggga gacttttgga gtaatcatgg aagaaagact atgaaatcag 1260

atattcttgg atttgaatgt tgcttctatc ccttcttaac ttaaatccat gggcattcat 1320

tatcatcagt gcctaacctg gcagaaccac agtctacaca taatctggca tctgtctttt 1380

ccatgctggc taccaaatgg ctgaatgttg gtggagaaac tctcacaacc aagtgaatgg 1440

ttatatttta atttcgctct tgttgcccag tctggagtgc aatggcacga tctcggctca 1500
 
ccgcaacctc tgcctcccga gttcaagcga ttctcctgcc tcagcctcct gagtagctgg 1560

aattacaggc acccaccacc atgcccggct aattttttgt atttttagta gagacggggt 1620

ttcttttttt ttcttctttt tttgtattat tttttagcaa aaaaaaaaaa aaaatacatg 1680

aagcaattcc agaaagcgat gaattaaatt aaatttgatt aaaggctgtc tgtgtacata 1740

gagaattgca atctaacttc atatgtaaac aaattgcatt ctaatctatt agtatattct 1800

tgtaacaagt agctgtctca gccaatcaca gcagctgagc ttcagccaac cacagcctgc 1860

tgatttatca gacaatgtcc acataaggca aatgtcaagc tataagcaat caagctgttt 1920

ttgtgcgtca ctcccttttt ctgtctataa atactcctgc tcatgttgct gagttgagct 1980

ctccgaactt ctcttggttc tgagtgctgc ctaaatcatt attattatta tcttatttca 2040

gcaataaata ctatttcatt gctgaaataa gctgctaaat ttaatttgtt gaaaattttt 2100

cttttaacaa aggttaagaa aaccattcac agtataaact cagaaatagg agcaagactc 2160

agaataaaag gacagatccc agtgagaaaa cttaagttta aataagtgtg gtaaaataat 2220

ctacaatatt aaatgtaagt tactcatcag atatattaat tttacagcat ataaattaag 2280

aaataaaatt ctccatcaat tggaaaaaaa aaaaaaaaaa aaaaaaaaaa aaaaaaaaaa 2340

aaaaaaaaaa aa 2352
<210> 14
   <211> 267
   <212> DNA
   <213> Homo sapiens
<220>
   <221> misc_feature
   <222> (7)..(7)
   <223> n is a, c, g, or t
<220>
   <221> misc_feature
   <222> (166)..(166)
   <223> n is a, c, g, or t
<220>
   <221> misc_feature
   <222> (194)..(194)
   <223> n is a, c, g, or t
<220>
   <221> misc_feature
   <222> (230)..(230)
   <223> n is a, c, g, or t
<400> 14 
gtgcggntcc cctttgctcc tttgactaga cgtaggattc ggccaatcct ggcaagacgg 60

atcactcgga acagggtaac agagatctct ggaggtgaaa cattgctctg aactggcatt 120

gattctagcc cagctcttgt agaccaatta ccaacttgtc catctncctc cagtgcctgg 180

gaactggacc tggnacacag gcaaagggtt atcaactgtg ctgcactctn aggagcttga 240
 ccggtttaat ctctagaaag gaattca 267
<210> 15
   <211> 1080
   <212> DNA
   <213> Homo sapiens
<400> 15 
gagtttaaga agaggttggt gttacatgga gcagcagcac agtcaagcag ggaacagatc 60

acgtaaagcc ctttagagag tgaagtcggc tttttatgct aggtgcaact gggagttctg 120

aacacggcaa agttggacat tccaaagatg gcgtagatga acatgaccag gaagagcagg 180

aggccgatgt taaacaacgc aggaagggac atcatcaaag caaagagcag cgtgcggatc 240

ccctttgctc ctttgactag acgtaggatt cggccaatcc tggcaagacg gatcactcgg 300

aacagggtaa cagagatctc tggaggtgaa acattgctct gaactggcat tgattctagc 360

ccagctcttg tagaccaatt accaacttgt ccatctccct ccagtgcctg gaactggacc 420

tggcacacag gcctctggct catcggaatt cataggttca gcctctgctt cttctccttc 480

tccaggcaaa gggttatcaa ctgtgctgca ctctgaggag cttgaccggt ttaatctcta 540

gaaaggaatt caccacccca ccaggtaaca gagatctctg gaggtgaaac attgctctga 600

actggcattg attctagccc agctcttgta gaccaattac caacttgtcc atctccctcc 660

agtgcctgga actggacctg gcacacaggc aaagggttat caactgtgct gcactctgag 720

gagcttgacc ggtttaatct ctagaaagga attcaccacc ccaccagaca caatgaagat 780

gaggtcattg tgtgggtcca aatccaatat aactggggtc cttataagaa gaaaagaccg 840

agacacacag ggagaaaact acgtgagtac cgggccagag attgggatga tgcagctgca 900

agctgcaagg attgccaaca acctccaaag ttagaaagca gcaaggcagg atacttacta 960

gagcctttag agaaatcatg gccttgctca gaccttgatt tcagacttct agcctccaga 1020

attggcaaaa gaataaattt ctgttgttgt aaaaaaaaaa aaaaaaaaaa aaaaaaaaaa 1080
<210> 16
   <211> 173
   <212> DNA
   <213> Homo sapiens
<400> 16 
tttttttttt tttttttttt tccaattgat ggagaatttt atttcttaat ttatatgctg 60

taaaattaat atatctgatg agtaacttac atttaatatt gtagattatt ttaccacact 120

tatttaaact taagttttct cactgggatc tgtcctttta ttctgagtct tgc 173
<210> 17
   <211> 618
   <212> DNA
   <213> Homo sapiens
<400> 17 
tgtacatgga gcagcagcac agtcaagcag ggaacagatc acgtaaagcc ctttagaaga 60

gtgaagtcgg ctttttatgc taggtgcaac tgggagttct gaacacggca aagttggaca 120

ttccaaagat ggcgtagatg aacatgacca ggaagagcag gaggccgatg ttaaacaacg 180

caggaaggga catcatcaaa gcaaagagca gcgtgcggat cccctttgct cctttgacta 240

gacgtaggat tcggccaatc ctggcaagac ggatcactcg gaacagggta acagagatct 300

ctggaggtga aacattgctc tgaactggca ttgattctag cccagctctt gtaagaccaa 360

ttaccaactt gtccatctcc ctccagtgcc tggaactgga cctggcacac aggcctctgg 420

tcaatcggaa ttcataggtt cagcctctgt tcttctcctt ctccaggcaa gggttatcaa 480

ctgtgctgca ctctgaggag cttgaccggt taaatctcta gaaaggaatt caccacccca 540

caggtaacca gagatctctg aggtgaacat tgtctgaacg gattgttcta gccagtctgt 600

aaccattaca tgtcattc 618
<210> 18
   <211> 871
   <212> DNA
   <213> Homo sapiens
<400> 18 
gaaactggcc ttttataagt aaatcattat gtagtttctt cctttggagg gcagctctgt 60

gtgactgctt gggcctctgt ggttagatct gtttggttaa cgggctttgg gctttcacac 120

aaagctatac tctccacaaa cttctgtgag ataaaaaggt gatatctgtc ttagtcctct 180

gaatattttt tctttgttca gaacctgaga aagatgaatg aaatttagtg tttcccatcc 240

ccgagagaca cccagcatcc acaacttcac actctgatgc cattgtgtat ttatgcaaga 300

aataccataa ctaaggtaat gtcttgagat ttttggttca ataatgtcct taaattctat 360

gtgtttttaa agcctaaaag aaattccccc tcttcactcc cccgtctctc cctacaacct 420

ggtgagacaa ttctctcatt ttctcctccc agtttccaat gctattgtta tgatctatta 480

gaataattgc tattattttt ttggccttat ctgaccctag aagtgattac agggaaaaaa 540

attactaacc aattgattag gggaactgga atataaagtg cagctcttat ggtttattca 600

cagaccaaga atatacatag tacttaatgt gataggtatt tgctcgttga ctgtggaacc 660

aggttagtct ataatacagg tttcaggcag aacagactta tttacctgaa ctctggactt 720

cggaaaaggt gagtaaggta tttaagagaa ctgaccacgt ttgtattcca cccatatagg 780

ggatttaaca gaaattcttc ctctttataa acaacgttcc caaagggctt tgtaaatgtg 840

cccttaaaaa tttgcgcccg tgggggacac t 871
<210> 19
   <211> 304
   <212> DNA
   <213> Homo sapiens
<220>
   <221> misc_feature
   <222> (1)..(1)
   <223> n is a, c, g, or t
<220>
   <221> misc_feature
   <222> (32)..(32)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (51)..(51)
   <223> n is a, c, g, or t
<220>
   <221> misc_feature
   <222> (58)..(58)
   <223> n is a, c, g, or t
<400> 19 
nctttcggag caagattgcg ccatttccaa antaggcgta gnagnaacag naccaggnaa 60

gacaggaggc cgatgttaaa caacgcagga agggacatca tcaaagcaaa gagcagcgtg 120

cggatcccct ttgctccttt gactagacgt aggattcggc caatcctggc aagacggatc 180

actcggaaca gggtagggga aacaaaatac gtttcaatca aatcagctag aaacatacct 240

gtatgtggag gaaaataata gaaataaaat atttaaagat gtatgctacc taaaaaaaaa 300

aaaa 304
<210> 20
   <211> 293
   <212> DNA
   <213> Homo sapiens
<400> 20 
ctggcaaagc tggcattcca agatggcgta gatgaacatg accaggaaga gcaggaggcc 60

gatgttaaac aacgcaggaa gggacgtcat caaagcaaag agcagcgtgc ggatcccctt 120

tgctcctttg actagacgta ggattcggcc aatcctggca agacggatca ctcggaacag 180

ggtaggggaa acaaaatacg tttcaatcaa atcagctaga aacatacctg tatgtggagg 240

aatataattg aaataaaata tttaaagatg tatgctacct aaaaaaaaaa aaa 293
<210> 21
   <211> 892
   <212> DNA
   <213> Homo sapiens
<220>
   <221> misc_feature
   <222> (663)..(663)
   <223> n is a, c, g, or t
<220>
   <221> misc_feature
   <222> (685)..(685)
   <223> n is a, c, g, or t
<220>
   <221> misc_feature
   <222> (784)..(784)
   <223> n is a, c, g, or t
<220>
   <221> misc_feature
   <222> (845)..(845)
   <223> n is a, c, g, or t
<400> 21 
tttctaggcg aagattggca cattccaaag atggcgtagc aggaacatga ccagggaaga 60

gcaggaggcc gatgttaaac aacgcaggaa gggacatcat caaagcaaag agcagcgtgc 120

ggatcccctt tgctcctttg actagacgta ggattcggcc aatcctggca agacggatca 180

ctcggaacag ggtaaccgag atctctggag gtgaaacatt gctctgaact ggcattgatt 240

ctagcccagc tcttgtagac caattaccaa cttgtccatc tccctccagt gcctggaact 300

ggacctggca cacaggcaaa gggttatcaa ctgtgctgca ctctgaggag cttgaccggt 360

ttaatctcta gaaaggagtt caccacccca ccagcacgcg gaacacaatc aggaaggagt 420

ggaagaagtc gttcatgtgc caccgtggga gcgtacagtc atcattgatc ttgcagacac 480

attctttgta gctcttacca aagagctgca tgccgaccac agcaaaaatg aagacgatga 540

tggccaacac taagaagctg tccattgggg agcatgaggg ctgagcgtcc atcaaccagg 600

gagaccacac cgttgcagtc cacagcactg tgcattttcc cgttcaccgg cagcattggt 660

ggngacctac tggcttggct gatgntactg ctgcgtcgct cctggggtct gtggagcaca 720

aacagtgagc cccttctgct ctcattgtct acaagatgct gtgctcatca tcggcaaatt 780

cagnctcaga tcctatatct cttcctcttg cctttgaact aaaaagactt gttctgctgc 840

ttcgncctgc agaaacaagg agcacgatgc tcgagtggga ctgcaaaaaa at 892
<210> 22
   <211> 260
   <212> DNA
   <213> Homo sapiens
<400> 22 
taacattgcc tggaagagca ggaggccgat gtttaacaac gcaggaaggg acatcatcaa 60

agcaaagagc agcgtgcgga tcccctttgc tcctttgact agacgtagga ttcggccaat 120
 
cctggcaaga cggatcactc ggaacagggt aggggaaaca aaatacgttt caatcaaatc 180

agctagaaac atacctgtat gtggaggaaa ataatagaaa taaaatattt aaagatgtat 240

gctacccaaa aaaaaaaaaa 260
<210> 23
   <211> 982
   <212> DNA
   <213> Homo sapiens
<400> 23 
cacgcgtccg gtcttagtcc tctgaatatt ttttctttgt tcagaacctg agaaagatga 60

atgaaattta gtgtttccca tccccgagag acacccagca tccacaactt cacactctga 120

tgccattgtg tatttatgca agaaatacca taactaagat gaggtctcgc tatgttgcct 180

aggctagtct tgaactcctg gcctcaagtg atccttctgc ctcagcatcg ggagtcactg 240

ggattaaagg catgagccac catgctcagg aacttgacat catcagacag taaatcaaga 300

ggtactgaca actgagactg agtgatggtc cctgcctagg atttggaatc tttgcatgaa 360

atagggactc tagccaccag cgacaaagaa gtcagggaga ctgcggtctc taggaagcac 420

ttccaggaaa caggaattca ggcaaagttg gacattccaa agatggcgta gatgaacatg 480

accaggaaga gcaggaggcc gatgttaaac aacgcaggaa gggacatcat caaagcaaag 540

agcagcgtgc ggatcccctt tgctcctttg actagacgta ggattcggcc aatcctggca 600

agacggatca ctcggaacag ggtaacagag atctctggag gtgaaacatt gctctgaact 660

ggcattgatt ctagcccagc tcttgtagac caattaccaa cttgtccatc tccctccagt 720

gcctggaact ggacctggca cacaggcctc tggctcatcg gaattcatag gttcagcctc 780

tgcttcttct ccttctccag gcaaaggggt tatcaactgt gctgcactct gaggagcttg 840

accggtttaa tctctagaaa ggaattcacc acccccccag cacgcggaac acaatcaggg 900

aaggagtggg aggaagtcct tcctgggccc acggggggag acggacaggc attcattgat 960

cttgcagaaa cctttctttg ta 982
<210> 24
   <211> 906
   <212> DNA
   <213> Homo sapiens
<400> 24 
taaggcttgt tcctaagact gggggcagat ctatccttgt atagtaaatg tttaaatgac 60

taaaaaacct tttgtcatct ctcttaaata tctattctac tataagaacc tgacttccta 120

ttttactcag gtcaaattaa aaaatctgat tattgggaga cttttggagt aatcatggaa 180

gaaagactat gaaatcagat attcttggat ttgaatgttg cttctatccc ttcttaagta 240

agtgaattat ctttaagcca ttaagcctta gctgctgcat ctgtaaaata cgtataagaa 300
 
tacctacttt ataaagttgt taaatagtat aaataggata ttgcatgtaa accacttgat 360

actgtgatcc acacattctg tactagctgc tcaaaaacag gagttattat aattagttaa 420

actattatac aatttgagat aaaactattg attgaattac acatcatcat tttatcttac 480

ttagaagaca cagataaaac aataaagcct tgcagtttga ggtgcaaatg taggtgaaaa 540

tgtgcatgaa aagctttcct gaaaacactc ctgaacctcg ggaaggatgg gcctggtaga 600

gtgagtatgg gagaggataa gatgcaattt tgcatacata aacatccaaa atattttaat 660

aaggatggat tactttagaa ttatccttta aactaatgct ggttcacatt tatcaaccac 720

tgaggagctg aattatatga atgggctctg gagctgtcta ttagcacact gctatactac 780

tgtatttaac acagtataag aatgatgaaa ggtaaagcaa tgcaggtagc tcactctagg 840

gttgtagaaa actttacaat aatttctaaa caactagaca taaaatatta agatgggttg 900

gcttca 906
<210> 25
   <211> 476
   <212> DNA
   <213> Mus musculus
<400> 25 
gatctgattc actgctaaag tcttctgtgt ttaagttctc aaagtcagac tctcccacag 60

caatgggcac agtcacagtg aggctggggt tgtttatgaa tgacatataa tcactctcat 120

caatgatgta cttctccaca ctgctgcccg tccctatgcc acttgtggtt ccattcacat 180

ctttcagaca gtccagatct ttcccaattt ctgttgtgtg gttagagata caattgtctt 240

ttctgttgtt tagatcatca agtggcttaa tttcatctag aatcttctgt ttcttaacaa 300

aggattgttg aatgaattca tatatttttc tttttacata agctattcct ttgtgcatcc 360

tgtccacagc aatctgcagg ttgttcatct cattgtcatc atcagtggct gcaaggttgt 420

ctgcactaaa tgagctcaga agcaaggcca gaaagaggtt caagaccttg aaaaca 476
<210> 26
   <211> 185
   <212> DNA
   <213> Mus musculus
<400> 26 
aggcaaagtt ggacatccca aagatggcgt agatgaacat gacgagaaaa agcaggaggc 60

cgatgttaaa cagcgcagga agggacatca tcagagcaaa gagcagcgtg cggatcccct 120

tggcaccttt gatcaggcgt aggattcgtc caatcctggc caggcggatg actcggaaca 180

gggta 185
<210> 27
   <211> 162
   <212> DNA
   <213> Mus musculus
<400> 27 
ccccacacag cacacggaac acgatcagga aggagtggaa gaagtcgttc atgtgccagc 60

gtgggagctt gcagttctcg ttgatcttgc aaacacactc cttgtagctc tttccaaaca 120

gctgcatgcc gaccacggca aaaatgaaga cgatgatggc ca 162
<210> 28
   <211> 94
   <212> DNA
   <213> Mus musculus
<400> 28 
ttacataccc tgaatctgtg ctgaaaccgc aaagaagagc atctttggat ccctccaagt 60

aataaaaata tcctgtttag gaagaatttg aaca 94
<210> 29
   <211> 36
   <212> DNA
   <213> Artificial Sequence
<220>
   <223> Antisense oligonucleotide
<400> 29
   mcmcmumamu mctttccccc ccctmamcmc mumumu 36
<210> 30
   <211> 20
   <212> DNA
   <213> Artificial Sequence
<220>
   <223> Antisense oligonucleotide
<400> 30
   tcggtgtcca ctctggcagt 20
<210> 31
   <211> 20
   <212> DNA
   <213> Artificial Sequence
<220>
   <223> Antisense oligonucleotide
<400> 31
   tgcactgtgg gagcctgtct 20
<210> 32
   <211> 20
   <212> DNA
   <213> Artificial Sequence
<220>
   <223> Antisense oligonucleotide
<400> 32
   gtagcactgt ggacatcggc 20
<210> 33
   <211> 21
   <212> DNA
   <213> Artificial Sequence
<220>
   <223> Antisense oligonucleotide
<400> 33
   gtagaagaac agcccgtagt g 21
<210> 34
   <211> 20
   <212> DNA
   <213> Artificial Sequence
<220>
   <223> Antisense oligonucleotide
<400> 34
   gtggtctctg cattctgtca 20
<210> 35
   <211> 21
   <212> DNA
   <213> Artificial Sequence
<220>
   <223> Antisense oligonucleotide
<400> 35
   gtggtatagg aactggcagc a 21
<210> 36
   <211> 19
   <212> DNA
   <213> Artificial Sequence
<220>
   <223> Antisense oligonucleotide
<400> 36
   gtccaatcat acagcagaa 19
<210> 37
   <211> 20
   <212> DNA
   <213> Artificial Sequence
<220>
   <223> Antisense oligonucleotide
<400> 37
   gtgactgtac caattgctgt 20
<210> 38
   <211> 19
   <212> DNA
   <213> Artificial Sequence
<220>
   <223> Antisense oligonucleotide
<400> 38
   acttcttcca ctccttcct 19
<210> 39
   <211> 20
   <212> DNA
   <213> Artificial Sequence
<220>
   <223> Antisense oligonucleotide
<400> 39
   gatgtccctt cctgcgttgt 20
<210> 40
   <211> 21
   <212> DNA
   <213> Artificial Sequence
<220>
   <223> Antisense oligonucleotide
<400> 40
   tgtggatgct gggtgtctct c 21
<210> 41
   <211> 20
   <212> DNA
   <213> Artificial Sequence
<220>
   <223> Antisense oligonucleotide
<400> 41
   tcccagtgac tcccgatgct 20
<210> 42
   <211> 21
   <212> DNA
   <213> Artificial Sequence
<220>
   <223> Antisense oligonucleotide
<400> 42
   agtctcagtt gtcagtacct c 21
<210> 43
   <211> 20
   <212> DNA
   <213> Artificial Sequence
<220>
   <223> Antisense oligonucleotide
<400> 43
   gttattgaat gccctggtgt 20
<210> 44
   <211> 21
   <212> DNA
   <213> Artificial Sequence
<220>
   <223> Antisense oligonucleotide
<400> 44
   tcggatcatc agggttgtag t 21
<210> 45
   <211> 21
   <212> DNA
   <213> Artificial Sequence
<220>
   <223> Antisense oligonucleotide
<400> 45
   gtggtatagg aactggcagc a 21
<210> 46
   <211> 20
   <212> DNA
   <213> Artificial Sequence
<220>
   <223> Antisense oligonucleotide
<400> 46
   tctgctcttc cctacattgg 20
<210> 47
   <211> 19
   <212> DNA
   <213> Artificial Sequence
<220>
   <223> Antisense oligonucleotide
<400> 47
   gtaatctgct cttccctac 19
<210> 48
   <211> 21
   <212> DNA
   <213> Artificial Sequence
<220>
   <223> Antisense oligonucleotide
<400> 48
   gggagaactt gagagcaaca g 21
<210> 49
   <211> 21
   <212> DNA
   <213> Artificial Sequence
<220>
   <223> Antisense oligonucleotide
<400> 49
   gccagtcaca aattcagatc a 21
<210> 50
   <211> 15
   <212> DNA
   <213> Artificial Sequence
<220>
   <223> Antisense oligonucleotide
<400> 50
   gtataggaac tggca 15
<210> 51
   <211> 15
   <212> DNA
   <213> Artificial Sequence
<220>
   <223> Antisense oligonucleotide
<400> 51
   gtggtatagg aactg 15
<210> 52
   <211> 31
   <212> DNA
   <213> Artificial Sequence
<220>
   <223> Antisense oligonucleotide
<400> 52
   mgmumggmua muaggaamct ggmcamgmcm a 31
<210> 53
   <211> 15
   <212> DNA
   <213> Artificial Sequence
<220>
   <223> Antisense oligonucleotide
<400> 53
   agaacttgag agcaa 15
<210> 54
   <211> 30
   <212> DNA
   <213> Artificial Sequence
<220>
   <223> Antisense oligonucleotide
<400> 54
   mgmgmgagaa mctmugagag mcaamcmamg 30
<210> 55
   <211> 15
   <212> DNA
   <213> Artificial Sequence
<220>
   <223> Antisense oligonucleotide
<400> 55
   gccagtcaca aattc 15
<210> 56
   <211> 15
   <212> DNA
   <213> Artificial Sequence
<220>
   <223> Antisense oligonucleotide
<400> 56
   cacaaattca gatca 15
<210> 57
   <211> 30
   <212> DNA
   <213> Artificial Sequence
<220>
   <223> Antisense oligonucleotide
<400> 57
   mgmcmcagtm cacaaamutm cagamumcma 30
<210> 58
   <211> 54
   <212> DNA
   <213> Artificial Sequence
<220>
   <223> Antisense oligonucleotide
<400> 58
   rarururura rararcrarc rgrgrararg rarcrururu rargrurarg rurg 54
<210> 59
   <211> 54
   <212> DNA
   <213> Artificial Sequence
<220>
   <223> Antisense oligonucleotide
<400> 59
   rurcrarcra rararururc rargrarurc rarcrcrcra rurcrururc rura 54
<210> 60
   <211> 15
   <212> DNA
   <213> Artificial Sequence
<220>
   <223> Antisense oligonucleotide
<400> 60
   gtggtatagg aactg 15
<210> 61
   <211> 31
   <212> DNA
   <213> Artificial Sequence
<220>
   <223> Antisense oligonucleotide
<400> 61
   mgmumggmua muaggaamct ggmcamgmcm a 31
<210> 62
   <211> 15
   <212> DNA
   <213> Artificial Sequence
<220>
   <223> Antisense oligonucleotide
<400> 62
   gccagtcaca aattc 15
<210> 63
   <211> 15
   <212> DNA
   <213> Artificial Sequence
<220>
   <223> Antisense oligonucleotide
<400> 63
   cacaaattca gatca 15
<210> 64
   <211> 37
   <212> DNA
   <213> Artificial Sequence
<220>
   <223> Antisense oligonucleotide
<400> 64
   mgmgmuamua ggmaacmugg mcagmcagmu gmumumg 37
<210> 65
   <211> 32
   <212> DNA
   <213> Artificial Sequence
<220>
   <223> Antisense oligonucleotide
<400> 65
   mumgmgtamu agmgaamctg gmcagcmamg mu 32
<210> 66
   <211> 31
   <212> DNA
   <213> Artificial Sequence
<220>
   <223> Antisense oligonucleotide
<400> 66
   mggtamuagg aamctggmca gmcagtgttm g 31
<210> 67
   <211> 32
   <212> DNA
   <213> Artificial Sequence
<220>
   <223> Antisense oligonucleotide
<400> 67
   mamagmcggm uataggaamc tggmcagmca mg 32
<210> 68
   <211> 21
   <212> DNA
   <213> Artificial Sequence
<220>
   <223> Antisense oligonucleotide
<400> 68
   gtggcatagg gacgggcagc a 21
<210> 69
   <211> 32
   <212> DNA
   <213> Artificial Sequence
<220>
   <223> Antisense oligonucleotide
<400> 69
   mgmumggmca muagmggamc gggmcamgmc ma 32
<210> 70
   <211> 36
   <212> DNA
   <213> Artificial Sequence
<220>
   <223> Antisense oligonucleotide
<400> 70
   mgmamgccag mucamcaaam utcagmatca mcmcmc 36
<210> 71
   <211> 29
   <212> DNA
   <213> Artificial Sequence
<220>
   <223> Antisense oligonucleotide
<400> 71
   maamugggag aamcmumuga gagmcmama 29
<210> 72
   <211> 37
   <212> DNA
   <213> Artificial Sequence
<220>
   <223> Antisense oligonucleotide
<400> 72
   mamcmamamg muggcatagg gacggmgmcm amgmcma 37
<210> 73
   <211> 34
   <212> DNA
   <213> Artificial Sequence
<220>
   <223> Antisense oligonucleotide
<400> 73
   mamcaagmug gmcatamggg amcgggmcag mcma 34
<210> 74
   <211> 34
   <212> DNA
   <213> Artificial Sequence
<220>
   <223> Antisense oligonucleotide
<400> 74
   maagmuggmc amuagmggam cgggmcagmc agmu 34
<210> 75
   <211> 35
   <212> DNA
   <213> Artificial Sequence
<220>
   <223> Antisense oligonucleotide
<400> 75
   mamamgmumg gcatagggac gggcamgmcm amgmu 35
<210> 76
   <211> 19
   <212> DNA
   <213> Artificial Sequence
<220>
   <223> Antisense oligonucleotide
<400> 76
   gtgactgtgc ccattgctg 19
<210> 77
   <211> 19
   <212> DNA
   <213> Artificial Sequence
<220>
   <223> Antisense oligonucleotide
<400> 77
   gccacttgat gatctaaac 19
<210> 78
   <211> 21
   <212> DNA
   <213> Artificial Sequence
<220>
   <223> Antisense oligonucleotide
<400> 78
   gtggacagga tgcacaaagg a 21
<210> 79
   <211> 23
   <212> DNA
   <213> Artificial Sequence
<220>
   <223> Antisense oligonucleotide
<400> 79
   mgtgacmugt gccmcattgc tmg 23
<210> 80
   <211> 21
   <212> DNA
   <213> Artificial Sequence
<220>
   <223> Antisense oligonucleotide
<400> 80
   mgtgactgtg cccattgctm g 21
<210> 81
   <211> 26
   <212> DNA
   <213> Artificial Sequence
<220>
   <223> Antisense oligonucleotide
<400> 81
   mcctcmuttc muggcmcttg mcttmc 26
<210> 82
   <211> 32
   <212> DNA
   <213> Artificial Sequence
<220>
   <223> Antisense oligonucleotide
<400> 82
   mgacaamcct tgmcagccam ctgamugatg ma 32
<210> 83
   <211> 20
   <212> DNA
   <213> Artificial Sequence
<220>
   <223> Antisense oligonucleotide
<400> 83
   tggtatagga actggcagca 20
<210> 84
   <211> 29
   <212> DNA
   <213> Artificial Sequence
<220>
   <223> Antisense oligonucleotide
<400> 84
   mumggmuamu aggaamctgg mcamgmcma 29
<210> 85
   <211> 28
   <212> DNA
   <213> Artificial Sequence
<220>
   <223> Antisense oligonucleotide
<400> 85
   mcmcagtmca caaamutmca gamumcma 28
<210> 86
   <211> 29
   <212> DNA
   <213> Artificial Sequence
<220>
   <223> Antisense oligonucleotide
<400> 86
   mumggmuamu aggaamctgg mcamgmcma 29
<210> 87
   <211> 34
   <212> DNA
   <213> Artificial Sequence
<220>
   <223> Antisense oligonucleotide
<400> 87
   mamgccagmu camcaaamut cagmatcamc mcmc 34
<210> 88
   <211> 24
   <212> DNA
   <213> Artificial Sequence
<220>
   <223> Antisense oligonucleotide
<400> 88
   mgmccagmuc amcaaamutc mamg 24
<210> 89
   <211> 22
   <212> DNA
   <213> Artificial Sequence
<220>
   <223> Antisense oligonucleotide
<400> 89
   mgmccagmuc amcaaamumu mc 22
<210> 90
   <211> 19
   <212> DNA
   <213> Artificial Sequence
<220>
   <223> Antisense oligonucleotide
<400> 90
   gccagmucam caamamutc 19
<210> 91
   <211> 15
   <212> DNA
   <213> Artificial Sequence
<220>
   <223> Antisense oligonucleotide
<400> 91
   gccagtcaca aattc 15
<210> 92
   <211> 17
   <212> DNA
   <213> Artificial Sequence
<220>
   <223> Antisense oligonucleotide
<400> 92
   gcmcagmuca mcamaat 17
<210> 93
   <211> 13
   <212> DNA
   <213> Artificial Sequence
<220>
   <223> Antisense oligonucleotide
<400> 93
   gccagtcaca aat 13
<210> 94
   <211> 11
   <212> DNA
   <213> Artificial Sequence
<220>
   <223> Antisense oligonucleotide
<400> 94
   gccagtcaca a 11
<210> 95
   <211> 48
   <212> DNA
   <213> Artificial Sequence
<220>
   <223> Reverse complement of the antisense oligonucleotide SEQ ID NO: 48
<400> 95
   rcrurarcru rararargru rcrururcrc rgrurgruru ruraraat 48
<210> 96
   <211> 48
   <212> DNA
   <213> Artificial Sequence
<220>
   <223> Reverse complement of the antisense oligonucleotide SEQ ID NO: 49
<400> 96
   rgrarargra rurgrgrgru rgrarurcru rgrararuru rurgruga 48

## Claims

1. An oligonucleotide for use as a therapeutic compound, wherein said oligonucleotide increases the expression of Sodium channel, voltage-gated, type I, alpha subunit (SCN1A), and wherein said oligonucleotide:
a. targets a Sodium channel, voltage-gated, type I, alpha subunit (SCN1A) natural antisense transcript, wherein the natural antisense transcript has the nucleic acid sequence as set forth in any one of SEQ ID NOs: 12 to 15 or 17 to 28, or a variant thereof which retains the function of the natural antisense transcript of any one of SEQ ID NOs: 12 to 15 or 17 to 28; or
b. is 10-30 nucleotides in length and binds to a reverse complement of SEQ ID NO: 16.

2. An oligonucleotide for use in the prevention or treatment of a Sodium channel, voltage-gated, type I, alpha subunit (SCN1A) associated disease or disorder, wherein said oligonucleotide increases the expression of Sodium channel, voltage-gated, type I, alpha subunit (SCN1A), and wherein said oligonucleotide:
a. targets a Sodium channel, voltage-gated, type I, alpha subunit (SCN1A) natural antisense transcript, wherein the natural antisense transcript has the nucleic acid sequence as set forth in any one of SEQ ID NOs: 12 to 15 or 17 to 28, or a variant thereof which retains the function of the natural antisense transcript of any one of SEQ ID NOs: 12 to 15 or 17 to 28; or
b. is 10-30 nucleotides in length and binds to a reverse complement of SEQ ID NO: 16.

3. Use of an oligonucleotide for the manufacture of a medicament for the prevention or treatment of a Sodium channel, voltage-gated, type I, alpha subunit (SCN1A) associated disease or disorder, wherein said oligonucleotide increases the expression of Sodium channel, voltage-gated, type I, alpha subunit (SCN1A), and wherein said oligonucleotide:
a. targets a Sodium channel, voltage-gated, type I, alpha subunit (SCN1A) natural antisense transcript, wherein the natural antisense transcript has the nucleic acid sequence as set forth in any one of SEQ ID NOs: 12 to 15 or 17 to 28, or a variant thereof which retains the function of the natural antisense transcript of any one of SEQ ID NOs: 12 to 15 or 17 to 28; or
b. is 10-30 nucleotides in length and binds to a reverse complement of SEQ ID NO: 16.

4. Use according to Claim 3, or an oligonucleotide for use according to Claim 1 or 2, wherein the disease or disorder is selected from the group consisting of a disease or disorder associated with abnormal function and/or expression of SCNA, Severe myoclonic epilepsy of infancy (SMEI or Dravet's syndrome), a neurological disease or disorder, convulsion, pain (including chronic pain), impaired electrical excitability involving sodium channel dysfunction, a disease or disorder associated with sodium channel dysfunction, a disease or disorder associated with misregulation of voltage-gated sodium channel alpha subunit activity (e.g., paralysis, hyperkalemic periodic paralysis, paramyotonia congenita, potassium-aggravated myotonia, long Q-T syndrome 3, motor endplate disease, ataxia etc.), a gastrointestinal tract disease due to dysfunction of the enteric nervous system (e.g., colitis, ileitis, inflammatory bowel syndrome etc.), a cardiovascular disease or disorder (e.g., hypertension, congestive heart failure etc.); a disease or disorder of the genitourinary tract involving sympathetic and parasympathetic innervation (e.g., benign prostrate hyperplasia, impotence); a disease or disorder associated with neuromuscular system (e.g., muscular dystrophy, multiple sclerosis, epilepsy, autism, migraine (e.g., Sporadic and familial hemiplegic migraines etc.), Generalised epilepsy with febrile seizure plus (GEFS+) etc.) and SCNA-related seizure disorders.

5. An *in vitro* method of increasing the expression of Sodium channel, voltage-gated, type I, alpha subunit (SCN1A) in patient cells or tissues comprising:
contacting said cells or tissues with an oligonucleotide which:
a. targets a Sodium channel, voltage-gated, type I, alpha subunit (SCN1A) natural antisense transcript; and wherein the natural antisense transcript has the nucleic acid sequence as set forth in any one of SEQ ID NOs: 12 to 15 or 17 to 28, or a variant thereof which retains the function of the natural antisense transcript of any one of SEQ ID NOs: 12 to 15 or 17 to 28; or
b. is 10-30 nucleotides in length and binds to a reverse complement of SEQ ID NO: 16;
thereby increasing the expression of Sodium channel, voltage-gated, type I, alpha subunit (SCN1A),

6. Use according to Claim 3 or 4, or an oligonucleotide for use according to any one of Claims 1, 2 or 4, or a method according to Claim 5, wherein the oligonucleotide is single stranded.

7. Use according to Claim 3 or 4, or an oligonucleotide for use according to any one of Claims 1, 2 or 4, or a method according to Claim 5, wherein the oligonucleotide is an siRNA compound.

8. Use according to any one of Claims 3, 4, 6 or 7, or an oligonucleotide for use according to any one of Claims 1, 2, 4, 6 or 7, or a method according to any one of Claims 5 to 7, wherein the oligonucleotide comprises at least one of SEQ ID NOs: 70, 93, 45, 57, 52, 40, 51, 47, 49, 48, 90 to 92, 94, 87, 69, 78, 82, 79, 61 to 63, 84, 30, 33, 35, 37, 46, 50, 55, 56, 59, 67, 76, 77 or 83.

9. Use according to any one of Claims 3, 4 or 6 to 8, or an oligonucleotide for use according to any one of Claims 1, 2, 4 or 6 to 8, or a method according to any one of Claims 5 to 8, wherein the expression of Sodium channel, voltage-gated, type I, alpha subunit (SCN1A) is increased by at least 10%.

10. Use according to any one of Claims 3, 4 or 6 to 9, or an oligonucleotide for use according to any one of Claims 1, 2, 4 or 6 to 9, or a method according to any one of Claims 3 to 9, wherein the oligonucleotide further comprises one or more modifications comprising:
a. at least one modified internucleoside linkage selected from: a phosphorothioate, alkylphosphonate, phosphorodithioate, alkylphosphonothioate, phosphoramidate, carbamate, carbonate, phosphate triester, acetamidate, carboxymethyl ester, and combinations thereof;
b. at least one modified nucleotide selected from: a peptide nucleic acid (PNA), a locked nucleic acid (LNA), an arabino-nucleic acid, an analogue, a derivative, and combinations thereof; or
c. at least one modified sugar moiety selected from: a 2'-O-methoxyethyl modified sugar moiety, a 2'-methoxy modified sugar moiety, a 2'-O-alkyl modified sugar moiety, a bicyclic sugar moiety, a 2'-fluoro moiety, and combinations thereof.

11. An oligonucleotide that:
a. is hybridisable to a natural antisense transcript that has the nucleic acid sequence as set forth in any one of SEQ ID NOs: 12 or 23, or a variant thereof which retains the function of the natural antisense transcript of any one of SEQ ID NOs: 12 or 23 wherein optionally the oligonucleotide is between 10 to 30 nucleotides in length; or
b. has at least 80% complementarity to a target region within a natural antisense transcript that has the nucleic acid sequence as set forth in any one of SEQ ID NOs: 14 or 24, or a variant thereof which retains the function of the natural antisense transcript of any of any one of SEQ ID NOs: 14 or 24, wherein optionally the oligonucleotide is between 10 to 30 nucleotides in length; or
c. is hybridisable to a natural antisense transcript that has the nucleic acid sequence as set forth in any one of SEQ ID NOs: 13 and 18, or a variant thereof which retains the function of the natural antisense transcript of any one of SEQ ID NOs: 13 or 18, wherein the oligonucleotide is between 10 to 30 nucleotides in length; or
d. is 10 to 30 nucleotides in length and is hybridisable to a reverse complement of SEQ ID NO: 16; or
e. comprises at least one of SEQ ID NOs: 93, 45, 57, 52, 40, 51, 47, 49, 48, 90 to 92, 94, 87, 69, 78, 82, 79, 61 to 63, 84, 30, 33, 35, 37, 46, 50, 55, 56, 59, 67, 70, 76, 77, 83, wherein optionally the oligonucleotide is between 10 to 30 nucleotides in length;
wherein said oligonucleotide increases the expression of Sodium channel, voltage-gated, type I, alpha subunit (SCN1A).

12. An oligonucleotide according to Claim 11, wherein the oligonucleotide is single stranded or an siRNA compound.

13. An oligonucleotide according to Claim 11 or Claim 12, wherein the oligonucleotide has at least 90% sequence identity to SEQ ID NO: 16.

14. An oligonucleotide according to any one of Claims 11 to 13, which further comprises one or more modifications comprising:
a. at least one modified internucleoside linkage selected from: a phosphorothioate, alkylphosphonate, phosphorodithioate, alkylphosphonothioate, phosphoramidate, carbamate, carbonate, phosphate triester, acetamidate, carboxymethyl ester, and combinations thereof;
b. at least one modified nucleotide selected from: a peptide nucleic acid (PNA), a locked nucleic acid (LNA), an arabino-nucleic acid, an analogue, a derivative, and combinations thereof; or
c. at least one modified sugar moiety selected from: a 2'-O-methoxyethyl modified sugar moiety, a 2'-methoxy modified sugar moiety, a 2'-O-alkyl modified sugar moiety, a bicyclic sugar moiety, a 2'-fluoro moiety and combinations thereof.

15. A pharmaceutical composition comprising at least one oligonucleotide that is:
a. hybridisable to a natural antisense transcript that has the nucleic acid sequence as set forth in any one of SEQ ID NOs: 12 to 15, 18 to 24 or a variant thereof which retains the function of the natural antisense transcript of any one of SEQ ID NOs: 12 to 15, 18 to 24, wherein said oligonucleotide increases the expression of SCN1A; or
b. 10 to 30 nucleotides in length and is hybridisable to a reverse complement of SEQ ID NO: 16, wherein said oligonucleotide increases the expression of SCN1A;
and a pharmaceutically acceptable excipient; wherein optionally the at least one oligonucleotide is an oligonucleotide according to any one of Claims 11 to 14.

## Patentansprüche

1. Oligonukleotid zur Verwendung als eine therapeutische Verbindung, wobei das genannte Oligonukleotid die Expression von SCN1A (Natriumkanal, spannungsabhängig, Typ I, Alpha-Untereinheit) steigert und wobei das genannte Oligonukleotid:
a. ein natürliches Antisense-Transkript von SCN1A (Natriumkanal, spannungsabhängig, Typ I, Alpha-Untereinheit) targetiert, wobei das natürliche Antisense-Transkript die in einer von SEQ ID NOs: 12 bis 15 oder 17 bis 28 dargelegte Nukleinsäureresequenz oder eine Variante davon, welche die Funktion des natürlichen Antisense-Transkripts von einer von SEQ ID NOs: 12 bis 15 oder 17 bis 28 beibehält, aufweist; oder
b. 10-30 Nukleotide lang ist und an ein reverses Komplement von SEQ ID NO: 16 bindet.

2. Oligonukleotid zur Verwendung bei der Prävention oder Behandlung einer mit SCN1A (Natriumkanal, spannungsabhängig, Typ I, Alpha-Untereinheit) assoziierten Erkrankung oder Störung, wobei das genannte Oligonukleotid die Expression von SCN1A (Natriumkanal, spannungsabhängig, Typ I, Alpha-Untereinheit) steigert und wobei das genannte Oligonukleotid:
a. ein natürliches Antisense-Transkript von SCN1A (Natriumkanal, spannungsabhängig, Typ I, Alpha-Untereinheit) targetiert, wobei das natürliche Antisense-Transkript die in einer von SEQ ID NOs: 12 bis 15 oder 17 bis 28 dargelegte Nukleinsäureresequenz oder eine Variante davon, welche die Funktion des natürlichen Antisense-Transkripts von einer von SEQ ID NOs: 12 bis 15 oder 17 bis 28 beibehält, aufweist; oder
b. 10-30 Nukleotide lang ist und an ein reverses Komplement von SEQ ID NO: 16 bindet.

3. Verwendung eines Oligonukleotids zur Herstellung eines Arzneimittels für die Prävention oder Behandlung einer mit SCN1A (Natriumkanal, spannungsabhängig, Typ I, Alpha-Untereinheit) assoziierten Erkrankung oder Störung, wobei das genannte Oligonukleotid die Expression von SCN1A (Natriumkanal, spannungsabhängig, Typ I, Alpha-Untereinheit) steigert und wobei das genannte Oligonukleotid:
a. ein natürliches Antisense-Transkript von SCN1A (Natriumkanal, spannungsabhängig, Typ I, Alpha-Untereinheit) targetiert, wobei das natürliche Antisense-Transkript die in einer von SEQ ID NOs: 12 bis 15 oder 17 bis 28 dargelegte Nukleinsäureresequenz oder eine Variante davon, welche die Funktion des natürlichen Antisense-Transkripts von einer von SEQ ID NOs: 12 bis 15 oder 17 bis 28 beibehält, aufweist; oder
b. 10-30 Nukleotide lang ist und an ein reverses Komplement von SEQ ID NO: 16 bindet.

4. Verwendung nach Anspruch 3 oder Oligonukleotid zur Verwendung nach Anspruch 1 oder 2, wobei die Erkrankung oder Störung ausgewählt ist aus der Gruppe, bestehend aus einer Erkrankung oder Störung, die mit einer abnormen Funktion und/oder Expression von SCNA assoziiert ist, schwerer myoklonischen Epilepsie des Kleinkindalters (SMEI oder Dravet-Syndrom), einer neurologischen Erkrankung oder Störung, Konvulsion, Schmerzen (einschließlich chronischer Schmerzen), gestörter elektrischer Erregungsbildung unter Beteiligung einer Natriumkanalfunktionsstörung, einer Erkrankung oder Störung, die mit einer Natriumkanalfunktionsstörung assoziiert ist, einer Erkrankung oder Störung, die mit einer Fehlregulation der Aktivität der Alpha-Untereinheit des spannungsabhängigen Natriumkanals assoziiert ist (z. B. Paralyse, hyperkaliämische periodische Paralyse, Paramyotonia congenita, kaliumsensitive Myotonie, Lang-Q-T-Syndrom 3, Krankheit der motorischen Endplatten, Ataxie usw.), einer durch Funktionsstörung des enterischen Nervensystems bedingten Erkrankung des Magen-Darm-Trakts (z. B. Kolitis, Ileitis, Reizdarmsyndrom usw.), einer kardiovaskulären Erkrankung oder Störung (z. B. Hypertonie, Herzinsuffizienz usw.); einer Erkrankung oder Störung der Urogenitaltrakts unter Beteiligung der sympathetischen und parasympathischen Innervation (z. B. benigne Prostatahyperplasie, Impotenz); einer mit dem neuromuskulären System assoziierten Erkrankung oder Störung (z. B. Muskeldystrophie, multiple Sklerose, Epilepsie, Autismus, Migräne (z. B. sporadische und familiäre hemiplegische Migränen usw.), generalisierte Epilepsie mit Fieberanfall plus (GEFS+) usw.) und mit SCNA verbundenen Anfallsstörungen.

5. In-vitro-Verfahren zur Steigerung der Expression von SCN1A (Natriumkanal, spannungsabhängig, Typ I, Alpha-Untereinheit) in Patientenzellen oder -geweben umfassend:
In-Kontakt-Bringen der genannten Zellen oder Gewebe mit einem Oligonukleotid, das:
a. ein natürliches Antisense-Transkript von SCN1A (Natriumkanal, spannungsabhängig, Typ I, Alpha-Untereinheit) targetiert; und wobei das natürliche Antisense-Transkript die in einer von SEQ ID NOs: 12 bis 15 oder 17 bis 28 dargelegte Nukleinsäureresequenz oder eine Variante davon, welche die Funktion des natürlichen Antisense-Transkripts von einer von SEQ ID NOs: 12 bis 15 oder 17 bis 28 beibehält, aufweist; oder
b. 10-30 Nukleotide lang ist und an ein reverses Komplement von SEQ ID NO: 16 bindet;
wobei auf diese Weise die Expression von SCN1A (Natriumkanal, spannungsabhängig, Typ I, Alpha-Untereinheit) gesteigert wird.

6. Verwendung nach Anspruch 3 oder 4 oder Oligonukleotid zur Verwendung nach einem der Ansprüche 1, 2 oder 4 oder Verfahren nach Anspruch 5, wobei das Oligonukleotid einzelsträngig ist.

7. Verwendung nach Anspruch 3 oder 4 oder Oligonukleotid zur Verwendung nach einem der Ansprüche 1, 2 oder 4 oder Verfahren nach Anspruch 5, wobei das Oligonukleotid eine siRNA-Verbindung ist.

8. Verwendung nach einem der Ansprüche 3, 4, 6 oder 7 oder Oligonukleotid zur Verwendung nach einem der Ansprüche 1, 2, 4, 6 oder 7 oder Verfahren nach einem der Ansprüche 5 bis 7, wobei das Oligonukleotid mindestens eine der folgenden SEQ ID NOs umfasst: 70, 93, 45, 57, 52, 40, 51, 47, 49, 48, 90 bis 92, 94, 87, 69, 78, 82, 79, 61 bis 63, 84, 30, 33, 35, 37, 46, 50, 55, 56, 59, 67, 76, 77 oder 83.

9. Verwendung nach einem der Ansprüche 3, 4 oder 6 bis 8 oder Oligonukleotid zur Verwendung nach einem der Ansprüche 1, 2, 4 oder 6 bis 8 oder Verfahren nach einem der Ansprüche 5 bis 8, wobei die Expression von SCN1A (Natriumkanal, spannungsabhängig, Typ I, Alpha-Untereinheit) um mindestens 10 % gesteigert wird.

10. Verwendung nach einem der Ansprüche 3, 4 oder 6 bis 9 oder Oligonukleotid zur Verwendung nach einem der Ansprüche 1, 2, 4 oder 6 bis 9 oder Verfahren nach einem der Ansprüche 3 bis 9, wobei das Oligonukleotid ferner eine oder mehr Modifikation(en) umfasst, umfassend:
a. mindestens eine modifizierte Internukleosid-Verknüpfung, ausgewählt aus: einem Phosphorothioat, Alkylphosphonat, Phosphorodithioat, Alkylphosphonothioat, Phosphoramidat, Carbamat, Carbonat, Phosphattriester, Acetamidat, Carboxymethylester und Kombinationen davon;
b. mindestens ein modifiziertes Nukleotid, ausgewählt aus: einer Peptidnukleinsäure (PNA), einer verbrückten Nukleinsäure (locked nucleic acid, LNA), einer Arabinonukleinsäure, einem Analogon, einem Derivat und Kombinationen davon; oder
c. mindestens einen modifizierten Zuckerteil, ausgewählt aus: einem 2'-O-Methoxyethyl-modifizierten Zuckerteil, einem 2'-Methoxy-modifizierten Zuckerteil, einem 2'-O-Alkyl-modifizierten Zuckerteil, einem bicyclischen Zuckerteil, einem 2'-Fluoroteil und Kombinationen davon.

11. Oligonukleotid, das:
a. an ein natürliches Antisense-Transkript hybridisierbar ist, das die in einer von SEQ ID NOs: 12 oder 23 dargelegte Nukleinsäureresequenz oder eine Variante davon, welche die Funktion des natürlichen Antisense-Transkripts von einer von SEQ ID NOs: 12 oder 23 beibehält, aufweist, wobei das Oligonukleotid optional zwischen 10 bis 30 Nukleotide lang ist; oder
b. eine mindestens 80%ige Komplementarität mit einer Zielregion innerhalb eines natürlichen Antisense-Transkripts aufweist, das die in einer von SEQ ID NOs: 14 oder 24 dargelegte Nukleinsäureresequenz oder eine Variante davon, welche die Funktion des natürlichen Antisense-Transkripts nach einer von SEQ ID NOs: 14 oder 24 beibehält, aufweist, und wobei das Oligonukleotid optional zwischen 10 bis 30 Nukleotide lang ist; oder
c. an ein natürliches Antisense-Transkript hybridisierbar ist, das die in einer von SEQ ID NOs: 13 und 18 dargelegte Nukleinsäureresequenz oder eine Variante davon, welche die Funktion des natürlichen Antisense-Transkripts nach einer von SEQ ID NOs: 13 oder 18 beibehält, aufweist, wobei das Oligonukleotid zwischen 10 bis 30 Nukleotide lang ist; oder
d. 10 bis 30 Nukleotide lang ist und an ein reverses Komplement von SEQ ID NO: 16 hybridisierbar ist; oder
e. mindestens eine der folgenden SEQ ID NOs umfasst: 93, 45, 57, 52, 40, 51, 47, 49, 48, 90 bis 92, 94, 87, 69, 78, 82, 79, 61 bis 63, 84, 30, 33, 35, 37, 46, 50, 55, 56, 59, 67, 70, 76, 77, 83, wobei das Oligonukleotid optional zwischen 10 bis 30 Nukleotide lang ist;
wobei das genannte Oligonukleotid die Expression von SCN1A (Natriumkanal, spannungsabhängig, Typ I, Alpha-Untereinheit) steigert.

12. Oligonukleotid nach Anspruch 11, wobei das Oligonukleotid einzelsträngig oder eine siRNA-Verbindung ist.

13. Oligonukleotid nach Anspruch 11 oder Anspruch 12, wobei das Oligonukleotid eine mindestens 90%ige Sequenzidentität mit SEQ ID NO: 16 aufweist.

14. Oligonukleotid nach einem der Ansprüche 11 bis 13, das ferner eine oder mehr Modifikation(en) umfasst, umfassend:
a. mindestens eine modifizierte Internukleosid-Verknüpfung, ausgewählt aus: einem Phosphorothioat, Alkylphosphonat, Phosphorodithioat, Alkylphosphonothioat, Phosphoramidat, Carbamat, Carbonat, Phosphattriester, Acetamidat, Carboxymethylester und Kombinationen davon;
b. mindestens ein modifiziertes Nukleotid, ausgewählt aus: einer Peptidnukleinsäure (PNA), einer verbrückten Nukleinsäure (locked nucleic acid, LNA), einer Arabinonukleinsäure, einem Analogon, einem Derivat und Kombinationen davon; oder
c. mindestens einen modifizierten Zuckerteil, ausgewählt aus: einem 2'-O-Methoxyethyl-modifizierten Zuckerteil, einem 2'-Methoxy-modifizierten Zuckerteil, einem 2'-O-Alkyl-modifizierten Zuckerteil, einem bicyclischen Zuckerteil, einem 2'-Fluoroteil und Kombinationen davon.

15. Pharmazeutische Zusammensetzung, umfassend mindestens ein Oligonukleotid, das:
a. an ein natürliches Antisense-Transkript hybridisierbar ist, das die in einer von SEQ ID NOs: 12 bis 15, 18 bis 24 dargelegte Nukleinsäureresequenz oder eine Variante davon, welche die Funktion des natürlichen Antisense-Transkripts von einer der SEQ ID NOs: 12 bis 15, 18 bis 24 beibehält, aufweist, wobei das genannte Oligonukleotid die Expression von SCN1A steigert; oder
b. 10 bis 30 Nukleotide lang ist und an ein reverses Komplement von SEQ ID NO: 16 hybridisierbar ist, wobei das genannte Oligonukleotid die Expression von SCN1A steigert;
und einen pharmazeutisch akzeptablen Hilfsstoff; wobei das mindestens eine Oligonukleotid optional ein Oligonukleotid nach einem der Ansprüche 11 bis 14 ist.

## Revendications

1. Oligonucléotide destiné à une utilisation comme composé thérapeutique, ledit oligonucléotide augmentant l'expression de la sous-unité alpha du canal sodique voltage-dépendant de type I (SCN1A), et ledit oligonucléotide :
a. ciblant un transcrit antisens naturel de la sous-unité alpha du canal sodique voltage-dépendant de type I (SCN1A), le transcrit antisens naturel ayant la séquence d'acide nucléique telle qu'énoncée dans l'une quelconque des SÉQ. ID n^{os} 12 à 15 ou 17 à 28, ou une variante de celle-ci qui conserve la fonction du transcrit antisens naturel ayant l'une quelconque des SÉQ. ID n^{os} 12 à 15 ou 17 à 28 ; ou
b. ayant une longueur de 10 à 30 nucléotides et se liant à un complément inverse de la SÉQ. ID n° 16.

2. Oligonucléotide destiné à une utilisation dans la prévention ou le traitement d'une maladie ou d'une affection associée à la sous-unité alpha du canal sodique voltage-dépendant de type I (SCN1A), ledit oligonucléotide augmentant l'expression de la sous-unité alpha du canal sodique voltage-dépendant de type I (SCN1A), et ledit oligonucléotide :
a. ciblant un transcrit antisens naturel de la sous-unité alpha du canal sodique voltage-dépendant de type I (SCN1A), le transcrit antisens naturel ayant la séquence d'acide nucléique telle qu'énoncée dans l'une quelconque des SÉQ. ID n^{os} 12 à 15 ou 17 à 28, ou une variante de celle-ci qui conserve la fonction du transcrit antisens naturel ayant l'une quelconque des SÉQ. ID n^{os} 12 à 15 ou 17 à 28 ; ou
b. ayant une longueur de 10 à 30 nucléotides et se liant à un complément inverse de la SÉQ. ID n° 16.

3. Utilisation d'un oligonucléotide pour la fabrication d'un médicament destiné à la prévention ou au traitement d'une maladie ou d'une affection associée à la sous-unité alpha du canal sodique voltage-dépendant de type I (SCN1A), ledit oligonucléotide augmentant l'expression de la sous-unité alpha du canal sodique voltage-dépendant de type I (SCN1A), et ledit oligonucléotide :
a. ciblant un transcrit antisens naturel de la sous-unité alpha du canal sodique voltage-dépendant de type I (SCN1A), le transcrit antisens naturel ayant la séquence d'acide nucléique telle qu'énoncée dans l'une quelconque des SÉQ. ID n^{os} 12 à 15 ou 17 à 28, ou une variante de celle-ci qui conserve la fonction du transcrit antisens naturel ayant l'une quelconque des SÉQ. ID n^{os} 12 à 15 ou 17 à 28 ; ou
b. ayant une longueur de 10 à 30 nucléotides et se liant à un complément inverse de la SÉQ. ID n° 16.

4. Utilisation selon la revendication 3, ou oligonucléotide destiné à une utilisation selon la revendication 1 ou 2, la maladie ou l'affection étant sélectionnée dans le groupe constitué d'une maladie ou d'une affection associée à une fonction et/ou une expression anormale de la SCNA, d'une épilepsie myoclonique sévère du nourrisson (EMSN ou syndrome de Dravet), d'une maladie ou d'une affection neurologique, de convulsions, d'une douleur (y compris douleur chronique), d'une altération de l'excitabilité électrique impliquant un dysfonctionnement du canal sodique, d'une maladie ou d'une affection associée à un dysfonctionnement du canal sodique, d'une maladie ou d'une affection associée à une régulation incorrecte de l'activité de la sous-unité alpha du canal sodique voltage-dépendant (par exemple paralysie, paralysie périodique hyperkaliémique, paramyotonie congénitale, myotonie aggravée par le potassium, syndrome du QT long familial, type 3, maladie de la plaque motrice héréditaire, ataxie etc.), d'une maladie du tractus gastro-intestinal due à un dysfonctionnement du système nerveux entérique (par exemple colite, iléite, syndrome inflammatoire de l'intestin, etc.), d'une maladie ou d'une affection cardiovasculaire (par exemple hypertension, insuffisance cardiaque congestive, etc.), d'une maladie ou d'une affection du tractus génito-urinaire impliquant une innervation sympathique et parasympathique (par exemple hyperplasie bénigne de la prostate, impuissance), d'une maladie ou d'une affection associée au système neuromusculaire (par exemple dystrophie musculaire, sclérose en plaques, épilepsie, autisme, migraines (par exemple migraines hémiplégiques sporadiques et familiales, etc.), d'une épilepsie généralisée avec des crises fébriles plus (GEFS+, etc.), et d'épilepsies liées à la SCNA.

5. Procédé *in vitro* d'augmentation de l'expression de la sous-unité alpha du canal sodique voltage-dépendant de type I (SCN1A) dans des cellules ou des tissus de patients, comprenant :
la mise en contact desdites cellules ou desdits tissus avec un oligonucléotide qui :
a. cible un transcrit antisens naturel de la sous-unité alpha du canal sodique voltage-dépendant de type I (SCN1A) ; et le transcrit antisens naturel ayant la séquence d'acide nucléique telle qu'énoncée dans l'une quelconque des SÉQ. ID n^{os} 12 à 15 ou 17 à 28, ou une variante de celle-ci qui conserve la fonction du transcrit antisens naturel ayant l'une quelconque des SÉQ. ID n^{os} 12 à 15 ou 17 à 28 ; ou
b. a une longueur de 10 à 30 nucléotides et se lie à un complément inverse de la SÉQ. ID n° 16 ;
en augmentant ainsi l'expression de la sous-unité alpha du canal sodique voltage-dépendant de type I (SCN1A).

6. Utilisation selon la revendication 3 ou 4, ou oligonucléotide destiné à une utilisation selon l'une quelconque des revendications 1, 2 ou 4, ou procédé selon la revendication 5, l'oligonucléotide étant monobrin.

7. Utilisation selon la revendication 3 ou 4, ou oligonucléotide destiné à une utilisation selon l'une quelconque des revendications 1, 2 ou 4, ou procédé selon la revendication 5, l'oligonucléotide étant un composé d'ARNsi.

8. Utilisation selon l'une quelconque des revendications 3, 4, 6 ou 7, ou oligonucléotide destiné à une utilisation selon l'une quelconque des revendications 1, 2, 4, 6 ou 7, ou procédé selon l'une quelconque des revendications 5 à 7, l'oligonucléotide comprenant au moins l'une des SÉQ. ID n^{os} 70, 93, 45, 57, 52, 40, 51, 47, 49, 48, 90 à 92, 94, 87, 69, 78, 82, 79, 61 à 63, 84, 30, 33, 35, 37, 46, 50, 55, 56, 59, 67, 76, 77 ou 83.

9. Utilisation selon l'une quelconque des revendications 3, 4 ou 6 à 8, ou oligonucléotide destiné à une utilisation selon l'une quelconque des revendications 1, 2, 4 ou 6 à 8, ou procédé selon l'une quelconque des revendications 5 à 8, l'expression de la sous-unité alpha du canal sodique voltage-dépendant de type I (SCN1A) étant augmentée d'au moins 10 %.

10. Utilisation selon l'une quelconque des revendications 3, 4 ou 6 à 9, ou oligonucléotide destiné à une utilisation selon l'une quelconque des revendications 1, 2, 4 ou 6 à 9, ou procédé selon l'une quelconque des revendications 3 à 9, l'oligonucléotide comprenant en outre une ou plusieurs modifications comprenant :
a. au moins une liaison internucléosidique modifiée sélectionnée parmi : un phosphorothioate, alkylphosphonate, phosphorodithioate, alkylphosphonothioate, phosphoramidate, carbamate, carbonate, triester de phosphate, acétamidate, ester carboxyméthylique, et des combinaisons de ceux-ci ;
b. au moins un nucléotide modifié sélectionné parmi : un acide nucléique peptidique (PNA), un acide nucléique bloqué (LNA), un acide arabino-nucléique, un analogue, un dérivé, et des combinaisons de ceux-ci ; ou
c. au moins une fraction sucre modifiée sélectionnée parmi : une fraction sucre modifiée par un groupe 2'-O-méthoxyéthyle, une fraction sucre modifiée par un groupe 2'-méthoxy, une fraction sucre modifiée par un groupe 2'-O-alkyle, une fraction sucre bicyclique, une fraction 2'-fluoro, et des combinaisons de celles-ci.

11. Oligonucléotide qui :
a. est capable de s'hybrider avec un transcrit antisens naturel qui a la séquence d'acide nucléique telle qu'énoncée dans l'une quelconque des SÉQ. ID n^{os} 12 ou 23, ou une variante de celle-ci qui conserve la fonction du transcrit antisens naturel ayant l'une quelconque des SÉQ. ID n^{os} 12 ou 23, l'oligonucléotide ayant optionnellement une longueur de 10 à 30 nucléides ; ou
b. a une complémentarité d'au moins 80 % avec une région cible dans un transcrit antisens naturel qui a la séquence d'acide nucléique telle qu'énoncée dans l'une quelconque des SÉQ. ID n^{os} 14 ou 24, ou une variante de celle-ci qui conserve la fonction du transcrit antisens naturel ayant l'une quelconque des SÉQ. ID n^{os} 14 ou 24, et l'oligonucléotide ayant optionnellement une longueur de 10 à 30 nucléotides ; ou
c. est capable de s'hybrider avec un transcrit antisens naturel qui a la séquence d'acide nucléique telle qu'énoncée dans l'une quelconque des SÉQ. ID n^{os} 13 ou 18, ou une variante de celle-ci qui conserve la fonction du transcrit antisens naturel ayant l'une quelconque des SÉQ. ID n^{os} 13 ou 18, l'oligonucléotide ayant une longueur de 10 à 30 nucléotides ; ou
d. a une longueur de 10 à 30 nucléotides et est capable de s'hybrider avec un complément inverse de la SÉQ. ID n° 16 ; ou
e. comprend au moins l'une des SÉQ. ID n^{os} 93, 45, 57, 52, 40, 51, 47, 49, 48, 90 à 92, 94, 87, 69, 78, 82, 79, 61 à 63, 84, 30, 33, 35, 37, 46, 50, 55, 56, 59, 67, 70, 76, 77 ou 83, l'oligonucléotide ayant optionnellement une longueur de 10 à 30 nucléotides ;
ledit oligonucléotide augmentant l'expression de la sous-unité alpha du canal sodique voltage-dépendant de type I (SCN1A).

12. Oligonucléotide selon la revendication 11, l'oligonucléotide étant monobrin ou étant un composé d'ARNsi.

13. Oligonucléotide selon la revendication 11 ou la revendication 12, l'oligonucléotide ayant une identité de séquence d'au moins 90 % avec la SÉQ. ID n° 16.

14. Oligonucléotide selon l'une quelconque des revendications 11 à 13, lequel comprend en outre une ou plusieurs modifications comprenant :
a. au moins une liaison internucléosidique modifiée sélectionnée parmi : un phosphorothioate, alkylphosphonate, phosphorodithioate, alkylphosphonothioate, phosphoramidate, carbamate, carbonate, triester de phosphate, acétamidate, ester carboxyméthylique, et des combinaisons de ceux-ci ;
b. au moins un nucléotide modifié sélectionné parmi : un acide nucléique peptidique (PNA), un acide nucléique bloqué (LNA), un acide arabino-nucléique, un analogue, un dérivé, et des combinaisons de ceux-ci ; ou
c. au moins une fraction sucre modifiée sélectionnée parmi : une fraction sucre modifiée par un groupe 2'-O-méthoxyéthyle, une fraction sucre modifiée par un groupe 2'-méthoxy, une fraction sucre modifiée par un groupe 2'-O-alkyle, une fraction sucre bicyclique, une fraction 2'-fluoro, et des combinaisons de celles-ci.

15. Composition pharmaceutique comprenant au moins un oligonucléotide qui est :
a. capable de s'hybrider avec un transcrit antisens naturel qui a la séquence d'acide nucléique telle qu'énoncée dans l'une quelconque des SÉQ. ID n^{os} 12 à 15, 18 à 24 ou une variante de celle-ci qui conserve la fonction du transcrit antisens naturel ayant l'une quelconque des SÉQ. ID n^{os} 12 à 15, 18 à 24, ledit oligonucléotide augmentant l'expression de la SCN1A; ou
b. a une longueur de 10 à 30 nucléotides et est capable de s'hybrider avec un complément inverse de la SÉQ. ID n° 16, ledit oligonucléotide augmentant l'expression de la SCN1A ;
et un excipient pharmaceutiquement acceptable ; dans laquelle, optionnellement, ledit au moins un oligonucléotide est un oligonucléotide selon l'une quelconque des revendications 11 à 14.
